# EUROPEAN PATENT APPLICATION

(11) **EP 4 789 541 A1**
(43) Date of publication of application: **12.08.2026**
(21) Application number: 24874689.3
(22) Date of filing: 03.10.2024
(51) Int. Cl.: A01N 43/76, A01N 43/50, A01N 43/56, A01N 43/78, A01N 43/647, A01N 43/653, A01N 43/713, A01N 43/836, A01P 3/00, C07D 231/12, C07D 249/04, C07D 249/08, C07D 257/04, C07D 263/32, C07D 271/06, C07D 277/22, C07D 277/24

(54) **BIARYL COMPOUND AND METHOD FOR CONTROLLING PLANT DISEASE**

(30) Priority: 06.10.2023 JP 2023174447; 30.04.2024 JP 2024073432
(71) Applicant: SUMITOMO CHEMICAL COMPANY, LIMITED, Tokyo 103-6020 (JP)
(72) Inventor: ARAI, Keisuke, Takarazuka-shi, Hyogo 665-8555 (JP); ICHIHASHI, Kazuki, Takarazuka-shi, Hyogo 665-8555 (JP); MAEHATA, Nao, Takarazuka-shi, Hyogo 665-8555 (JP); YAMAMOTO, Masaoki, Takarazuka-shi, Hyogo 665-8555 (JP)
(74) Representative: Vossius & Partner Patentanwälte Rechtsanwälte mbB
(86) International application number: PCT/JP2024/035447
(87) International publication number: WO 2025/075089

(57) **Abstract**

The present invention provides a method having excellent efficacy for controlling plant diseases. A compound represented by formula (I) [wherein the ring A⁵ represents a five(5) membered nonaromatic nitrogen-containing heterocyclic group, etc., (with the proviso that in the ring A⁵, an atom binding to D represents a carbon atom or a nitrogen atom, and among the atoms which are adjacent to the atom binding to the D and constitute the ring A⁵, at least one thereof represents a nitrogen atom),
D represents CR¹R², etc.,
n is 0, 1, 2, or 3,
m is 0, 1, 2, or 3,
q is 0, 1, 2, 3, 4, or 5,
R¹ and R² are identical to or different from each other and each represents a C1-C6 chain hydrocarbon group , etc.,
R¹ and R² may combine together with a carbon atom to which they are attached to form a C3-C8 alicyclic hydrocarbon,
R³ and R⁵ are identical to or different from each other and each represents a C1-C6 chain hydrocarbon group, etc.,
R⁴ represents a C1-C6 chain hydrocarbon group, etc.,
R⁶ represents a C1-C6 chain hydrocarbon group, etc.,
when q is 2, 3, 4, or 5, the neighboring two R⁶ and R⁶ may combine together with carbon atoms to which they are attached to form a C5-C6 alicyclic hydrocarbon, etc.] or an N-oxide or a salt thereof
can be used to control plant diseases.

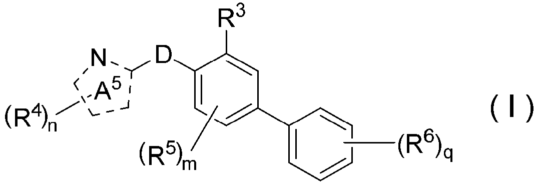

## Description

### TECHNICAL FIELD

This application claims priority to and the benefit of Japanese Patent Application No. 2023-174447 filed October 6, 2023 and Japanese Patent Application No. 2024-073432 filed April 30, 2024, the entire contents of which are incorporated herein by reference.

The present invention relates to a biaryl compound and a method for controlling plant diseases.

### BACKGROUND ART

Patent Literature 1 describes a biaryl compound.

### PRIOR ART DOCUMENT

### PATENT DOCUMENT

Patent DOCUMENT 1: WO 2011/005674 A1

### SUMMARY OF THE INVENTION

### (PROBLEMS TO BE SOLVED BY INVENTION)

An object of the present invention is to provide a method having excellent efficacy for controlling plant diseases.

### SOLUTIONS TO THE PROBLEMS

The present inventors have intensively studied to find out a method having excellent efficacy for controlling plant diseases, and as a result, found that a compound represented by the following formula (I) has an excellent efficacy for controlling plant diseases.

That is, the present invention encompasses the followings.
[1] A method for controlling a plant disease which comprises applying a compound represented by formula (I): 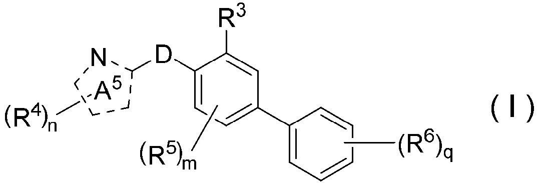 [wherein
   the ring A⁵ represents a five(5) membered nonaromatic nitrogen-containing heterocycle or a five(5) membered aromatic nitrogen-containing heterocycle (with the proviso that in the ring A⁵, an atom binding to D represents a carbon atom or a nitrogen atom, and among the atoms which is adjacent to the atom binding to the D and constitutes the ring A⁵, at least one thereof represents a nitrogen atom),
   D represents CR¹R², C=O, C=S, or C=N-OR⁷,
   n is 0, 1, 2, or 3,
   m is 0, 1, 2, or 3,
   q is 0, 1, 2, 3, 4, or 5,
   R¹ and R² are identical to or different from each other and each represents a C1-C6 chain hydrocarbon group optionally substituted with one or more substituent(s) selected from Group A, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B}, a phenyl group, a five(5) membered aromatic heterocyclic group {the phenyl group and the five (5) membered aromatic heterocyclic group may be optionally substituted with one or more substituents selected from Group C}, OR⁸, SR⁹, S(O)R¹⁰, S(O)₂R¹¹, NR¹²R¹³, C(O)R¹⁴, C(R¹⁵)=NOR¹⁶, a cyano group, a nitro group, a halogen atom, or a hydrogen atom,
   R¹ and R² may combine together with a carbon atom to which they are attached to form a C3-C8 alicyclic hydrocarbon, or a three(3)- eight(8) membered nonaromatic heterocycle {the C3-C8 alicyclic hydrocarbon, and the three(3)- eight(8) membered nonaromatic heterocycle may be optionally substituted with one or more substituents selected from Group B},
   R³ and R⁵ are identical to or different from each other and each represents a C1-C6 chain hydrocarbon group optionally substituted with one or more substituent(s) selected from Group A, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B}, a five(5)- six(6) membered aromatic heterocyclic group optionally substituted with one or more substituents selected from Group C, OR¹⁷, SR¹⁸, S(O)R¹⁹, S(O)₂R²⁰, NR²¹R²², C(O)R²³, C(R²⁴)=NOR²⁵, a cyano group, a nitro group, or a halogen atom,
   R⁴ represents a C1-C6 chain hydrocarbon group optionally substituted with one or more substituent(s) selected from Group A, a C3-C8 alicyclic hydrocarbon group, a three(3)-eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)-six(6) membered aromatic heterocyclic group {the phenyl group, and the five(5)-six(6) membered aromatic heterocyclic group may be optionally substituted with one or more substituents selected from Group C}, OR²⁶, NR²⁷R²⁸, C(O)R²⁹, C(R³⁰)=NOR³¹, a cyano group, a nitro group, or a halogen atom,
   R⁶ represents a C1-C6 chain hydrocarbon group optionally substituted with one or more substituent(s) selected from Group D, a C3-C8 alicyclic hydrocarbon group, a three(3)-eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, OR³², SR³³, S(O)R³⁴, S(O)₂R³⁵, NR³⁶R³⁷, C(O)R³⁸, C(R³⁹)=NOR⁴⁰, a cyano group, a nitro group, or a halogen atom,
   R⁸, R¹², R¹³, R¹⁷, R²¹, R²², R²⁶, R²⁷, R²⁸, R³², R³⁶, and R³⁷ are identical to or different from each other and each represents a C1-C6 chain hydrocarbon group optionally substituted with one or more substituent(s) selected from Group A, a C1-C6 alkylsulfonyl group, a (C1-C6 alkyl)carbonyl group, a (C1-C6 alkoxy)carbonyl group, a (C1-C6 alkylamino) carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, or a hydrogen atom,
   R⁷, R⁹, R¹⁶, R¹⁸, R²⁵, R³¹, R³³, and R⁴⁰ are identical to or different from each other and each represents a C1-C6 chain hydrocarbon group optionally substituted with one or more substituent(s) selected from Group A, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, or a hydrogen atom,
   R¹⁰, R¹¹, R¹⁹, R²⁰, R³⁴, and R³⁵ are identical to or different from each other and each represents a C1-C6 chain hydrocarbon group optionally substituted with one or more substituent(s) selected from Group A, a C1-C6 alkylamino group, a C2-C8 dialkylamino group {the C1-C6 alkylamino group, and the C2-C8 dialkylamino group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, or a five(5)- six(6) membered aromatic heterocyclic group {the phenyl group, and the five(5)- six(6) membered aromatic heterocyclic group may be optionally substituted with one or more substituents selected from Group C},
   R¹⁴, R²³, R²⁹, and R³⁸ are identical to or different from each other and each represents a C1-C6 chain hydrocarbon group, a C1-C6 alkoxy group, a C1-C6 alkylamino group, a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms}, or a hydroxy group,
   R¹⁵, R²⁴, R³⁰, and R³⁹ are identical to or different from each other and each represents a C1-C6 chain hydrocarbon group optionally substituted with one or more substituent(s) selected from Group A, or a hydrogen atom,
   when q is 2, 3, 4, or 5, the neighboring two R⁶ and R⁶ may combine together with carbon atoms to which they are attached to form a C5-C6 alicyclic hydrocarbon, a five(5)-six(6) membered nonaromatic heterocycle {the C5-C6 alicyclic hydrocarbon, and the five(5)- six(6) membered nonaromatic heterocycle may be optionally substituted with one or more substituents selected from Group B}, a benzene ring, or five(5)- six(6) membered aromatic heterocycle {the benzene ring, and the five(5)- six(6) membered aromatic heterocycle may be optionally substituted with one or more substituents selected from Group C}.

Group A: a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, a halogen atom, a cyano group, a nitro group, a hydroxy group, and a sulfanyl group.

Group B: a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.

Group C: a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino) carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino) carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group optionally substituted with one or more substituents selected from Group B, an amino group, a nitro group, a cyano group, a hydroxy group, a sulfanyl group, and a halogen atom.

Group D: a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5) membered aromatic heterocyclic group {the phenyl group, and the five(5) membered aromatic heterocyclic group may be optionally substituted with one or more substituents selected from Group C}, a halogen atom, a cyano group, a nitro group, a hydroxy group, and a sulfanyl group. (with the proviso that the following compounds are excluded:
3-((3,5-difluoro-[1,1'-biphenyl]-4-yl)methyl)-1,2,4-thiadiazole,
3-((3-fluoro-[1,1'-biphenyl]-4-yl)methyl)-1,2,4-thiadiazole,
3-((3,5-difluoro-[1,1'-biphenyl]-4-yl)difluoromethyl)-1,2,4-thiadiazole,
3-((3,5-difluoro-[1,1'-biphenyl]-4-yl)methyl)-1,3,4-thiadiazole-2(3H)-one,
2-((3,5-difluoro-[1,1'-biphenyl]-4-yl)methyl)-1,3,4-thiadiazole,
(3,5-difluoro-[1,1'-biphenyl]-4-yl)(1,2,4-thiadiazole-3-yl)methanone,
2-((3-fluoro-[1,1'-biphenyl]-4-yl)methyl)-1,3,4-oxadiazole,
(E)-(3,5-difluoro-[1,1'-biphenyl]-4-yl)(1,2,4-thiadiazole-3-yl)methanone O-methyl oxime,
(Z)-(3,5-difluoro-[1,1'-biphenyl]-4-yl)(1,2,4-thiadiazole-3-yl)methanone O-methyl oxime,
3-((3,5-difluoro-[1,1'-biphenyl]-4-yl)methyl)-5-ethyl-1,2,4-thiadiazole,
5-cyclopropyl-3-((3,5-difluoro-[1,1'-biphenyl]-4-yl)methyl)-1,2,4-thiadiazole,
3-((3,5-difluoro-[1,1'-biphenyl]-4-yl)methyl)-5-(trifluoromethyl)-1,2,4-thiadiazole,
3-((3,5-difluoro-[1,1'-biphenyl]-4-yl)methyl)-1,2,4-thiadiazole-5-carbonitrile,
3-((3,5-difluoro-[1,1'-biphenyl]-4-yl)methyl)isoxazole 5(4H)-one,
5-chloro-3-((3,5-difluoro-[1,1'-biphenyl]-4-yl)methyl)isoxazole,
3-((3,5-difluoro-[1,1'-biphenyl]-4-yl)methyl)-1,2,4-thiadiazole-5-ol,
3-((3,5-difluoro-[1,1'-biphenyl]-4-yl)methyl)-5-methoxy-1,2,4-thiadiazole,
(2-chlorothiazole-4-yl)(3-fluoro-[1,1'-biphenyl]-4-yl)methanol,
2-chloro-4-((3-fluoro-[1,1'-biphenyl]-4-yl)methyl)thiazole,
5-chloro-3-((3,5-difluoro-[1,1'-biphenyl]-4-yl)methyl)-1,2,4-thiadiazole,
5-bromo-3-((3,5-difluoro-[1,1'-biphenyl]-4-yl)methyl)- -1,2,4-thiadiazole, and
3-((3,5-difluoro-[1,1'-biphenyl]-4-yl)methyl)-1,2,4-thiadiazole-5-carboxamide)]

(hereinafter, referred to as "Present compound A") or an N-oxide or a salt thereof (hereinafter, the Present compound A or an N-oxide or a salt thereof is referred to as "Present compound")
to a plant or soil for cultivating a plant.

[2] The method for controlling a plant disease according to [1] wherein the compound represented by formula (I) or an N-oxide or a salt thereof according to claim 1 is a compound or an N-oxide or a salt thereof, in which the ring A⁵ represents a group represented by formula H1, formula H2, formula H3, or formula H4, 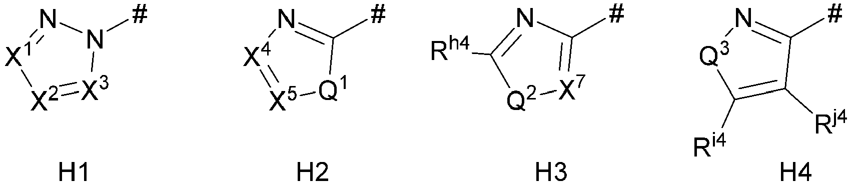 [wherein
   # represents a binding position to D,
   X¹ represents a nitrogen atom, or CR^{a4}, X² represents a nitrogen atom, or CR^{b4}, X³ represents a nitrogen atom, or CR^{c4} (with the proviso that among X¹, X², and X³, the number of the nitrogen atom is 0, or 1],
   a combination of Q¹, X⁴, and X⁵ represents
      a combination wherein X⁴ represents a nitrogen atom, Q¹ represents NR^{a1}, and X⁵ represents CR^{d4}, or
      a combination wherein X⁴ represents CRe4, Q¹ represents an oxygen atom, a sulfur atom, or NR^{b1}, and X⁵ represents nitrogen atom, or CR^{f4};
   Q² represents an oxygen atom, or NR^{c1}, X⁷ represents a nitrogen atom, or CR^{g4},
   Q³ represents a sulfur atom, or NR^{d1},
   R^{a4}, R^{b4}, R^{c4}, R^{d4}, R^{e4}, R^{f4}, R^{g4}, R^{h4}, Rⁱ⁴, and R^{j4} are identical to or different from each other and each represents a C1-C6 chain hydrocarbon group optionally substituted with one or more substituents selected from Group A, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, OR²⁶, NR²⁷R²⁸, C(O)R²⁹, C(R³⁰)=NOR³¹, a cyano group, a nitro group, a halogen atom, or a hydrogen atom,
   R^{a1}, R^{b1}, R^{c1}, and R^{d1} are identical to or different from each other and each represents a C1-C6 chain hydrocarbon group optionally substituted with one or more substituents selected from Group A, a C3-C8 alicyclic hydrocarbon group, a three(3)- eight(8) membered nonaromatic heterocyclic group {the C3-C8 alicyclic hydrocarbon group, and the three (3)-eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, C(O)R²⁹, or a hydrogen].
[3] The method for controlling a plant disease according to [2] wherein the compound represented by formula (I) or an N-oxide or a salt thereof is a compound represented by formula (I) in which the ring A⁵ represents the group represented by formula H1.
[4] The method for controlling a plant disease according to [2] wherein the compound represented by formula (I) or an N-oxide or a salt thereof is a compound represented by formula (I) in which the ring A⁵ represents the group represented by formula H2.
[5] The method for controlling a plant disease according to [2] wherein the compound represented by formula (I) or an N-oxide or a salt thereof is a compound represented by formula (I) in which the ring A⁵ represents the group represented by formula H3.
[6] The method for controlling a plant disease according to [2] wherein the compound represented by formula (I) or an N-oxide or a salt thereof is a compound represented by formula (I) in which the ring A⁵ represents the group represented by formula H4.
[7] A compound represented by formula (II) 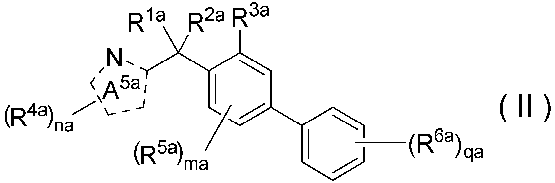 [wherein
   the ring A^{5a} represents a five(5) membered aromatic nitrogen-containing heterocyclic group (with the proviso that in the ring A^{5a}, the binding atom to CR^{1a}R^{2a} represents a carbon atom or a nitrogen atom, and among the atoms which are adjacent to the atom binding to CR^{1a}R^{2a} and constitute the ring A^{5a}, at least one thereof represents a nitrogen atom),
   na is 0, 1, 2, or 3,
   ma is 0, 1, 2, or 3,
   qa is 0, 1, 2, 3, 4, or 5,
   R^{1a} and R^{2a} are identical to or different from each other and each represents a C1-C6 chain hydrocarbon group optionally substituted with one or more substituents selected from Group A^{a}, a C3-C8 alicyclic hydrocarbon group, a three(3)- eight(8) membered nonaromatic heterocyclic group {the C3-C8 alicyclic hydrocarbon group, and the three (3)-eight(8) membered nonaromatic heterocyclic group may be optionally substituted with one or more substituents selected from Group B^{a}}, a phenyl group optionally substituted with one or more substituents selected from Group C^{a}, OR^{8a}, SR^{9a}, S(O)R^{10a}, S(O)₂R^{11a}, NR^{12a}R^{13a}, C(O)R^{14a}, C(R^{15a})=NOR^{16a}, a cyano group, a nitro group, a halogen atom, or a hydrogen atom,
   R^{1a} and R^{2a} may combine together with a carbon atom to which they are attached to form a C3-C8 alicyclic hydrocarbon, or a three(3)- eight(8) membered nonaromatic heterocycle {the C3-C8 alicyclic hydrocarbon, and the three(3)- eight(8) membered nonaromatic heterocycle may be optionally substituted with one or more substituents selected from Group Ba}
   R^{3a} and R^{5a} are identical to or different from each other and each represents a C1-C6 chain hydrocarbon group optionally substituted with one or more substituents selected from Group A^{a}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}}, a five(5)- six(6) membered aromatic heterocyclic group optionally substituted with one or more substituents selected from Group C^{a}, OR^{17a}, SR^{18a}, S(O)R^{19a}, S(O)₂R^{20a}, NR^{21a}R^{22a}, C(O)R^{23a}, C(R^{24a}) =NOR^{25a}, a cyano group, a nitro group, or a halogen atom,
   R^{4a} represents a C1-C6 chain hydrocarbon group optionally substituted with one or more substituents selected from Group D^{a}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}}, OR^{26a}, NR^{27a}R^{28a}, C(R^{29a})=NOR^{30a}, a cyano group, a nitro group, or a halogen atom,
   R^{6a} represents a C1-C6 chain hydrocarbon group optionally substituted with one or more substituents selected from Group E^{a}, a C3-C8 alicyclic hydrocarbon group optionally substituted with one or more substituents selected from Group B^{a}, a phenyl group, a five(5)- six(6) membered aromatic heterocyclic group {the phenyl group, and the five(5)- six(6) membered aromatic heterocyclic group may be optionally substituted with one or more substituents selected from Group C^{a}}, OR^{31a}, SR^{32a}, S(O)R^{33a}, S(O)₂R^{34a}, NR^{35a}R^{36a}, C(O)R^{37a}, C(R^{38a}) =NOR^{39a}, a cyano group, a nitro group, or a halogen atom,
   R^{8a}, R^{12a}, R^{13a}, R^{17a}, R^{31a}, R^{21a}, R^{22a}, and R^{35a} are identical to or different from each other and each represents a C1-C6 chain hydrocarbon group optionally substituted with one or more substituents selected from Group A^{a}, a C1-C6 alkylsulfonyl group, a (C1-C6 alkyl)carbonyl group, a (C1-C6 alkoxy)carbonyl group, a (C1-C6 alkylamino)carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino)carbonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a phenyl group, a five(5)- six(6) membered aromatic heterocyclic group {the phenyl group, and the five(5)- six(6) membered aromatic heterocyclic group may be optionally substituted with one or more substituents selected from Group C^{a}}, or a hydrogen atom,
   R^{36a} represents a C1-C6 chain hydrocarbon group optionally substituted with one or more substituents selected from Group A^{a}, a C1-C6 alkylsulfonyl group, a (C1-C6 alkyl)carbonyl group, a (C1-C6 alkoxy)carbonyl group, a (C1-C6 alkylamino)carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a phenyl group, or a five(5)- six(6) membered aromatic heterocyclic group{the phenyl group, and the five(5)- six(6) membered aromatic heterocyclic group may be optionally substituted with one or more substituents selected from Group C^{a}},
   R^{7a}, R^{9a}, R^{10a}, R^{18a}, R^{19a}, R^{25a}, R^{32a}, R^{33a}, R^{34a}, and R^{39a} are identical to or different from each other and each represents a C1-C6 chain hydrocarbon group optionally substituted with one or more substituents selected from Group A^{a}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a phenyl group, or a five(5)- six(6) membered aromatic heterocyclic group{the phenyl group, and the five(5)- six(6) membered aromatic heterocyclic group may be optionally substituted with one or more substituents selected from Group C^{a}},
   R^{11a} and R^{20a} are identical to or different from each other and each represents a C1-C6 chain hydrocarbon group optionally substituted with one or more substituents selected from Group A^{a}, a C1-C6 alkylamino group, a C2-C8 dialkylamino group {the C1-C6 alkylamino group, and the C2-C8 dialkylamino group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}}, a phenyl group, or a five(5)- six(6) membered aromatic heterocyclic group {the phenyl group, and the five(5)- six(6) membered aromatic heterocyclic group may be optionally substituted with one or more substituents selected from Group C^{a}},
   R^{14a} and R^{23a} are identical to or different from each other and each represents a C1-C6 chain hydrocarbon group, a C1-C6 alkoxy group, a C1-C6 alkylamino group, a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms}, or a hydroxy group,
   R^{15a}, R^{24a}, and R^{38a} are identical to or different from each other and each represents a C1-C6 chain hydrocarbon group optionally substituted with one or more substituents selected from Group A^{a}, or a hydrogen atom,
   R^{26a}, R^{27a}, and R^{28a} are identical to or different from each other and each represents a C1-C6 chain hydrocarbon group optionally substituted with one or more substituents selected from Group D^{a}, a C1-C6 alkylsulfonyl group, a (C1-C6 alkyl)carbonyl group, a (C1-C6 alkoxy)carbonyl group, a (C1-C6 alkylamino)carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, or a hydrogen atom,
   R^{30a} represents a C1-C6 chain hydrocarbon group optionally substituted with one or more substituents selected from Group D^{a}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}},
   R^{29a} represents a C1-C6 chain hydrocarbon group optionally substituted with one or more substituents selected from Group D^{a}, or a hydrogen atom,
   R^{37a} represents a C1-C6 chain hydrocarbon group, a C1-C6 alkoxy group, a C1-C6 alkylamino group, or a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms}.

Group A^{a}: a group consisting of a C1-C6 alkoxy group, a a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a phenyl group, a five(5)- six(6) membered aromatic heterocyclic group{the phenyl group, and the five(5)- six(6) membered aromatic heterocyclic group may be optionally substituted with one or more substituents selected from Group C^{a}}, a halogen atom, a cyano group, a nitro group, a hydroxy group, and a sulfanyl group.

Group B^{a}: a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.

Group C^{a}: a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group optionally substituted with one or more substituents selected from Group B^{a}, an amino group, a nitro group, a cyano group, a hydroxy group, a sulfanyl group, and a halogen atom.

Group D^{a}: a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a halogen atom, a cyano group, a nitro group, a hydroxy group, and a sulfanyl group.

Group E^{a}: a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a phenyl group, five(5) membered aromatic heterocyclic group, a halogen atom, a cyano group, a nitro group, a hydroxy group, and a sulfanyl group], (with the proviso that the following compounds are excluded:
4-((3,4,5-tribromo-1H-pyrazole-1-yl)methyl)-[1,1'-biphenyl]-3-ol,
5-(1-(3-fluoro-[1,1'-biphenyl]-4-yl)ethyl)-3-methyl-1,2,4-oxadiazole,
3-(chloromethyl)-5-(1-(3-fluoro-[1,1'-biphenyl]-4-yl)ethyl)-1,2,4-oxadiazole,
3-((3,5-difluoro-[1,1'-biphenyl]-4-yl)methyl)-1,2,4-thiadiazole,
3-((3-fluoro-[1,1'-biphenyl]-4-yl)methyl)-1,2,4-thiadiazole,
3-((3,5-difluoro-[1,1'-biphenyl]-4-yl)difluoromethyl)-1,2,4-thiadiazole,
3-((3,5-difluoro-[1,1'-biphenyl]-4-yl)methyl)-1,3,4-thiadiazole-2(3H)-one,
2-((3,5-difluoro-[1,1'-biphenyl]-4-yl)methyl)-1,3,4-thiadiazole,
(3,5-difluoro-[1,1'-biphenyl]-4-yl) (1,2,4-thiadiazole-3-yl)methanone,
2-((3-fluoro-[1,1'-biphenyl]-4-yl)methyl)-1,3,4-oxadiazole,
(E)-(3,5-difluoro-[1,1'-biphenyl]-4-yl)(1,2,4-thiadiazole-3-yl)methanone O-methyl oxime,
(Z)-(3,5-difluoro-[1,1'-biphenyl]-4-yl)(1,2,4-thiadiazole-3-yl)methanone O-methyl oxime,
3-((3,5-difluoro-[1,1'-biphenyl]-4-yl)methyl)-5-ethyl-1,2,4-thiadiazole,
5-cyclopropyl-3-((3,5-difluoro-[1,1'-biphenyl]-4-yl)methyl)-1,2,4-thiadiazole,
3-((3,5-difluoro-[1,1'-biphenyl]-4-yl)methyl)-5-(trifluoromethyl)-1,2,4-thiadiazole,
3-((3,5-difluoro-[1,1'-biphenyl]-4-yl)methyl)-1,2,4-thiadiazole-5-carbonitrile,
3-((3,5-difluoro-[1,1'-biphenyl]-4-yl)methyl)isoxazole-5(4H)-one,
5-chloro-3-((3,5-difluoro-[1,1'-biphenyl]-4-yl)methyl)isoxazole,
3-((3,5-difluoro-[1,1'-biphenyl]-4-yl)methyl)-1,2,4-thiadiazole-5-ol,
3-((3,5-difluoro-[1,1'-biphenyl]-4-yl)methyl)-5-methoxy-1,2,4-thiadiazole,
(2-chlorothiazole-4-yl)(3-fluoro-[1,1'-biphenyl]-4-yl)methanol,
2-chloro-4-((3-fluoro-[1,1'-biphenyl]-4-yl)methyl)thiazole,
5-chloro-3-((3,5-difluoro-[1,1'-biphenyl]-4-yl)methyl)-1,2,4-thiadiazole,
5-bromo-3-((3,5-difluoro-[1,1'-biphenyl]-4-yl)methyl)-1,2,4-thiadiazole, and
3-((3,5-difluoro-[1,1'-biphenyl]-4-yl)methyl)-1,2,4-thiadiazole-5-carboxamide)
(hereinafter, referred to as "Compound A of the present invention") or an N-oxide or a salt thereof (hereinafter, the Compound A of the present invention or an N-oxide or a salt thereof is referred to as "Compound of the present invention").
[8] The compound or an N-oxide thereof, or a salt thereof according to [7], wherein
   the ring A^{5a} represents a group represented by formula H1a, formula H2a, formula H3a, or formula H4a, 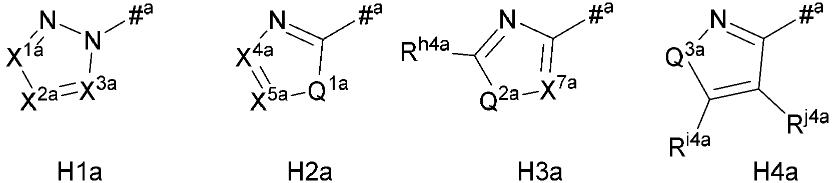 [wherein
   #^{a} represents a binding position to CR^{1a}R^{2a},
   X^{1a} represents a nitrogen atom, or CR^{a4a}, X^{2a} represents a nitrogen atom, or CR^{b4a}, X^{3a} represents a nitrogen atom, or CR^{c4a} (with the proviso that among X^{1a}, X^{2a}, and X^{3a}, the number of the nitrogen atom is 0, or 1],
   a combination of Q^{1a}, X^{4a}, and X^{5a} represents
      a combination wherein X^{4a} represents a nitrogen atom, Q¹ represents NR^{a1a}, and X^{5a} represents CR^{d4a}, or
      a combination wherein X^{4a} represents CR^{e4a}, Q^{1a} represents an oxygen atom, a sulfur atom, or NR^{b1a}, and X^{5a} represents a nitrogen atom, or CR^{f4a};
   Q^{2a} represents an oxygen atom, or NR^{c1a}, X^{7a} represents a nitrogen atom, or CR^{g4a},
   Q^{3a} represents a sulfur atom, or NR^{d1a},
   Ra4a, Rb4a, Rc4a, Rd4a, R^{e4a}, R^{f4a}, R^{g4a}, R^{h4a}, R^{i4a}, and R^{j4a} are identical to or different from each other and each represents a C1-C6 chain hydrocarbon group optionally substituted with one or more substituents selected from Group D^{a}, a C3-C8 alicyclic hydrocarbon group, a three (3)- eight (8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, OR^{26a}, NR^{27a}R^{28a}, C(R^{29a})=NOR^{30a}, a cyano group, a nitro group, a halogen atom, or a hydrogen atom,
   R^{a1a}, R^{b1a}, R^{c1a}, and R^{d1a} are identical to or different from each other and each represents a C1-C6 chain hydrocarbon group optionally substituted with one or more substituents selected from Group D^{a}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}), a cyano group, or a hydrogen atom].
[9] A composition comprising the compound or an N-oxide thereof, or a salt thereof according to [7] or [8], and an inert carrier.
[10] A composition comprising one or more ingredient(s) selected from the group consisting of Group (a), Group (b), Group (c), and Group (d), and the compound or an N-oxide thereof, or a salt thereof according to [7] or [8]:
   Group (a): a group consisting of insecticidal active ingredients, miticidal active ingredients, and nematicidal active ingredients;
   Group (b): fungicidal active ingredients;
   Group (c): plant growth regulatory ingredients;
   Group (d): repellent ingredients.
[11] A method for controlling a plant disease which comprises applying an effective amount of the compound or an N-oxide thereof, or a salt thereof according to [7] or [8] or an effective amount of the composition according to [10], to a plant or soil for cultivating a plant.
[12] Use of the compound or an N-oxide thereof, or a salt thereof according to [7] or [8] or the composition according to [10] for controlling a plant disease.
[13] A seed or a vegetative reproductive organ holding an effective amount of the compound or an N-oxide thereof, or a salt thereof according to [7] or [8] or an effective amount of the composition according to [10].
[14] A compound represented by formula (III): 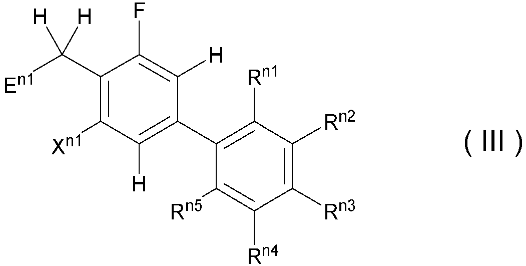 [wherein
   Xⁿ¹ represents a halogen atom,
   Rⁿ¹, Rⁿ², Rⁿ³, and Rⁿ⁵ are identical to or different from each other and each represents a hydrogen atom, a halogen atom, a methyl group, or a methoxy group,
   a combination of Eⁿ¹ and Rⁿ⁴ represents
      a combination wherein Eⁿ¹ represents a halogen atom, and Rⁿ⁴ represents a hydrogen atom, a methyl group, or a methoxy group; or
      a combination wherein Eⁿ¹ represents a cyano group, C(O)OH, or C(NH₂)=NORⁿ⁶, and Rⁿ⁴ represents a hydrogen atom, a halogen atom, a methyl group, or a methoxy group,
   Rⁿ⁶ represents a C1-C6 alkylcarbonyl group optionally substituted with one or more halogen atoms, or a hydrogen atom].
   (hereinafter, referred to as "Intermediate compound A").
[15] A compound represented by formula (IV): 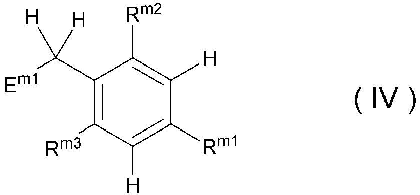 [wherein
   R^{m1} represents a chlorine atom, a bromine atom, or an iodine atom,
   R^{m2} represents a halogen atom,
   R^{m3} represents a hydrogen atom, a halogen atom, a methyl group, or a methoxy group,
   E^{m1} represents a 1,2,4-thiadiazole-3-yl group, a 1,2,4-oxadiazole-3-yl group {the 1,2,4-thiadiazole-3-yl group, and the 1,2,4-oxadiazole-3-yl group may be optionally substituted with one or more substituents selected from Group A^{m1}}, 1,2,3-triazole-2-yl group, or an isothiadiazole-3-yl group {the 1,2,3-triazole-2-yl group, and the isothiadiazole-3-yl group may be optionally substituted with one or more substituents selected from Group B^{m1}}.
   Group A^{m1}: a group consisting of a C1-C4 alkyl group, a C1-C4 alkoxy group, a cyclopropyl group, and a CF₃ group.
   Group B^{m1}: a group consisting of a halogen atom, a C1-C4 alkyl group, a C1-C4aalkoxy group, a C1-C4 haloalkyl group, a C1-C4 haloalkoxy group, and a cyclopropyl group].
   (hereinafter, referred to as "Intermediate compound B").

### [EFFECTS OF THE INVENTION]

The present invention can control plant diseases.

### DETAILED DESCRIPTION

The substituents as described herein are explained as follows.

The term "halogen atom" represents a fluorine atom, a chlorine atom, a bromine atom, or an iodine atom.

When a substituent has two or more halogen atoms or substituents, the halogen atoms or the substituents may be identical to or different from each other.

Regarding the expression of "(may be) optionally substituted with one or more substituents selected from Group X (wherein X represents any one of A, B, C, D, A^{a}, B^{a}, C^{a}, D^{a}, and E^{a})" as described herein represents that when two or more of the substituents selected from Group X are present, the substituents may be identical to or different from each other.

The expression of (R⁴)ₙ as described herein represents that when n is 2 or 3, a plural of R⁴ may be identical to or different from each other.

The expression of (R⁵)ₘ as described herein represents that when m is 2 or 3, a plural of R³ may be identical to or different from each other.

The expression of (R⁶)_{q} as described herein represents that when q is 2, 3, 4 or 5, a plural of R⁶ may be identical to or different from each other.

The expression of (R^{4a})ₙₐ as described herein represents that when na is 2 or 3, a plural of R^{4a} may be identical to or different from each other.

The expression of (R^{5a})ₘₐ as described herein represents that when ma is 2 or 3, a plural of R^{5a} may be identical to or different from each other.

The expression of (R^{6a})_{qa} as described herein represents that when qa is 2, 3, 4, or 5, a plural of R^{6a} may be identical to or different from each other.

The expression "CX-CY" as described herein represents that the number of carbon atoms is from X to Y. For example, the expression "C1-C6" represents that the number of carbon atoms is from 1 to 6.

The term of "chain hydrocarbon group" represents an alkyl group, an alkenyl group, or an alkynyl group.

Examples of the term of "alkyl group" include a methyl group, an ethyl group, a propyl group, an isopropyl group, a 1,1-dimethylpropyl group, a 1,2-dimethylpropyl group, a butyl group, a sec-butyl group, a tert-butyl group, a pentyl group, and a hexyl group.

Examples of the term of "alkenyl group" include a vinyl group, a 1-propenyl group, a 2-propenyl group, a 1-methyl-1-propenyl group, a 1-methyl-2-propenyl group, a 1,2-dimethyl-1-propenyl group, a 3-butenyl group, a 4-pentenyl group, and a 5-hexenyl group.

Examples of the term of "alkynyl group" include an ethynyl group, a 1-propynyl group, a 2-propynyl group, a 1-methyl-2-propynyl group, a 1,1-dimethyl-2-propynyl group, a 2-butynyl group, a 4-pentynyl group, and a 5-hexynyl group.

Examples of the term of "alkoxy group" include a methoxy group, an ethoxy group, a propoxy group, an isopropoxy group, a butoxy group, a tert-butoxy group, a pentyloxy group, and a hexyloxy group.

Examples of the term of "alkylsulfanyl group" include a methyl sulfanyl group, an ethyl sulfanyl group, an isopropyl sulfanyl group, and a hexyl sulfanyl group.

Examples of the term of "alkylsulfinyl group" include a methyl sulfinyl group, an ethyl sulfinyl group, an isopropyl sulfinyl group, and a hexylsulfinyl group.

Examples of the term of "alkylsulfonyl group" include a methane sulfonyl group, an ethane sulfonyl group, an isopropane sulfonyl group, and a hexane sulfonyl group.

Examples of the term of "alkylcarbonyl group" include an acetyl group, and a propionyl group.

Examples of the term of "alkoxycarbonyl group" include a methoxycarbonyl group, an isopropoxycarbonyl group, and a hexyloxycarbonyl group,

Examples of the term of "alicyclic hydrocarbon" include a cyclobutene ring, a cyclopentene ring, a cyclopentadiene ring, a cyclohexene ring, a cyclohexadiene ring, and a cycloheptene ring.

Examples of the term of "alicyclic hydrocarbon group" include a cycloalkyl group and a cycloalkenyl group.

Examples of the term of "cycloalkyl group" include a cyclopropyl group, a cyclobutyl group, a cyclopentyl group, and a cyclohexyl group.

Examples of the term of "cycloalkenyl group" include a cyclopentenyl group, and a cyclohexenyl group.

Examples of the term of "nonaromatic heterocycle" include a dihydrofuran ring, a dihydrothiophene ring, a dihydropyrrole ring, a 1,3-dioxole ring, a pyran ring, and a dihydropyran ring.

Examples of the term of "aromatic heterocycle" include a furan ring, a thiophene ring, a pyrrole ring, a pyrazole ring, an imidazole ring, an oxazole ring, an isoxazole ring, a thiazole ring, an isothiazole ring, a triazole ring, an oxadiazole ring, a thiadiazole ring, a pyridine ring, a pyridazine ring, a pyrimidine ring, and a pyrazine ring.

Examples of the term of "aromatic heterocyclic group" include five(5) membered aromatic heterocyclic group such as a pyrrolyl group, a furanyl group, a thienyl group, a pyrazolyl group, an imidazolyl group, a triazolyl group, a tetrazolyl group, an oxazolyl group, an isoxazolyl group, a thiazolyl group, an isothiazolyl group, an oxadiazolyl group, and a thiadiazolyl group; and six(6) membered aromatic heterocyclic group such as a pyridyl group, a pyridazinyl group, a pyrimidinyl group, a pyrazinyl group, a triazinyl group, and a tetrazinyl group.

Examples of the term of "nonaromatic heterocyclic group" include an aziridinyl group, an oxiranyl group, a thiranyl group, an azetidinyl group, an oxetanyl group, a thietanyl group, a pyrrolidinyl group, a tetrahydrofuranyl group, a tetrahydrothienyl group, a pyrazolynyl group, a pyrazolidinyl group, an imidazolinyl group, an imidazolidinyl group, an oxazolinyl group, a thiazolinyl group, an oxazolidinyl group, a thiazolidinyl group, an isoxazolinyl group, an isoxazolidinyl group, an isothiazolinyl group, an isothiazolidinyl group, a dioxolanyl group, a dioxanyl group, a piperidyl group, a piperazinyl group, a morpholinyl group, a thiomorpholinyl group, a pyranyl group, a dihydropyranyl group, a tetrahydropyranyl group, a tetrahydrothiopyranyl group, an azepanyl group, an oxepanyl group, and a thiepanyl group.

The ring A⁵ in the formula (I) means a group represented by formula (A⁵-1): 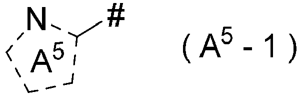
[wherein # is the same as defined above]
, and represents a five(5) membered nonaromatic nitrogen-containing heterocycle or a five(5) membered aromatic nitrogen-containing heterocycle (with the proviso that in the ring A⁵, the atom bound to D represents a carbon atom or a nitrogen atom, and among the atoms which are adjacent to the atom bound to the D and constitute the ring A⁵, at least one thereof represents a nitrogen atom).

As described in the formula (I), the ring A⁵ is substituted with the number of n of R⁴, and the ring A⁵ substituted with the number of n of R⁴ means a partial structure of formula (A⁵-2): 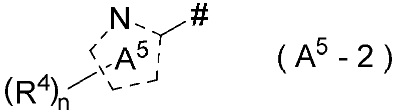 [wherein #, R⁴ and n are the same as defined above].

The ring A⁵ substituted with the number of n of R⁴, which is represented by formula (A⁵-2) represents, that is, a five(5) membered nonaromatic nitrogen-containing heterocycle which is substituted with the number of n of R⁴, or a five(5)-membered aromatic nitrogen-containing heterocycle which is substituted with the number of n of R⁴ (with the proviso that in the ring A⁵, the atom bound to D represents a carbon atom or a nitrogen atom, and among the atoms which are adjacent to the atom bound to the D and constitutes the ring A⁵, at least one thereof represents a nitrogen atom).

Examples of the group represented by formula (A⁵-2) include a group represented by formula H1, a group represented by formula H2, a group represented by formula H3, and a group represented by formula H4: 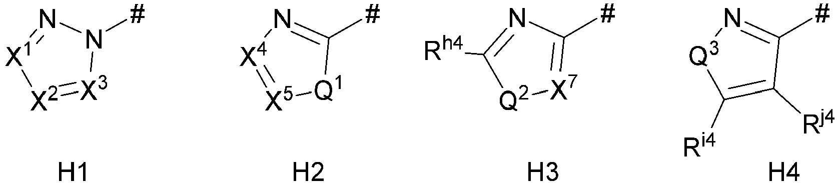 [wherein the symbols are the same as defined above].

The ring A^{5a} in the formula (II) represents group represented by formula (A^{5a}-1): 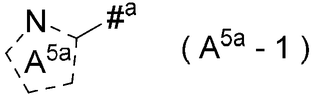
[wherein #^{a} is the same as defined above]
, and represents a five(5) membered aromatic nitrogen-containing heterocycle (with the proviso that in the ring A^{5a}, the atom bound to CR^{1a}R^{2a} represents a carbon atom or a nitrogen atom, and among the atoms which are adjacent to the atom bound to the CR^{1a}R^{2a} and constitute the ring A^{5a}, at least one thereof represents a nitrogen atom).

As described in the formula (II), the ring A^{5a} is substituted with the number of na of R^{4a}, and the ring A^{5a} substituted with the number of n of R^{4a} means a partial structure of formula (A^{5a}-2): 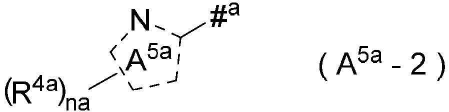 [wherein #^{a}, R^{4a} and na are the same as defined above].

The ring A^{5a} substituted with the number of na of R^{4a}, which is represented by formula (A^{5a}-2) represents, that is, a five(5) membered aromatic nitrogen-containing heterocycle which is substituted with the number of na of R^{4a} (with the proviso that in the ring A^{5a}, the atom bound to CR^{1a}R^{2a} represents a carbon atom or a nitrogen atom, and among the atoms which are adjacent to the atom bound to the CR^{1a}R^{2a} and constitutes the ring A^{5a}, at least one thereof represents a nitrogen atom).

Examples of the group represented by formula (A^{5a}-2) include a group represented by formula H1a, a group represented by formula H2a, a group represented by formula H3a, and a group represented by formula H4a: 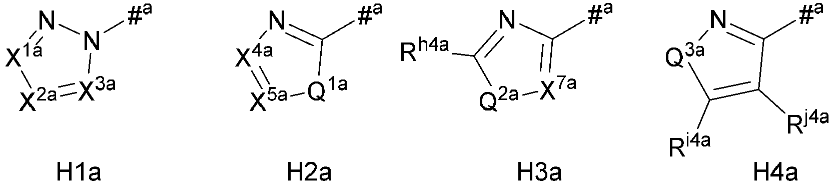 [wherein the symbols are the same as defined above].

4-((3,4,5-Tribromo-1H-pyrazole-1-yl)methyl)-[1,1'-biphenyl]-3-ol is a compound represented by the following formula.

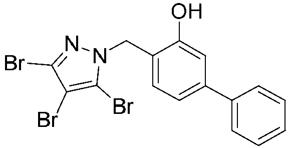

5-(1-(3-Fluoro-[1,1'-biphenyl]-4-yl)ethyl)-3-methyl-1,2,4-oxadiazole is a compound represented by the following formula.

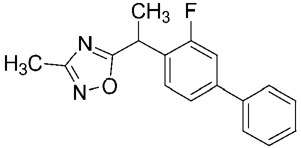

3-(Chloromethyl)-5-(1-(3-fluoro-[1,1'-biphenyl]-4-yl)ethyl)-1,2,4-oxadiazole is a compound represented by the following formula.

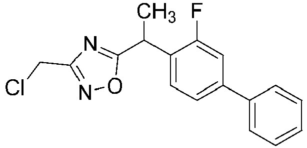

3-((3,5-Difluoro-[1,1'-biphenyl]-4-yl)methyl)-1,2,4-thiadiazole is a compound represented by the following formula.

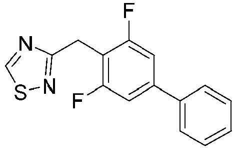

3-((3-Fluoro-[1,1'-biphenyl]-4-yl)methyl)-1,2,4-thiadiazole is a compound represented by the following formula.

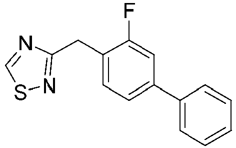

3-((3,5-Difluoro-[1,1'-biphenyl]-4-yl)difluoromethyl)-1,2,4-thiadiazole is a compound represented by the following formula.

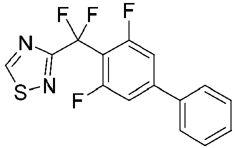

3-((3,5-Difluoro-[1,1'-biphenyl]-4-yl)methyl)-1,3,4-thiadiazole-2(3H)-one is a compound represented by the following formula.

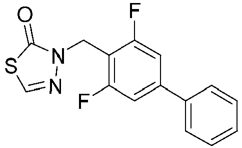

2-((3,5-Difluoro-[1,1'-biphenyl]-4-yl)methyl)-1,3,4-thiadiazole is a compound represented by the following formula.

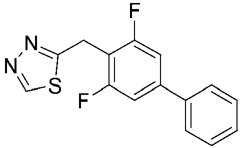

(3,5-Difluoro-[1,1'-biphenyl]-4-yl)(1,2,4-thiadiazole-3-yl)methanone is a compound represented by the following formula.

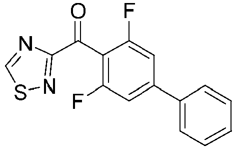

2-((3-Fluoro-[1,1'-biphenyl]-4-yl)methyl)-1,3,4-oxadiazole is a compound represented by the following formula.

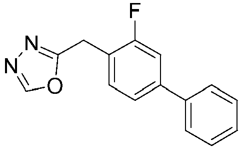

(E)-(3,5-Difluoro-[1,1'-biphenyl]-4-yl)(1,2,4-thiadiazole-3-yl)methanone O-methyl oxime is a compound represented by the following formula.

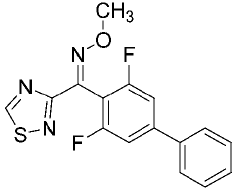

(Z)-(3,5-Difluoro-[1,1'-biphenyl]-4-yl)(1,2,4-thiadiazole-3-yl)methanone O-methyl oxime is a compound represented by the following formula.

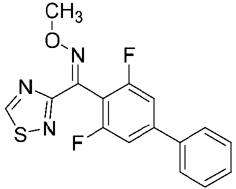

3-((3,5-Difluoro-[1,1'-biphenyl]-4-yl)methyl)-5-ethyl-1,2,4-thiadiazole is a compound represented by the following formula.

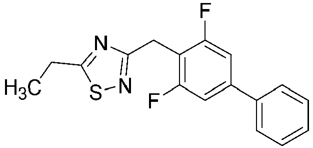

5-Cyclopropyl-3-((3,5-difluoro-[1,1'-biphenyl]-4-yl)methyl)-1,2,4-thiadiazole is a compound represented by the following formula.

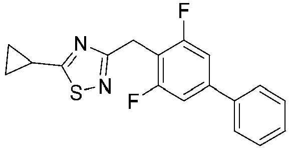

3-((3,5-Difluoro-[1,1'-biphenyl]-4-yl)methyl)-5-(trifluoromethyl)-1,2,4-thiadiazole is a compound represented by the following formula.

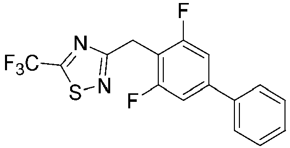

3-((3,5-Difluoro-[1,1'-biphenyl]-4-yl)methyl)-1,2,4-thiadiazole-5-carbonitrile is a compound represented by the following formula.

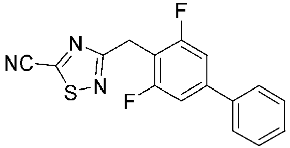

3-((3,5-Difluoro-[1,1'-biphenyl]-4-yl)methyl)isoxazole-5(4H)-one is a compound represented by the following formula.

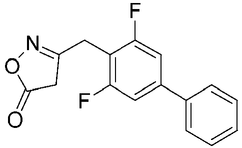

5-Chloro-3-((3,5-difluoro-[1,1'-biphenyl]-4-yl)methyl)isoxazole is a compound represented by the following formula.

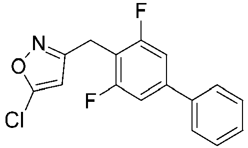

3-((3,5-Difluoro-[1,1'-biphenyl]-4-yl)methyl)-1,2,4-thiadiazole-5-ol is a compound represented by the following formula.

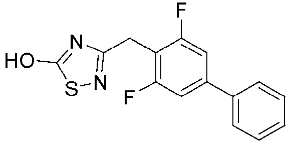

3-((3,5-Difluoro-[1,1'-biphenyl]-4-yl)methyl)-5-methoxy-1,2,4-thiadiazole is a compound represented by the following formula.

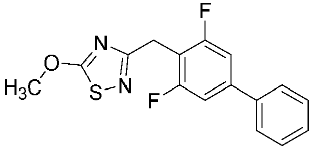

(2-Chlorothiazole-4-yl)(3-fluoro-[1,1'-biphenyl]-4-yl)methanol is a compound represented by the following formula.

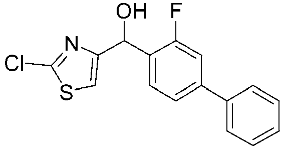

2-Chloro-4-((3-fluoro-[1,1'-biphenyl]-4-yl)methyl)thiazole is a compound represented by the following formula.

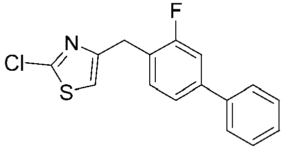

5-Chloro-3-((3,5-difluoro-[1,1'-biphenyl]-4-yl)methyl)-1,2,4-thiadiazole is a compound represented by the following formula.

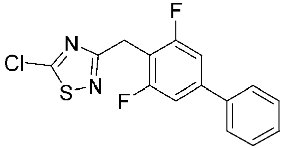

5-Bromo-3-((3,5-difluoro-[1,1'-biphenyl]-4-yl)methyl)-1,2,4-thiadiazole is a compound represented by the following formula.

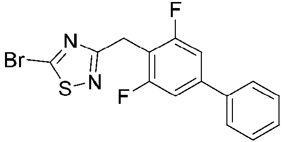

3-((3,5-Difluoro-[1,1'-biphenyl]-4-yl)methyl)-1,2,4-thiadiazole-5-carboxamide is a compound represented by the following formula.

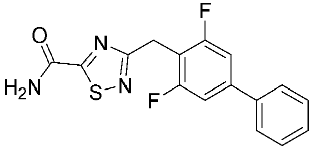

An N-oxide of the compound represented by formula (I) or formula (II) means a structure in which at least one nitrogen atom contained in the compound represented by formula (I) or formula (II) is substituted with an oxo group.

The Present compound or the Compound of the present invention, the Intermediate compound A, or the Intermediate compound B may optionally have one or more stereoisomer(s). Examples of the stereoisomer(s) include enantiomers, diastereomers, atropisomers, and geometric isomers. The present invention encompasses each stereoisomer and mixtures of stereoisomers at any ratio.

The compound represented by formula (I) or formula (II), or an N-oxide thereof may optionally be mixed with an acid such as hydrochloric acid, sulfuric acid, nitric acid, phosphoric acid, acetic acid, and benzoic acid to form an acid addition salt such as hydrochloride, sulfate, nitrate, phosphate, acetate, and benzoate.

In the Present compound, examples of the compound wherein when q is 2, 3, 4 or 5, the neighboring two R⁶ and R⁶ may combine together with carbon atoms to which they are attached to form a C5-C6 alicyclic hydrocarbon, a five(5)-six(6) membered nonaromatic heterocycle {the C5-C6 alicyclic hydrocarbon, and the five(5)- six(6) membered nonaromatic heterocycle may be optionally substituted with one or more substituents selected from Group B}, a benzene ring, or a five(5)- six(6) membered aromatic heterocycle {the benzene ring, and the five(5)- six(6) membered aromatic heterocycle may be optionally substituted with one or more substituents selected from Group C} include specifically
1) a compound forming a formula (I-A): 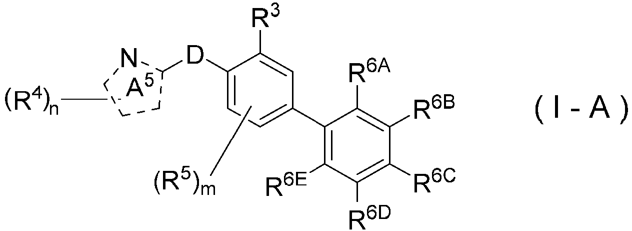 wherein
   the ring A⁵, the R³, the R⁴, the R⁵, the D, m and n are the same as defined in [1],
   the R^{6A}, the R^{6B}, and the R^{6C} are identical to or different from each other and each represents the same meanings as R⁶ in [1] or a hydrogen atom,
   R^{6D} and R^{6E} may combine together with carbon atoms to which they are attached to form a C5-C6 alicyclic hydrocarbon, a five(5)- six(6) membered nonaromatic heterocycle {the C5-C6 alicyclic hydrocarbon, and the five(5)- six(6) membered nonaromatic heterocycle may be optionally substituted with one or more substituents selected from Group B}, a benzene ring, or a five(5)- six(6) membered aromatic heterocycle {the benzene ring, and the five(5)- six(6) membered aromatic heterocycle may be optionally substituted with one or more substituents selected from Group C}; or
2) a compound forming a formula (I-A):
   the ring A⁵, the R³, the R⁴, the R⁵, the D, m and n are the same as defined in [1],
   the R^{6A}, the R^{6B}, and the R^{6E} are identical to or different from each other and each represents the same meanings as R⁶ in [1] or a hydrogen atom,
   R^{6C} and R^{6D} may combine together with carbon atoms to which they are attached to form a C5-C6 alicyclic hydrocarbon, a five(5)- six(6) membered nonaromatic heterocycle {the C5-C6 alicyclic hydrocarbon, and the five(5)- six(6) membered nonaromatic heterocycle may be optionally substituted with one or more substituents selected from Group B}, a benzene ring, or a five(5)- six(6) membered aromatic heterocycle {the benzene ring, and the five(5)- six(6) membered aromatic heterocycle may be optionally substituted with one or more substituents selected from Group C}.

Aspects of the Present compound A include the following compounds.
[Aspect A1] The Present compound A wherein the ring A⁵ represents a five(5) membered nonaromatic nitrogen-containing heterocycle.
[Aspect A2] The Present compound A wherein the ring A⁵ represents a five(5) membered aromatic nitrogen-containing heterocycle.
[Aspect A3] The Present compound A wherein the ring A⁵ represents a group represented by formula H1.
[Aspect A4] The Present compound A wherein the ring A⁵ represents a group represented by formula H2.
[Aspect A5] The Present compound A wherein the ring A⁵ represents a group represented by formula H3.
[Aspect A6] The Present compound A wherein the ring A⁵ represents a group represented by formula H4.
[Aspect A7] The Present compound A wherein the ring A⁵ represents a group represented by formula H1, a group represented by formula H2, a group represented by formula H3 or a group represented by formula H4.
[Aspect A8] The Present compound A wherein D represents CR¹R².
[Aspect A9] The Present compound A wherein D represents C=O.
[Aspect A10] The Present compound A wherein R¹ and R² are identical to or different from each other and each represents a C1-C6 chain hydrocarbon group optionally substituted with one or more substituents selected from Group A, or a hydrogen atom.
[Aspect A11] The Present compound A wherein R¹ and R² are identical to or different from each other and each represents a C1-C6 chain hydrocarbon group optionally substituted with one or more halogen atoms, or a hydrogen atom.
[Aspect A12] The Present compound A wherein R¹ and R² are identical to or different from each other and each represents OR⁸, or a hydrogen atom.
[Aspect A13] The Present compound A wherein R¹ and R² are identical to or different from each other and each represents SR⁹, S(O)R¹⁹, S(O)₂R¹¹, NR¹²R¹³, or a hydrogen atom.
[Aspect A14] The Present compound A wherein R¹ and R² are identical to or different from each other and each represents C(O)R¹⁴, C(R¹⁵)=NOR¹⁶, a cyano group, a nitro group, or a hydrogen atom.
[Aspect A15] The Present compound A wherein R¹ and R² are identical to or different from each other and each represents a halogen atom, or a hydrogen atom.
[Aspect A16] The Present compound A wherein R¹ and R² represent a hydrogen atom.
[Aspect A17] The Present compound A wherein R¹ and R² are identical to or different from each other and each represents a C1-C6 chain hydrocarbon group optionally substituted with one or more halogen atoms, OR⁸, a halogen atom, or a hydrogen atom.
[Aspect A18] The Present compound A wherein R¹ and R² are identical to or different from each other and each represents OR⁸, a halogen atom, or a hydrogen atom.
[Aspect A19] The Present compound A wherein R¹ and R² are identical to or different from each other and each represents a C1-C6 chain hydrocarbon group optionally substituted with one or more halogen atoms, OR⁸, or a hydrogen atom.
[Aspect A20] The Present compound A wherein R³ and R⁵ are identical to or different from each other and each represents a C1-C6 chain hydrocarbon group optionally substituted with one or more substituents selected from Group A.
[Aspect A21] The Present compound A wherein R³ and R⁵ are identical to or different from each other and each represents a C1-C6 chain hydrocarbon group optionally substituted with one or more halogen atoms.
[Aspect A22] The Present compound A wherein R³ and R⁵ are identical to or different from each other and each represents a C3-C8 alicyclic hydrocarbon group, or a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}.
[Aspect A23] The Present compound A wherein R³ and R⁵ are identical to or different from each other and each represents a five(5)- six(6) membered aromatic heterocyclic group optionally substituted with one or more substituents selected from Group C.
[Aspect A24] The Present compound A wherein R³ and R⁵ are identical to or different from each other and each represents OR¹⁷, SR¹⁸, S(O)R¹⁹, S(O)₂R²⁰, NR²¹R²², C(O)R²³, C(R²⁴)=NOR²⁵, a cyano group, or a nitro group.
[Aspect A25] The Present compound A wherein R³ and R⁵ are identical to or different from each other and each represents a halogen atom.
[Aspect A26] The Present compound A wherein R³ and R⁵ are identical to or different from each other and each represents a C1-C6 chain hydrocarbon group optionally substituted with one or more halogen atoms, or a halogen atom.
[Aspect A27] The Present compound A wherein R⁴ represents a C1-C6 chain hydrocarbon group optionally substituted with one or more substituents selected from Group A.
[Aspect A28] The Present compound A wherein R⁴ represents a C1-C6 chain hydrocarbon group optionally substituted with one or more halogen atoms.
[Aspect A29] The Present compound A wherein R⁴ represents a C3-C8 alicyclic hydrocarbon group, or a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}.
[Aspect A30] The Present compound A wherein R⁴ represents OR²⁶, NR²⁷R²⁸, C(O)R²⁹, C(R³⁰)=NOR³¹, a cyano group, or a nitro group.
[Aspect A31] The Present compound A wherein R⁴ represents a halogen atom.
[Aspect A32] The Present compound A wherein R⁴ represents a C1-C6 chain hydrocarbon group optionally substituted with one or more halogen atoms, or a halogen atom.
[Aspect A33] The Present compound A wherein R⁶ represents a C1-C6 chain hydrocarbon group optionally substituted with one or more substituents selected from Group A.
[Aspect A34] The Present compound A wherein R⁶ represents a C1-C6 chain hydrocarbon group optionally substituted with one or more halogen atoms.
[Aspect A35] The Present compound A wherein R⁶ represents a C3-C8 alicyclic hydrocarbon group, or a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}.
[Aspect A36] The Present compound A wherein R⁶ represents a phenyl group, or a five(5)- six(6) membered aromatic heterocyclic group {the phenyl group, and the a five(5)-six(6) membered aromatic heterocyclic group may be optionally substituted with one or more substituents selected from Group C}.
[Aspect A37] The Present compound A wherein R⁶ represents OR³², SR³³, S(O)R³⁴, S(O)₂R³⁵, NR³⁶R³⁷, C(O)R³⁸, C(R³⁹) =NOR⁴⁰, a cyano group, or a nitro group.
[Aspect A38] The Present compound A wherein R⁶ represents a halogen atom.
[Aspect A39] The Present compound A wherein R⁶ represents a C1-C6 chain hydrocarbon group optionally substituted with one or more halogen atoms, or a halogen atom.
[Aspect A40] The Present compound A wherein the neighboring two R⁶ and R⁶ may combine together with carbon atoms to which they are attached to form a C5-C6 alicyclic hydrocarbon, or a five(5)- six(6) membered nonaromatic heterocycle {the C5-C6 alicyclic hydrocarbon, and the a five(5)- six(6) membered nonaromatic heterocycle may be optionally substituted with one or more halogen atoms}.
[Aspect A41] The Present compound A wherein the neighboring two R⁶ and R⁶ may combine together with carbon atoms to which they are attached to form a benzene ring, or a five(5)- six(6) membered aromatic heterocyce {the benzene ring, and the a five(5)- six(6) membered aromatic heterocycle optionally substituted with one or more halogen atoms}.
[Aspect A42] The Present compound A wherein n is 0.
[Aspect A43] The Present compound A wherein n is 1.
[Aspect A44] The Present compound A wherein n is 2.
[Aspect A45] The Present compound A wherein n is 3.
[Aspect A46] The Present compound A wherein m is 0.
[Aspect A47] The Present compound A wherein m is 1.
[Aspect A48] The Present compound A wherein m is 2.
[Aspect A49] The Present compound A wherein m is 3.
[Aspect A50] The Present compound A wherein m is 0, or 1.
[Aspect A51] The Present compound A wherein q is 0.
[Aspect A52] The Present compound A wherein q is 1.
[Aspect A53] The Present compound A wherein q is 2.
[Aspect A54] The Present compound A wherein q is 3.
[Aspect A55] The Present compound A wherein q is 4.
[Aspect A56] The Present compound A wherein q is 5.
[Aspect A57] The Present compound A wherein the ring A⁵ is a group represented by formula H1, a group represented by formula H2, a group represented by formula H3, or a group represented by formula H4, D represents CR¹R², R¹ and R² are identical to or different from each other and each represents a C1-C6 chain hydrocarbon group optionally substituted with one or more halogen atoms, OR⁸, a halogen atom, or a hydrogen atom, R³ and R⁵ are identical to or different from each other and each represents a C1-C6 chain hydrocarbon group optionally substituted with one or more halogen atoms, or a halogen atom, R⁴ represents a C1-C6 chain hydrocarbon group optionally substituted with one or more halogen atoms, or a halogen atom, and R⁶ represents a C1-C6 chain hydrocarbon group optionally substituted with one or more halogen atoms, or a halogen atom.
[Aspect A58] The Present compound A wherein R¹ and R² may combine together with a carbon atom to which they are attached to form a C3-C8 alicyclic hydrocarbon, or a three(3)- eight(8) membered nonaromatic heterocycle {the C3-C8 alicyclic hydrocarbon, and the three(3)- eight(8) membered nonaromatic heterocycle may be optionally substituted with one or more substituents selected from Group B}.
[Aspect A59] The Present compound A wherein the ring A⁵ represents a group represented by formula H1 or a group represented by formula H2.
[Aspect A60] The Present compound A wherein the ring A⁵ represents a group represented by formula H1 or a group represented by formula H3.
[Aspect A61] The Present compound A wherein the ring A⁵ represents a group represented by formula H1 or a group represented by formula H4.
[Aspect A62] The Present compound A wherein the ring A⁵ represents a group represented by formula H2 or a group represented by formula H3.
[Aspect A63] The Present compound A wherein the ring A⁵ represents a group represented by formula H2 or a group represented by formula H4.
[Aspect A64] The Present compound A wherein the ring A⁵ represents a group represented by formula H3 or a group represented by formula H4.
[Aspect A65] The Present compound A wherein the ring A⁵ represents a group represented by formula H1, a group represented by formula H2, a group represented by formula H3, or a group represented by formula H4, a tetrazole-1-yl group, or a tetrazole-2-yl group.

Aspects of the Present compound A include the following compounds.
[Aspect AX1] The Present compound A wherein the ring A⁵ represents a group represented by formula H1-1, formula H1-2, formula H1-3, formula H1-4, formula H1-5, formula H1-6, formula H2-1, formula H2-2, formula H3-1, formula H3-2, formula H4-1, or formula H4-2. 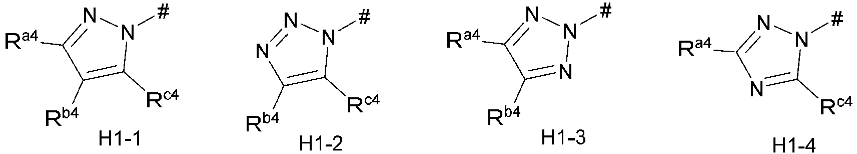 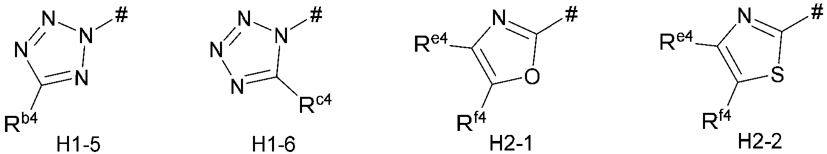 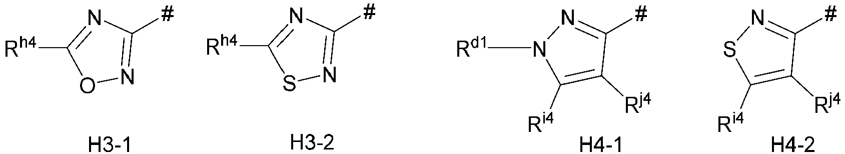 [wherein the symbols are the same as defined above]
[Aspect AX2] The Present compound A wherein the ring A⁵ represents a group represented by formula H1-1, formula H1-2, formula H1-3, formula H1-4, formula H1-5, formula H1-6, formula H2-1, formula H2-2, formula H3-1, formula H3-2, formula H4-1, or formula H4-2,
   R^{a4}, R^{b4}, R^{c4}, R^{e4}, R^{f4}, R^{h4}, Rⁱ⁴, and R^{j4} are identical to or different from each other and each represents a C1-C6 chain hydrocarbon group optionally substituted with one or more halogen atoms, OR²⁶, a halogen atom, or a hydrogen atom,
   R^{d1} represents a C1-C6 chain hydrocarbon group optionally substituted with one or more halogen atoms,
   D represents CR¹R², or C=O,
   R¹ and R² are identical to or different from each other and each represents a halogen atom, a hydroxy group, or a hydrogen atom,
   R³ and R⁵ are identical to or different from each other and each represents a C1-C6 chain hydrocarbon group optionally substituted with one or more halogen atoms, a five(5)- six(6) membered aromatic heterocyclic group optionally substituted with one or more substituent(s) selected from Group A^{x}, OR¹⁷, or a halogen atom,
   R⁶ represents a C1-C6 chain hydrocarbon group optionally substituted with one or more halogen atoms, or a halogen atom,
   R¹⁷ and R²⁶ are identical to or different from each other and each represents a C1-C6 chain hydrocarbon group optionally substituted with one or more halogen atoms, or a hydrogen atom.
   Group A^{X}: a group consisting of a halogen atom, C1-C4 alkyl group, a C1-C4 haloalkyl group, a C1-C4 alkoxy group, and a C1-C4 haloalkoxy group.
[Aspect AX3] The Present compound A wherein the ring A⁵ represents a group represented by formula H1-1, formula H1-2, formula H1-3, formula H1-4, formula H1-5, formula H1-6, formula H2-1, formula H2-2, formula H3-1, formula H3-2, formula H4-1, or formula H4-2,
   R^{a4}, R^{b4}, R^{c4}, R^{e4}, R^{f4}, R^{h4}, Rⁱ⁴, and R^{j4} are identical to or different from each other and each represents a C1-C6 chain hydrocarbon group optionally substituted with one or more halogen atoms, a C1-C4 alkoxy group, a halogen atom, or a hydrogen atom,
   R^{d1} represents a C1-C6 chain hydrocarbon group,
   D represents CR¹R², or C=O,
   R¹ and R² are identical to or different from each other and each represents a halogen atom, a hydroxy group, or a hydrogen atom,
   R³ and R⁵ are identical to or different from each other and each represents a C1-C6 chain hydrocarbon group, a five(5)- six(6) membered aromatic heterocyclic group, C1-C4 alkoxy group, or a halogen atom,
   R⁶ represents a C1-C6 chain hydrocarbon group, or a halogen atom.
[Aspect AX4] The compound of the aspect AX2, aspect AX3, or the Present compound A wherein the ring A⁵ represents a group represented by formula H1-1, formula H1-2, formula H1-3, formula H1-4, formula H1-5, formula H1-6, formula H2-1, formula H2-2, formula H3-1, formula H4-1, or formula H4-2.
[Aspect AX5] The compound of the aspect AX2, aspect AX3, or the Present compound A wherein the ring A⁵ represents a group represented by formula H1-1, formula H1-2, formula H1-3, formula H1-5, formula H1-6, formula H2-1, formula H2-2, formula H3-1, formula H4-1, or formula H4-2.
[Aspect AX5] The compound of the aspect AX2, aspect AX3, or the Present compound A wherein the ring A⁵ represents a group represented by formula H1-1.
[Aspect AX6] The compound of the aspect AX2, aspect AX3, or the Present compound A wherein the ring A⁵ represents a group represented by formula H1-2.
[Aspect AX7] The compound of the aspect AX2, aspect AX3, or the Present compound A wherein the ring A⁵ represents a group represented by formula H1-3.
[Aspect AX8] The compound of the aspect AX2, aspect AX3, or the Present compound A wherein the ring A⁵ represents a group represented by formula H1-4.
[Aspect AX9] The compound of the aspect AX2, aspect AX3, or the Present compound A wherein the ring A⁵ represents a group represented by formula H1-5.
[Aspect AX10] The compound of the aspect AX2, aspect AX3, or the Present compound A wherein the ring A⁵ represents a group represented by formula H1-6.
[Aspect AX11] The compound of the aspect AX2, aspect AX3, or the Present compound A wherein the ring A⁵ represents a group represented by formula H2-1.
[Aspect AX12] The compound of the aspect AX2, aspect AX3, or the Present compound A wherein the ring A⁵ represents a group represented by formula H2-2.
[Aspect AX13] The compound of the aspect AX2, aspect AX3, or the Present compound A wherein the ring A⁵ represents a group represented by formula H3-1.
[Aspect AX14] The compound of the aspect AX2, aspect AX3, or the Present compound A wherein the ring A⁵ represents a group represented by formula H3-2.
[Aspect AX15] The compound of the aspect AX2, aspect AX3, or the Present compound A wherein the ring A⁵ represents a group represented by formula H4-1.
[Aspect AX16] The compound of the aspect AX2, aspect AX3, or the Present compound A wherein the ring A⁵ represents a group represented by formula H4-2.

Aspects of the Compound A of the present invention include the following compounds.
[Aspect B1] The Compound A of the present invention wherein the ring A^{5a} represents a five(5) membered aromatic nitrogen-containing heterocycle.
[Aspect B2] The Compound A of the present invention wherein the ring A^{5a} represents a group represented by formula H1a.
[Aspect B3] The Compound A of the present invention wherein the ring A^{5a} represents a group represented by formula H2a.
[Aspect B4] The Compound A of the present invention wherein the ring A^{5a} represents a group represented by formula H3a.
[Aspect B5] The Compound A of the present invention wherein the ring A^{5a} represents a group represented by formula H4a.
[Aspect B6] The Compound A of the present invention wherein the ring A^{5a} represents a group represented by formula H1a, H2a, H3a, or H4a.
[Aspect B7] The Compound A of the present invention wherein R^{1a} and R^{2a} are identical to or different from each other and each represents a C1-C6 chain hydrocarbon group optionally substituted with one or more substituents selected from Group A^{a}, or a hydrogen atom.
[Aspect B8] The Compound A of the present invention wherein R^{1a} and R^{2a} are identical to or different from each other and each represents a C1-C6 chain hydrocarbon group optionally substituted with one or more halogen atoms, or a hydrogen atom.
[Aspect B9] The Compound A of the present invention wherein R^{1a} and R^{2a} are identical to or different from each other and each represents OR^{8a}, or a hydrogen atom.
[Aspect B10] The Compound A of the present invention wherein R^{1a} and R^{2a} are identical to or different from each other and each represents SR^{9a}, S(O)R^{10a}, S(O)₂R^{11a}, NR^{12a}R^{13a}, or a hydrogen atom.
[Aspect B11] The Compound A of the present invention wherein R^{1a} and R^{2a} are identical to or different from each other and each represents C(O)R^{14a}, C(R^{15a})=NOR^{16a}, a cyano group, a nitro group, or a hydrogen atom.
[Aspect B12] The Compound A of the present invention wherein R^{1a} and R^{2a} are identical to or different from each other and each represents a halogen atom, or a hydrogen atom.
[Aspect B13] The Compound A of the present invention wherein R^{1a} and R^{2a} represent a hydrogen atom.
[Aspect B14] The Compound A of the present invention wherein R^{1a} and R^{2a} are identical to or different from each other and each represents a C1-C6 chain hydrocarbon group optionally substituted with one or more halogen atoms, OR^{8a}, halogen atom, or a hydrogen atom.
[Aspect B15] The Compound A of the present invention wherein R^{1a} and R^{2a} are identical to or different from each other and each represents OR^{8a}, a halogen atom, or a hydrogen atom.
[Aspect B16] The Compound A of the present invention wherein R^{1a} and R^{2a} are identical to or different from each other and each represents a C1-C6 chain hydrocarbon group optionally substituted with one or more halogen atoms, OR^{8a}, or a hydrogen atom.
[Aspect B17] The Compound A of the present invention wherein R^{3a} and R^{5a} are identical to or different from each other and each represents a C1-C6 chain hydrocarbon group optionally substituted with one or more substituents selected from Group A^{a}.
[Aspect B18] The Compound A of the present invention wherein R^{3a} and R^{5a} are identical to or different from each other and each represents a C1-C6 chain hydrocarbon group optionally substituted with one or more halogen atoms.
[Aspect B19] The Compound A of the present invention wherein R^{3a} and R^{5a} are identical to or different from each other and each represents a C3-C8 alicyclic hydrocarbon group, or a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}.
[Aspect B20] The Compound A of the present invention wherein R^{3a} and R^{5a} are identical to or different from each other and each represents a five(5)- six(6) membered aromatic heterocyclic group optionally substituted with one or more substituents selected from Group C^{a}.
[Aspect B21] The Compound A of the present invention wherein R^{3a} and R^{5a} are identical to or different from each other and each represents OR^{17a}, SR^{18a}, S(O)R^{19a}, S(O)₂^{R20a}, NR^{21a}R^{22a}, C(O)R^{23a}, C(^{R24a})=NOR^{25a}, a cyano group, or a nitro group.
[Aspect B22] The Compound A of the present invention wherein R^{3a} and R^{5a} are identical to or different from each other and each represents a halogen atom.
[Aspect B23] The Compound A of the present invention wherein R^{3a} and R^{5a} are identical to or different from each other and each represents a C1-C6 chain hydrocarbon group optionally substituted with one or more halogen atoms, or a halogen atom.
[Aspect B24] The Compound A of the present invention wherein R^{4a} represents a C1-C6 chain hydrocarbon group optionally substituted with one or more substituents selected from Group D^{a}.
[Aspect B25] The Compound A of the present invention wherein R^{4a} represents a C1-C6 chain hydrocarbon group optionally substituted with one or more halogen atoms.
[Aspect B26] The Compound A of the present invention wherein R^{4a} represents a C3-C8 alicyclic hydrocarbon group, or a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}.
[Aspect B27] The Compound A of the present invention wherein R^{4a} represents OR^{26a}, NR^{27a}R^{28a}, C(O)R^{29a}, C(R^{30a})=NOR^{31a}, a cyano group, or a nitro group.
[Aspect B28] The Compound A of the present invention wherein R^{4a} represents a halogen atom.
[Aspect B29] The Compound A of the present invention wherein R^{4a} represents a C1-C6 chain hydrocarbon group optionally substituted with one or more halogen atoms, or a halogen atom.
[Aspect B30] The Compound A of the present invention wherein R^{6a} represents a C1-C6 chain hydrocarbon group optionally substituted with one or more substituents selected from Group E^{a}.
[Aspect B31] The Compound A of the present invention wherein R^{6a} represents a C1-C6 chain hydrocarbon group optionally substituted with one or more halogen atoms.
[Aspect B32] The Compound A of the present invention wherein R^{6a} represents a C3-C8 alicyclic hydrocarbon group optionally substituted with one or more substituents selected from Group B^{a}.
[Aspect B33] The Compound A of the present invention wherein R^{6a} represents a phenyl group, or a five(5)- six(6) membered aromatic heterocyclic group {the phenyl group, and the a five(5)- six(6) membered aromatic heterocyclic group may be optionally substituted with one or more substituents selected from Group C^{a}}.
[Aspect B34] The Compound A of the present invention wherein R^{6a} represents OR^{31a}, SR^{32a}, S(O)R^{33a}, S(O)₂R^{34a}, NR^{35a}R^{36a}, C(O)R^{37a}, C(R^{38a}) =NOR^{39a}, a cyano group, or a nitro group.
[Aspect B35] The Compound A of the present invention wherein R^{6a} represents a halogen atom.
[Aspect B36] The Compound A of the present invention wherein R^{6a} represents a C1-C6 chain hydrocarbon group optionally substituted with one or more halogen atoms, or a halogen atom.
[Aspect B37] The Compound A of the present invention wherein na is 0.
[Aspect B38] The Compound A of the present invention wherein na is 1.
[Aspect B39] The Compound A of the present invention wherein na is 2.
[Aspect B40] The Compound A of the present invention wherein na is 3.
[Aspect B41] The Compound A of the present invention wherein ma is 0.
[Aspect B42] The Compound A of the present invention wherein ma is 1.
[Aspect B43] The Compound A of the present invention wherein ma is 2.
[Aspect B44] The Compound A of the present invention wherein ma is 3.
[Aspect B45] The Compound A of the present invention wherein ma is 0, or 1.
[Aspect B46] The Compound A of the present invention wherein qa is 0.
[Aspect B47] The Compound A of the present invention wherein qa is 1.
[Aspect B48] The Compound A of the present invention wherein qa is 2.
[Aspect B49] The Compound A of the present invention wherein qa is 3.
[Aspect B50] The Compound A of the present invention wherein qa is 4.
[Aspect B51] The Compound A of the present invention wherein qa is 5.
[Aspect B52] The Compound A of the present invention wherein the R^{1a} and R^{2a} may combine together with a carbon atom to which they are attached to form a C3-C8 alicyclic hydrocarbon, or a three(3)- eight(8) membered nonaromatic heterocycle {the C3-C8 alicyclic hydrocarbon, and the three(3)- eight(8) membered nonaromatic heterocycle may be optionally substituted with one or more substituents selected from Group B^{a}}.
[Aspect B53] The Compound A of the present invention wherein the ring A^{5a} represents a group represented by formula H1a, or H2a.
[Aspect B54] The Compound A of the present invention wherein the ring A^{5a} represents a group represented by formula H1a, or H3a.
[Aspect B55] The Compound A of the present invention wherein the ring A^{5a} represents a group represented by formula H1a, or H4a.
[Aspect B56] The Compound A of the present invention wherein the ring A^{5a} represents a group represented by formula H2a, or H3a.
[Aspect B57] The Compound A of the present invention wherein the ring A^{5a} represents a group represented by formula H2a, or H4a.
[Aspect B58] The Compound A of the present invention wherein the ring A^{5a} represents a group represented by formula H3a, or H4a.
[Aspect B59] The Compound A of the present invention wherein the ring A^{5a} represents a group represented by formula H1a, H2a, H3a, or H4a, a tetrazole-1-yl group, or a tetrazole-2-yl group.

Also, aspects of the Compound A of the present invention include the following compounds.
[Aspect BB1] A compound represented by formula (II-1A) 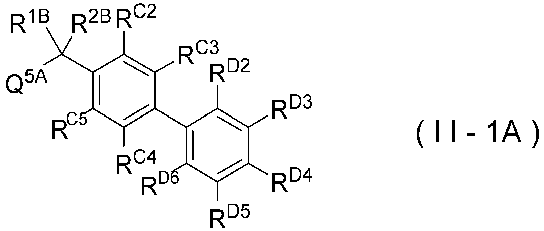 [wherein
   Q^{5A} represents a group representd by formula H1a, H2a, H3a, or H4a,
   R^{1B} and R^{2B} are identical to or different from each other and each represents a C1-C6 chain hydrocarbon group optionally substituted with one or more halogen atoms, OR^{8a}, SR^{9a}, S (O)R^{10a}, S(O)₂R¹¹³, NR^{12a}R^{13a}, C (O)R^{14a}, C (R^{15a})=NOR^{16a}, a cyano group, a nitro group, a halogen atom, or a hydrogen atom,
   R^{C2} represents a C1-C6 chain hydrocarbon group optionally substituted with one or more substituents selected from Group A^{a}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}}, a five(5)- six(6) membered aromatic heterocyclic group optionally substituted with one or more substituents selected from Group C^{a}, OR^{17a}, SR^{18a}, S(O)R^{19a}, S(O)₂R^{20a}, NR^{21a}R^{22a}, C(O)R^{23a}, C(R^{24a})=NOR^{25a}, a cyano group, a nitro group, or a halogen atom,
   R^{C3}, R^{C4}, and R^{C5} are identical to or different from each other and each represents a C1-C6 chain hydrocarbon group optionally substituted with one or more substituents selected from Group A^{a}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}}, a five(5)- six(6) membered aromatic heterocyclic group optionally substituted with one or more substituents selected from Group C^{a}, OR^{17a}, SR^{18a}, S(O)R^{19a}, S(O)₂R^{20a}, NR^{21a}R^{22a}, C(O)R^{23a}, C(R^{24a})=NOR^{25a}, a cyano group, a nitro group, a halogen atom, or a hydrogen atom,
   R^{D2}, R^{D3}, R^{D4}, R^{D5}, and R^{D6} are identical to or different from each other and each represents a C1-C6 chain hydrocarbon group optionally substituted with one or more substituents selected from Group E^{a}, a C3-C8 alicyclic hydrocarbon group optionally substituted with one or more substituents selected from Group B^{a}, a phenyl group, a five(5)- six(6) membered aromatic heterocyclic group {the a phenyl group, and the five(5)- six(6) membered aromatic heterocyclic group may be optionally substituted with one or more substituents selected from Group C^{a}}, OR^{31a}, SR^{32a}, S(O)R^{33a}, S(O)₂R^{34a}, NR^{35a}R^{36a}, C(O)R^{37a}, C(R^{38a})=NOR^{39a}, a cyano group, a nitro group, a halogen atom, or a hydrogen atom, and
   the other symbols are the same as defined in [7] and [8]].
[Aspect BB2] The compound of Aspect BB1 wherein R^{D2}, R^{D3}, R^{D4}, R^{D5}, and R^{D6} are identical to or different from each other and each represents a C1-C4 chain hydrocarbon group optionally substituted with one or more halogen atoms, OR^{31a}, SR^{32a}, S(O)R^{33a}, S(O)₂R^{34a}, a cyano group, a halogen atom, or a hydrogen atom, and R^{32a}, R^{33a}, R^{34a}, and R^{35a} are identical to or different from each other and each represents a C1-C4 alkyl group optionally substituted with one or more halogen atoms.
[Aspect BB3] The compound of Aspect BB1 wherein R^{D2}, R^{D3}, R^{D4}, R^{D5}, and R^{D6} are identical to or different from each other and each represents a C1-C4 chain hydrocarbon group optionally substituted with one or more halogen atoms, a C1-C4 alkoxy group optionally substituted with one or more halogen atoms, a halogen atom, or a hydrogen atom.
[Aspect BB4] The compound of Aspect BB1 wherein R^{D2}, R^{D3}, R^{D4}, R^{D5}, and R^{D6} are identical to or different from each other and each represents a halogen atom, or a hydrogen atom.
[Aspect BB5] The compound of Aspect BB1 wherein R^{c2} represents a C1-C4 chain hydrocarbon group optionally substituted with one or more halogen atoms, a C1-C4 alkoxy group optionally substituted with one or more halogen atoms, or a halogen atom, R^{C3} and R^{C4} are identical to or different from each other and each represents a halogen atom or a hydrogen atom, R^{C5} represents a C1-C4 chain hydrocarbon group optionally substituted with one or more halogen atoms, a C1-C4 alkoxy group optionally substituted with one or more halogen atoms, a halogen atom, or a hydrogen atom.
[Aspect BB6] The compound of Aspect BB2 wherein R^{c2} represents a C1-C4 chain hydrocarbon group optionally substituted with one or more halogen atoms, a C1-C4 alkoxy group optionally substituted with one or more halogen atoms, or a halogen atom, R^{C3} and R^{C4} are identical to or different from each other and each represents a halogen atom or a hydrogen atom, and R^{C5} represents a C1-C4 chain hydrocarbon group optionally substituted with one or more halogen atoms, a C1-C4 alkoxy group optionally substituted with one or more halogen atoms, a halogen atom, or a hydrogen atom.
[Aspect BB7] The compound of Aspect BB3 wherein R^{c2} represents a C1-C4 chain hydrocarbon group optionally substituted with one or more halogen atoms, a C1-C4 alkoxy group optionally substituted with one or more halogen atoms, or a halogen atom, R^{C3} and R^{C4} are identical to or different from each other and each represents a halogen atom or a hydrogen atom, and R^{C5} represents a C1-C4 chain hydrocarbon group optionally substituted with one or more halogen atoms, a C1-C4 alkoxy group optionally substituted with one or more halogen atoms, a halogen atom, or a hydrogen atom.
[Aspect BB8] The compound of Aspect BB4 wherein R^{c2} represents a C1-C4 chain hydrocarbon group optionally substituted with one or more halogen atoms, a C1-C4 alkoxy group optionally substituted with one or more halogen atoms, or a halogen atom, R^{C3} and R^{C4} are identical to or different from each other and each represents a halogen atom or a hydrogen atom, and R^{C5} represents a C1-C4 chain hydrocarbon group optionally substituted with one or more halogen atoms, a C1-C4 alkoxy group optionally substituted with one or more halogen atoms, a halogen atom, or a hydrogen atom.
[Aspect BB9] The compound of Aspect BB1 wherein R^{c2} represents a C1-C4 chain hydrocarbon group optionally substituted with one or more halogen atoms, a C1-C4 alkoxy group optionally substituted with one or more halogen atoms, or a halogen atom, and R^{C3}, R^{C4}, and R^{C5} are identical to or different from each other and each represents a halogen atom or a hydrogen atom.
[Aspect BB10] The compound of Aspect BB2 wherein R^{c2} represents a C1-C4 chain hydrocarbon group optionally substituted with one or more halogen atoms, a C1-C4 alkoxy group optionally substituted with one or more halogen atoms, or a halogen atom, and R^{C3}, R^{e4}, and R^{C5} are identical to or different from each other and each represents a halogen atom or a hydrogen atom.
[Aspect BB11] The compound of Aspect BB3 wherein R^{c2} represents a C1-C4 chain hydrocarbon group optionally substituted with one or more halogen atoms, a C1-C4 alkoxy group optionally substituted with one or more halogen atoms, or a halogen atom, and R^{C3}, R^{C4}, and R^{C5} are identical to or different from each other and each represents a halogen atom or a hydrogen atom.
[Aspect BB12] The compound of Aspect BB4 wherein R^{c2} represents a C1-C4 chain hydrocarbon group optionally substituted with one or more halogen atoms, a C1-C4 alkoxy group optionally substituted with one or more halogen atoms, or a halogen atom, and R^{C3}, R^{C4}, and R^{C5} are identical to or different from each other and each represents a halogen atom or a hydrogen atom.
[Aspect BB13] The compound of Aspect BB1 wherein R^{c2} represents halogen atom, and R^{C3}, R^{C4}, and R^{C5} are identical to or different from each other and each represents a halogen atom or a hydrogen atom.
[Aspect BB14] The compound of Aspect BB2 wherein R^{c2} represents halogen atom, and R^{C3}, R^{C4}, and R^{C5} are identical to or different from each other and each represents a halogen atom or a hydrogen atom.
[Aspect BB15] The compound of Aspect BB3 wherein R^{c2} represents halogen atom, and R^{C3}, R^{C4}, and R^{C5} are identical to or different from each other and each represents a halogen atom or a hydrogen atom.
[Aspect BB16] The compound of Aspect BB4 wherein R^{c2} represents halogen atom, and R^{C3}, R^{C4}, and R^{C5} are identical to or different from each other and each represents a halogen atom or a hydrogen atom.
[Aspect BB17] The compound of Aspect BB1 wherein R^{c2} represents a C1-C4 chain hydrocarbon group optionally substituted with one or more halogen atoms, a C1-C4 alkoxy group optionally substituted with one or more halogen atoms, or a halogen atom, R^{C3} and R^{C4} represent a hydrogen atom, and R^{C5} represents a C1-C4 chain hydrocarbon group optionally substituted with one or more halogen atoms, a C1-C4 alkoxy group optionally substituted with one or more halogen atoms, a halogen atom, or a hydrogen atom.
[Aspect BB18] The compound of Aspect BB2 wherein R^{c2} represents a C1-C4 chain hydrocarbon group optionally substituted with one or more halogen atoms, a C1-C4 alkoxy group optionally substituted with one or more halogen atoms, or a halogen atom, R^{c3} and R^{C4} represent a hydrogen atom, and R^{C5} represents a C1-C4 chain hydrocarbon group optionally substituted with one or more halogen atoms, a C1-C4 alkoxy group optionally substituted with one or more halogen atoms, a halogen atom, or a hydrogen atom.
[Aspect BB19] The compound of Aspect BB3 wherein R^{c2} represents a C1-C4 chain hydrocarbon group optionally substituted with one or more halogen atoms, a C1-C4 alkoxy group optionally substituted with one or more halogen atoms, or a halogen atom, R^{C3} and R^{C4} represent a hydrogen atom, and R^{C5} represents a C1-C4 chain hydrocarbon group optionally substituted with one or more halogen atoms, a C1-C4 alkoxy group optionally substituted with one or more halogen atoms, a halogen atom, or a hydrogen atom.
[Aspect BB20] The compound of Aspect BB4 wherein R^{c2} represents a C1-C4 chain hydrocarbon group optionally substituted with one or more halogen atoms, a C1-C4 alkoxy group optionally substituted with one or more halogen atoms, or a halogen atom, R^{C3} and R^{C4} represent a hydrogen atom, and R^{C5} represents a C1-C4 chain hydrocarbon group optionally substituted with one or more halogen atoms, a C1-C4 alkoxy group optionally substituted with one or more halogen atoms, a halogen atom, or a hydrogen atom.
[Aspect BB21] The compound of Aspect BB1 wherein R^{c2} represents a halogen atom, R^{c3} and R^{c4} represent a hydrogen atom, and R^{c5} represents a halogen atom or a hydrogen atom.
[Aspect BB22] The compound of Aspect BB2 wherein R^{c2} represents a halogen atom, R^{c3} and R^{c4} represent a hydrogen atom, and R^{c5} represents a halogen atom or a hydrogen atom.
[Aspect BB23] The compound of Aspect BB3 wherein R^{c2} represents a halogen atom, R^{c3} and R^{c4} represent a hydrogen atom, and R^{c5} represents a halogen atom or a hydrogen atom.
[Aspect BB24] The compound of Aspect BB4 wherein R^{c2} represents a halogen atom, R^{c3} and R^{c4} represent a hydrogen atom, and R^{c5} represents a halogen atom or a hydrogen atom.
[Aspect BB25] The compound of Aspect BB1 wherein R^{c2} and R^{c5} are identical to or different from each other and each represents a C1-C4 chain hydrocarbon group optionally substituted with one or more halogen atoms, a C1-C4 alkoxy group optionally substituted with one or more halogen atoms, or a halogen atom, and R^{c3} and R^{c4} represent a hydrogen atom.
[Aspect BB26] The compound of Aspect BB2 wherein R^{c2} and R^{c5} are identical to or different from each other and each represents a C1-C4 chain hydrocarbon group optionally substituted with one or more halogen atoms, a C1-C4 alkoxy group optionally substituted with one or more halogen atoms, or a halogen atom, and R^{c3} and R^{c4} represent a hydrogen atom.
[Aspect BB27] The compound of Aspect BB3 wherein R^{c2} and R^{c5} are identical to or different from each other and each represents a C1-C4 chain hydrocarbon group optionally substituted with one or more halogen atoms, a C1-C4 alkoxy group optionally substituted with one or more halogen atoms, or a halogen atom, and R^{c3} and R^{c4} represent a hydrogen atom.
[Aspect BB28] The compound of Aspect BB4 wherein R^{c2} and R^{c5} are identical to or different from each other and each represents a C1-C4 chain hydrocarbon group optionally substituted with one or more halogen atoms, a C1-C4 alkoxy group optionally substituted with one or more halogen atoms, or a halogen atom, and R^{c3} and R^{c4} represent a hydrogen atom.
[Aspect BB29] The compound of Aspect BB1 wherein R^{c2} and R^{c5} represent a halogen atom, and R^{c3} and R^{c4} represent a hydrogen atom.
[Aspect BB30] The compound of Aspect BB2 wherein R^{c2} and R^{c5} represent a halogen atom, and R^{c3} and R^{c4} represent a hydrogen atom.
[Aspect BB31] The compound of Aspect BB3 wherein R^{c2} and R^{c5} represent a halogen atom, and R^{c3} and R^{c4} represent a hydrogen atom.
[Aspect BB32] The compound of Aspect BB4 wherein R^{c2} and R^{c5} represent a halogen atom, and R^{c3} and R^{c4} represent a hydrogen atom.
[Aspect BB33] The compound of Aspect BB1 wherein R^{c2} represents a fluorine atom, R^{c3} and R^{c4} represent a hydrogen atom, and R^{c5} represents a fluorine atom or hydrogen atom.
[Aspect BB34] The compound of Aspect BB2 wherein R^{c2} represents a fluorine atom, R^{c3} and R^{c4} represent a hydrogen atom, and R^{c5} represents a fluorine atom or hydrogen atom.
[Aspect BB35] The compound of Aspect BB3 wherein R^{c2} represents a fluorine atom, R^{c3} and R^{c4} represent a hydrogen atom, and R^{c5} represents a fluorine atom or hydrogen atom.
[Aspect BB36] The compound of Aspect BB4 wherein R^{c2} represents a fluorine atom, R^{c3} and R^{c4} represent a hydrogen atom, and R^{c5} represents a fluorine atom or hydrogen atom.
[Aspect BB37] The compound of Aspect BB1 wherein R^{c2} and R^{c5} represent a fluorine atom, and R^{c3} and R^{c4} represent a hydrogen atom.
[Aspect BB38] The compound of Aspect BB2 wherein R^{c2} and R^{c5} represent a fluorine atom, and R^{c3} and R^{c4} represent a hydrogen atom.
[Aspect BB39] The compound of Aspect BB3 wherein R^{c2} and R^{c5} represent a fluorine atom, and R^{c3} and R^{c4} represent a hydrogen atom.
[Aspect BB40] The compound of Aspect BB4 wherein R^{c2} and R^{c5} represent a fluorine atom, and R^{c3} and R^{c4} represent a hydrogen atom.
[Aspect BB41] The compound of any one of Aspect BB1 to Aspect BB40 wherein R^{1B} and R^{2B} are identical to or different from each other and each represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB42] The compound of any one of Aspect BB1 to Aspect BB40 wherein R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB43] The compound of any one of Aspect BB1 to Aspect BB40 wherein R^{1B} represents a hydrogen atom, R^{2B} represents OR^{8a} or a hydrogen atom, and R^{8a} represents a hydrogen atom.
[Aspect BB44] The compound of any one of Aspect BB1 to Aspect BB40 wherein R^{1B} represents a hydrogen atom, R^{2B} represents OR^{8a}, and R^{8a} represents a hydrogen atom.
[Aspect BB45] The compound of any one of Aspect BB1 to Aspect BB40 wherein R^{1B} and R^{2B} represents a hydrogen atom.
[Aspect BB46] The compound of any one of Aspect BB1 to Aspect BB40 wherein Q^{5A} represents a group represented by formula HQ1, formula HQ2, formula HQ3, formula HQ4, formula HQ5, formula HQ6, formula HQ7, or formula HQ8.

The groups represented by formula HQ1, formula HQ2, formula HQ3, formula HQ4, formula HQ5, formula HQ6, formula HQ7, or formula HQ8 are the gorups of the following groups. 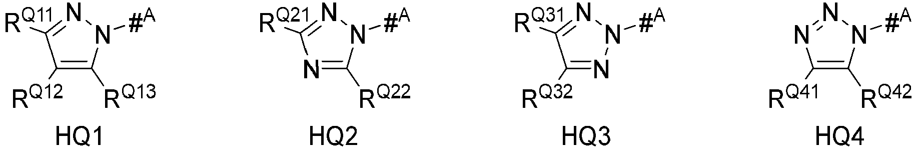 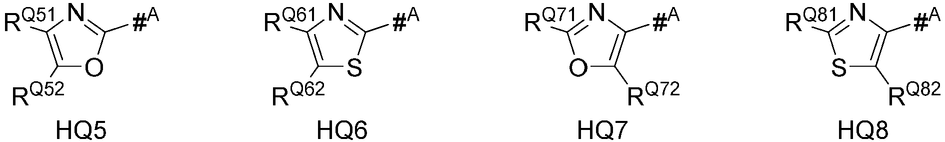 [wherein
#A represents a binding position to CR^{1B}R^{2B}, and R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, R^{Q62}, R^{Q71}, R^{Q72}, R^{Q81}, and R^{Q82} are identical to or different from each other and each represents a C1-C6 chain hydrocarbon group optionally substituted with one or more substituents selected from Group D^{a}, C3-C8 alicyclic hydrocarbon group, a three(3)- eight(8) membered nonaromatic heterocyclic group{the C3-C8 alicyclic hydrocarbon group, and the three(3)- eight(8) membered nonaromatic heterocyclic group may be optionally substituted with one or more substituents selected from Group B^{a}}, OR^{26a}, NR^{27a}R^{28a}, C(R^{29a})=NOR^{30a}, a cyano group, a nitro group, a halogen atom, or a hydrogen atom].
[Aspect BB47] The compound of any one of Aspect BB1 to Aspect BB40 wherein Q^{5A} represents a group represented by formula HQ1, formula HQ2, formula HQ3, formula HQ4, formula HQ5, or formula HQ6.
[Aspect BB48] The compound of any one of Aspect BB1 to Aspect BB40 wherein Q^{5A} represents a group represented by formula HQ1, formula HQ2, formula HQ3, formula HQ4, formula HQ5, or formula HQ6, and R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom.
[Aspect BB49] The compound of any one of Aspect BB1 to Aspect BB40 wherein Q^{5A} represents a group represented by formula HQ1, formula HQ2, formula HQ3, formula HQ4, formula HQ5, or formula HQ6, and R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms or a hydrogen atom.
[Aspect BB50] The compound of any one of Aspect BB1 to Aspect BB40 wherein Q^{5A} represents a group represented by formula HQ1, formula HQ2, formula HQ3, formula HQ4, formula HQ5, or formula HQ6, and R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} represents a hydrogen atom.
[Aspect BB51] The compound of any one of Aspect BB1 to Aspect BB40 wherein Q^{5A} represents a group represented by formula HQ1, formula HQ2, formula HQ3, or formula HQ4.
[Aspect BB52] The compound of any one of Aspect BB1 to Aspect BB40 wherein Q^{5A} represents a group represented by formula HQ1, formula HQ2, formula HQ3, or formula HQ4, and R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom.
[Aspect BB53] The compound of any one of Aspect BB1 to Aspect BB40 wherein Q^{5A} represents a group represented by formula HQ1, formula HQ2, formula HQ3, or formula HQ4, and R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom.
[Aspect BB54] The compound of any one of Aspect BB1 to Aspect BB40 wherein Q^{5A} represents a group represented by formula HQ1, formula HQ2, formula HQ3, or formula HQ4, and R^{Q11}, R^{Q12}, R^{Q13}, R^{Q21}, R^{Q22}, R^{Q31}, R^{Q32}, R^{Q41}, and R^{Q42} represents a hydrogen atom.
[Aspect BB55] The compound of any one of Aspect BB1 to Aspect BB40 wherein Q^{5A} represents a group represented by formula HQ1.
[Aspect BB56] The compound of any one of Aspect BB1 to Aspect BB40 wherein Q^{5A} represents a group represented by formula HQ1, and R^{Q11}, R^{Q12}, and R^{Q13} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom.
[Aspect BB57] The compound of any one of Aspect BB1 to Aspect BB40 wherein Q^{5A} represents a group represented by formula HQ1, and R^{Q11}, R^{Q12}, and R^{Q13} are identical to or different from each other and each represents a C1-C4 alkyl group optionally substituted with one or more halogen atoms, a halogen atom, or a hydrogen atom.
[Aspect BB58] The compound of any one of Aspect BB1 to Aspect BB40 wherein Q^{5A} represents a group represented by formula HQ1, and R^{Q11}, R^{Q12}, and R^{Q13} represents a hydrogen atom.
[Aspect BB59] The compound of any one of Aspect BB1 to Aspect BB40 wherein Q^{5A} represents a group represented by formula HQ2, HQ3, or HQ4.
[Aspect BB60] The compound of any one of Aspect BB1 to Aspect BB40 wherein Q^{5A} represents a group represented by formula HQ2, HQ3, or HQ4, and R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom.
[Aspect BB61] The compound of any one of Aspect BB1 to Aspect BB40 wherein Q^{5A} represents a group represented by formula HQ2, HQ3, or HQ4, and R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom.
[Aspect BB62] The compound of any one of Aspect BB1 to Aspect BB40 wherein Q^{5A} represents a group represented by formula HQ2, HQ3, or HQ4, and R^{Q21}, R^{Q22}, R^{Q31}, R^{Q32}, R^{Q41}, and R^{Q42} represents a hydrogen atom.
[Aspect BB63] The compound of any one of Aspect BB1 to Aspect BB40 wherein Q^{5A} represents a group represented by formula HQ2.
[Aspect BB64] The compound of any one of Aspect BB1 to Aspect BB40 wherein Q^{5A} represents a group represented by formula HQ2, and R^{Q21} and R^{Q22} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom.
[Aspect BB65] The compound of any one of Aspect BB1 to Aspect BB40 wherein Q^{5A} represents a group represented by formula HQ2, and R^{Q21} and R^{Q22} are identical to or different from each other and each represents a C1-C4 alkyl group optionally substituted with one or more halogen atoms, a halogen atom, or a hydrogen atom.
[Aspect BB66] The compound of any one of Aspect BB1 to Aspect BB40 wherein Q^{5A} represents a group represented by formula HQ2, and R^{Q21} and R^{Q22} represents a hydrogen atom.
[Aspect BB67] The compound of any one of Aspect BB1 to Aspect BB40 wherein Q^{5A} represents a group represented by formula HQ3.
[Aspect BB68] The compound of any one of Aspect BB1 to Aspect BB40 wherein Q^{5A} represents a group represented by formula HQ3, and R^{Q31} and R^{Q32} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom.
[Aspect BB69] The compound of any one of Aspect BB1 to Aspect BB40 wherein Q^{5A} represents a group represented by formula HQ3, and R^{Q31} and R^{Q32} are identical to or different from each other and each represents a C1-C4 alkyl group optionally substituted with one or more halogen atoms, a halogen atom, or a hydrogen atom.
[Aspect BB70] The compound of any one of Aspect BB1 to Aspect BB40 wherein Q^{5A} represents a group represented by formula HQ3, and R^{Q31} and R^{Q32} represent a hydrogen atom.
[Aspect BB71] The compound of any one of Aspect BB1 to Aspect BB40 wherein Q^{5A} represents a group represented by formula HQ4.
[Aspect BB72] The compound of any one of Aspect BB1 to Aspect BB40 wherein Q^{5A} represents a group represented by formula HQ4, and R^{Q41} and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom.
[Aspect BB73] The compound of any one of Aspect BB1 to Aspect BB40 wherein Q^{5A} represents a group represented by formula HQ4, and R^{Q41} and R^{Q42} are identical to or different from each other and each represents a C1-C4 alkyl group optionally substituted with one or more halogen atoms, a halogen atom, or a hydrogen atom.
[Aspect BB74] The compound of any one of Aspect BB1 to Aspect BB40 wherein Q^{5A} represents a group represented by formula HQ4, and R^{Q41} and R^{Q42} represent a hydrogen atom.
[Aspect BB75] The compound of any one of Aspect BB1 to Aspect BB40 wherein Q^{5A} represents a group represented by formula HQ5 or formula HQ6.
[Aspect BB76] The compound of any one of Aspect BB1 to Aspect BB40 wherein Q^{5A} represents a group represented by formula HQ5 or formula HQ6, and R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom.
[Aspect BB77] The compound of any one of Aspect BB1 to Aspect BB40 wherein Q^{5A} represents a group represented by formula HQ5 or formula HQ6, and R^{Q51}, R^{Q52}, R^{Q61}, and R^{Q62} are identical to or different from each other and each represents a C1-C4 alkyl group optionally substituted with one or more halogen atoms, a halogen atom, or a hydrogen atom.
[Aspect BB78] The compound of any one of Aspect BB1 to Aspect BB40 wherein Q^{5A} represents a group represented by formula HQ5 or formula HQ6, and R^{Q51}, R^{Q52}, R^{Q61}, and R^{Q62} represent a hydrogen atom.
[Aspect BB79] The compound of any one of Aspect BB1 to Aspect BB40 wherein Q^{5A} represents a group represented by formula HQ5.
[Aspect BB80] The compound of any one of Aspect BB1 to Aspect BB40 wherein Q^{5A} represents a group represented by formula HQ5, and R^{Q51} and R^{Q52} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom.
[Aspect BB81] The compound of any one of Aspect BB1 to Aspect BB40 wherein Q^{5A} represents a group represented by formula HQ5, and R^{Q51} and R^{Q52} are identical to or different from each other and each represents a C1-C4 alkyl group optionally substituted with one or more halogen atoms, a halogen atom, or a hydrogen atom.
[Aspect BB82] The compound of any one of Aspect BB1 to Aspect BB40 wherein Q^{5A} represents a group represented by formula HQ5, and R^{Q51} and R^{Q52} represent a hydrogen atom.
[Aspect BB84] The compound of any one of Aspect BB1 to Aspect BB40 wherein Q^{5A} represents a group represented by formula HQ6, and R^{Q61} and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom.
[Aspect BB85] The compound of any one of Aspect BB1 to Aspect BB40 wherein Q^{5A} represents a group represented by formula HQ6, and R^{Q61} and R^{Q62} are identical to or different from each other and each represents, a C1-C4 alkyl group optionally substituted with one or more halogen atoms, a halogen atom, or a hydrogen atom.
[Aspect BB86] The compound of any one of Aspect BB1 to Aspect BB40 wherein Q^{5A} represents a group represented by formula HQ6, and R^{Q61} and R^{Q62} represent a hydrogen atom.
[Aspect BB87] The compound of any one of Aspect BB1 to Aspect BB40 wherein Q^{5A} represents a group represented by formula HQ1, formula HQ2, formula HQ3, formula HQ4, formula HQ5, formula HQ6, formula HQ7, or formula HQ8, R^{1B} and R^{2B} are identical to or different from each other and each represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB88] The compound of any one of Aspect BB1 to Aspect BB40 wherein Q^{5A} represents a group represented by formula HQ1, formula HQ2, formula HQ3, formula HQ4, formula HQ5, or formula HQ6, and R^{8a} represents a C1-C4 alkyl group optionally substituted with one or more halogen atoms, a (C1-C4 alkyl)carbonyl group, or a hydrogen atom.
[Aspect BB89] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB90] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms or a hydrogen atom, and R^{8a} represents a C1-C4 alkyl group optionally substituted with one or more halogen atoms, a (C1-C4 alkyl)carbonyl group, or a hydrogen atom.
[Aspect BB91] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} represent a hydrogen atom, and R^{8a} represents a C1-C4 alkyl group optionally substituted with one or more halogen atoms, a (C1-C4 alkyl)carbonyl group, or a hydrogen atom.
[Aspect BB92] The compound of any one of Aspect BB1 to Aspect BB40 wherein Q^{5A} represents a group represented by formula HQ1, formula HQ2, formula HQ3, or formula HQ4, and R^{8a} represents a C1-C4 alkyl group optionally substituted with one or more halogen atoms, a (C1-C4 alkyl)carbonyl group, or a hydrogen atom.
[Aspect BB93] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB94] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} are identical to or different from each other and each represents, a C1-C4 alkyl group optionally substituted with one or more halogen atoms, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB95] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31} R^{Q32}, R^{Q41}, and R^{Q42} represent a hydrogen atom, and R^{8a} represents a C1-C4 alkyl group optionally substituted with one or more halogen atoms, a (C1-C4 alkyl)carbonyl group, or a hydrogen atom.
[Aspect BB96] The compound of any one of Aspect BB1 to Aspect BB40 wherein Q^{5A} represents a group represented by formula HQ1, and R^{8a} represents a C1-C4 alkyl group optionally substituted with one or more halogen atoms, a (C1-C4 alkyl)carbonyl group, or a hydrogen atom.
[Aspect BB97] The compound of any one of Aspect BB1 to Aspect BB40 wherein Q^{5A} represents a group represented by formula HQ1, R^{Q11}, R^{Q12}, and R^{Q13} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB98] The compound of any one of Aspect BB1 to Aspect BB40 wherein Q^{5A} represents a group represented by formula HQ1, R^{Q11}, R^{Q12}, and R^{Q13} are identical to or different from each other and each represents a C1-C4 alkyl group optionally substituted with one or more halogen atoms, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB99] The compound of any one of Aspect BB1 to Aspect BB40 wherein Q^{5A} represents a group represented by formula HQ1, R^{Q11}, R^{Q12}, and R^{Q13} represent a hydrogen atom, and R^{8a} represents a C1-C4 alkyl group optionally substituted with one or more halogen atoms, a (C1-C4 alkyl)carbonyl group, or a hydrogen atom.
[Aspect BB100] The compound of any one of Aspect BB1 to Aspect BB40 wherein Q^{5A} represents a group represented by formula HQ2, formula HQ3, or formula HQ4, and R^{8a} represents a C1-C4 alkyl group optionally substituted with one or more halogen atoms, a (C1-C4 alkyl)carbonyl group, or a hydrogen atom.
[Aspect BB101] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB102] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB103] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} represent a hydrogen atom, and R^{8a} represents a C1-C4 alkyl group optionally substituted with one or more halogen atoms, a (C1-C4 alkyl)carbonyl group, or a hydrogen atom.
[Aspect BB104] The compound of any one of Aspect BB1 to Aspect BB40 wherein Q^{5A} represents a group represented by formula HQ2, and R^{8a} represents a C1-C4 alkyl group optionally substituted with one or more halogen atoms, a (C1-C4 alkyl)carbonyl group, or a hydrogen atom.
[Aspect BB105] The compound of any one of Aspect BB1 to Aspect BB40 wherein Q^{5A} represents a group represented by formula HQ2, and R^{Q21} and R^{Q22} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB106] The compound of any one of Aspect BB1 to Aspect BB40 wherein Q^{5A} represents a group represented by formula HQ2, and R^{Q21} and R^{Q22} are identical to or different from each other and each represents a C1-C4 alkyl group optionally substituted with one or more halogen atoms, halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB107] The compound of any one of Aspect BB1 to Aspect BB40 wherein Q^{5A} represents a group represented by formula HQ2, and R^{Q21} and R^{Q22} represent a hydrogen atom, and R^{8a} represents a C1-C4 alkyl group optionally substituted with one or more halogen atoms, a (C1-C4 alkyl)carbonyl group, or a hydrogen atom.
[Aspect BB108] The compound of any one of Aspect BB1 to Aspect BB40 wherein Q^{5A} represents a group represented by formula HQ3, and R^{8a} represent a C1-C4 alkyl group optionally substituted with one or more halogen atoms, a (C1-C4 alkyl)carbonyl group, or a hydrogen atom.
[Aspect BB109] The compound of any one of Aspect BB1 to Aspect BB40 wherein Q^{5A} represents a group epresented by formula HQ3, R^{Q31} and R^{Q32} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB110] The compound of any one of Aspect BB1 to Aspect BB40 wherein Q^{5A} represents a group epresented by formula HQ3, R^{Q31} and R^{Q32} are identical to or different from each other and each represents a C1-C4 alkyl group optionally substituted with one or more halogen atoms, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB111] The compound of any one of Aspect BB1 to Aspect BB40 wherein Q^{5A} represents a group epresented by formula HQ3, R^{Q31} and R^{Q32} represent a hydrogen atom, and R^{8a} represents a C1-C4 alkyl group optionally substituted with one or more halogen atoms, a (C1-C4 alkyl)carbonyl group, or a hydrogen atom.
[Aspect BB112] The compound of any one of Aspect BB1 to Aspect BB40 wherein Q^{5A} represents a group epresented by formula HQ4, and R^{8a} represents a C1-C4 alkyl group optionally substituted with one or more halogen atoms, a C1-C4 alkyl group optionally substituted with one or more halogen atoms, or a hydrogen atom.
[Aspect BB113] The compound of any one of Aspect BB1 to Aspect BB40 wherein Q^{5A} represents a group epresented by formula HQ4, R^{Q41} and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB114] The compound of any one of Aspect BB1 to Aspect BB40 wherein Q^{5A} represents a group epresented by formula HQ4, R^{Q41} and R^{Q42} are identical to or different from each other and each represents a C1-C4 alkyl group optionally substituted with one or more halogen atoms, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB115] The compound of any one of Aspect BB1 to Aspect BB40 wherein Q^{5A} represents a group epresented by formula HQ4, R^{Q41} and R^{Q42} represent a hydrogen atom, and R^{8a} represents a C1-C4 alkyl group optionally substituted with one or more halogen atoms, a (C1-C4 alkyl)carbonyl group, or a hydrogen atom.
[Aspect BB116] The compound of any one of Aspect BB1 to Aspect BB40 wherein Q^{5A} represents a group epresented by formula HQ5, or formula HQ6, and R^{8a} represents a C1-C4 alkyl group optionally substituted with one or more halogen atoms, a (C1-C4 alkyl)carbonyl group, or a hydrogen atom.
[Aspect BB117] The compound of any one of Aspect BB1 to Aspect BB40 wherein Q^{5A} represents a group epresented by formula HQ5, or formula HQ6, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB118] The compound of any one of Aspect BB1 to Aspect BB40 wherein Q^{5A} represents a group epresented by formula HQ5, or formula HQ6, R^{Q51}, R^{Q52}, R^{Q61}, and R^{Q62} are identical to or different from each other and each represents a C1-C4 alkyl group optionally substituted with one or more halogen atoms, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB119] The compound of any one of Aspect BB1 to Aspect BB40 wherein Q^{5A} represents a group epresented by formula HQ5, or formula HQ6, R^{Q51}, R^{Q52}, R^{Q61}, and R^{Q62} represent a hydrogen atom, and R^{8a} represents a C1-C4 alkyl group optionally substituted with one or more halogen atoms, a (C1-C4 alkyl)carbonyl group, or a hydrogen atom.
[Aspect BB120] The compound of any one of Aspect BB1 to Aspect BB40 wherein Q^{5A} represents a group epresented by formula HQ5, and R^{8a} represents a C1-C4 alkyl group optionally substituted with one or more halogen atoms, a (C1-C4 alkyl)carbonyl group, or a hydrogen atom.
[Aspect BB121] The compound of any one of Aspect BB1 to Aspect BB40 wherein Q^{5A} represents a group epresented by formula HQ5, R^{Q51} and R^{Q52} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB122] The compound of any one of Aspect BB1 to Aspect BB40 wherein Q^{5A} represents a group epresented by formula HQ5, R^{Q51} and R^{Q52} are identical to or different from each other and each represents a C1-C4 alkyl group optionally substituted with one or more halogen atoms, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB123] The compound of any one of Aspect BB1 to Aspect BB40 wherein Q^{5A} represents a group epresented by formula HQ5, R^{Q51} and R^{Q52} represent a hydrogen atom, and R^{8a} represents a C1-C4 alkyl group optionally substituted with one or more halogen atoms, a (C1-C4 alkyl)carbonyl group, or a hydrogen atom.
[Aspect BB124] The compound of any one of Aspect BB1 to Aspect BB40 wherein Q^{5A} represents a group epresented by formula HQ6, and R^{8a} represents a C1-C4 alkyl group optionally substituted with one or more halogen atoms, a (C1-C4 alkyl)carbonyl group, or a hydrogen atom.
[Aspect BB125] The compound of any one of Aspect BB1 to Aspect BB40 wherein Q^{5A} represents a group epresented by formula HQ6, R^{Q61} and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB126] The compound of any one of Aspect BB1 to Aspect BB40 wherein Q^{5A} represents a group epresented by formula HQ6, R^{Q61} and R^{Q62} are identical to or different from each other and each represents a C1-C4 alkyl group optionally substituted with one or more halogen atoms, a halogen atom, or a hydrogen atom, and R^{8a} represents a C1-C4 alkyl group optionally substituted with one or more halogen atoms, a (C1-C4 alkyl)carbonyl group, or a hydrogen atom.
[Aspect BB127] The compound of any one of Aspect BB1 to Aspect BB40 wherein Q^{5A} represents a group epresented by formula HQ6, R^{Q61} and R^{Q62} represent a hydrogen atom, and R^{8a} represents a C1-C4 alkyl group optionally substituted with one or more halogen atoms, a (C1-C4 alkyl)carbonyl group, or a hydrogen atom.
[Aspect BB128] The compound of any one of Aspect BB1 to Aspect BB40 wherein Q^{5A} represents a group represented by formula HQ1, formula HQ2, formula HQ3, formula HQ4, formula HQ5, formula HQ6, formula HQ7, or formula HQ8, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB129] The compound of any one of Aspect BB1 to Aspect BB40 wherein Q^{5A} represents a group represented by formula HQ1, formula HQ2, formula HQ3, formula HQ4, formula HQ5, or formula HQ6, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB130] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB131] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms or hydrogen atom, R^{1B} represents a hydrogen atom, R^{2B} represents OR^{8a} or a hydrogen atom, R^{8a} represents a C1-C4 alkyl group optionally substituted with one or more halogen atoms, a (C1-C4 alkyl)carbonyl group, or a hydrogen atom.
[Aspect BB132] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} represent a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB133] The compound of any one of Aspect BB1 to Aspect BB40 wherein Q^{5A} represents a group represented by formula HQ1, formula HQ2, formula HQ3, or formula HQ4, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB134] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB135] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB136] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} represent a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom,
[Aspect BB137] The compound of any one of Aspect BB1 to Aspect BB40 wherein Q^{5A} represents a group represented by formula HQ1, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB138] The compound of any one of Aspect BB1 to Aspect BB40 wherein Q^{5A} represents a group represented by formula HQ1, R^{Q11}, R^{Q12}, and R^{Q13} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB139] The compound of any one of Aspect BB1 to Aspect BB40 wherein Q^{5A} represents a group represented by formula HQ1, R^{Q11}, R^{Q12}, and R^{Q13} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB140] The compound of any one of Aspect BB1 to Aspect BB40 wherein Q^{5A} represents a group represented by formula HQ1, R^{Q11}, R^{Q12}, and R^{Q13} represents a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB141] The compound of any one of Aspect BB1 to Aspect BB40 wherein Q^{5A} represents a group represented by formula HQ2, formula HQ3, or formula HQ4, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB142] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB143] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB144] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} represent a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB145] The compound of any one of Aspect BB1 to Aspect BB40 wherein Q^{5A} represents a group represented by formula HQ2, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB146] The compound of any one of Aspect BB1 to Aspect BB40 wherein Q^{5A} represents a group represented by formula HQ2, R^{Q21} and R^{Q22} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, R^{8a} represents a C1-C4 alkyl group optionally substituted with one or more halogen atoms, a (C1-C4 alkyl)carbonyl group, or a hydrogen atom
[Aspect BB147] The compound of any one of Aspect BB1 to Aspect BB40 wherein Q^{5A} represents a group represented by formula HQ2, R^{Q21} and R^{Q22} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB148] The compound of any one of Aspect BB1 to Aspect BB40 wherein Q^{5A} represents a group represented by formula HQ2, R^{Q21} and R^{Q22} represent a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB149] The compound of any one of Aspect BB1 to Aspect BB40 wherein Q^{5A} represents a group represented by formula HQ3, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB150] The compound of any one of Aspect BB1 to Aspect BB40 wherein Q^{5A} represents a group represented by formula HQ3, R^{Q31} and R^{Q32} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB151] The compound of any one of Aspect BB1 to Aspect BB40 wherein Q^{5A} represents a group represented by formula HQ3, R^{Q31} and R^{Q32} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB152] The compound of any one of Aspect BB1 to Aspect BB40 wherein Q^{5A} represents a group represented by formula HQ3, R^{Q31} and R^{Q32} represent a hydrogen atom, R^{1B} represents a hydrogen atom, R^{2B} represent OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB153] The compound of any one of Aspect BB1 to Aspect BB40 wherein Q^{5A} represents a group represented by formula HQ4, R^{1B} represents a hydrogen atom, R^{2B} represents OR^{8a} or a hydrogen atom, R^{8a} represents a C1-C4 alkyl group optionally substituted with one or more halogen atoms, a (C1-C4 alkyl)carbonyl group, or a hydrogen atom.
[Aspect BB154] The compound of any one of Aspect BB1 to Aspect BB40 wherein Q^{5A} represents a group represented by formula HQ4, R^{Q41} and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB155] The compound of any one of Aspect BB1 to Aspect BB40 wherein Q^{5A} represents a group represented by formula HQ4, R^{Q41} and R^{Q42} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB156] The compound of any one of Aspect BB1 to Aspect BB40 wherein Q^{5A} represents a group represented by formula HQ4, R^{Q41} and R^{Q42} represent a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB157] The compound of any one of Aspect BB1 to Aspect BB40 wherein Q^{5A} represents a group represented by formula HQ5 or formula HQ6, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB158] The compound of any one of Aspect BB1 to Aspect BB40 wherein Q^{5A} represents a group represented by formula HQ5 or formula HQ6, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB159] The compound of any one of Aspect BB1 to Aspect BB40 wherein Q^{5A} represents a group represented by formula HQ5 or formula HQ6, R^{Q51}, R^{Q52}, R^{Q61}, and R^{Q62} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB160] The compound of any one of Aspect BB1 to Aspect BB40 wherein Q^{5A} represents a group represented by formula HQ5 or formula HQ6, R^{Q51}, R^{Q52}, R^{Q61}, and R^{Q62} represent a hydrogen atom, R^{1B} represents a hydrogen atom, R^{2B} represents OR^{8a} or a hydrogen atom, R^{8a} represents a C1-C4 alkyl group optionally substituted with one or more halogen atoms, a (C1-C4 alkyl)carbonyl group, or a hydrogen atom.
[Aspect BB161] The compound of any one of Aspect BB1 to Aspect BB40 wherein Q^{5A} represents a group represented by formula HQ5, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB162] The compound of any one of Aspect BB1 to Aspect BB40 wherein Q^{5A} represents a group represented by formula HQ5, R^{Q51} and R^{Q52} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB163] The compound of any one of Aspect BB1 to Aspect BB40 wherein Q^{5A} represents a group represented by formula HQ5, R^{Q51} and R^{Q52} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB164] The compound of any one of Aspect BB1 to Aspect BB40 wherein Q^{5A} represents a group represented by formula HQ5, R^{Q51} and R^{Q52} represent a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB165] The compound of any one of Aspect BB1 to Aspect BB40 wherein Q^{5A} represents a group represented by formula HQ6, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB166] The compound of any one of Aspect BB1 to Aspect BB40 wherein Q^{5A} represents a group represented by formula HQ6, R^{Q61} and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB167] The compound of any one of Aspect BB1 to Aspect BB40 wherein Q^{5A} represents a group represented by formula HQ6, R^{Q61} and R^{Q62} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB168] The compound of any one of Aspect BB1 to Aspect BB40 wherein Q^{5A} represents a group represented by formula HQ6, R^{Q61} and R^{Q62} represent a hydrogen atom, R^{1B} represents a hydrogen atom, R^{2B} represents OR^{8a} or a hydrogen atom, and R^{8a} represents a C1-C4 alkyl group optionally substituted with one or more halogen atoms, a (C1-C4 alkyl)carbonyl group, or a hydrogen atom.
[Aspect BB169] The compound of any one of Aspect BB1 to Aspect BB40 wherein Q^{5A} represents a group represented by formula HQ1, formula HQ2, formula HQ3, formula HQ4, formula HQ5, formula HQ6, formula HQ7, or formula HQ8, R^{1B} represents a hydrogen atom, R^{2B} represents OR^{8a} or a hydrogen atom, and R^{8a} represents a hydrogen atom.
[Aspect BB170] The compound of any one of Aspect BB1 to Aspect BB40 wherein Q^{5A} represents a group represented by formula HQ1, formula HQ2, formula HQ3, formula HQ4, formula HQ5, or formula HQ6, R^{1B} represents a hydrogen atom, R^{2B} represents OR^{8a} or a hydrogen atom, and R^{8a} represents a hydrogen atom.
[Aspect BB171] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a hydrogen atom.
[Aspect BB172] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms or a hydrogen atom, R^{1B} represents a hydrogen atom, R^{2B} represents OR^{8a} or a hydrogen atom, and R^{8a} represents hydrogen atom.
[Aspect BB173] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} represent a hydrogen atom, R^{1B} represents a hydrogen atom, R^{2B} represents OR^{8a} or a hydrogen atom, and R^{8a} represents a hydrogen atom.
[Aspect BB174] The compound of any one of Aspect BB1 to Aspect BB40 wherein Q^{5A} represents a group represented by formula HQ1, formula HQ2, formula HQ3, or formula HQ4, R^{1B} represents a hydrogen atom, R^{2B} represents OR^{8a} or a hydrogen atom, and R^{8a} represents a hydrogen atom.
[Aspect BB175] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a hydrogen atom.
[Aspect BB176] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a hydrogen atom.
[Aspect BB177] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} represent a hydrogen atom, R^{1B} represents a hydrogen atom, R^{2B} represents OR^{8a} or a hydrogen atom, and R^{8a} represents a hydrogen atom.
[Aspect BB178] The compound of any one of Aspect BB1 to Aspect BB40 wherein Q^{5A} represents a group represented by formula HQ1, R^{1B} represents a hydrogen atom, R^{2B} represents OR^{8a} or a hydrogen atom, and R^{8a} represents a hydrogen atom.
[Aspect BB179] The compound of any one of Aspect BB1 to Aspect BB40 wherein Q^{5A} represents a group represented by formula HQ1, R^{Q11}, R^{Q12}, and R^{Q13} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a hydrogen atom.
[Aspect BB180] The compound of any one of Aspect BB1 to Aspect BB40 wherein Q^{5A} represents a group represented by formula HQ1, R^{Q11}, R^{Q12}, and R^{Q13} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a hydrogen atom.
[Aspect BB181] The compound of any one of Aspect BB1 to Aspect BB40 wherein Q^{5A} represents a group represented by formula HQ1, R^{Q11}, R^{Q12}, and R^{Q13} represent a hydrogen atom, R^{1B} represents a hydrogen atom, R^{2B} represents OR^{8a} or a hydrogen atom, and R^{8a} represents a hydrogen atom.
[Aspect BB182] The compound of any one of Aspect BB1 to Aspect BB40 wherein Q^{5A} represents a group represented by formula HQ2, formula HQ3, or formula HQ4, R^{1B} represents a hydrogen atom, R^{2B} represents OR^{8a} or a hydrogen atom, and R^{8a} represents a hydrogen atom.
[Aspect BB183] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a hydrogen atom.
[Aspect BB184] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a hydrogen atom.
[Aspect BB185] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} represent a hydrogen atom, R^{1B} represents a hydrogen atom, R^{2B} represents OR^{8a} or a hydrogen atom, and R^{8a} represents a hydrogen atom.
[Aspect BB186] The compound of any one of Aspect BB1 to Aspect BB40 wherein Q^{5A} represents a group represented by formula HQ2, R^{1B} represents a hydrogen atom, R^{2B} represents OR^{8a} or a hydrogen atom, and R^{8a} represents a hydrogen atom.
[Aspect BB187] The compound of any one of Aspect BB1 to Aspect BB40 wherein Q^{5A} represents a group represented by formula HQ2, R^{Q21} and R^{Q22} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a hydrogen atom.
[Aspect BB188] The compound of any one of Aspect BB1 to Aspect BB40 wherein Q^{5A} represents a group represented by formula HQ2, R^{Q21} and R^{Q22} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a hydrogen atom.
[Aspect BB189] The compound of any one of Aspect BB1 to Aspect BB40 wherein Q^{5A} represents a group represented by formula HQ2, R^{Q21} and R^{Q22} represent a hydrogen atom, R^{1B} represents a hydrogen atom, R^{2B} represents OR^{8a} or a hydrogen atom, and R^{8a} represents a hydrogen atom.
[Aspect BB190] The compound of any one of Aspect BB1 to Aspect BB40 wherein Q^{5A} represents a group represented by formula HQ3, R^{1B} represents a hydrogen atom, R^{2B} represents OR^{8a} or a hydrogen atom, and R^{8a} represents a hydrogen atom.
[Aspect BB191] The compound of any one of Aspect BB1 to Aspect BB40 wherein Q^{5A} represents a group represented by formula HQ3, R^{Q31} and R^{Q32} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a hydrogen atom.
[Aspect BB192] The compound of any one of Aspect BB1 to Aspect BB40 wherein Q^{5A} represents a group represented by formula HQ3, R^{Q31} and R^{Q32} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a hydrogen atom.
[Aspect BB193] The compound of any one of Aspect BB1 to Aspect BB40 wherein Q^{5A} represents a group represented by formula HQ3, R^{Q31} and R^{Q32} represent a hydrogen atom, R^{1B} represents a hydrogen atom, R^{2B} represents OR^{8a} or a hydrogen atom, and R^{8a} represents a hydrogen atom.
[Aspect BB194] The compound of any one of Aspect BB1 to Aspect BB40 wherein Q^{5A} represents a group represented by formula HQ4, R^{1B} represents a hydrogen atom, R^{2B} represents OR^{8a} or a hydrogen atom, and R^{8a} represents a hydrogen atom.
[Aspect BB195] The compound of any one of Aspect BB1 to Aspect BB40 wherein Q^{5A} represents a group represented by formula HQ4, R^{Q41} and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a hydrogen atom.
[Aspect BB196] The compound of any one of Aspect BB1 to Aspect BB40 wherein Q^{5A} represents a group represented by formula HQ4, R^{Q41} and R^{Q42} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a hydrogen atom.
[Aspect BB197] The compound of any one of Aspect BB1 to Aspect BB40 wherein Q^{5A} represents a group represented by formula HQ4, R^{Q41} and R^{Q42} represent a hydrogen atom, R^{1B} represents a hydrogen atom, R^{2B} represents OR^{8a} or a hydrogen atom, and R^{8a} represents a hydrogen atom.
[Aspect BB198] The compound of any one of Aspect BB1 to Aspect BB40 wherein Q^{5A} represents a group represented by formula HQ5 or formula HQ6, R^{1B} represents a hydrogen atom, R^{2B} represents OR^{8a} or a hydrogen atom, and R^{8a} represents a hydrogen atom.
[Aspect BB199] The compound of any one of Aspect BB1 to Aspect BB40 wherein Q^{5A} represents a group represented by formula HQ5 or formula HQ6, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a hydrogen atom.
[Aspect BB200] The compound of any one of Aspect BB1 to Aspect BB40 wherein Q^{5A} represents a group represented by formula HQ5 or formula HQ6, R^{Q51}, R^{Q52}, R^{Q61}, and R^{Q62} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a hydrogen atom.
[Aspect BB201] The compound of any one of Aspect BB1 to Aspect BB40 wherein Q^{5A} represents a group represented by formula HQ5 or formula HQ6, R^{Q51}, R^{Q52}, R^{Q61}, and R^{Q62} represent a hydrogen atom, R^{1B} represents a hydrogen atom, R^{2B} represents OR^{8a} or a hydrogen atom, and R^{8a} represents a hydrogen atom.
[Aspect BB202] The compound of any one of Aspect BB1 to Aspect BB40 wherein Q^{5A} represents a group represented by formula HQ5, R^{1B} represents a hydrogen atom, R^{2B} represents OR^{8a} or a hydrogen atom, and R^{8a} represents a hydrogen atom.
[Aspect BB203] The compound of any one of Aspect BB1 to Aspect BB40 wherein Q^{5A} represents a group represented by formula HQ5, R^{Q51} and R^{Q52} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a hydrogen atom.
[Aspect BB204] The compound of any one of Aspect BB1 to Aspect BB40 wherein Q^{5A} represents a group represented by formula HQ5, R^{Q51} and R^{Q52} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a} or a hydrogen atom, and R^{8a} represents a hydrogen atom.
[Aspect BB205] The compound of any one of Aspect BB1 to Aspect BB40 wherein Q^{5A} represents a group represented by formula HQ5, R^{Q51} and R^{Q52} represent a hydrogen atom, R^{1B} represents a hydrogen atom, R^{2B} represents OR^{8a} or a hydrogen atom, and R^{8a} represents a hydrogen atom.
[Aspect BB206] The compound of any one of Aspect BB1 to Aspect BB40 wherein Q^{5A} represents a group represented by formula HQ6, R^{1B} represents a hydrogen atom, R^{2B} represents OR^{8a} or a hydrogen atom, and R^{8a} represents a hydrogen atom.
[Aspect BB207] The compound of any one of Aspect BB1 to Aspect BB40 wherein Q^{5A} represents a group represented by formula HQ6, R^{Q61} and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a} or a hydrogen atom, and R^{8a} represents a hydrogen atom.
[Aspect BB208] The compound of any one of Aspect BB1 to Aspect BB40 wherein Q^{5A} represents a group represented by formula HQ6, R^{Q61} and R^{Q62} are identical to or different from each other and each represents a C1-C4 alkyl group optionally substituted with one or more halogen atoms, halogen atom, or a hydrogen atom, R^{1B} represents a hydrogen atom, R^{2B} represents OR^{8a} or a hydrogen atom, and R^{8a} represents a hydrogen atom.
[Aspect BB209] The compound of any one of Aspect BB1 to Aspect BB40 wherein Q^{5A} represents a group represented by formula HQ6, R^{Q61} and R^{Q62} represent a hydrogen atom, R^{1B} represents a hydrogen atom, R^{2B} represents OR^{8a} or a hydrogen atom, and R^{8a} represents a hydrogen atom.
[Aspect BB210] The compound of any one of Aspect BB1 to Aspect BB40 wherein Q^{5A} represents a group represented by formula HQ1, formula HQ2, formula HQ3, formula HQ4, formula HQ5, formula HQ6, formula HQ7, or formula HQ8, R^{1B} represents a hydrogen atom, R^{2B} represents OR^{8a}, and R^{8a} represents a hydrogen atom.
[Aspect BB211] The compound of any one of Aspect BB1 to Aspect BB40 wherein Q^{5A} represents a group represented by formula HQ1, formula HQ2, formula HQ3, formula HQ4, formula HQ5, or formula HQ6, R^{1B} represents a hydrogen atom, R^{2B} represents OR^{8a}, and R^{8a} represents a hydrogen atom.
[Aspect BB212] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a}, and R^{8a} represents a hydrogen atom.
[Aspect BB213] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms or a hydrogen atom, R^{1B} represents a hydrogen atom, R^{2B} represents OR^{8a}, and R^{8a} represents a hydrogen atom.
[Aspect BB214] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} represent a hydrogen atom, R^{1B} represents a hydrogen atom, R^{2B} represents OR^{8a}, and R^{8a} represents a hydrogen atom.
[Aspect BB215] The compound of any one of Aspect BB1 to Aspect BB40 wherein Q^{5A} represents a group represented by formula HQ1, formula HQ2, formula HQ3, or formula HQ4, R^{1B} represents a hydrogen atom, R^{2B} represents OR^{8a}, and R^{8a} represents a hydrogen atom.
[Aspect BB216] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a}, and R^{8a} represents a hydrogen atom.
[Aspect BB217] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a}, and R^{8a} represents a hydrogen atom.
[Aspect BB218] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} represent a hydrogen atom, R^{1B} represents a hydrogen atom, R^{2B} represents OR^{8a}, and R^{8a} represents a hydrogen atom.
[Aspect BB219] The compound of any one of Aspect BB1 to Aspect BB40 wherein Q^{5A} represents a group represented by formula HQ1, R^{1B} represents a hydrogen atom, R^{2B} represents OR^{8a}, and R^{8a} represents a hydrogen atom.
[Aspect BB220] The compound of any one of Aspect BB1 to Aspect BB40 wherein Q^{5A} represents a group represented by formula HQ1, R^{Q11}, R^{Q12}, and R^{Q13} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a}, and R^{8a} represents a hydrogen atom.
[Aspect BB221] The compound of any one of Aspect BB1 to Aspect BB40 wherein Q^{5A} represents a group represented by formula HQ1, R^{Q11}, R^{Q12}, and R^{Q13} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a}, and R^{8a} represents a hydrogen atom.
[Aspect BB222] The compound of any one of Aspect BB1 to Aspect BB40 wherein Q^{5A} represents a group represented by formula HQ1, R^{Q11}, R^{Q12}, and R^{Q13} represent a hydrogen atom, R^{1B} represents a hydrogen atom, R^{2B} represents OR^{8a}, and R^{8a} represents a hydrogen atom.
[Aspect BB223] The compound of any one of Aspect BB1 to Aspect BB40 wherein Q^{5A} represents a group represented by formula HQ2, formula HQ3, or formula HQ4, R^{1B} represents a hydrogen atom, R^{2B} represents OR^{8a}, and R^{8a} represents a hydrogen atom.
[Aspect BB224] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a}, and R^{8a} represents a hydrogen atom.
[Aspect BB225] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a}, and R^{8a} represents a hydrogen atom.
[Aspect BB226] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} represent a hydrogen atom, R^{1B} represents a hydrogen atom, R^{2B} represents OR^{8a}, and R^{8a} represents a hydrogen atom.
[Aspect BB227] The compound of any one of Aspect BB1 to Aspect BB40 wherein Q^{5A} represents a group represented by formula HQ2, R^{1B} represents a hydrogen atom, R^{2B} represents OR^{8a}, and R^{8a} represents a hydrogen atom.
[Aspect BB228] The compound of any one of Aspect BB1 to Aspect BB40 wherein Q^{5A} represents a group represented by formula HQ2, R^{Q21} and R^{Q22} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a}, and R^{8a} represents a hydrogen atom.
[Aspect BB229] The compound of any one of Aspect BB1 to Aspect BB40 wherein Q^{5A} represents a group represented by formula HQ2, R^{Q21} and R^{Q22} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a}, and R^{8a} represents a hydrogen atom.
[Aspect BB230] The compound of any one of Aspect BB1 to Aspect BB40 wherein Q^{5A} represents a group represented by formula HQ2, R^{Q21} and R^{Q22} represent a hydrogen atom, R^{1B} represents a hydrogen atom, R^{2B} represents OR^{8a}, and R^{8a} represents a hydrogen atom.
[Aspect BB231] The compound of any one of Aspect BB1 to Aspect BB40 wherein Q^{5A} represents a group represented by formula HQ3, R^{1B} represents a hydrogen atom, R^{2B} represents OR^{8a}, and R^{8a} represents a hydrogen atom.
[Aspect BB232] The compound of any one of Aspect BB1 to Aspect BB40 wherein Q^{5A} represents a group represented by formula HQ3, R^{Q31} and R^{Q32} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a}, and R^{8a} represents a hydrogen atom.
[Aspect BB233] The compound of any one of Aspect BB1 to Aspect BB40 wherein Q^{5A} represents a group represented by formula HQ3, R^{Q31} and R^{Q32} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a}, and R^{8a} represents a hydrogen atom.
[Aspect BB234] The compound of any one of Aspect BB1 to Aspect BB40 wherein Q^{5A} represents a group represented by formula HQ3, R^{Q31} and R^{Q32} represent a hydrogen atom, R^{1B} represents a hydrogen atom, R^{2B} represents OR^{8a}, and R^{8a} represents a hydrogen atom.
[Aspect BB235] The compound of any one of Aspect BB1 to Aspect BB40 wherein Q^{5A} represents a group represented by formula HQ4, R^{1B} represents a hydrogen atom, R^{2B} represents OR^{8a}, and R^{8a} represents a hydrogen atom.
[Aspect BB236] The compound of any one of Aspect BB1 to Aspect BB40 wherein Q^{5A} represents a group represented by formula HQ4, R^{Q41} and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a}, and R^{8a} represents a hydrogen atom.
[Aspect BB237] The compound of any one of Aspect BB1 to Aspect BB40 wherein Q^{5A} represents a group represented by formula HQ4, R^{Q41} and R^{Q42} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a}, and R^{8a} represents a hydrogen atom.
[Aspect BB238] The compound of any one of Aspect BB1 to Aspect BB40 wherein Q^{5A} represents a group represented by formula HQ4, R^{Q41} and R^{Q42} represent a hydrogen atom, R^{1B} represents a hydrogen atom, R^{2B} represents OR^{8a}, and R^{8a} represents a hydrogen atom.
[Aspect BB239] The compound of any one of Aspect BB1 to Aspect BB40 wherein Q^{5A} represents a group represented by formula HQ5 or formula HQ6, R^{1B} represents a hydrogen atom, R^{2B} represents OR^{8a}, and R^{8a} represents a hydrogen atom.
[Aspect BB240] The compound of any one of Aspect BB1 to Aspect BB40 wherein Q^{5A} represents a group represented by formula HQ5 or formula HQ6, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a}, and R^{8a} represents a hydrogen atom.
[Aspect BB241] The compound of any one of Aspect BB1 to Aspect BB40 wherein Q^{5A} represents a group represented by formula HQ5 or formula HQ6, R^{Q51}, R^{Q52}, R^{Q61}, and R^{Q62} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a}, and R^{8a} represents a hydrogen atom.
[Aspect BB242] The compound of any one of Aspect BB1 to Aspect BB40 wherein Q^{5A} represents a group represented by formula HQ5 or formula HQ6, R^{Q51}, R^{Q52}, R^{Q61}, and R^{Q62} represent a hydrogen atom, R^{1B} represents a hydrogen atom, R^{2B} represents OR^{8a}, and R^{8a} represents a hydrogen atom.
[Aspect BB243] The compound of any one of Aspect BB1 to Aspect BB40 wherein Q^{5A} represents a group represented by formula HQ5, R^{1B} represents a hydrogen atom, R^{2B} represents OR^{8a}, and R^{8a} represents a hydrogen atom.
[Aspect BB244] The compound of any one of Aspect BB1 to Aspect BB40 wherein Q^{5A} represents a group represented by formula HQ5, R^{Q51} and R^{Q52} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a}, and R^{8a} represents a hydrogen atom.
[Aspect BB245] The compound of any one of Aspect BB1 to Aspect BB40 wherein Q^{5A} represents a group represented by formula HQ5, R^{Q51} and R^{Q52} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a}, and R^{8a} represents a hydrogen atom.
[Aspect BB246] The compound of any one of Aspect BB1 to Aspect BB40 wherein Q^{5A} represents a group represented by formula HQ5, R^{Q51} and R^{Q52} represent a hydrogen atom, R^{1B} represents a hydrogen atom, R^{2B} represents OR^{8a}, and R^{8a} represents a hydrogen atom.
[Aspect BB247] The compound of any one of Aspect BB1 to Aspect BB40 wherein Q^{5A} represents a group represented by formula HQ6, R^{1B} represents a hydrogen atom, R^{2B} represents OR^{8a}, and R^{8a} represents a hydrogen atom.
[Aspect BB248] The compound of any one of Aspect BB1 to Aspect BB40 wherein Q^{5A} represents a group represented by formula HQ6, R^{Q61} and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, R^{1B} represents a hydrogen atom, R^{2B} represents OR^{8a}, and R^{8a} represents a hydrogen atom.
[Aspect BB249] The compound of any one of Aspect BB1 to Aspect BB40 wherein Q^{5A} represents a group represented by formula HQ6, R^{Q61} and R^{Q62} are identical to or different from each other and each represents a C1-C4 alkyl group optionally substituted with one or more halogen atoms, a halogen atom, or a hydrogen atom, R^{1B} represents a hydrogen atom, R^{2B} represents OR^{8a}, and R^{8a} represents a hydrogen atom.
[Aspect BB250] The compound of any one of Aspect BB1 to Aspect BB40 wherein Q^{5A} represents a group represented by formula HQ6, R^{Q61} and R^{Q62} represent a hydrogen atom, R^{1B} represents a hydrogen atom, R^{2B} represents OR^{8a}, and R^{8a} represents a hydrogen atom.
[Aspect BB251] The compound of any one of Aspect BB1 to Aspect BB40 wherein Q^{5A} represents a group represented by formula HQ1, formula HQ2, formula HQ3, formula HQ4, formula HQ5, formula HQ6, formula HQ7, or formula HQ8, and R^{1B} and R^{2B} represent a hydrogen atom.
[Aspect BB252] The compound of any one of Aspect BB1 to Aspect BB40 wherein Q^{5A} represents a group represented by formula HQ1, formula HQ2, formula HQ3, formula HQ4, formula HQ5, or formula HQ6, and R^{1B} and R^{2B} represent a hydrogen atom.
[Aspect BB253] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{1B} and R^{2B} represent a hydrogen atom.
[Aspect BB254] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} are identical to or different from each other and each represents a C1-C4 alkyl group optionally substituted with one or more halogen atoms or a hydrogen atom, and R^{1B} and R^{2B} represent a hydrogen atom.
[Aspect BB255] The compound of any one of Aspect BB1 to Aspect BB40 wherein Q^{5A} represents a group represented by formula HQ1, formula HQ2, formula HQ3, formula HQ4, formula HQ5, or formula HQ6, R^{Q11}, R^{Q12}, R^{Q13}, R^{Q21}, R^{Q22}, R^{Q31}, R^{Q32}, R^{Q41}, R^{Q42}, R^{Q51}, R^{Q52}, R^{Q61}, and R^{Q62} represent a hydrogen atom, and R^{1B} and R^{2B} represent a hydrogen atom.
[Aspect BB256] The compound of any one of Aspect BB1 to Aspect BB40 wherein Q^{5A} represents a group represented by formula HQ1, formula HQ2, formula HQ3, or formula HQ4, and R^{1B} and R^{2B} represent a hydrogen atom.
[Aspect BB257] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{1B} and R^{2B} represent a hydrogen atom.
[Aspect BB258] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom, and R^{1B} and R^{2B} represent a hydrogen atom.
[Aspect BB259] The compound of any one of Aspect BB1 to Aspect BB40 wherein Q^{5A} represents a group represented by formula HQ1, formula HQ2, formula HQ3, or formula HQ4, R^{Q11}, R^{Q12}, R^{Q13}, R^{Q21}, R^{Q22}, R^{Q31}, R^{Q32}, R^{Q41}, and R^{Q42} represent a hydrogen atom, and R^{1B} and R^{2B} represent a hydrogen atom.
[Aspect BB260] The compound of any one of Aspect BB1 to Aspect BB40 wherein Q^{5A} represents a group represented by formula HQ1, and R^{1B} and R^{2B} represent a hydrogen atom.
[Aspect BB261] The compound of any one of Aspect BB1 to Aspect BB40 wherein Q^{5A} represents a group represented by formula HQ1, R^{Q11}, R^{Q12}, and R^{Q13} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{1B} and R^{2B} represent a hydrogen atom.
[Aspect BB262] The compound of any one of Aspect BB1 to Aspect BB40 wherein Q^{5A} represents a group represented by formula HQ1, R^{Q11}, R^{Q12}, and R^{Q13} are identical to or different from each other and each represents a C1-C4 alkyl group optionally substituted with one or more halogen atoms, a halogen atom, or a hydrogen atom, and R^{1B} and R^{2B} represent a hydrogen atom.
[Aspect BB263] The compound of any one of Aspect BB1 to Aspect BB40 wherein Q^{5A} represents a group represented by formula HQ1, R^{Q11}, R^{Q12}, and R^{Q13} represent a hydrogen atom, and R^{1B} and R^{2B} represent a hydrogen atom.
[Aspect BB264] The compound of any one of Aspect BB1 to Aspect BB40 wherein Q^{5A} represents a group represented by formula HQ2, formula HQ3, or formula HQ4, and R^{1B} and R^{2B} represent a hydrogen atom.
[Aspect BB265] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{1B} and R^{2B} represent a hydrogen atom.
[Aspect BB266] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} are identical to or different from each other and each represents a C1-C4 alkyl group optionally substituted with one or more halogen atoms, a halogen atom, or a hydrogen atom, and R^{1B} and R^{2B} represent a hydrogen atom.
[Aspect BB267] The compound of any one of Aspect BB1 to Aspect BB40 wherein Q^{5A} represents a group represented by formula HQ2, formula HQ3, or formula HQ4, R^{Q21}, R^{Q22}, R^{Q31}, R^{Q32}, R^{Q41}, and R^{Q42} represent a hydrogen atom, and R^{1B} and R^{2B} represent a hydrogen atom.
[Aspect BB268] The compound of any one of Aspect BB1 to Aspect BB40 wherein Q^{5A} represents a group represented by formula HQ2, and R^{1B} and R^{2B} represent a hydrogen atom.
[Aspect BB269] The compound of any one of Aspect BB1 to Aspect BB40 wherein Q^{5A} represents a group represented by formula HQ2, R^{Q21} and R^{Q22} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{1B} and R^{2B} represent a hydrogen atom.
[Aspect BB270] The compound of any one of Aspect BB1 to Aspect BB40 wherein Q^{5A} represents a group represented by formula HQ2, R^{Q21} and R^{Q22} are identical to or different from each other and each represents a C1-C4 alkyl group optionally substituted with one or more halogen atoms, a halogen atom, or a hydrogen atom, and R^{1B} and R^{2B} represent a hydrogen atom.
[Aspect BB271] The compound of any one of Aspect BB1 to Aspect BB40 wherein Q^{5A} represents a group represented by formula HQ2, R^{Q21} and R^{Q22} represent a hydrogen atom, and R^{1B} and R^{2B} represent a hydrogen atom.
[Aspect BB272] The compound of any one of Aspect BB1 to Aspect BB40 wherein Q^{5A} represents a group represented by formula HQ3, and R^{1B} and R^{2B} represent a hydrogen atom.
[Aspect BB273] The compound of any one of Aspect BB1 to Aspect BB40 wherein Q^{5A} represents a group represented by formula HQ3, R^{Q31} and R^{Q32} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{1B} and R^{2B} represent a hydrogen atom.
[Aspect BB274] The compound of any one of Aspect BB1 to Aspect BB40 wherein Q^{5A} represents a group represented by formula HQ3, R^{Q31} and R^{Q32} are identical to or different from each other and each represents a C1-C4 alkyl group optionally substituted with one or more halogen atoms, a halogen atom, or a hydrogen atom, and R^{1B} and R^{2B} represent a hydrogen atom.
[Aspect BB275] The compound of any one of Aspect BB1 to Aspect BB40 wherein Q^{5A} represents a group represented by formula HQ3, R^{Q31} and R^{Q32} represent a hydrogen atom, and R^{1B} and R^{2B} represent a hydrogen atom.
[Aspect BB276] The compound of any one of Aspect BB1 to Aspect BB40 wherein Q^{5A} represents a group represented by formula HQ4, and R^{1B} and R^{2B} represent a hydrogen atom.
[Aspect BB277] The compound of any one of Aspect BB1 to Aspect BB40 wherein Q^{5A} represents a group represented by formula HQ4, R^{Q41} and R^{Q42} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{1B} and R^{2B} represent a hydrogen atom.
[Aspect BB278] The compound of any one of Aspect BB1 to Aspect BB40 wherein Q^{5A} represents a group represented by formula HQ4, R^{Q41} and R^{Q42} are identical to or different from each other and each represents a C1-C4 alkyl group optionally substituted with one or more halogen atoms, a halogen atom, or a hydrogen atom, and R^{1B} and R^{2B} represent a hydrogen atom.
[Aspect BB279] The compound of any one of Aspect BB1 to Aspect BB40 wherein Q^{5A} represents a group represented by formula HQ4, R^{Q41} and R^{Q42} represent a hydrogen atom, and R^{1B} and R^{2B} represent a hydrogen atom.
[Aspect BB280] The compound of any one of Aspect BB1 to Aspect BB40 wherein Q^{5A} represents a group represented by formula HQ5 or formula HQ6, and R^{1B} and R^{2B} represent a hydrogen atom.
[Aspect BB281] The compound of any one of Aspect BB1 to Aspect BB40 wherein Q^{5A} represents a group represented by formula HQ5 or formula HQ6, R^{Q51}, R^{Q52}, R^{Q61}, and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{1B} and R^{2B} represent a hydrogen atom.
[Aspect BB282] The compound of any one of Aspect BB1 to Aspect BB40 wherein Q^{5A} represents a group represented by formula HQ5 or formula HQ6, R^{Q51}, R^{Q52}, R^{Q61}, and R^{Q62} are identical to or different from each other and each represents a C1-C4 alkyl group optionally substituted with one or more halogen atoms, a halogen atom, or a hydrogen atom, and R^{1B} and R^{2B} represent a hydrogen atom.
[Aspect BB283] The compound of any one of Aspect BB1 to Aspect BB40 wherein Q^{5A} represents a group represented by formula HQ5 or formula HQ6, R^{Q51}, R^{Q52}, R^{Q61}, and R^{Q62} represent a hydrogen atom, and R^{1B} and R^{2B} represent a hydrogen atom.
[Aspect BB284] The compound of any one of Aspect BB1 to Aspect BB40 wherein Q^{5A} represents a group represented by formula HQ5, and R^{1B} and R^{2B} represent a hydrogen atom.
[Aspect BB285] The compound of any one of Aspect BB1 to Aspect BB40 wherein Q^{5A} represents a group represented by formula HQ5, R^{Q51} and R^{Q52} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{1B} and R^{2B} represent a hydrogen atom.
[Aspect BB286] The compound of any one of Aspect BB1 to Aspect BB40 wherein Q^{5A} represents a group represented by formula HQ5, R^{Q51} and R^{Q52} are identical to or different from each other and each represents a C1-C4 alkyl group optionally substituted with one or more halogen atoms, a halogen atom, or a hydrogen atom, and R^{1B} and R^{2B} represent a hydrogen atom.
[Aspect BB287] The compound of any one of Aspect BB1 to Aspect BB40 wherein Q^{5A} represents a group represented by formula HQ5, R^{Q51} and R^{Q52} represent a hydrogen atom, and R^{1B} and R^{2B} represent a hydrogen atom.
[Aspect BB288] The compound of any one of Aspect BB1 to Aspect BB40 wherein Q^{5A} represents a group represented by formula HQ6, and R^{1B} and R^{2B} represent a hydrogen atom.
[Aspect BB289] The compound of any one of Aspect BB1 to Aspect BB40 wherein Q^{5A} represents a group represented by formula HQ6, R^{Q61} and R^{Q62} are identical to or different from each other and each represents a C1-C4 alkyl group optionally substituted with one or more halogen atoms, a C3-C6 cycloalkyl group optionally substituted with one or more halogen atoms, a C1-C4 alkoxy group optionally substituted with one or more halogen atoms, a cyano group, a halogen atom, or a hydrogen atom, and R^{1B} and R^{2B} represent a hydrogen atom.
[Aspect BB290] The compound of any one of Aspect BB1 to Aspect BB40 wherein Q^{5A} represents a group represented by formula HQ6, R^{Q61} and R^{Q62} are identical to or different from each other and each represents a C1-C4 alkyl group optionally substituted with one or more halogen atoms, a halogen atom, or a hydrogen atom, and R^{1B} and R^{2B} represent a hydrogen atom.
[Aspect BB291] The compound of any one of Aspect BB1 to Aspect BB40 wherein Q^{5A} represents a group represented by formula HQ6, R^{Q61} and R^{Q62} represent a hydrogen atom, and R^{1B} and R^{2B} represent a hydrogen atom.

Also, aspects of the Compound A of the present invention include also the following compounds.
[Aspect BX1] The Compound A of the present invention wherein the ring A^{5a} represents a group represented by formula H1a-1, formula H1a-2, formula H1a-3, formula H1a-4, formula H1a-5, formula H1a-6, formula H2a-1, formula H2a-2, formula H3a-1, formula H3a-2, formula H4a-1, or formula H4a-2. 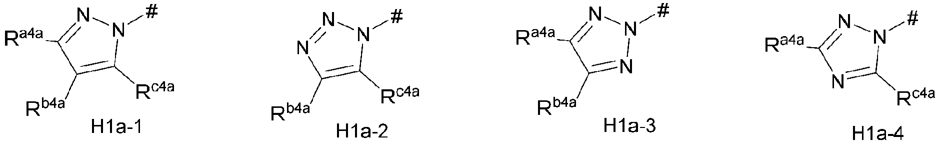 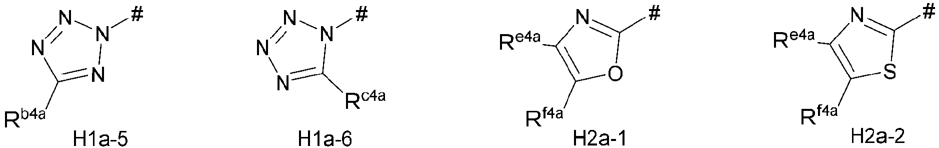 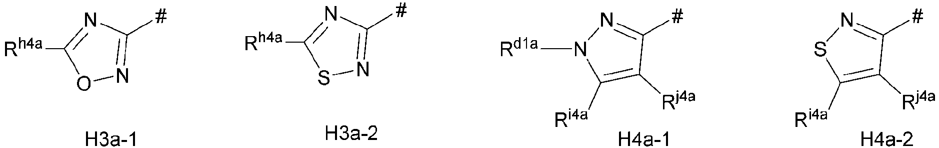 [wherein the symbols are the same as defined above.]
[Aspect BX2] The Compound A of the present invention wherein the ring A^{5a} represents a group represented by formula H1a-1, formula H1a-2, formula H1a-3, formula H1a-4, formula H1a-5, formula H1a-6, formula H2a-1, formula H2a-2, formula H3a-1, formula H3a-2, formula H4a-1, or formula H4a-2,
   R^{a4a}, R^{b4a}, R^{c4a}, R^{e4a}, R^{f4a}, R^{h4a}, R^{i4a}, and R^{j4a} are identical to or different from each other and each represents a C1-C6 chain hydrocarbon group optionally substituted with one or more halogen atoms, OR^{26a}, a halogen atom, or a hydrogen atom,
   R^{d1a} represents a C1-C6 chain hydrocarbon group optionally substituted with one or more halogen atoms,
   R^{1a} and R^{2a} are identical to or different from each other and each represents a halogen atom, a hydroxy group, or a hydrogen atom,
   R^{3a} and R^{5a} are identical to or different from each other and each represents a C1-C6 chain hydrocarbon group optionally substituted with one or more halogen atoms, a five (5) - six(6) membered aromatic heterocyclic group optionally substituted with one or more substituents selected from the Group A^{Xa}, OR^{17a}, or a halogen atom,
   R^{6a} represents a C1-C6 chain hydrocarbon group optionally substituted with one or more halogen atoms, or a halogen atom,
   R^{17a} and R^{26a} are identical to or different from each other and each represents a C1-C6 chain hydrocarbon group optionally substituted with one or more halogen atoms, or a hydrogen atom,
      Group A^{Xa}: a group consisting of a halogen atom, a C1-C4 alkyl group, a C1-C4 haloalkyl group, a C1-C4 alkoxy group, and a C1-C4 haloalkoxy group.
[Aspect BX3] The Compound A of the present invention wherein the ring A^{5a} represents a group represented by formula H1a-1, formula H1a-2, formula H1a-3, formula H1a-4, formula H1a-5, formula H1a-6, formula H2a-1, formula H2a-2, formula H3a-1, formula H3a-2, formula H4a-1, or formula H4a-2,
   R^{a4a}, R^{b4a}, R^{c4a}, R^{e4a}, R^{f4a}, R^{h4a}, R^{i4a}, and R^{j4a} are identical to or different from each other and each represents a C1-C6 chain hydrocarbon group a C1-C4 alkoxy group optionally substituted with one or more halogen atoms, a halogen atom, or a hydrogen atom,
   R^{d1a} represents a C1-C6 chain hydrocarbon group optionally substituted with one or more halogen atoms,
   R^{1a} and R^{2a} are identical to or different from each other and each represents a halogen atom, a hydroxy group, or a hydrogen atom,
   R^{3a} and R^{5a} are identical to or different from each other and each represents a C1-C6 chain hydrocarbon group optionally substituted with one or more halogen atoms, a five(5)- six(6) membered aromatic heterocyclic group, a C1-C4 alkoxy group, or a halogen atom, and
   R^{6a} represents a C1-C6 chain hydrocarbon group, or a halogen atom.
[Aspect BX4] The compound of Aspect BX2, Aspect BX3, or the Compound A of the present invention wherein the ring A^{5a} represents a group represented by formula H1a-1, formula H1a-2, formula H1a-3, formula H1a-4, formula H1a-5, formula H1a-6, formula H2a-1, formula H2a-2, formula H3a-1, formula H3a-2, formula H4a-1, or formula H4a-2.
[Aspect BX5] The compound of Aspect BX2, Aspect BX3, or the Compound A of the present invention wherein the ring A^{5a} represents a group represented by formula H1a-1.
[Aspect BX6] The compound of Aspect BX2, Aspect BX3, or the Compound A of the present invention wherein the ring A^{5a} represents a group represented by formula H1a-2.
[Aspect BX7] The compound of Aspect BX2, Aspect BX3, or the Compound A of the present invention wherein the ring A^{5a} represents a group represented by formula H1a-3.
[Aspect BX8] The compound of Aspect BX2, Aspect BX3, or the Compound A of the present invention wherein the ring A^{5a} represents a group represented by formula H1a-4.
[Aspect BX9] The compound of Aspect BX2, Aspect BX3, or the Compound A of the present invention wherein the ring A^{5a} represents a group represented by formula H1a-5.
[Aspect BX10] The compound of Aspect BX2, Aspect BX3, or the Compound A of the present invention wherein the ring A^{5a} represents a group represented by formula H1a-6.
[Aspect BX11] The compound of Aspect BX2, Aspect BX3, or the Compound A of the present invention wherein the ring A^{5a} represents a group represented by formula H2a-1.
[Aspect BX12] The compound of Aspect BX2, Aspect BX3, or the Compound A of the present invention wherein the ring A^{5a} represents a group represented by formula H2a-2.
[Aspect BX13] The compound of Aspect BX2, Aspect BX3, or the Compound A of the present invention wherein the ring A^{5a} represents a group represented by formula H3a-1.
[Aspect BX14] The compound of Aspect BX2, Aspect BX3, or the Compound A of the present invention wherein the ring A^{5a} represents a group represented by formula H3a-2.
[Aspect BX15] The compound of Aspect BX2, Aspect BX3, or the Compound A of the present invention wherein the ring A^{5a} represents a group represented by formula H4a-1.
[Aspect BX16] The compound of Aspect BX2, Aspect BX3, or the Compound A of the present invention wherein the ring A^{5a} represents a group represented by formula H4a-2.
[Aspect C1] A method for controlling a soybean rust disease fungus (*Phakopsora pachyrhiz*) which comprises applying the Present compound to a soybean or soil for cultivating a soybean.
[Aspect C2] A method for controlling a soybean rust disease fungus (*Phakopsora pachyrhiz*) which comprises applying the Compound of the present invention to a soybean or soil for cultivating a soybean.

Next, production methods of the Present compounds (including Compounds of the present invention) are described.

### Production method A

A compound represented by formula (I) (Present compound A) can be prepared by reacting a compound represented by formula (B1) (hereinafter, referred to as "Compound (B1)") with a compound represented by formula (M1) (hereinafter, referred to as "Compound (M1)") in the presence of a palladium catalyst and a base. 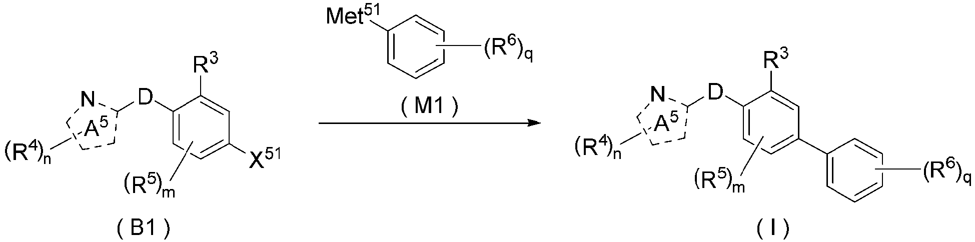 [wherein
X⁵¹ represents a chlorine atom, a bromine atom, an iodine atom, or a triflyloxy group, Met¹ represents B(OH)₂ or a 4,4,5,5-tetramethyl-1,3,2-dioxaborolan-2-yl group, and the other symbols are the same as defined above.]

The reaction is usually carried out in a solvent. Examples of the solvent to be used in the reaction include hydrocarbons such as hexane, toluene, and xylene (hereinafter collectively referred to as "hydrocarbons"); ethers such as methyl tert-butyl ether (hereinafter referred to as "MTBE"), tetrahydrofuran (hereinafter referred to as "THF"), and 1,2-dimethoxyethane (hereinafter referred to as "DME") (hereinafter referred to as "ethers"); halogenated hydrocarbons such as dichloromethane, chloroform, and chlorobenzene (hereinafter collectively referred to as "halogenated hydrocarbons"); amides such as dimethylformamide (hereinafter referred to as "DMF") and N-methylpyrrolidone (hereinafter referred to as "NMP") (hereinafter collectively referred to as "amides"); esters such as methyl acetate and ethyl acetate (hereinafter collectively referred to as "esters"); nitriles such as acetonitrile and propionitrile (hereinafter collectively referred to as "nitriles"); dimethyl sulfoxide (hereinafter referred to as "DMSO"); water; and mixtures of two or more of them.

Examples of the palladium catalyst to be used in the reaction include palladium(II) acetate and [1,1'-bis(diphenylphosphino)ferrocene]palladium(II) dichloride.

Examples of the base to be used in the reaction include organic bases such as triethylamine and pyridine (hereinafter collectively referred to as "organic bases"); alkali metal carbonates such as sodium carbonate and potassium carbonate (hereinafter collectively referred to as "alkali metal carbonates"); alkali metal hydrogen carbonates such as sodium hydrogen carbonate and potassium hydrogen carbonate (hereinafter collectively referred to as "alkali metal hydrogen carbonates"); alkali metal hydrogen carbonates; sodium fluoride, and tripotassium phosphate.

In the reaction, the Compound (M1) is usually used at a ratio of 0.5 to 2 mols, the palladium catalyst is usually used at a ratio of 0.01 to 0.3 mols, and the base is usually used at a ratio of 1 to 10 mols, relative to 1 mol of the Compound (B1).

The reaction temperature is usually within the range of 0 to 150°C. The reaction time is usually within the range of 0.1 to 120 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to isolate the Present compound A.

The Compound (M1) is known or may be prepared according to known method(s).

### Production method B

A compound represented by formula (I) (Present compound A) can be prepared by reacting a compound represented by formula (B2) (hereinafter, referred to as "Compound (B2)") with a compound represented by formula (M2) (hereinafter, referred to as "Compound (M2)") in the presence of a palladium catalyst and a base. 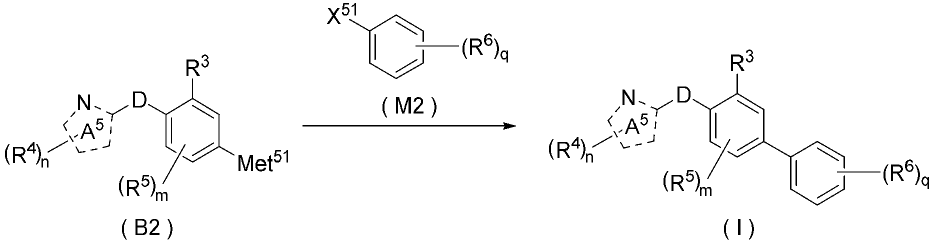 [wherein
the symbols are the same as defined above.]

The reaction may be carried out according to the Production method A by using the Compound (M2) instead of the Compound (B1) and using the Compound (B2) instead of the Compound (M1).

The Compound (M2) is known or may be prepared according to known method(s).

### Production method C

A compound represented by formula (I-a) (hereinafter, referred to as "Compound (I-a)"; with the proviso that for the Compound (I-a), in the ring A⁵, the atom binding to C(OH)(R¹) is a carbon atom) can be prepared by reacting an organolithium reactant (which is produced by reacting a compound represented by formula (B3) (hereinafter, referred to as "Compound (B3)") with a compound represented by formula (M3) (hereinafter, referred to as "Compound (M3)") with a compound represented by formula (M4) (hereinafter, referred to as "Compound (M4)").

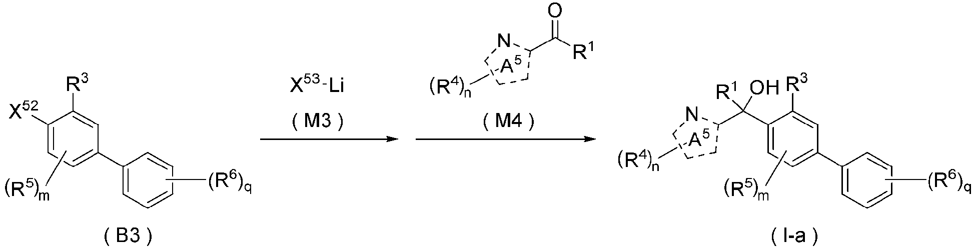

X⁵² represents a bromine atom, or an iodine atom, X⁵³ represents a butyl group, a sec-butyl group, or a tert-butyl group, and the other symbols are the same as defined above. With the proviso that for the Compound (M4) and the Compound (I-a), an atom on the ring A⁵ which is attached to a carbon atom to which R¹ is attached is a carbon atom.]

Firstly, a production method for preparing an organolithium reactant from the Compound (B3) is described.

The reaction is usually carried out in a solvent. Examples of the solvent to be used in the reaction include hydrocarbons, ethers, and mixtures of two or more of them.

In the reaction, the Compound (M3) is usually used at a ratio of 0.5 to 2 mols relative to 1 mol of the Compound (B3).

The reaction temperature is usually within the range of -78°C to a boiling point of the solvent. The reaction time is usually within the range of 0.1 to 120 hour(s).

Next, a production method for preparing the Compound (I-a) from the organolithium reactant is described.

The reaction is usually carried out in a solvent. Examples of the solvent to be used in the reaction include hydrocarbons, ethers, and mixtures of two or more of them.

In the reaction, the Compound (M4) is usually used at a ratio of 0.5 to 2 mols relative to 1 mol of the organolithium reactant.

The reaction temperature is usually within the range of -78°C to a boiling point of the solvent. The reaction time is usually within the range of 0.1 to 120 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to isolate the Compound (I-a).

The Compound (B3), the Compound (M3), and the Compound (M4) are known or may be prepared according to known method (s) .

### Production method D

The Compound (I-a) can be also prepared by reacting a Grignard reactant (which is produced by reacting the Compound (B3) with a compound represented formula (M5) (hereinafter, referred to as "Compound (M5)") with the Compound (M4). 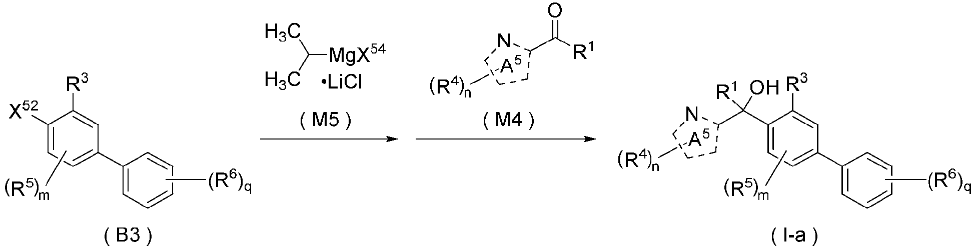 [wherein
X⁵⁴ represents a chlorine atom, or a bromine atom, and the other symbols are the same as defined above. With the proviso that for the Compound (M4) and the Compound (I-a), an atom on the ring A⁵ which is attached to a carbon atom to which R¹ is attached is a carbon atom.]

The reaction can be carried out by using the Compound (M5) instead of the Compound (M3) according to the Production method C.

The Compound (M5) is a commercially available compound or may be prepared by using known method(s).

### Production method E

The Compound (I-a) can be also prepared by reacting an organolithium reactant (which is produced by reacting a compound represented by formula (M6) (hereinafter, referred to as "Compound (M6)") with the Compound (M3)) with a compound represented by formula (M7) (hereinafter, referred to as "Compound (M7)"). 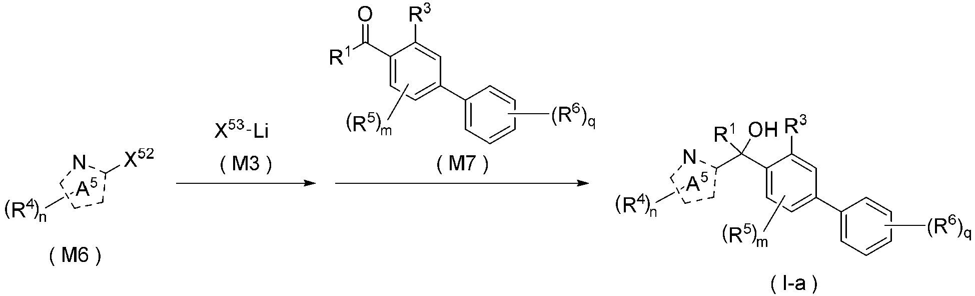 [wherein
the symbols are the same as defined above. With the proviso that for the Compound (M6) and the Compound (I-a), an atom on the ring A⁵ which is attached to a carbon atom to which R¹ is attached is a carbon atom.]

The reaction can be carried out by using the Compound (M6) instead of the Compound (B3) and using the Compound (M7) instead of the Compound (M4) according to the Production method C.

The Compound (M6) and the Compound (M7) are known or may be prepared according to known method(s).

### Production method F

The Compound (I-a) can be also prepared by reacting a Grignard reactant (which is produced by reacting the Compound (M6) with the Compound (M5)) with the Compound (M7). 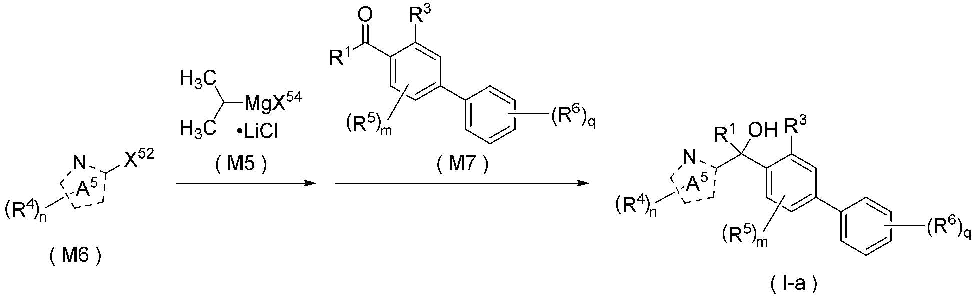 [wherein
the symbols are the same as defined above.]

The reaction can be carried out by using the Compound (M6) instead of the Compound (B3), using the Compound (M5) instead of the Compound (M3), and using the Compound (M7) instead of the Compound (M4) according to the Production method C.

### Production method G

A compound represented by formula (I-b) (hereinafter, referred to as "Compound (I-b)") can be prepared by subjecting the Compound (I-a) to a dehydroxylation. 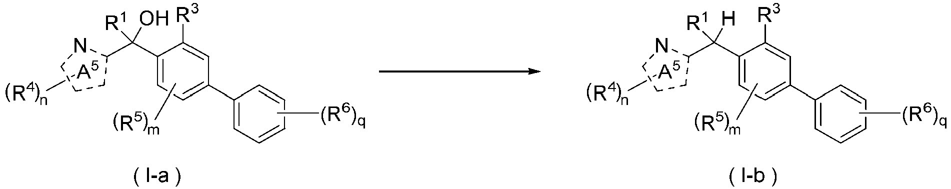 [wherein
the symbols are the same as defined above. With the proviso that for the Compound (I-a) and the Compound (I-b), an atom on the ring A⁵ which is attached to a carbon atom to which R¹ is attached is a carbon atom.]

Examples of the method for dehydroxylation include a catalytic hydrogen reduction reaction and a reaction with a phosphorus tribromide.

Firstly, a production method for preparing the Compound (I-b) from the Compound (I-a) by the catalytic hydrogen reduction reaction is described.

The catalytic hydrogen reduction reaction can be carried out by reacting the Compound (I-a) with a hydrogen in the presence of a solvent and a catalyst.

Examples of the solvent to be used in the reaction include alcohols such as methanol and ethanol (hereinafter collectively referred to as "alcohols"); esters; acetic acid, and mixtures of two or more of them.

The catalyst to be used in the reaction include a palladium on carbon.

The pressure of the hydrogen in the reaction is usually 1 to 5 atmospheric pressures.

In the reaction, the catalyst is usually used at a ratio of 0.01 to 1 mol relative to 1 mol of the Compound (I-a).

The reaction temperature is usually within the range of 10 to 50°C. The reaction time is usually within the range of 0.1 to 120 hour(s).

Next, a production method for preparing the Compound (I-b) from the Compound (I-a) by reacting with the phosphorus tribromide is described.

The reaction is usually carried out in a solvent. Examples of the solvent to be used in the reaction include hydrocarbons, ethers, and mixtures of two or more of them.

In the reaction, the phosphorus tribromide is usually used at a ratio of 1 to 10 mols relative to 1 mol of the Compound (I-a).

The reaction temperature is usually within the range of 0°C to the boiling point of the solvent. The reaction time is usually within the range of 0.1 to 120 hour(s).

When the reaction is completed, the reaction mixture may be subjected to a work-up such as adding water to the reaction mixture, as needed, washing the organic layer with an aqueous alkaline solution (such as an aqueous solution of saturated sodium hydrogen carbonate), and drying and/or concentrating the resulting organic layer to isolate the Compound (I-b).

### Production method H

A compound represented by formula (I-c) (hereinafter, referred to as "Compound (I-c)") can be prepared by subjecting the Compound (I-a) to an oxidation reaction. 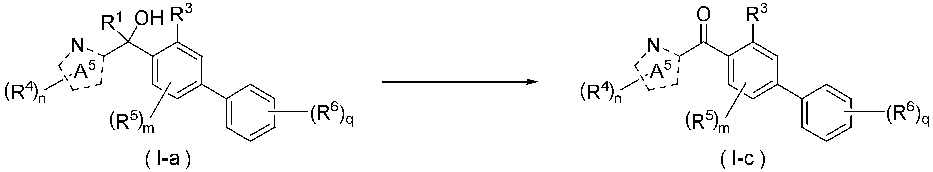 [wherein
the symbols are the same as defined above. With the proviso that for the Compound (I-a) and the Compound (I-c), an atom on the ring A⁵ which is attached to a carbon atom to which R¹ is attached is a carbon atom.]

The oxidation can be carried out according to a method described in Natural Product Reports, 2011, 28, 1722-1754.

### Production method I

The Compound (I-a) can be prepared by subjecting the Compound (I-c) to a reduction reaction. 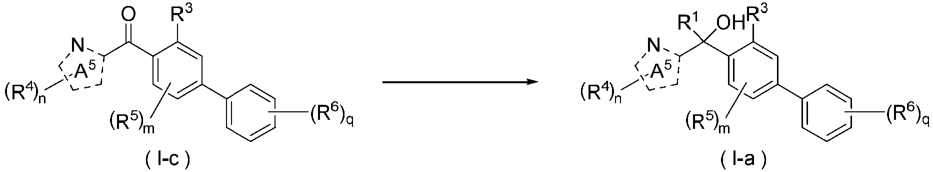 [wherein
the symbols are the same as defined above.]

The reduction reaction is usually carried out in a solvent. Examples of the solvent to be used in the reaction include ethers, alcohols, and mixtures of two or more of them.

Examples of the reducing agent to be used in the reaction include sodium borohydride.

In the reaction, the reducing agent is usually used at a ratio of 0.5 to 2 mols relative to 1 mol of the Compound (I-c).

The reaction temperature is usually within the range of 0 to 100°C. The reaction time is usually within the range of 0.1 to 120 hour(s).

When the reaction is completed, the reaction mixture may be subjected to a work-up such as adding water to the reaction mixture, and drying and/or concentrating the resulting organic layer to isolate the Compound (I-a).

### Production method J

A compound represented by formula (I-d) (hereinafter, referred to as "Compound (I-d)"; with the proviso that for the Compound (I-a), in the ring A⁵, the atom binding to C(OH)(R¹) is a carbon atom) can be prepared by reacting the Compound (I-a) with a compound represented by formula (M8) (hereinafter, referred to as "Compound (M8)") in the presence of a base. 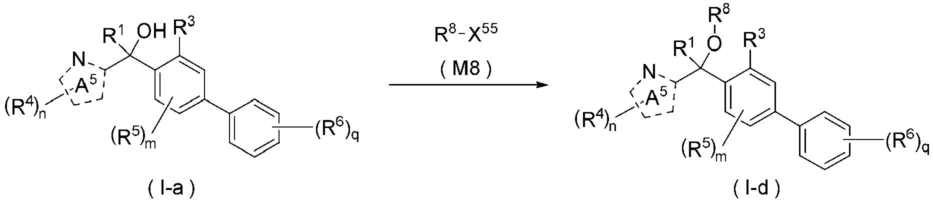 [wherein,
X⁵⁵ represents a fluorine atom, a chlorine atom, a bromine atom, an iodine atom, a mesyloxy group or a triflyloxy group, and the other symbols are the same as defined above.}

The reaction is usually carried out in a solvent. Examples of the solvent to be used in the reaction include alcohols, nitriles, ethers, hydrocarbons, amides, DMSO, water, and mixtures of two or more of them.

Examples of the base include alkali metal hydrides, alkali metal carbonates, organic bases, sodium hydride, and tripotassium phosphate.

When X⁵⁵ represents a bromine atom, an iodine atom, or a triflyloxy group, a metal catalyst and/or a ligand may be used as needed.

Examples of the metal catalyst to be used in the reaction include palladium catalysts such as palladium(II) acetate and [1,1'-bis(diphenylphosphino)ferrocene]palladium(II) dichloride; nickel catalysts such as bis (cyclooctadiene) nickel (0) and nickel(II) chloride; and copper catalysts such as copper(I) iodide and copper(I) chloride. When a metal catalyst is used in the reaction, the metal catalyst is usually used at a ratio of 0.01 to 1 mol relative to 1 mol of the Compound (I-a).

Examples of the ligand to be used in the reaction include triphenylphosphine, Xantphos, 2,2'-bis(diphenylphosphino)-1,1'-binaphthyl, 1,1'-bis(diphenylphosphino)ferrocene, 2-dicyclohexylphosphino-2',4',6'-triisopropylbiphenyl, 2-dicyclohexylphosphino-2',6'-dimethoxybiphenyl, 1,2-bis(diphenylphosphino)ethane, 2,2'-bipyridine, 2-aminoethanol, 8-hydroxyquinoline, and 1,10-phenanthroline. When a ligand is used in the reaction, the ligand is usually used at a ratio of 0.01 to 1 mol relative to 1 mol of the Compound (I-a).

In the reaction, the Compound (M8) is usually used at a ratio of 1 to 10 mols, and the base is usually used at a ratio of 1 to 10 mols, relative to 1 mol of the Compound (I-a).

The reaction temperature is usually within the range of -20°C to 200°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to obtain the Compound (I-d).

The Compound (M8) is known or may be prepared according to known method(s).

### Production method K

A compound represented by formula (I-e) (hereinafter, referred to as "Compound (I-e)") can be prepared by reacting a compound represented by formula (B4) (hereinafter, referred to as "Compound (B4)") with a compound represented by formula (M9) (hereinafter, referred to as "Compound (M9)") in the presence of a condensation agent. 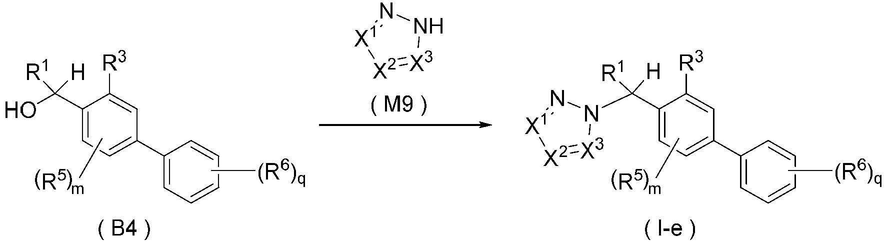 [wherein,
the symbols are the same as defined above.}

The reaction is usually carried out in a solvent. Examples of the solvent to be used in the reaction include nitriles, ethers, hydrocarbons, amides, DMSO, water, and mixtures of two or more of them.

Examples of the condensation agent include cyanomethylene tributylphosphorane; and a mixture of azodicarboxylates (such as diethyl azodicarboxylate and dimethoxy-ethyl azodicarboxylate) and triphenyl Phosphine.

In the reaction, the Compound (M9) is usually used at a ratio of 1 to 5 mols, and the condensation agent is usually used at a ratio of 1 to 5 mols, relative to 1 mol of the Compound (B4).

The reaction temperature is usually within the range of 0°C to 150°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to obtain the Compound (I-e).

The Compound (M9) is known or may be prepared according to known method(s).

### Production method L

The Compound (I-e) can be also prepared by reacting a compound represented by formula (B5) (hereinafter, referred to as "Compound (B5)") with the Compound (M9) in the presence of a base. 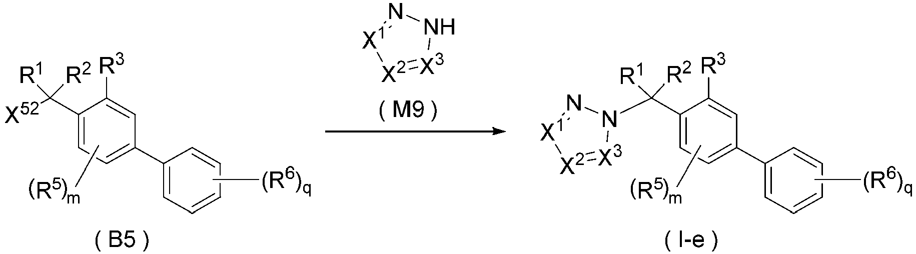 [wherein the symbols are the same as defined above.]

The reaction is usually carried out in a solvent. Examples of the solvent to be used in the reaction include alcohols, nitriles, ethers, hydrocarbons, amides, DMSO, water, and mixtures of two or more of them.

Examples of the base include alkali metal hydrides, alkali metal carbonates, organic bases, sodium hydride, and tripotassium phosphate.

In the reaction, the Compound (M9) is usually used at a ratio of 1 to 5 mols, and the base is usually used at a ratio of 1 to 5 mols, relative to 1 mol of the Compound (B5).

The reaction temperature is usually within the range of -20°C to 150°C. The reaction time is usually within the range of 0.1 to 72 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to obtain the Compound (I-e).

The Compound (B5) is known or may be prepared according to known method(s).

### Reference production method A

A compound represented by formula (C2) (hereinafter referred to as "Compound (C2)") can be prepared by reacting an organolithium reactant (which is produced by reacting the Compound (M6) with the Compound (M3)) with a compound represented by formula (C1) (hereinafter referred to as "Compound (C1)"). 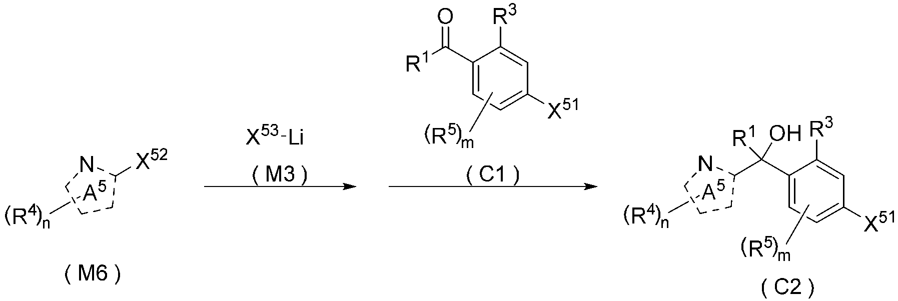 [wherein
the symbols are the same as defined above.]

The reaction can be carried out by using the Compound (M6) instead of the Compound (B3) and using the Compound (C1) instead of the Compound (M4) according to the Production method C.

The Compound (C1) is known or may be prepared according to known method(s).

### Reference production method B

The Compound (C2) can be also prepared by reacting a Grignard reactant (which is produced by reacting the Compound (M6) with the Compound (M5)) with the Compound (C1). 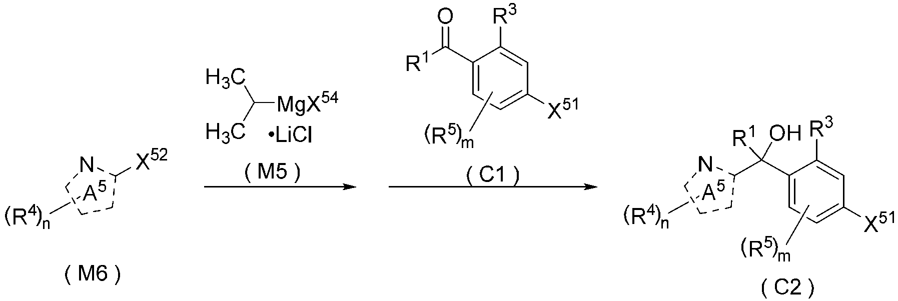 [wherein,
the symbols are the same as defined above.]

The reaction can be carried out by using the Compound (M6) instead of the Compound (B3) and using the Compound (C1) instead of the Compound (M4) according to the Production method D.

### Reference production method C

A compound represented by formula (C4) (hereinafter referred to as "Compound (C4)") can be prepared by reacting with a compound represented by formula (C3) (hereinafter referred to as "Compound (C3)") with the Compound (M9) in the presence of a condensation agent. 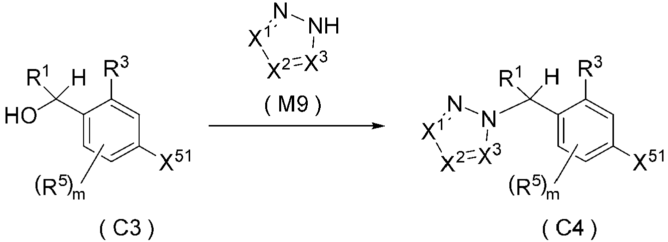 [wherein,
the symbols are the same as defined above.]

The reaction can be carried out by using the Compound (C3) instead of the Compound (B4) according to the Production method K.

The Compound (C3) is known or may be prepared according to known method(s).

### Reference production method D

The Compound (C6) can be prepared by reacting with a compound represented by formula (C5) (hereinafter referred to as "Compound (C5)") with the Compound (M9) in the presence of a base. 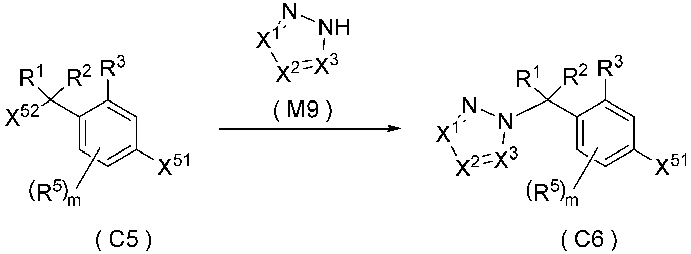 [wherein the symbols are the same as defined above.]

The reaction can be carried out by using the Compound (C5) instead of the Compound (B5) according to the Production method L.

The Compound (C5) is known or may be prepared according to known method(s).

### Reference production method E

A compound represented by formula (C7) (hereinafter referred to as "Compound (C7)") can be prepared by reacting the Compound (B1) with bis(pinacolato)diboron in the presence of a base and a palladium catalyst. 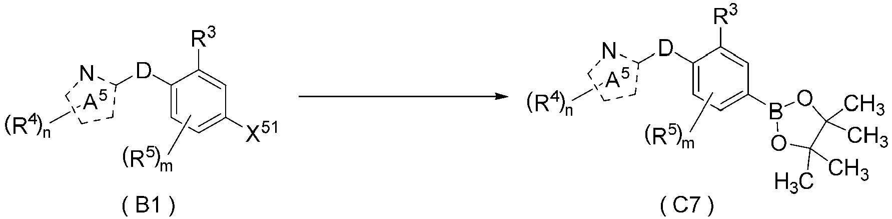 [wherein,
the symbols are the same as defined above.]

The reaction can be carried out by using bis(pinacolato)diboron instead of the Compound (M1) according to the Production method A.

### Reference production method F

The Compound (B4) can be prepared by subjecting a compound represented by formula (C8) (hereinafter, referred to as "Compound (C8)") to a reduction reaction. 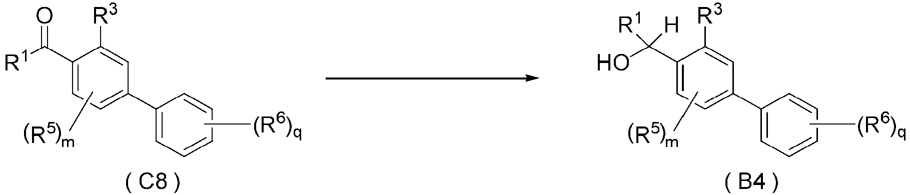 [wherein
the symbols are the same as defined above.]

The reduction reaction is usually carried out in a solvent. Examples of the solvent to be used in the reaction include ethers, alcohols, and mixtures of two or more of them.

Examples of the reducing agent to be used in the reaction include sodium borohydride.

In the reaction, the reducing agent is usually used at a ratio of 0.5 to 2 mols relative to 1 mol of the Compound (C8).

The reaction temperature is usually within the range of 0 to 100°C. The reaction time is usually within the range of 0.1 to 120 hour(s).

When the reaction is completed, the reaction mixture may be subjected to a work-up such as adding water to the reaction mixture, and drying and/or concentrating the resulting organic layer to isolate the Compound (B4).

The Compound (C8) is known or may be prepared according to known method(s).

### Reference production method G

A compound represented by formula (C10) (hereinafter, referred to as "Compound (C10)") can be prepared by subjecting a compound represented by formula (C9) (hereinafter, referred to as "Compound (C8)") to an oxidation reaction. 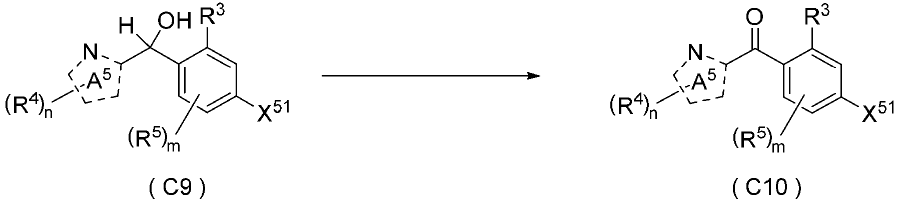 [wherein,
the symbols are the same as defined above.]

The oxidation can be carried out according to the method described in Natural Product Reports, 2011, 28, 1722-1754.

The Compound (C9) can be prepared according to the Reference production method A, or the Reference production method B.

### Reference production method H

A compound represented by formula (C11) (hereinafter, referred to as "Compound (C11)") can be prepared by subjecting the Compound (C10) to a thiocarbonylation reaction. 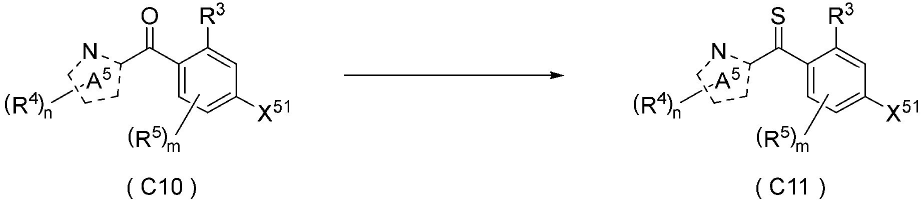 [wherein,
the symbols are the same as defined above.]

The thiocarbonylation reaction can be caried out according to the method described in Tetrahedron, 1985, 41, 5061-5087.

### Reference production method I

A compound represented by formula (C12) (hereinafter, referred to as "Compound (C12)") can be prepared by reacting the Compound (C10) with a compound represented by formula (M10) (hereinafter, referred to as "Compound (M10)") or a salt of the Compound (M10). 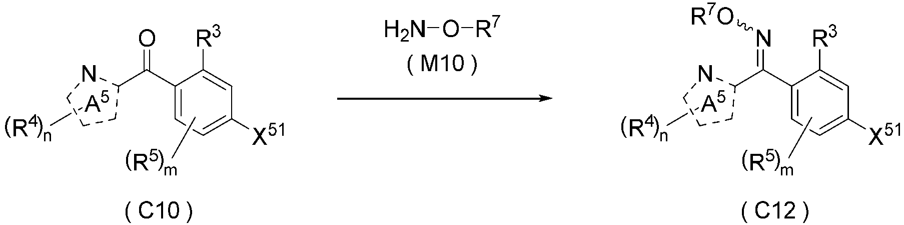 [wherein
the symbols are the same as defined above.]

Examples of the salt of the Compound (M10) include a hydrochloride salt and a sulfate salt.

The reaction is usually carried out in a solvent. Examples of the solvent to be used in the reaction include hydrocarbons, ethers, halogenated hydrocarbons, amides, esters, nitriles, alcohols, and mixtures of two or more of them.

A base may be added to the reaction as needed. Examples of the base to be used in the reaction include organic bases; alkali metal carbonates; alkali metal hydrogen carbonates; sodium hydride, and tripotassium phosphate.

When a base is used in the reaction, the base is usually used at a ratio of 1 to 10 mols relative to 1 mol of the Compound (C10).

In the reaction, the Compound (M9) is usually used at a ratio of 1 to 10 mols relative to 1 mol of the Compound (C10).

The reaction temperature is usually within the range of 0 to 150°C. The reaction time is usually within the range of 0.1 to 120 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to isolate the Compound (C12).

The salt of the Compound (M10) is known or may be prepared according to known method(s).

### Reference production method J

A compound represented by formula (C13) (hereinafter, referred to as "Compound (C13)") can be prepared by reacting the Compound (M6) with a compound represented by formula (M11) (hereinafter, referred to as "Compound (M11)"). 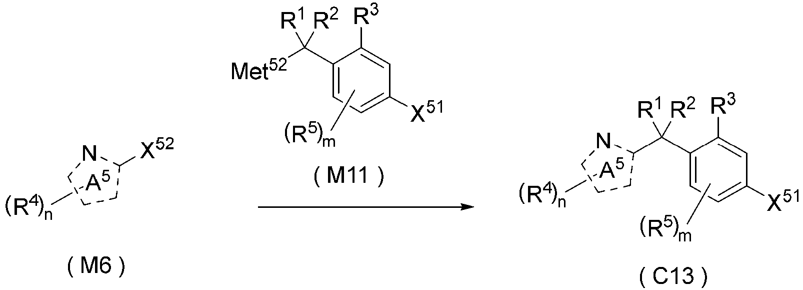 [wherein
Met⁵² represents B(OH)₂ or a 4,4,5,5-tetramethyl-1,3,2-dioxaborolan-2-yl group, or ZnBr and the other symbols are the same as defined above.]

The reaction can be carried out by using the Compound (M6) instead of the Compound (B1) and using the Compound (M11) instead of the Compound (M1) according to the Production method A.

Also, the reaction can be also carried out according to the method described in Journal of Medicinal Chemistry, 2017, 60 (23), 9769-9789, or WO 2011/075515.

The Compound (M11) is known or may be prepared according to known method(s).

### Reference production method K

A compound represented by formula (B1-1) (hereinafter, referred to as "Compound (B1-1)") can be prepared by subjecting a compound represented by formula (C14) (hereinafter, referred to as "Compound (C14)" to a cyclization reaction. 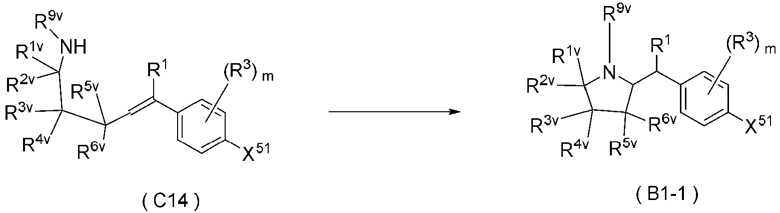 [wherein,
R^{1v}, R^{2v}, R^{3v}, R^{4v}, R^{5v}, and R^{6v} are identical to or different from each other and each represents a C1-C6 chain hydrocarbon group optionally substituted with one or more substituents selected from Group A, a C3-C8 alicyclic hydrocarbon group, a three(3) - eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the a phenyl group, and the a five(5)-six(6) membered aromatic heterocyclic group may be optionally substituted with one or more substituents selected from Group C}, OR²⁶, NR²⁷R²⁸, C(O)R²⁹, C(R³⁰)=NOR³¹, a cyano group, a nitro group, a halogen atom, or a hydrogen atom, and R^{9v} represents a C1-C6 chain hydrocarbon group optionally substituted with one or more substituents selected from Group A, a C3-C8 alicyclic hydrocarbon group, a three(3)- eight(8) membered nonaromatic heterocyclic group {the C3-C8 alicyclic hydrocarbon group, and the three (3)-eight(8) membered nonaromatic heterocyclic group may be optionally substituted with one or more substituents selected from Group B}, a phenyl group optionally substituted with one or more substituents selected from Group C, C(O)R²⁹, or a hydrogen atom, and the other symbols are the same as defined above.]

The reaction is usually carried out in a solvent. Examples of the solvent to be used in the reaction include alcohols, hydrocarbons, ethers, halogenated hydrocarbons, amides, esters, nitriles, DMSO, water, and mixtures of two or more of them.

A base may be added to the reaction as needed.

Examples of the base to be used in the reaction include organic bases, alkali metal carbonates, alkali metal hydrogen carbonates, or phosphazene bases such as 1-tert-butyl-4,4,4-tris(dimethylamino)-2,2-bis[tris(dimethylamino)phosphoranylidene amino]-2λ5, 4λ5-catenadi (phosphazenes).

In the reaction, the base is usually used at a ratio of 0.01 to 10 mols relative to 1 mol of the Compound (C14).

The reaction temperature is usually within the range of 0 to 150°C. The reaction time is usually within the range of 0.1 to 120 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to isolate the Compound (B1-1).

Also, the reaction can be also carried out according to the method described in Tetrahedron Letters, 2022, 89, 153599; Org. Lett, 2022, 24, 51, 9447; or J. Am. Chem. Soc. 2014, 136, 35, 12217 and the like.

The Compound (C14) can be prepared according to the known methods, for example, a method described in Tetrahedron Letters, 2022, 89, 153599; Org. Lett, 2022, 24, 51, 9447; or J. Am. Chem. Soc. 2014, 136, 35, 12217 and the like.

### Reference production method L

A compound represented by formula (B1-2) (hereinafter, referred to as "Compound (B1-2)") can be prepared by reacting a compound represented by formula (C15) (hereinafter, referred to as "Compound (C15)") with a compound represented by formula (M1-1) (hereinafter, referred to as "Compound (M1-1)"). 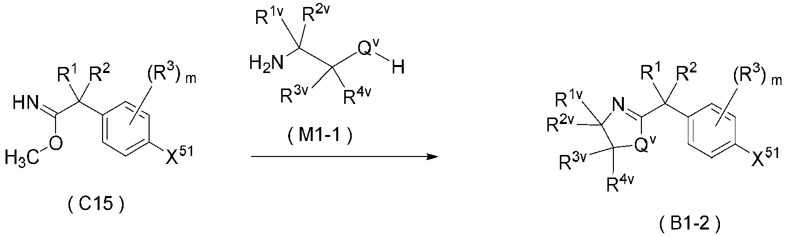 [wherein,
Q^{v} represents an oxygen atom, a sulfur atom, or NR^{9v}, and the other symbols are the same as defined above.]

The reaction is usually carried out in a solvent. Examples of the solvent to be used in the reaction include alcohols, hydrocarbons, ethers, halogenated hydrocarbons, amides, esters, nitriles, DMSO, water, and mixtures of two or more of them.

A base may be added to the reaction as needed.

Examples of the base to be used in the reaction include organic bases, alkali metal carbonates, and alkali metal hydrogen carbonates.

In the reaction, the Compound (M1-1) is usually used at a ratio of 0.5 to 10 mols and the base is usually used at a ratio of 1 to 10 moles, relative to 1 mole of the Compound (C15).

The reaction temperature is usually within the range of 0 to 150°C. The reaction time is usually within the range of 0.1 to 120 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to isolate the Compound (B1-2).

Also, the reaction can be also carried out according to the method described in J. Chem. Soc., Perkin Trans.1, 1977, 2328-2332.

The Compound (C15) is known or may be prepared according to known method(s).

### Reference production method M

A compound represented by formula (B1-3) (hereinafter, referred to as "Compound (B1-3)") can be prepared by reacting a compound represented by formula (C16) (hereinafter, referred to as "Compound (C16)") with a compound represented by formula (M1-2) (hereinafter, referred to as "Compound (M1-2)") in the presence of sodium hypochlorite. 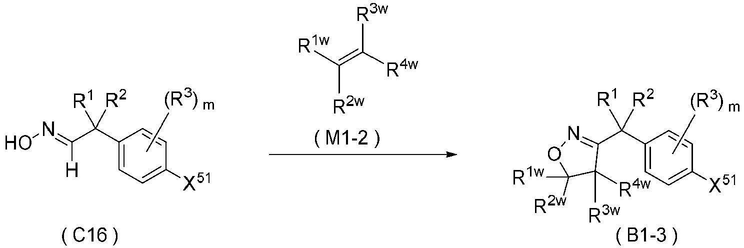 [wherein,
R^{1v}, R^{2v}, R^{3v}, and R^{4v} are identical to or different from each other and each represents a C1-C6 chain hydrocarbon group optionally substituted with one or more substituents selected from Group A, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B}, a phenyl group, a five(5)- six(6) membered aromatic heterocyclic group {the a phenyl group, and the a five(5)- six(6) membered aromatic heterocyclic group may be optionally substituted with one or more substituents selected from Group C}, OR²⁶, NR²⁷R²⁸, C(O)R²⁹, C(R³⁰)=NOR³¹, a cyano group, a nitro group, a halogen atom, or a hydrogen atom, and the other symbols are the same as defined above.]

The reaction is usually carried out in a solvent. Examples of the solvent to be used in the reaction include alcohols, hydrocarbons, ethers, halogenated hydrocarbons, amides, esters, nitriles, DMSO, water, and mixtures of two or more of them.

Examples of the base to be used in the reaction include organic bases, alkali metal carbonates, and alkali metal hydrogen carbonates.

In the reaction, the Compound (M1-2) is usually used at a ratio of 0.5 to 10 mols and sodium hypochlorite is usually used at a ratio of 1 to 10 mols, relative to 1 mol of the Compound (C16).

The reaction temperature is usually within the range of 0 to 150°C. The reaction time is usually within the range of 0.1 to 120 hour(s).

When the reaction is completed, the reaction mixture may be subjected to a work-up such as adding water to the reaction mixture, subjecting the resulting mixture to extraction with organic solvent(s), and drying and/or concentrating the resulting organic layer to isolate the Compound (B1-3).

The Compound (C16) is known or may be prepared according to known method(s).

The Compound of the present invention may be mixed with or used in combination with one or more ingredient(s) selected from the group consisting of the following Group (a), Group (b), Group (c), and Group (d) (hereinafter referred to as "Present ingredient").

When the Compound of the present invention is mixed with or used in combination with the Present ingredient, they are used simultaneously, separately, or at time intervals with each other.

When the Compound of the present invention is used simultaneously with the Present ingredient, the Compound of the present invention and Present ingredient may be contained in separate formulations or contained in one formulation.

One aspect of the present invention provides a composition comprising one or more ingredient (s) selected from the group consisting of Group (a), Group (b), Group (c), and Group (d) (i.e., Present ingredient), and the Compound of the present invention (hereinafter referred to as "Composition A").

Group (a) is a group consisting of acetylcholinesterase inhibitors (for example, carbamate insecticides and organophosphate insecticides), GABA-gated chloride channel blockers (for example, phenylpyrazole insecticides), sodium channel modulators (for example, pyrethroid insecticides), nicotinic acetylcholine receptor competitive modulators (for example, neonicotinoid insecticides), nicotinic acetylcholine receptor allosteric modulators, glutamate-gated chloride channel allosteric modulators (for example, macrolide insecticides), juvenile hormone mimics, multisite inhibitors, chordotonal organ TRPV channel modulators, mite growth inhibitors, microbial disruptors of insect midgut membranes, inhibitors of mitochondrial ATP synthase, uncouplers of oxidative phosphorylation, nicotinic acetylcholine receptor channel blockers (for example, nereistoxin insecticides), inhibitors of chitin biosynthesis, moulting disruptors, ecdysone receptor agonists, octopamine receptor agonists, mitochondrial complexes I, II, III, and IV electron transport inhibitors, voltage-dependent sodium channel blockers, inhibitors of acetyl CoA carboxylase, ryanodine receptor modulators (for example, diamide insecticides), chordotonal organ modulators, and microbial insecticides, and other insecticidal active ingredients, miticidal active ingredients, and nematicidal active ingredients. These ingredients are described in the classification on the basis of action mechanism by IRAC.

Group (b) is a group consisting of nucleic acids synthesis inhibitors (for example, phenylamide fungicides and acylamino acid fungicides), cell division and cytoskeleton inhibitors (for example, MBC fungicides), respiration inhibitors (for example, QoI fungicides and QiI fungicides), amino acids synthesis and protein synthesis inhibitors (for example, anilino-pyrimidine fungicides), signal transduction inhibitors, lipid synthesis and membrane synthesis inhibitors, sterol biosynthesis inhibitors (for example, DMI fungicides such as triazole fungicides), cell wall biosynthesis inhibitors, melanin synthesis inhibitors, plant defense inducers, fungicides with multi-site contact activity, microbial fungicides, and other fungicidal active ingredients. These ingredients are described in the classification on the basis of action mechanism by FRAC.

Group (c) is a group of plant growth regulatory ingredients (including mycorrhizal fungi and root nodule bacteria).

Group (d) is a group of repellent ingredients.

Hereinafter, examples of the combination of the Present ingredient and the Compound of the present invention are described. For example, "alanycarb + SX" indicates a combination of alanycarb and SX.

The abbreviation of "SX" indicates any one of the Compound of the present invention selected from the Compound groups SX1 to SX98. Also, all of the following Present ingredient are known ingredients, and may be obtained from commercially available formulations, or may be prepared by known methods. When the Present ingredient is a microorganism, it may also be available from a bacterial authority depository. Further, the number in parentheses represents the CAS RN (registered trademark).

Combinations of the Present ingredient in the above Group (a) and the Compound of the present invention:
abamectin + SX, acephate + SX, acequinocyl + SX, acetamiprid + SX, acetoprole + SX, acrinathrin + SX, acynonapyr + SX, afidopyropen + SX, afoxolaner + SX, alanycarb + SX, aldicarb + SX, allethrin + SX, alpha-cypermethrin + SX, alpha-endosulfan + SX, aluminium phosphide + SX, amitraz + SX, azadirachtin + SX, azamethiphos + SX, azinphos-ethyl + SX, azinphos-methyl + SX, azocyclotin + SX, bark of Celastrus angulatus + SX, bendiocarb + SX, benfluthrin + SX, benfuracarb + SX, bensultap + SX, bentioflumin + SX, benzoximate + SX, benzpyrimoxan + SX, beta-cyfluthrin + SX, beta-cypermethrin + SX, bifenazate + SX, bifenthrin + SX, bioallethrin + SX, bioresmethrin + SX, bistrifluron + SX, bisulflufen + SX, borax + SX, boric acid + SX, broflanilide + SX, bromopropylate + SX, buprofezin + SX, butocarboxim + SX, butoxycarboxim + SX, cadusafos + SX, calcium phosphide + SX, carbaryl + SX, carbofuran + SX, carbosulfan + SX, cartap hydrochloride + SX, cartap + SX, chinomethionat + SX, chlorantraniliprole + SX, chlordane + SX, chlorethoxyfos + SX, chlorfenapyr + SX, chlorfenvinphos + SX, chlorfluazuron + SX, chlormephos + SX, chloropicrin + SX, chlorpyrifos + SX, chlorpyrifos-methyl + SX, chromafenozide + SX, clofentezine + SX, clothianidin + SX, concanamycin A + SX, coumaphos + SX, cryolite + SX, cyanophos + SX, cyantraniliprole + SX, cybenzoxasulfyl + SX, cyclaniliprole + SX, cyclobutrifluram + SX, cycloprothrin + SX, cycloxaprid + SX, cyenopyrafen + SX, cyetpyrafen + SX, cyflumetofen + SX, cyfluthrin + SX, cyhalodiamide + SX, cyhalothrin + SX, cyhexatin + SX, cypermethrin + SX, cyphenothrin + SX, cyproflanilide + SX, cyromazine + SX, dazomet + SX, deltamethrin + SX, demeton-S-methyl + SX, diafenthiuron + SX, diazinon + SX, dichlorvos + SX, dicloromezotiaz + SX, dicofol + SX, dicrotophos + SX, diflovidazin + SX, diflubenzuron + SX, dimefluthrin + SX, dimethoate + SX, dimethylvinphos + SX, dimpropyridaz + SX, dinotefuran + SX, disodium octaborate + SX, disulfoton + SX, DNOC(2-methyl-4,6-dinitrophenol) + SX, doramectin + SX, dried leaves of Dryopteris filix-mas + SX, emamectin-benzoate + SX, empenthrin + SX, endosulfan + SX, EPN(O-ethyl O-(4-nitrophenyl) phenylphosphonothioate) + SX, epsilon-metofluthrin + SX, epsilon-momfluorothrin + SX, esfenvalerate + SX, ethiofencarb + SX, ethion + SX, ethiprole + SX, ethoprophos + SX, etofenprox + SX, etoxazole + SX, extract of Artemisia absinthium + SX, extract of Azadirachta indica + SX, extract of Cassia nigricans + SX, extract of clitoria ternatea + SX, extract of Symphytum officinale + SX, extract of Chenopodium ambrosioides + SX, extract of Tanacetum vulgare + SX, extract of Urtica dioica + SX, extract of Viscum album + SX, famphur + SX, fenamiphos + SX, fenazaquin + SX, fenbutatin oxide + SX, fenitrothion + SX, fenmezoditiaz + SX, fenobucarb + SX, fenoxycarb + SX, fenpropathrin + SX, fenpyroximate + SX, fenthion + SX, fenvalerate + SX, fipronil + SX, flometoquin + SX, flonicamid + SX, fluacrypyrim + SX, fluazaindolizine + SX, fluazuron + SX, flubendiamide + SX, fluchlordiniliprole + SX, flucycloxuron + SX, flucythrinate + SX, fluensulfone + SX, flufenoprox + SX, flufenoxuron + SX, flufiprole + SX, flumethrin + SX, flupentiofenox + SX, flupyradifurone + SX, flupyrimin + SX, flupyroxystrobin + SX, fluralaner + SX, fluvalinate + SX, fluxametamide + SX, formetanate + SX, fosthiazate + SX, furamethrin + SX, furathiocarb + SX, gamma-cyhalothrin + SX, GS-omega/kappa HXTX-Hv1a peptide + SX, halfenprox + SX, halofenozide + SX, heptafluthrin + SX, heptenophos + SX, hexaflumuron + SX, hexythiazox + SX, potassium salt of hop beta acid + SX, hydramethylnon + SX, hydroprene + SX, imicyafos + SX, imidacloprid + SX, imidaclothiz + SX, imiprothrin + SX, indazapyroxamet + SX, indoxacarb + SX, isocycloseram + SX, isofenphos + SX, isoflualanam + SX, isoprocarb + SX, isopropyl-O-(methoxyaminothiophosphoryl) salicylate + SX, isoxathion + SX, ivermectin + SX, kadethrin + SX, kappa-tefluthrin + SX, kappa-bifenthrin + SX, kinoprene + SX, lambda-cyhalothrin + SX, ledprona + SX, lenoremycin + SX, lepimectin + SX, lime sulfur + SX, lotilaner + SX, lufenuron + SX, machine oil + SX, malathion + SX, mecarbam + SX, meperfluthrin + SX, metaflumizone + SX, metam + SX, methamidophos + SX, methidathion + SX, methiocarb + SX, methomyl + SX, methoprene + SX, methoxychlor + SX, methoxyfenozide + SX, methyl bromide + SX, metofluthrin + SX, metolcarb + SX, metoxadiazone + SX, mevinphos + SX, milbemectin + SX, milbemycin oxime + SX, mivorilaner + SX, modoflaner + SX, momfluorothrin + SX, monocrotophos + SX, moxidectin + SX, naled + SX, nicofluprole + SX, nicotine + SX, nicotine-sulfate + SX, nitenpyram + SX, novaluron + SX, noviflumuron + SX, oil of the seeds of Chenopodium anthelminticum + SX, omethoate + SX, oxamyl + SX, oxazosulfyl + SX, oxydemeton-methyl + SX, parathion + SX, parathion-methyl + SX, permethrin + SX, phenothrin + SX, phenthoate + SX, phorate + SX, phosalone + SX, phosmet + SX, phosphamidon + SX, phosphine + SX, phoxim + SX, pioxaniliprole + SX, piperflanilide + SX, pirimicarb + SX, pirimiphos-methyl + SX, prallethrin + SX, profenofos + SX, profluthrin + SX, propargite + SX, propetamphos + SX, propoxur + SX, propylene glycol alginate + SX, prothiofos + SX, pyflubumide + SX, pymetrozine + SX, pyraclofos + SX, pyrethrins + SX, pyridaben + SX, pyridalyl + SX, pyridaphenthion + SX, pyrifluquinazone + SX, pyrimidifen + SX, pyriminostrobin + SX, pyriprole + SX, pyriproxyfen + SX, quinalphos + SX, resmethrin + SX, rotenone + SX, ryanodine + SX, sarolaner + SX, selamectin + SX, sigma-cypermethrin + SX, silafluofen + SX, sodium borate + SX, sodium metaborate + SX, spidoxamat + SX, spinetoram + SX, spinosad + SX, spirobudifen + SX, spirodiclofen + SX, spiromesifen + SX, spiropidion + SX, spirotetramat + SX, sulfiflumin + SX, sulfluramid + SX, sulfotep + SX, sulfoxaflor + SX, sulfur + SX, sulfuryl fluoride + SX, tartar emetic + SX, tau-fluvalinate + SX, tebufenozide + SX, tebufenpyrad + SX, tebupirimfos + SX, teflubenzuron + SX, tefluthrin + SX, temephos + SX, terbufos + SX, terpene constituents of the extract of chenopodium ambrosioides near ambrosioides + SX, tetrachlorantraniliprole + SX, tetrachlorvinphos + SX, tetradifon + SX, tetramethrin + SX, tetramethylfluthrin + SX, tetraniliprole + SX, theta-cypermethrin + SX, thiacloprid + SX, thiamethoxam + SX, thiocyclam + SX, thiodicarb + SX, thiofanox + SX, thiometon + SX, thiosultap-disodium + SX, thiosultap-monosodium + SX, tiapyrachlor + SX, tigolaner + SX, tiorantraniliprole + SX, tioxazafen + SX, tolfenpyrad + SX, tralomethrin + SX, transfluthrin + SX, triazamate + SX, triazophos + SX, trichlorfon + SX, trifluenfuronate + SX, triflumezopyrim + SX, triflumuron + SX, trimethacarb + SX, tyclopyrazoflor + SX, umifoxolaner + SX, vadescana + SX, vamidothion + SX, wood extract of Quassia amara + SX, XMC (3,5-dimethylphenyl N-methylcarbamate) + SX, xylylcarb + SX, zeta-cypermethrin + SX, zinc phosphide + SX, 4-[5-(3,5-dichlorophenyl)-5-(trifluoromethyl)-4,5-dihydro-1,2-oxazol-3-yl]-2-methyl-N-(1-oxothietan-3-yl)benzamide (1241050-20-3) + SX, 3-methoxy-N-(5-{5-(trifluoromethyl)-5-[3-(trifluoromethyl)phenyl]-4,5-dihydro-1,2-oxazol-3-yl}indan-1-yl)propanamide (1118626-57-5) + SX, N-{4-chloro-3-[(1-cyanocyclopropyl)carbamoyl]phenyl}-1-methyl-4-(methanesulfonyl)-3-(1,1,2,2,2-pentafluoroethyl)-1H-pyrazole-3-carboxamide (1400768-21-9) + SX, N-[3-chloro-1-(pyridin-3-yl)-1H-pyrazol-4-yl]-2-(methanesulfonyl)propanamide (2396747-83-2) + SX, N-[4-chloro-2-(pyridin-3-yl)-1,3-thiazol-5-yl]-N-ethyl-3-(methanesulfonyl)propanamide + SX, 1,4-dimethyl-2-[2-(pyridin-3-yl)-2H-indazol-5-yl]-1,2,4-triazolidine-3,5-dione (2171099-09-3) + SX, 2-isopropyl-5-[(3,4,4-trifluoro-3-buten-1-yl)sulfonyl]-1,3,4-thiadiazole (2058052-95-0) + SX, N-({2-fluoro-4-[(2S,3S)-2-hydroxy-3-(3,4,5-trichlorophenyl)-3-(trifluoromethyl)pyrrolidin-1-yl]phenyl}methyl)cyclopropanecarboxamide + SX, 7-fluoro-N-[1-(methylsulfanyl)-2-methylpropan-2-yl]-2-(pyridin-3-yl)-2H-indazole-4-carboxamide + SX, 7-fluoro-N-[1-(methanesulfinyl)-2-methylpropan-2-yl]-2-(pyridin-3-yl)-2H-indazole-4-carboxamide + SX, 7-fluoro-N-[1-(methanesulfonyl)-2-methylpropan-2-yl]-2-(pyridin-3-yl)-2H-indazole-4-carboxamide + SX, N-[1-(difluoromethyl)cyclopropyl]-2-(pyridin-3-yl)-2H-indazole-4-carboxamide + SX, 2,9-dihydro-9-(methoxymethyl)-2-(pyridin-3-yl)-10H-pyrazolo[3,4-f]pyrido[2,3-b][1,4]oxazepin-10-one (2607927-97-7) + SX, Bacillus thuringiensis Cry1Ab protein + SX, Bacillus thuringiensis Cry1Ac protein + SX, Bacillus thuringiensis Cry1Fa protein + SX, Bacillus thuringiensis Cry1A.105 protein + SX, Bacillus thuringiensis Cry2Ab protein + SX, Bacillus thuringiensis Vip3A protein + SX, Bacillus thuringiensis mCry3A protein + SX, Bacillus thuringiensis Cry3Ab protein + SX, Bacillus thuringiensis Cry3Bb protein + SX, Bacillus thuringiensis Cry34Ab1/Cry35Ab1 protein + SX, Adoxophyes orana granulovirus virus strain BV-0001 + SX, Anticarsia gemmatalis mNPV + SX, Autographa californica mNPV + SX, Cydia pomonella GV strain V15 + SX, Cydia pomonella GV strain V22 + SX, Cryptophlebia leucotreta GV + SX, Dendrolimus punctatus cypovirus + SX, Helicoverpa armigera NPV strain BV-0003 + SX, Helicoverpa zea NPV + SX, Lymantria dispar NPV + SX, Mamestra brassicae NPV + SX, Mamestra configurata NPV + SX, Neodiprion abietis NPV + SX, Neodiprion lecontei NPV + SX, Neodiprion sertifer NPV + SX, Nosema locustae + SX, Orgyia pseudotsugata NPV + SX, Pieris rapae GV + SX, Plodia interpunctella GV + SX, Spodoptera exigua MNPV + SX, Spodoptera littoralis mNPV + SX, Spodoptera litura mNPV + SX, Arthrobotrys dactyloides + SX, Bacillus firmus strain GB-126 + SX, Bacillus firmus strain I-1582 + SX, Bacillus firmus strain NCIM2637 + SX, Bacillus megaterium + SX, Bacillus sp. strain AQ175 + SX, Bacillus sp. strain AQ177 + SX, Bacillus sp. strain AQ178 + SX, Bacillus sphaericus strain 2362 serotype H5a5b + SX, Bacillus sphaericus strain ABTS1743 + SX, Bacillus thuringiensis strain AQ52 + SX, Bacillus thuringiensis strain BD#32 + SX, Bacillus thuringiensis strain CR-371 + SX, Bacillus thuringiensis subsp. Aizawai strain ABTS-1857 + SX, Bacillus thuringiensis subsp. Aizawai strain AM65-52 + SX, Bacillus thuringiensis subsp. Aizawai strain GC-91 + SX, Bacillus thuringiensis subsp. Aizawai strain NB200 + SX, Bacillus thuringiensis subsp. Aizawai Serotype strain H-7 + SX, Bacillus thuringiensis subsp. Kurstaki strain ABTS351 + SX, Bacillus thuringiensis subsp. Kurstaki strain BMP123 + SX, Bacillus thuringiensis subsp. Kurstaki strain CCT1306 + SX, Bacillus thuringiensis subsp. Kurstaki strain EG2348 + SX, Bacillus thuringiensis subsp. Kurstaki strain EG7841 + SX, Bacillus thuringiensis subsp. Kurstaki strain EVB113-19 + SX, Bacillus thuringiensis subsp. Kurstaki strain F810 + SX, Bacillus thuringiensis subsp. Kurstaki strain HD-1 + SX, Bacillus thuringiensis subsp. Kurstaki strain PB54 + SX, Bacillus thuringiensis subsp. Kurstaki strain SA-11 + SX, Bacillus thuringiensis subsp. Kurstaki strain SA-12 + SX, Bacillus thuringiensis subsp. Tenebriosis strain NB176 + SX, Bacillus thuringiensis subsp. Thuringiensis strain MPPL002 + SX, Bacillus thuringiensis subsp. morrisoni + SX, Bacillus thuringiensis var. colmeri + SX, Bacillus thuringiensis var. darmstadiensis strain 24-91 + SX, Bacillus thuringiensis var. dendrolimus + SX, Bacillus thuringiensis var. galleriae + SX, Bacillus thuringiensis var. israelensis strain BMP144 + SX, Bacillus thuringiensis var. israelensis serotype strain H-14 + SX, Bacillus thuringiensis var. japonensis strain buibui + SX, Bacillus thuringiensis var. san diego strain M-7 + SX, Bacillus thuringiensis var. 7216 + SX, Bacillus thuringiensis var. aegypti + SX, Bacillus thuringiensis var. T36 + SX, Beauveria bassiana strain ANT-03 + SX, Beauveria bassiana strain ATCC74040 + SX, Beauveria bassiana strain GHA + SX, Beauveria brongniartii + SX, Burkholderia rinojensis strain A396 + SX, Chromobacterium subtsugae strain PRAA4-1T + SX, Dactyllela ellipsospora + SX, Dectylaria thaumasia + SX, Hirsutella minnesotensis + SX, Hirsutella rhossiliensis + SX, Hirsutella thompsonii + SX, Lagenidium giganteum + SX, Lecanicillium lecanii strain KV01 + SX, Lecanicillium lecanii conidia of strain DAOM198499 + SX, Lecanicillium lecanii conidia of strain DAOM216596 + SX, Lecanicillium muscarium strain Ve6 + SX, Metarhizium anisopliae strain F52 + SX, Metarhizium anisopliae var. acridum + SX, Metarhizium anisopliae var. anisopliae BIPESCO 5/F52 + SX, Metarhizium flavoviride + SX, Monacrosporium phymatopagum + SX, Paecilomyces fumosoroseus Apopka strain 97 + SX, Paecilomyces lilacinus strain 251 + SX, Paecilomyces tenuipes strain T1 + SX, Paenibacillus popilliae + SX, Pasteuria nishizawae strain Pn1 + SX, Pasteuria penetrans + SX, Pasteuria usgae + SX, Pasteuria thornei + SX, Serratia entomophila + SX, Verticillium chlamydosporium + SX, Verticillium lecani strain NCIM1312 + SX, Wolbachia pipientis + SX.

Combination of the Present ingredient in the above Group (b) and the Compound of the present invention:
acibenzolar-S-methyl + SX, aldimorph + SX, ametoctradin + SX, aminopyrifen + SX, amisulbrom + SX, anilazine + SX, azaconazole + SX, azoxystrobin + SX, basic copper sulfate + SX, benalaxyl + SX, benalaxyl-M + SX, benodanil + SX, benomyl + SX, benthiavalicarb + SX, benthiavalicarb-isopropyl + SX, benzovindiflupyr + SX, binapacryl + SX, biphenyl + SX, bitertanol + SX, bixafen + SX, blasticidin-S + SX, Bordeaux mixture + SX, boscalid + SX, bromothalonil + SX, bromuconazole + SX, bupirimate + SX, captafol + SX, captan + SX, carbendazim + SX, carboxin + SX, carpropamid + SX, chinomethionat + SX, chitin + SX, chloroinconazide + SX, chloroneb + SX, chlorothalonil + SX, chlozolinate + SX, colletochlorin B + SX, copper(II) acetate + SX, copper(II) hydroxide + SX, copper oxychloride + SX, copper(II) sulfate + SX, coumoxystrobin + SX, cyazofamid + SX, cyflufenamid + SX, cymoxanil + SX, cyproconazole + SX, cyprodinil + SX, dichlobentiazox + SX, dichlofluanid + SX, diclocymet + SX, diclomezine + SX, dicloran + SX, diethofencarb + SX, difenoconazole + SX, diflumetorim + SX, dimethachlone + SX, dimethirimol + SX, dimethomorph + SX, dimoxystrobin + SX, diniconazole + SX, diniconazole-M + SX, dinocap + SX, dipotassium hydrogenphosphite + SX, dipymetitrone + SX, dithianon + SX, dodecylbenzenesulphonic acid bisethylenediamine copper(II) salt + SX, dodemorph + SX, dodine + SX, edifenphos + SX, enoxastrobin + SX, epoxiconazole + SX, etaconazole + SX, ethaboxam + SX, ethirimol + SX, etridiazole + SX, extract of Melaleuca alternifolia + SX, extract of Reynoutria sachalinensis + SX, extract of the cotyledons of lupine plantlets ("BLAD") + SX, extract of Allium sativum) + SX, extract of Equisetum arvense + SX, extract of Tropaeolum majus + SX, famoxadone + SX, fenamidone + SX, fenaminstrobin + SX, fenarimol + SX, fenbuconazole + SX, feneptamidoquin + SX, fenfuram + SX, fenhexamid + SX, fenopyramid + SX, fenoxanil + SX, fenpiclonil + SX, fenpicoxamid + SX, fenpropidin + SX, fenpropimorph + SX, fenpyrazamine + SX, fentin acetate + SX, fentin chloride + SX, fentin hydroxide + SX, ferbam + SX, ferimzone + SX, florylpicoxamid + SX, fluazinam + SX, flubeneteram + SX, fludioxonil + SX, flufenoxadiazam + SX, flufenoxystrobin + SX, fluindapyr + SX, flumetylsulforim + SX, flumorph + SX, fluopicolide + SX, fluopyram + SX, fluopimomide + SX, fluoroimide + SX, fluoxapiprolin + SX, fluoxastrobin + SX, fluoxytioconazole + SX, fluquinconazole + SX, flusilazole + SX, flusulfamide + SX, flutianil + SX, flutolanil + SX, flutriafol + SX, fluxapyroxad + SX, folpet + SX, fosetyl + SX, fosetyl-aluminium + SX, fuberidazole + SX, furalaxyl + SX, furametpyr + SX, galquin + SX, guazatine + SX, hexaconazole + SX, hymexazole + SX, imazalil + SX, imibenconazole + SX, iminoctadine + SX, iminoctadine triacetate + SX, inpyrfluxam + SX, iodocarb + SX, ipconazole + SX, ipfentrifluconazole + SX, ipflufenoquin + SX, iprobenfos + SX, iprodione + SX, iprovalicarb + SX, isofetamid + SX, isoflucypram + SX, isoprothiolane + SX, isopyrazam + SX, isotianil + SX, kasugamycin + SX, kresoxim-methyl + SX, laminarin + SX, leaves and bark of Quercus + SX, mancozeb + SX, mandestrobin + SX, mandipropamid + SX, maneb + SX, mefentrifluconazole + SX, mepanipyrim + SX, mepronil + SX, meptyldinocap + SX, metalaxyl + SX, metalaxyl-M + SX, metarylpicoxamid + SX, metconazole + SX, methasulfocarb + SX, metiram + SX, metominostrobin + SX, metrafenone + SX, metyltetraprole + SX, myclobutanil + SX, naftifine + SX, nuarimol + SX, octhilinone + SX, ofurace + SX, orysastrobin + SX, oxadixyl + SX, oxathiapiprolin + SX, oxine-copper + SX, oxolinic acid + SX, oxpoconazole + SX, oxpoconazole fumarate + SX, oxycarboxin + SX, oxytetracycline + SX, pefurazoate + SX, penconazole + SX, pencycuron + SX, penflufen + SX, penthiopyrad + SX, phenamacril + SX, phosphorous acid + SX, phthalide + SX, picarbutrazox + SX, picoxystrobin + SX, piperalin + SX, polyoxins + SX, potassium hydrogencarbonate + SX, potassium dihydrogenphosphite + SX, probenazole + SX, prochloraz + SX, procymidone + SX, propamidine + SX, propamocarb + SX, propiconazole + SX, propineb + SX, proquinazid + SX, prothiocarb + SX, prothioconazole + SX, pydiflumetofen + SX, pyraclostrobin + SX, pyrametostrobin + SX, pyraoxystrobin + SX, pyrapropoyne + SX, pyraziflumid + SX, pyrazophos + SX, pyribencarb + SX, pyributicarb + SX, pyridachlometyl + SX, pyrifenox + SX, pyrimethanil + SX, pyrimorph + SX, pyriofenone + SX, pyrisoxazole + SX, pyroquilon + SX, Quillaja extract + SX, quinconazole + SX, quinofumelin + SX, quinoxyfen + SX, quintozene + SX, Saponins of Chenopodium quinoa + SX, seboctylamine + SX, sedaxane + SX, silthiofam + SX, simeconazole + SX, sodium hydrogencarbonate + SX, spiroxamine + SX, streptomycin + SX, sulfur + SX, tebuconazole + SX, tebufloquin + SX, teclofthalam + SX, tecnazene + SX, terbinafine + SX, tetraconazole + SX, thiabendazole + SX, thifluzamide + SX, thiophanate + SX, thiophanate-methyl + SX, thiram + SX, thymol + SX, tiadinil + SX, tolclofos-methyl + SX, tolfenpyrad + SX, tolprocarb + SX, tolylfluanid + SX, triadimefon + SX, triadimenol + SX, triazoxide + SX, triclopyricarb + SX, tricyclazole + SX, tridemorph + SX, trifloxystrobin + SX, triflumizole + SX, triforine + SX, triticonazole + SX, validamycin + SX, valifenalate + SX, vinclozolin + SX, yellow mustard powder + SX, zinc thiazole + SX, zineb + SX, ziram + SX, zoxamide + SX, N'-[4-({3-[(4-chlorophenyl)methyl]-1,2,4-thiadiazol-5-yl}oxy)-2,5-dimethylphenyl]-N-ethyl-N-methylmethanimidamide (1202781-91-6) + SX, N'-{4-[(4,5-dichlorothiazol-2-yl)oxy]-2,5-dimethylphenyl}-N-ethyl-N-methylmethanimidamide (929908-57-6) + SX, N'-(2,5-dimethyl-4-phenoxyphenyl)-N-ethyl-N-methylmethanimidamide (1052688-31-9) + SX, N'-[5-chloro-4-(2-fluorophenoxy)-2-methylphenyl]-N-ethyl-N-methylmethanimidamide (2055589-28-9) + SX, N'-[2-chloro-4-(2-fluorophenoxy)-5-methylphenyl]-N-ethyl-N-methylmethanimidamide (2055756-21-1) + SX, N'-(2-chloro-4-phenoxy-5-methylphenyl)-N-ethyl-N-methylmethanimidamide (2062599-39-5) + SX, N'-[4-(1-hydroxy-1-phenyl-2,2,2-trifluoroethyl)-2-methyl-5-methoxyphenyl]-N-isopropyl-N-methylmethanimidamide (2101814-55-3) + SX, N'-[5-bromo-6-(1-methyl-2-propoxyethoxy)-2-methylpyridin-3-yl]-N-ethyl-N-methylmethanimidamide (1817828-69-5) + SX, 4-(2-bromo-4-fluorophenyl)-N-(2-chloro-6-fluorophenyl)-1,3-dimethyl-1H-pyrazol-5-amine (1362477-26-6) + SX, 2-[6-(3-fluoro-4-methoxyphenyl)-5-methylpyridin-2-yl]quinazoline (1257056-97-5) + SX, ethyl (2Z)-3-amino-2-cyano-3-phenylacrylate (39491-78-6) + SX, N-[(2-chlorothiazol-5-yl)methyl]-N-ethyl-6-methoxy-3-nitropyridin-2-amine (1446247-98-8) + SX, 5-(4-chlorobenzyl)-2-(chloromethyl)-2-methyl-1-(1H-1,2,4-triazol-1-ylmethyl)cyclopentan-1-ol (1394057-11-4) + SX, (1R, 2S, 5S)-5-(4-chlorobenzyl)-2-(chloromethyl)-2-methyl-1-(1H-1,2,4-triazol-1-ylmethyl)cyclopentan-1-ol (1801930-06-2) + SX, (1S, 2R, 5R)-5-(4-chlorobenzyl)-2-(chloromethyl)-2-methyl-1-(1H-1,2,4-triazol-1-ylmethyl)cyclopentan-1-ol (1801930-07-3) + SX, 2-(chloromethyl)-5-(4-fluorobenzyl)-2-methyl-1-(1H-1,2,4-triazol-1-ylmethyl)cyclopentan-1-ol (1394057-13-6) + SX, (1R, 2S, 5S)-2-(chloromethyl)-5-(4-fluorobenzyl)-2-methyl-1-(1H-1,2,4-triazol-1-ylmethyl)cyclopentan-1-ol (1801930-08-4) + SX, (1S, 2R, 5R)-2-(chloromethyl)-5-(4-fluorobenzyl)-2-methyl-1-(1H-1,2,4-triazol-1-ylmethyl)cyclopentan-1-ol (1801930-09-5) + SX, methyl 3-[(4-chlorophenyl)methyl]-2-hydroxy-1-methyl-2-(1H-1,2,4-triazol-1-ylmethyl)cyclopentan-1-carboxylate (1791398-02-1) + SX, 1-(2,4-difluorophenyl)-2-(1H-1,2,4-triazol-1-yl)-1-[1-(4-bromo-2,6-difluorophenoxy)cyclopropyl]ethanol (2019215-86-0) + SX, 1-(2,4-difluorophenyl)-2-(1H-1,2,4-triazol-1-yl)-1-[1-(4-chloro-2,6-difluorophenoxy)cyclopropyl]ethanol (2019215-84-8) + SX, 1-[2-(1-chlorocyclopropyl)-3-(2-fluorophenyl)-2-hydroxypropyl]-1H-imidazole-5-carbonitrile (2018316-13-5) + SX, 1-[2-(1-chlorocyclopropyl)-3-(2,3-difluorophenyl)-2-hydroxypropyl]-1H-imidazole-5-carbonitrile (2018317-25-2) + SX, 2-[6-(4-bromophenoxy)-2-(trifluoromethyl)pyridin-3-yl]-1-(1H-1,2,4-triazol-1-yl)propan-2-ol (2082661-43-4) + SX, 2-[6-(4-chlorophenoxy)-2-(trifluoromethyl)pyridin-3-yl]-1-(1H-1,2,4-triazol-1-yl)propan-2-ol (2082660-27-1) + SX, methyl ({2-methyl-5-[1-(4-methoxy-2-methylphenyl)-1H-pyrazol-3-yl]phenyl}methyl)carbamate (1605879-98-8) + SX, 2-(difluoromethyl)-N-[1,1,3-trimethyl-2,3-dihydro-1H-inden-4-yl]pyridine-3-carboxamide (1616239-21-4) + SX, 2-(difluoromethyl)-N-[3-ethyl-1,1-dimethyl-2,3-dihydro-1H-inden-4-yl]pyridine-3-carboxamide (1847460-02-9) + SX, 2-(difluoromethyl)-N-[3-propyl-1,1-dimethyl-2,3-dihydro-1H-inden-4-yl]pyridine-3-carboxamide (1847460-05-2) + SX, (2E,3Z)-5-{[1-(4-chlorophenyl)-1H-pyrazol-3-yl]oxy}-2-(methoxyimino)-N,3-dimethylpent-3-enamide (1445331-27-0) + SX, (2E,3Z)-5-{[1-(2,4-dichlorophenyl)-1H-pyrazol-3-yl]oxy}-2-(methoxyimino)-N,3-dimethylpent-3-enamide (1445331-54-3) + SX, 5-chloro-4-({2-[6-(4-chlorophenoxy)pyridin-3-yl]ethyl}amino)-6-methylpyrimidine (1605340-92-8) + SX, 4,4-dimethyl-2-({4-[5-(trifluoromethyl)-1,2,4-oxadiazol-3-yl]phenyl}methyl)isoxazolidin-3-one (2098918-25-1) + SX, 5,5-dimethyl-2-({4-[5-(trifluoromethyl)-1,2,4-oxadiazol-3-yl]phenyl}methyl)isoxazolidin-3-one (2098918-26-2) + SX, N-ethyl-2-methyl-N-({4-[5-(trifluoromethyl)-1,2,4-oxadiazol-3-yl]phenyl}methyl)propanamide + SX, N,2-dimethoxy-N-({4-[5-(trifluoromethyl)-1,2,4-oxadiazol-3-yl]phenyl}methyl)propanamide + SX, N-methoxy-N-({4-[5-(trifluoromethyl)-1,2,4-oxadiazol-3-yl]phenyl}methyl)cyclopropanecarboxamide + SX, N-methoxy-N'-methyl-N-({4-[5-(trifluoromethyl)-1,2,4-oxadiazol-3-yl]phenyl}methyl)urea + SX, N'-ethyl-N-methoxy-N-({4-[5-(trifluoromethyl)-1,2,4-oxadiazol-3-yl]phenyl}methyl)urea + SX, N,N'-dimethoxy-N-({4-[5-(trifluoromethyl)-1,2,4-oxadiazol-3-yl]phenyl}methyl)urea + SX, N-acetyl-2-(ethanesulfonyl)-N-[2-(methoxycarbonyl)-4-(trifluoromethoxy)phenyl]-4-(trifluoromethyl)benzamide (2043675-28-9) + SX, 3-(4-bromo-7-fluoroindol-1-yl)butan-2-yl N-[(3-hydroxy-4-methoxypyridin-2-yl)carbonyl]-L-alaninate + SX, 3-(7-bromoindol-1-yl)butan-2-yl N-[(3-hydroxy-4-methoxypyridin-2-yl)carbonyl]-L-alaninate + SX, 3-(7-bromo-4-fluoroindol-1-yl)butan-2-yl N-[(3-hydroxy-4-methoxypyridin-2-yl)carbonyl]-L-alaninate + SX, 3-(3,5-dichloropyridin-2-yl)butan-2-yl N-[(3-hydroxy-4-methoxypyridin-2-yl)carbonyl]-L-alaninate + SX, 3-(3,5-dichloropyridin-2-yl)butan-2-yl N-{[3-(acetoxymethoxy)-4-methoxypyridin-2-yl]carbonyl}-L-alaninate + SX, (1S)-1-[1-(naphthalen-1-yl)cyclopropyl]ethyl N-[(3-hydroxy-4-methoxypyridin-2-yl)carbonyl]-L-alaninate + SX, (1S)-1-[1-(naphthalen-1-yl)cyclopropyl]ethyl N-[(3-acetoxy-4-methoxypyridin-2-yl)carbonyl]-L-alaninate + SX, (1S)-1-[1-(naphthalen-1-yl)cyclopropyl]ethyl N-{[3-(acetoxymethoxy)-4-methoxypyridin-2-yl]carbonyl}-L-alaninate + SX, N-({4-[5-(trifluoromethyl)-1,2,4-oxadiazol-3-yl]phenyl}methyl)cyclopropanecarboxamide + SX, N-allyl-N-({4-[5-(trifluoromethyl)-1,2,4-oxadiazol-3-yl]phenyl}methyl)acetamide + SX, N-allyl-N-({4-[5-(trifluoromethyl)-1,2,4-oxadiazol-3-yl]phenyl}methyl)propanamide + SX, N-({4-[5-(trifluoromethyl)-1,2,4-oxadiazol-3-yl]phenyl}methyl)propanamide + SX, 3,3,3-trifluoro-N-({2-fluoro-4-[5-(trifluoromethyl)-1,2,4-oxadiazol-3-yl]phenyl}methyl)propanamide + SX, 3,3,3-trifluoro-N-({3-fluoro-4-[5-(trifluoromethyl)-1,2,4-oxadiazol-3-yl]phenyl}methyl)propanamide + SX, 3,3,3-trifluoro-N-({2,3-difluoro-4-[5-(trifluoromethyl)-1,2,4-oxadiazol-3-yl]phenyl}methyl)propanamide + SX, N-({2,3-difluoro-4-[5-(trifluoromethyl)-1,2,4-oxadiazol-3-yl]phenyl}methyl)butanamide + SX, N-methoxy-N-methyl-N'-({ 4-[5-(trifluoromethyl)-1,2,4-oxadiazol-3-yl]phenyl}methyl)urea + SX, N,N-diethyl-N'-({ 4-[5-(trifluoromethyl)-1,2,4-oxadiazol-3-yl]phenyl}methyl)urea + SX, N-methyl-N'-({ 4-[5-(trifluoromethyl)-1,2,4-oxadiazol-3-yl]phenyl}methyl)urea + SX, 1-({4-[5-(trifluoromethyl)-1,2,4-oxadiazol-3-yl]phenyl}methyl)pyrrolidin-2-one + SX, 1-({4-[5-(trifluoromethyl)-1,2,4-oxadiazol-3-yl]phenyl}methyl)piperidin-2-one + SX, 4-({4-[5-(trifluoromethyl)-1,2,4-oxadiazol-3-yl]phenyl}methyl)morpholin-3-one + SX, 2-({4-[5-(trifluoromethyl)-1,2,4-oxadiazol-3-yl]phenyl}methyl)isoxazolidin-3-one + SX, 3,3-dimethyl-1-({4-[5-(trifluoromethyl)-1,2,4-oxadiazol-3-yl]phenyl}methyl)piperidin-2-one + SX, 2-({4-[5-(trifluoromethyl)-1,2,4-oxadiazol-3-yl]phenyl}methyl)-1,2-oxazinan-3-one + SX, 1-({3-fluoro-4-[5-(trifluoromethyl)-1,2,4-oxadiazol-3-yl]phenyl}methyl)azepan-2-one + SX, 4,4-dimethyl-1-({4-[5-(trifluoromethyl)-1,2,4-oxadiazol-3-yl]phenyl}methyl)pyrrolidin-2-one + SX, 5-methyl-1-({4-[5-(trifluoromethyl)-1,2,4-oxadiazol-3-yl]phenyl}methyl)pyrrolidin-2-one + SX, ethyl 1-({4-[5-(trifluoromethyl)-1,2,4-oxadiazol-3-yl]phenyl}methyl)-1H-pyrazole-4-carboxylate + SX, N-methyl-1-({4-[5-(trifluoromethyl)-1,2,4-oxadiazol-3-yl]phenyl}methyl)-1H-pyrazole-4-carboxamide + SX, N-propyl-1-({4-[5-(trifluoromethyl)-1,2,4-oxadiazol-3-yl]phenyl}methyl)-1H-pyrazole-4-carboxamide + SX, N-methoxy-1-({4-[5-(trifluoromethyl)-1,2,4-oxadiazol-3-yl]phenyl}methyl)-1H-pyrazole-4-carboxamide + SX, N-methoxy-N-methyl-1-({4-[5-(trifluoromethyl)-1,2,4-oxadiazol-3-yl]phenyl}methyl)-1H-pyrazole-4-carboxamide + SX, N,N-dimethyl-1-({4-[5-(trifluoromethyl)-1,2,4-oxadiazol-3-yl]phenyl}methyl)-1H-1,2,4-triazol-3-amine + SX, N-methyl-4-[5-(trifluoromethyl)-1,2,4-oxadiazol-3-yl]benzamide + SX, methyl 2-[2-chloro-4-(4-chlorophenoxy)phenyl]-2-hydroxy-3-(1H-1,2,4-triazol-1-yl)propanoate + SX, ethyl 2-[2-chloro-4-(4-chlorophenoxy)phenyl]-2-hydroxy-3-(1H-1,2,4-triazol-1-yl)propanoate + SX, methyl 2-[2-(trifluoromethyl)-4-(4-chlorophenoxy)phenyl]-2-hydroxy-3-(1H-1,2,4-triazol-1-yl)propanoate + SX, 1-(2,3-dimethylpyridin-5-yl)-4,4-difluoro-3,3-dimethyl-3,4-dihydroisoquinoline + SX, 1-[2-(difluoromethyl)-3-methylpyridin-5-yl]-4,4-difluoro-3,3-dimethyl-3,4-dihydroisoquinoline + SX, 2,2-difluoro-N-[6-({[1-(1-methyl-1H-tetrazol-5-yl)benzimidazol-2-yl]oxy}methyl)pyridin-2-yl]-2-phenoxyacetamide + SX, 1-[2-(1-chlorocyclopropyl)-3-(3-chloro-2-fluorophenyl)-2-hydroxypropyl]-1H-imidazole-5-carbonitrile + SX, ethyl 1-[(4-{[2-(trifluoromethyl)-1,3-dioxolan-2-yl]methoxy}phenyl)methyl]-1H-pyrazole-4-carboxylate + SX, ethyl 1-[(4-{[(1Z)-2-ethoxy-3,3,3-trifluoro-1-propen-1-yl]oxy}phenyl)methyl]-1H-pyrazole-4-carboxylate + SX, 6-chloro-3-(3-cyclopropyl-2-fluorophenoxy)-N-[2-(2,4-dimethylphenyl)-2,2-difluoroethyl]-5-methylpyridazine-4-carboxamide + SX, 6-chloro-3-(3-cyclopropyl-2-fluorophenoxy)-N-[2-(3,4-dimethylphenyl)-2,2-difluoroethyl]-5-methylpyridazine-4-carboxamide + SX, 6-chloro-N-[2-(2-chloro-4-methylphenyl)-2,2-difluoroethyl]-3-(3-cyclopropyl-2-fluorophenoxy)-5-methylpyridazine-4-carboxamide + SX, 2-[cyano(2,6-difluoropyridin-4-yl)amino]-N-(2,2-dimethylcyclobutyl)-5-methylthiazole-4-carboxamide + SX, 2-[cyano(2,6-difluoropyridin-4-yl)amino]-N-(spiro[3.4]octan-1-yl)-5-methylthiazole-4-carboxamide + SX, 2-[cyano(2,6-difluoropyridin-4-yl)amino]-N-hexyl-5-methylthiazole-4-carboxamide + SX, 2-[acetyl(2,6-difluoropyridin-4-yl)amino]-N-(2,2-dimethylcyclobutyl)-5-methylthiazole-4-carboxamide + SX, 2-[(2-methoxyacetyl)(2,6-difluoropyridin-4-yl)amino]-N-(2,2-dimethylcyclobutyl)-5-methylthiazole-4-carboxamide + SX, 2-[(2-methylpropanoyl)(2,6-difluoropyridin-4-yl)amino]-N-(2,2-dimethylcyclobutyl)-5-methylthiazole-4-carboxamide + SX, 2-[(2,6-difluoropyridin-4-yl)amino]-N-(2,2-dimethylcyclobutyl)-5-methylthiazole-4-carboxamide + SX, 2-{[(oxetan-3-yl)carbonyl](2,6-difluoropyridin-4-yl)amino}-N-(2,2-dimethylcyclobutyl)-5-methylthiazole-4-carboxamide + SX, 2-{[(oxolan-3-yl)carbonyl](2,6-difluoropyridin-4-yl)amino}-N-(2,2-dimethylcyclobutyl)-5-methylthiazole-4-carboxamide + SX, 2-{[(oxan-4-yl)carbonyl](2,6-difluoropyridin-4-yl)amino}-N-(2,2-dimethylcyclobutyl)-5-methylthiazole-4-carboxamide + SX, 5-[5-(difluoromethyl)-1,3,4-oxadiazol-2-yl]-N-[1-(2-fluorophenyl)ethyl]pyrimidin-2-amine + SX, 5-[5-(difluoromethyl)-1,3,4-oxadiazol-2-yl]-N-[1-(2,6-difluorophenyl)ethyl]pyrimidin-2-amine + SX, 5-[5-(difluoromethyl)-1,3,4-oxadiazol-2-yl]-N-[1-(3,5-difluorophenyl)ethyl]pyrimidin-2-amine + SX, 5-[5-(difluoromethyl)-1,3,4-oxadiazol-2-yl]-N-[1-(6-chloropyridin-3-yl)ethyl]pyrimidin-2-amine + SX, 5-[5-(difluoromethyl)-1,3,4-oxadiazol-2-yl]-N-[1-(2-fluorophenyl)cyclopropyl]pyrimidin-2-amine + SX, 5-[5-(difluoromethyl)-1,3,4-oxadiazol-2-yl]-N-[1-(2,6-difluorophenyl)cyclopropyl]pyrimidin-2-amine + SX, 5-[5-(difluoromethyl)-1,3,4-oxadiazol-2-yl]-N-[1-(2-fluoro-3-methoxyphenyl)cyclopropyl]pyrimidin-2-amine + SX, 5-[5-(difluoromethyl)-1,3,4-oxadiazol-2-yl]-2-{[1-(2,6-difluorophenyl)cyclopropyl]oxy}pyrimidine + SX, 3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, (5S)-3-[3-(3-cyclopropyl-2-fluorophenoxy)-6-methylpyridazin-4-yl]-5-[(2,4-dimethylphenyl)methyl]-5,6-dihydro-4H-1,2,4-oxadiazine + SX, 3-[3-(3-chloro-2-fluorophenoxy)-6-methylpyridazin-4-yl]-5-[(4-bromo-2-methylphenyl)methyl]-5,6-dihydro-4H-1,2,4-oxadiazine + SX, 3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5S)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, (5R)-3-[3-(3-chloro-2-fluorophenoxy)-6-methylpyridazin-4-yl]-5-[(2-chloro-4-methylphenyl)methyl]-5,6-dihydro-4H-1,2,4-oxadiazine + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2-methylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}-3-methylbutan-2-yl)-2,2-dimethylpropanamide + SX, N-((2S)-1-{3-[2-(5-fluoro-2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(2-methoxyphenyl)-2-hydroxyethyl]-5-[(E)-1-(isopropoxyimino)ethyl]-2,6-dioxo-3,6-dihydropyrimidin-1(2H)-yl}propan-2-yl)-2-methylpropanamide + SX, N-((2S)-1-{3-[2-(5-fluoro-2-methoxyphenyl)-2-(2-cyanoethoxy)ethyl]-5-[1-(isopropoxyimino)ethyl]-2,6-dioxo-3,6-dihydropyrimidin-1(2H)-yl}propan-2-yl)-2-methylpropanamide + SX, methyl ({5-[1-(2,6-difluoro-4-isopropylphenyl)-1H-pyrazol-3-yl]-2-methylphenyl}methyl)carbamate + SX, methyl ({5-[1-(2,6-difluoro-4-cyclopropylphenyl)-1H-pyrazol-3-yl]-2-methylphenyl}methyl)carbamate + SX, methyl ({5-[1-(2,6-difluoro-4-methoxyphenyl)-1H-pyrazol-3-yl]-2-methylphenyl}methyl)carbamate + SX, methyl (Z)-2-(5-cyclopentyl-2-methylphenoxy)-3-methoxyprop-2-enoate + SX, methyl (Z)-2-(5-cyclohexyl-2-methylphenoxy)-3-methoxyprop-2-enoate + SX, methyl (Z)-2-[(3-isopropyl-1H-pyrazol-1-yl)-2-methylphenoxy]-3-methoxyprop-2-enoate + SX, 1-(4,5-dimethyl-1H-benzimidazol-1-yl)-4,4-difluoro-3,3-dimethyl-3,4-dihydroisoquinoline + SX, 1-(4,5-dimethyl-1H-benzimidazol-1-yl)-4,4,5-trifluoro-3,3-dimethyl-3,4-dihydroisoquinoline + SX, 1-(pyrazolo[1,5-a]pyridin-3-yl)-4,4-difluoro-3,3-dimethyl-3,4-dihydroisoquinoline + SX, 1-(6,7-dimethylpyrazolo[1,5-a]pyridin-3-yl)-6-fluoro-3,3-dimethylisoquinolin-4(3H)-one + SX, methyl (2Z)-3-methoxy-2-[(4-methyl[1,1'-biphenyl]-3-yl)oxy]prop-2-enoate + SX, Agrobacterium radiobactor strain K1026 + SX, Agrobacterium radiobactor strain K84 + SX, Bacillus amyloliquefaciens strain PTA-4838 (Aveo (registered trademark) EZ Nematicide) + SX, Bacillus amyloliquefaciens strain AT332 + SX, Bacillus amyloliquefaciens strain B3 + SX, Bacillus amyloliquefaciens strain D747 + SX, Bacillus amyloliquefaciens strain DB101 + SX, Bacillus amyloliquefaciens strain DB102 + SX, Bacillus amyloliquefaciens strain GB03 + SX, Bacillus amyloliquefaciens strain FZB24 + SX, Bacillus amyloliquefaciens strain FZB42 + SX, Bacillus amyloliquefaciens strain IN937a + SX, Bacillus amyloliquefaciens strain MBI600 + SX, Bacillus amyloliquefaciens strain QST713 + SX, Bacillus amyloliquefaciens isolate strain B246 + SX, Bacillus amyloliquefaciens strain F727 + SX, Bacillus amyloliquefaciens subsp. plantarum strain D747 + SX, Bacillus licheniformis strain HB-2 + SX, Bacillus licheniformis strain SB3086 + SX, Bacillus pumilus strain AQ717 + SX, Bacillus pumilus strain BUF-33 + SX, Bacillus pumilus strain GB34 + SX, Bacillus pumilus strain QST2808 + SX, Bacillus simplex strain CGF2856 + SX, Bacillus subtilis strain AQ153 + SX, Bacillus subtilis strain AQ743 + SX, Bacillus subtilis strain BU1814 + SX, Bacillus subtilis strain D747 + SX, Bacillus subtilis strain DB101 + SX, Bacillus subtilis strain FZB24 + SX, Bacillus subtilis strain GB03 + SX, Bacillus subtilis strain HAI0404 + SX, Bacillus subtilis strain IAB/BS03 + SX, Bacillus subtilis strain MBI600 + SX, Bacillus subtilis strain QST30002/AQ30002 + SX, Bacillus subtilis strain QST30004/AQ30004 + SX, Bacillus subtilis strain QST713 + SX, Bacillus subtilis strain QST714 + SX, Bacillus subtilis var. Amyloliquefaciens strain FZB24 + SX, Bacillus subtilis strain Y1336 + SX, Burkholderia cepacia + SX, Burkholderia cepacia type Wisconsin strain J82 + SX, Burkholderia cepacia type Wisconsin strain M54 + SX, Candida oleophila strain O + SX, Candida saitoana + SX, Chaetomium cupreum + SX, Clonostachys rosea + SX, Coniothyrium minitans strain CGMCC8325 + SX, Coniothyrium minitans strain CON/M/91-8 + SX, cryptococcus albidus + SX, Erwinia carotovora subsp. carotovora strain CGE234M403 + SX, Fusarium oxysporum strain Fo47 + SX, Gliocladium catenulatum strain J1446 + SX, Paenibacillus polymyxa strain AC-1 + SX, Paenibacillus polymyxa strain BS-0105 + SX, Pantoea agglomerans strain E325 + SX, Phlebiopsis gigantea strain VRA1992 + SX, Pseudomonas aureofaciens strain TX-1 + SX, Pseudomonas chlororaphis strain 63-28 + SX, Pseudomonas chlororaphis strain AFS009 + SX, Pseudomonas chlororaphis strain MA342 + SX, Pseudomonas fluorescens strain 1629RS + SX, Pseudomonas fluorescens strain A506 + SX, Pseudomonas fluorescens strain CL145A + SX, Pseudomonas fluorescens strain G7090 + SX, Pseudomonas sp. strain CAB-02 + SX, Pseudomonas syringae strain 742RS + SX, Pseudomonas syringae strain MA-4 + SX, Pseudozyma flocculosa strain PF-A22UL + SX, Pseudomonas rhodesiae strain HAI-0804 + SX, Pythium oligandrum strain DV74 + SX, Pythium oligandrum strain M1 + SX, Streptomyces griseoviridis strain K61 + SX, Streptomyces lydicus strain WYCD108US + SX, Streptomyces lydicus strain WYEC108 + SX, Talaromyces flavus strain SAY-Y-94-01 + SX, Talaromyces flavus strain V117b + SX, Trichoderma asperellum strain ICC012 + SX, Trichoderma asperellum SKT-1 + SX, Trichoderma asperellum strain T25 + SX, Trichoderma asperellum strain T34 + SX, Trichoderma asperellum strain TV1 + SX, Trichoderma atroviride strain CNCM 1-1237 + SX, Trichoderma atroviride strain LC52 + SX, Trichoderma atroviride strain IMI 206040 + SX, Trichoderma atroviride strain SC1 + SX, Trichoderma atroviride strain SKT-1 + SX, Trichoderma atroviride strain T11 + SX, Trichoderma gamsii strain ICC080 + SX, Trichoderma harzianum strain 21 + SX, Trichoderma harzianum strain DB104 + SX, Trichoderma harzianum strain DSM 14944 + SX, Trichoderma harzianum strain ESALQ-1303 + SX, Trichoderma harzianum strain ESALQ-1306 + SX, Trichoderma harzianum strain IIHR-Th-2 + SX, Trichoderma harzianum strain ITEM908 + SX, Trichoderma harzianum strain kd + SX, Trichoderma harzianum strain MO1 + SX, Trichoderma harzianum strain SF + SX, Trichoderma harzianum strain T22 + SX, Trichoderma harzianum strain T39 + SX, Trichoderma harzianum strain T78 + SX, Trichoderma harzianum strain TH35 + SX, Trichoderma polysporum strain IMI206039 + SX, trichoderma stromaticum + SX, Trichoderma virens strain G-41 + SX, Trichoderma virens strain GL-21 + SX, Trichoderma viride + SX, Variovorax paradoxus strain CGF4526 + SX, Harpin protein + SX.

Combination of the Present ingredient in the above Group (c) and the Compound of the present invention:
1-methylcyclopropene + SX, 1,3-diphenylurea + SX, 2,3,5-triiodobenzoic acid + SX, IAA ((1H-indol-3-yl)acetic acid) + SX, IBA (4-(1H-indol-3-yl)butyric acid) + SX, MCPA (2-(4-chloro-2-methylphenoxy)acetic acid) + SX, MCPB (4-(4-chloro-2-methylphenoxy)butyric acid) + SX, 4-CPA (4-chlorophenoxyacetic acid) + SX, 5-aminolevulinic acid hydrochloride + SX, 6-benzylaminopurine + SX, abscisic acid + SX, AVG (aminoethoxyvinylglycine) + SX, anisiflupurin + SX, ancymidol + SX, butralin + SX, calcium carbonate + SX, calcium chloride + SX, calcium formate + SX, calcium peroxide + SX, calcium polysulfide + SX, calcium sulfate + SX, chlormequat-chloride + SX, chlorpropham + SX, choline chloride + SX, cloprop + SX, cyanamide + SX, cyclanilide + SX, daminozide + SX, decan-1-ol + SX, dichlorprop + SX, dikegulac + SX, dimethipin + SX, diquat + SX, ethephon + SX, ethychlozate + SX, flumetralin + SX, flurprimidol + SX, forchlorfenuron + SX, formononetin + SX, Gibberellin A + SX, Gibberellin A3 + SX, inabenfide + SX, Kinetin + SX, lipochitooligosaccharide SP104 + SX, maleic hydrazide + SX, mefluidide + SX, mepiquat-chloride + SX, oxidized glutathione + SX, paclobutrazol + SX, pendimethalin + SX, prohexadione-calcium + SX, prohydrojasmon + SX, pyraflufen-ethyl + SX, sintofen + SX, sodium 1-naphthaleneacetate + SX, sodium cyanate + SX, thidiazuron + SX, triapenthenol + SX, tribufos + SX, trinexapac-ethyl + SX, uniconazole-P + SX, 2-(naphthalen-1-yl)acetamide + SX, [4-oxo-4-(2-phenylethyl)amino]butylate + SX, methyl 5-(trifluoromethyl)benzo[b]thiophene-2-carboxylate + SX, 3-[(6-chloro-4-phenylquinazolin-2-yl)amino]propan-1-ol + SX, Claroideoglomus etunicatum + SX, Claroideoglomus claroideum + SX, Funneliformis mosseae + SX, Gigaspora margarita + SX, Gigaspora rosea + SX, Glomus aggregatum + SX, Glomus deserticola + SX, Glomus monosporum + SX, Paraglomus brasillianum + SX, Rhizophagus clarus + SX, Rhizophagus intraradices RTI-801 + SX, Rhizophagus irregularis DAOM 197198 + SX, Azorhizobium caulinodans + SX, Azospirillum amazonense + SX, Azospirillum brasilense XOH + SX, Azospirillum brasilense Ab-V5 + SX, Azospirillum brasilense Ab-V6 + SX, Azospirillum caulinodans + SX, Azospirillum halopraeferens + SX, Azospirillum irakense + SX, Azospirillum lipoferum + SX, Bradyrhizobium elkanii SEMIA 587 + SX, Bradyrhizobium elkanii SEMIA 5019 + SX, Bradyrhizobium japonicum TA-11 + SX, Bradyrhizobium japonicum USDA 110 + SX, Bradyrhizobium liaoningense + SX, Bradyrhizobium lupini + SX, Delftia acidovorans RAY209 + SX, Mesorhizobium ciceri + SX, Mesorhizobium huakii + SX, Mesorhizobium loti + SX, Rhizobium etli + SX, Rhizobium galegae + SX, Rhizobium leguminosarum bv. Phaseoli + SX, Rhizobium leguminosarum bv. Trifolii + SX, Rhizobium leguminosarum bv. Viciae + SX, Rhizobium trifolii + SX, Rhizobium tropici + SX, Sinorhizobium fredii + SX, Sinorhizobium meliloti + SX, Zucchini Yellow Mosaik Virus weak strain + SX.

Combination of the Present ingredient in the above Group (d) and the Compound of the present invention:
anthraquinone + SX, deet + SX, icaridin + SX.

The ratio of the Compound of the present invention to the Present ingredient includes, but not limited thereto, as a ratio by weight (the Compound of the present invention : the Present ingredient) 1,000 : 1 to 1 : 1,000, 500 : 1 to 1 : 500, 100 : 1 to 1 : 100, 50 : 1, 20 : 1, 10 : 1, 9 : 1, 8 : 1, 7 : 1, 6 : 1, 5 : 1, 4 : 1, 3 : 1, 2 : 1, 1 : 1, 1 : 2, 1 : 3, 1 : 4, 1 : 5, 1 : 6, 1 : 7, 1 : 8, 1 : 9, 1 : 10, 1 : 20, 1 : 50, and the others.

The Present Compound, the Compound of the present invention, or the Composition A can control plant diseases caused by plant pathogenic microorganisms such as fungi, Oomycete, Phytomyxea, and bacteria. Examples of the fungi include Ascomycota, Basidiomycota, Blastocladiomycota, Chytridiomycota, Mucoromycota, and Olpidiomycota. Specific examples thereof include the followings. The scientific name of plant pathogenic microorganism which causes each disease is shown in parentheses.
Rice diseases:
   blast (Pyricularia oryzae), brown spot (Cochliobolus miyabeanus), sheath blight (Rhizoctonia solani), bakanae disease (Gibberella fujikuroi), downy mildew (Sclerophthora macrospora), false blast and head blight (Epicoccum nigrum), seedling blight (Trichoderma viride and Rhizopus oryzae), pseudo sheath blight (Waitea circinate, Ceratobasidium setariae, and Thanatephorus cucumeris), Rice false smut (Ustilaginoidea virens), and Kernel smut (Tilletia horrida and Tilletia barclayana);
Wheat diseases:
   powdery mildew (Blumeria graminis), fusarium blight (Fusarium graminearum, Fusarium avenaceum, Fusarium culmorum, and Microdochium nivale), stripe rust (Puccinia striiformis), stem rust (Puccinia graminis), leaf rust (Puccinia recondita), snow mould (Microdochium nivale and Microdochium majus), typhula snow blight (Typhula incarnata and Typhula ishikariensis), Sclerotinia snow blight (Sclerotinia borealis), Browning root rot (Pythium spp.), loose smut (Ustilago tritici), stinking smut (Tilletia caries and Tilletia controversa), eyespot (Pseudocercosporella herpotrichoides), leaf blotch (Septoria tritici), glume blotch (Stagonospora nodorum), tan spot (Pyrenophora tritici-repentis), rhizoctonia seeding blight (Rhizoctonia solani), take-all disease (Gaeumannomyces graminis), and blast (Pyricularia graminis-tritici);
Barley diseases:
   powdery mildew (Blumeria graminis), fusarium head blight (Fusarium graminearum, Fusarium avenaceum, Fusarium culmorum, and Microdochium nivale), stripe rust (Puccinia striiformis), stem rust (Puccinia graminis), dwarf leaf rust (Puccinia hordei), loose smut (Ustilago nuda), scald (Rhynchosporium secalis), net blotch (Pyrenophora teres), spot blotch (Cochliobolus sativus), stripe (Pyrenophora graminea), Ramularia disease (Ramularia collo-cygni), and rhizoctonia seeding blight (Rhizoctonia solani);
Corn diseases:
   rust (Puccinia sorghi), southern rust (Puccinia polysora), northern leaf blight (Setosphaeria turcica), tropical rust (Physopella zeae), southern leaf blight (Cochliobolus heterostrophus), anthracnose (Colletotrichum graminicola), gray leaf spot (Cercospora zeae-maydis), eyespot (Kabatiella zeae), phaeosphaeria leaf spot (Phaeosphaeria maydis), diplodia rot (Stenocarpella maydis and Stenocarpella macrospora), stalk rot (Fusarium graminearum, Fusarium verticilioides, and Colletotrichum graminicola), smut (Ustilago maydis), Physoderma brown spot and Physoderma stalk rot (Physoderma maydis), and tar spot disease (Phyllachora maydis);
Cotton diseases:
   anthracnose (Colletotrichum gossypii), grey mildew (Ramularia areola), alternaria leaf spot (Alternaria macrospora and Alternaria gossypii), and black root rot (Thielaviopsis basicola);
Coffee diseases:
   rust (Hemileia vastatrix) and leaf spot (Cercospora coffeicola);
Rape seed diseases:
   sclerotinia rot (Sclerotinia sclerotiorum), gray leaf spot (Alternaria brassicae), root rot (Phoma lingam), and light leaf spot (Pyrenopeziza brassicae);
Sugar cane diseases:
   rust (Puccinia melanocephela and Puccinia kuehnii), and smut (Ustilago scitaminea);
Sunflower diseases:
   rust (Puccinia helianthi) and downy mildew (Plasmopara halstedii);
Citrus diseases:
   melanose (Diaporthe citri), scab (Elsinoe fawcetti), green mold (Penicillium digitatum), blue mold (Penicillium italicum), Phytophthora rot (Phytophthora parasitica and Phytophthora citrophthora), aspergillus rot (Aspergillus niger);
Apple diseases:
   blossom blight (Monilinia mali), valsa canker (Valsa ceratosperma), powdery mildew (Podosphaera leucotricha), alternaria leaf spot (Alternaria alternata apple pathotype), scab (Venturia inaequalis), bitter rot (Glomerella cingulata and Colletotrichum acutatum), blotch (Diplocarpon mali), ring rot (Botryosphaeria berengeriana), crown rot (Phytophtora cactorum), and rust (Gymnosporangium juniperi-virginianae and Gymnosporangium yamadae);
Pear diseases:
   scab (Venturia nashicola and Venturia pirina), black spot (Alternaria alternata Japanese pear pathotype), and rust (Gymnosporangium haraeanum);
Peach diseases:
   brown rot (Monilinia fructicola), scab (Cladosporium carpophilum), Phomopsis rot (Phomopsis sp.), leaf curl (Taphrina deformans), and powdery mildew (Podosphaera leucotricha);
Grapes diseases:
   anthracnose (Elsinoe ampelina), ripe rot (Glomerella cingulata and Colletotrichum acutatum), powdery mildew (Uncinula necator), rust (Neophysopella sp.), black rot (Guignardia bidwellii), downy mildew (Plasmopara viticola), leaf blight (Pseudocercospora vitis), and white rot (Coniella castaneicola);
Japanese persimmon diseases:
   anthracnose (Gloeosporium kaki and Colletotrichum acutatum), leaf spot (Cercospora kaki and Mycosphaerella nawae), and powdery mildew (Phyllactinia kakicola);
Fig disease:
   rust (Phakopsora nishidana);
Diseases of gourd family:
   anthracnose (Colletotrichum lagenarium), powdery mildew (Sphaerotheca fuliginea), gummy stem blight (Didymella bryoniae), Corynespora leaf spot (Corynespora cassiicola), fusarium wilt (Fusarium oxysporum), downy mildew (Pseudoperonospora cubensis), phytophthora rot (Phytophthora capsici), damping-off (Pythium sp.), and Black root rot (Phomopsis sp.);
Tomato diseases:
   early blight (Alternaria solani), leaf mold (Cladosporium fulvum), Cercospora leaf mold (Pseudocercospora fuligena), late blight (Phytophthora infestans), and powdery mildew (Leveillula taurica);
Eggplant diseases:
   brown spot (Phomopsis vexans), powdery mildew (Erysiphe cichoracearum), black blight (Corynespora melongenae), leaf mold (Mycovellosiella nattrassii), and brown leaf spot (Thanatephorus cucumeris);
Cruciferous vegetables diseases:
   alternaria leaf spot (Alternaria japonica), white spot (Cercosporella brassicae), clubroot (Plasmodiophora brassicae), downy mildew (Peronospora parasitica), white rust (Albugo candida), and Alternaria sooty spot (Alternaria brassicicola);
Welsh onion diseases:
   rust (Puccinia allii), black spot (Alternaria porri), white rot (Sclerotium cepivorum), leaf blight (Botrytis squamosa), leaf spot (Stemphylium vesicarium and Stemphylium botryosum), southern blight (Sclerotium rolfsii), leaf blight (Botrytis squamosa);
Soybean diseases:
   purple stain (Cercospora kikuchii), sphaceloma scab (Elsinoe glycines), pod and stem blight (Diaporthe phaseolorum var. sojae), rust (Phakopsora pachyrhizi), target spot (Corynespora cassiicola), anthracnose (Colletotrichum glycines and Colletotrichum truncatum), Rhizoctonia rot (Rhizoctonia solani), septoria brown spot (Septoria glycines), Cercospora leaf spot (Cercospora sojina), stem rot (Sclerotinia sclerotiorum), powdery mildew (Microsphaera diffusa), phytophthora stem and root rot (Phytophthora sojae), downy mildew (Peronospora manshurica), sudden death syndrome (Fusarium virguliforme), red crown rot (Calonectria ilicicola), and Diaporthe/Phomopsis complex (Diaporthe longicolla, Diaporthe phaseolorum, Phomopsis longicolla), and pod anomalies caused by various causative bacteria;
Kidney bean diseases:
   stem rot (Sclerotinia sclerotiorum), rust (Uromyces appendiculatus), angular leaf spot (Phaeoisariopsis griseola), anthracnose (Colletotrichum lindemuthianum), and Fusarium root-rot (Fusarium solani);
Peanut diseases:
   leaf spot (Cercospora personata), brown leaf spot (Cercospora arachidicola), southern blight (Sclerotium rolfsii), and Cylindrocladium black rot (Calonectria ilicicola) ;
Garden pea diseases:
   powdery mildew (Erysiphe pisi) and root rot (Fusarium solani);
Potato diseases:
   early blight (Alternaria solani), late blight (Phytophthora infestans), Pink rot (Phytophthora erythroseptica), powdery scab (Spongospora subterranea f. sp. subterranea), verticillium wilt (Verticillium albo-atrum, Verticillium dahliae, and Verticillium nigrescens), dry rot (Fusarium solani), and potato wart (Synchytrium endobioticum);
Strawberry diseases:
   powdery mildew (Sphaerotheca humuli) and crown rot (Glomerella cingulata and Colletotrichum acutatum);
Tea diseases:
   net blister blight (Exobasidium reticulatum), white scab (Elsinoe leucospila), gray blight (Pestalotiopsis sp.), and anthracnose (Colletotrichum theae-sinensis);
Tobacco diseases:
   brown spot (Alternaria longipes), anthracnose (Colletotrichum tabacum), blue mold (Peronospora tabacina), and black shank (Phytophthora nicotianae);
Sugar beet diseases:
   cercospora leaf spot (Cercospora beticola), leaf blight (Thanatephorus cucumeris), root rot (Thanatephorus cucumeris), aphanomyces root rot (Aphanomyces cochlioides), and rust (Uromyces betae);
Rose diseases:
   black spot (Diplocarpon rosae) and powdery mildew (Sphaerotheca pannosa);
Chrysanthemum diseases:
   leaf blight (Septoria chrysanthemi-indici) and white rust (Puccinia horiana);
Onion diseases:
   botrytis leaf blight (Botrytis cinerea, Botrytis byssoidea, and Botrytis squamosa), gray-mold neck rot (Botrytis allii), and small sclerotial neck rot (Botrytis squamosa);
Various crops diseases:
   Botrytis rot (Botrytis cinerea), sclerotinia rot (Sclerotinia sclerotiorum), white rot (Sclerotium cepivorum), southern blight (Sclerotium rolfsii), and seedling blight (Pythium aphanidermatum, Pythium irregulare, and Pythium ultimum);
Japanese radish disease:
   alternaria leaf spot (Alternaria brassicicola);
Sweet potato diseases:
   stem rot (Fusarium oxysporum f. sp. batatas), foot rot (Diaporthe destruens), and black rot (Ceratocystis fimbriata);
Turfgrass diseases:
   dollar spot (Sclerotinia homoeocarpa), brown patch and large patch (Rhizoctonia solani), pythium blight (Pythium aphanidermatum), and anthracnose (Colletotrichum caudatum);
Banana disease:
   Sigatoka disease (Mycosphaerella fijiensis and Mycosphaerella musicola);
Lentils disease:
   ascochyta blight (Ascochyta lentis);
Chickpea disease:
   ascochyta blight (Ascochyta rabiei);
Green pepper diseases:
   anthracnose (Colletotrichum scovillei), powdery mildew (Leveillula taurica), and southern blight (Sclerotium rolfsii);
Mango disease:
   anthracnose (Colletotrichum acutatum);
Cacao diseases:
   Witches' broom (Moniliophthora perniciosa) and Frosty pod rot (Moniliophthora roreri);
Pecan disease:
   scab (Venturia effusa);
Lychee diseases:
   downy mildew (Peronophythora litchii) and anthracnose (Colletotrichum siemense);
Fruit trees diseases:
   white root rot (Rosellinia necatrix) and violet root rot (Helicobasidium mompa);
Postharvest disease of fruits (for example, apple and pear) :
   Mucor rot diseases (Mucor piriformis);
Seed diseases or diseases in the early stages of the growth of various plants caused by Aspergillus spp., Penicillium spp., Fusarium spp., Gibberella spp., Tricoderma spp., Thielaviopsis spp., Rhizopus spp., Mucor spp., Corticium spp., Phoma spp., Rhizoctonia spp. or Diplodia spp., or the like;
Viral diseases:
   Lettuce big-vein disease transmitted by Olpidium brassicae, and viral diseases of several crops transmitted by Polymyxa spp. (e.g., Polymyxa betae and Polymyxa graminis);
Diseases caused by bacteria:
   bacterial seedling blight of rice (Burkholderia plantarii), bacterial palea browning of rice (Pantoea ananatis), Bacterial leaf blight of rice (Xanthomonas oryzae pv. oryzae.), bacterial spot of cucumber (Pseudomonas syringae pv. lachrymans), bacterial wilt of eggplant (Ralstonia solanacearum), canker of citrus (Xanthomonas citri), bacterial soft rot of Chinese cabbage (Erwinia carotovora), scab of potato (Streptomyces scabiei), black leg of potato (Pectobacterium carotovorum and Dickeya sp.), bacterial leaf spot of peach (Pseudomona syringae, Erwinia nigrifluens, and Xanthomonas campestris), bacterial canker of Japanese apricot (Prunus mume) (Pseudomonas syringae pv. morsprunorum and Erwinia sp.), Goss's wilt of corn (Clavibacter michiganensis), Pierce's disease of grapes, olive, peach, and the like (Xylella fastidiosa), and crown gall of Rosaceae plants such as apple, peach, and cherries (Agrobacterium tumefaciens) ;
and the others.

A rust fungus of soybean having an amino acid substitution of F129L in the mitochondrial cytochrome b protein means a rust fungus of soybean (scientific name: Phakopsora pachyrhizi) which has a mutation in the mitochondrial cytochrome b gene encoding the mitochondrial cytochrome protein, and as a result of said mutation, has an amino acid substitution of F129L, thereby shows resistance to QoI fungicides.

One embodiment of the present invention is a method for controlling soybean rust fungus by applying the Present compound to a plant or soil for cultivating a plant

Examples of the method for controlling a plant disease of the present invention include the method for controlling a plant disease of the present invention is carried out by applying an effective amount of the Present compound, the Compound of the present invention, or the Composition A to a plant or soil for cultivating a plant. Examples of the treatment method include foliage treatment, soil treatment, and seed treatment.

The Present compound, the Compound of the present invention, or the Composition A is usually used by mixing it with inert carrier(s) such as solid carrier(s) and liquid carrier(s), surfactant(s), and the like, and as needed, adding thereto auxiliary agent(s) for formulation such as binder(s), dispersant(s), and stabilizer(s) to be formulated into an aqueous suspension formulation, an oily suspension formulation, an oil solution, an emulsifiable concentrate, an emulsion formulation, a microemulsion formulation, a microcapsule formulation, a wettable powder, a granular wettable powder, a dust formulation, a granule, or the like. In addition to these formulations, the Present compound or the Composition A may be used by formulating it into a dosage form described in Manual on development and use of FAO and WHO Specifications for pesticides, FAO Plant Production and Protection Papers- 271-276, prepared by the FAO/WHO Joint Meeting on Pesticide Specifications, 2016, ISSN:0259-2517.

These formulations usually comprise 0.0001% to 99% by weight ratio of the Present compound, the Compound of the present invention, or the Composition A.

Examples of the solid carrier include fine powders and granules of clays (for example, pyrophyllite clay and kaolin clay), talc, calcium carbonate, diatomaceous earth, zeolite, bentonite, acid white clay, attapulgite, white carbon, ammonium sulfate, vermiculite, perlite, pumice, silica sand, chemical fertilizers (for example, ammonium sulfate, ammonium phosphate, ammonium nitrate, urea, and ammonium chloride), and the others; as well as resins (for example, polyethylene, polypropylene, polyester, polyurethane, polyamide, and polyvinyl chloride).

Examples of the liquid carrier include water, alcohols (for example, ethanol, cyclohexanol, benzyl alcohol, propylene glycol, and polyethylene glycol), ketones (for example, acetone and cyclohexanone), aromatic hydrocarbons (for example, xylene, phenyl xylyl ethane, and methylnaphthalene), aliphatic hydrocarbons (for example, hexane and cyclohexane), esters (for example, ethyl acetate, methyl oleate, and propylene carbonate), nitriles (for example, acetonitrile), ethers (for example, ethylene glycol dimethyl ether), amides (for example, N,N-dimethylformamide and N,N-dimethyloctanamide), sulfoxides (for example, dimethyl sulfoxide), lactams (for example, N-methylpyrrolidone and N-octylpyrrolidone), fatty acids (for example, oleic acid), and vegetable oils (for example, soybean oil).

Examples of the surfactant include nonionic surfactants (for example, polyoxyethylene alkyl ethers, polyoxyethylene alkyl aryl ethers, and polyethylene glycol fatty acid esters), and anionic surfactants (for example, alkyl sulfonates, alkyl aryl sulfonates, and alkyl sulfates).

Examples of the other auxiliary agent for formulation include binders, dispersants, colorants, and stabilizers, and the specific examples thereof include polysaccharides (for example, starch, gum arabic, cellulose derivatives, and alginic acid), lignin derivatives, water-soluble synthetic polymers (for example, polyvinyl alcohol, polyvinyl pyrrolidone, and polyacrylic acids), acidic isopropyl phosphate, and dibutylhydroxytoluene.

Also, adjuvant(s) may be used as ingredient(s) for enhancing or assisting the efficacy of the Present compound, the Compound of the present invention, or the Composition A. Specific examples thereof include Nimbus (registered trademark), Assist (registered trademark), Aureo (registered trademark), Iharol (registered trademark), Silwet L-77 (registered trademark), BreakThru (registered trademark), SundanceII (registered trademark), Induce (registered trademark), Penetrator (registered trademark), AgriDex (registered trademark), Lutensol A8 (registered trademark), NP-7 (registered trademark), Triton (registered trademark), Nufilm (registered trademark), Emulgator NP7 (registered trademark), Emulad (registered trademark), TRITON X 45 (registered trademark), AGRAL 90 (registered trademark), AGROTIN (registered trademark), ARPON (registered trademark), EnSpray N (registered trademark), and BANOLE (registered trademark).

In the present invention, the plants include whole plants and specific parts of plants. Examples of the specific parts of plants include foliages, flowers, ears, fruits, tree stems, branches, tree crowns, seeds, vegetative reproductive organs, and nursery plants.

A vegetative reproductive organ means a part of plant such as root, stem, and leaf which has a growth capability even when said part is separated from the plant body and placed into soil. Examples of the vegetative reproductive organ include tuberous root, creeping root, bulb, corm or solid bulb, tuber, rhizome, stolon, rhizophore, cane cuttings, propagule, and vine cutting. Stolon is also referred to as "runner", and propagule is also referred to as "propagulum" and categorized into broad bud and bulbil. Vine cutting means a shoot (collective term of leaf and stem) of sweet potato, glutinous yam, or the like. Bulb, corm or solid bulb, tuber, rhizome, cane cuttings, rhizophore, and tuberous root are also collectively referred to as "bulb". For example, cultivation of potato starts with planting a tuber into soil, and the tuber to be used is generally referred to as "seed potato".

Nursery plants include seedlings and saplings.

Examples of a method for controlling plant diseases by applying an effective amount of the Present compound, the Compound of the present invention, or the Composition A to soil include a method of applying an effective amount of the Present compound, the Compound of the present invention, or the Composition A to soil before planting plants or after planting plants. Specifically, examples of the application method include planting hole treatment (for example, spraying into planting holes and soil mixing after planting hole treatment), plant foot treatment (for example, plant foot spraying, soil mixing after plant foot treatment, irrigation at plant foot, and plant foot treatment at a later seeding raising stage), planting furrow treatment (for example, planting furrow spraying and soil mixing after planting furrow treatment), planting row treatment (for example, planting row spraying, soil mixing after planting row treatment, and planting row spraying at a growing stage), planting row treatment at the time of sowing (for example, planting row spraying at the time of sowing and soil mixing after planting row treatment at the time of sowing), broadcast treatment (for example, overall soil surface spraying and soil mixing after broadcast treatment), side-article treatment, treatment of water surface (for example, application to water surface and application to water surface after flooding), other soil spraying treatment (for example, spraying of a granular formulation on leaves at a growing stage, spraying under a canopy or around a tree stem, spraying on the soil surface, mixing with surface soil, spraying into seed holes, spraying on the ground surfaces of furrows, and spraying between plants), other irrigation treatment (for example, soil irrigation, irrigation at a seedling raising stage, chemical solution injection treatment, irrigation of a plant part just above the ground, chemical solution drip irrigation, and chemigation), seedling raising box treatment (for example, spraying into a seedling raising box, irrigation of a seedling raising box, and flooding into a seedling raising box with chemical solution), seedling raising tray treatment (for example, spraying on a seedling raising tray, irrigation of a seedling raising tray, and flooding into a seedling raising tray with chemical solution), seedbed treatment (for example, spraying on a seedbed, irrigation of a seedbed, spraying on a lowland rice nursery, and immersion of seedlings), seedbed soil incorporation treatment (for example, mixing with seedbed soil, mixing with seedbed soil before sowing, spraying at sowing before covering with soil, spraying at sowing after covering with soil, and mixing with covering with soil), and other treatment (for example, mixing with culture soil, plowing under, mixing with surface soil, mixing with soil at the place where raindrops fall from a canopy, treatment at a planting position, spraying of a granule formulation on flower clusters, and mixing with a paste fertilizer).

Examples of the application to seeds (or seed treatments) include an application of the Present compound, the Compound of the present invention,, or the Composition A to seeds or vegetative reproductive organs, and specific examples thereof include spraying treatment in which a suspension of the Present compound, the Compound of the present invention, , or the Composition A is sprayed onto seed surface or the vegetative reproductive organ surface in the form of mist; smearing treatment in which the Present compound, the Compound of the present invention, , or the Composition A is coated on a surface of seeds or the vegetative reproductive organs; a soaking treatment in which the seeds or vegetative reproductive organs are soaked into the solution of the Present compound, the Compound of the present invention,, or the Composition A for a certain time; and a method for coating the seeds or the vegetative reproductive organs with a carrier containing the Present compound, the Compound of the present invention, , or the Composition A (for example, film coating treatment and pellet coating treatment). Examples of the above-described vegetative reproductive organ include particularly seed potato.

As used herein, seeds or vegetative reproductive organs holding the Present compound, the Compound of the present invention, or the Composition A mean seeds or vegetative reproductive organs in the state where the Present compound, the Compound of the present invention, or the Composition A is adhered to a surface of the seeds or the vegetative reproductive organs. The above-described seeds or vegetative reproductive organs holding the Present compound, the Compound of the present invention, or the Composition A may be adhered by any other materials that are different from the Present compound, the Compound of the present invention, or the Composition A before or after being adhered the Present compound, the Compound of the present invention, or the Composition A to the seeds or vegetative reproductive organs.

Also, when the Composition A is adhered in a form of layer(s) to a surface of seeds or vegetative reproductive organs, the layer(s) is/are composed of one layer or multiple layers. Also, when multiple layers are formed, each of the layer may be composed of a layer comprising one or more active ingredients, or a combination of a layer comprising one or more active ingredients and a layer not comprising an active ingredient.

Seeds or vegetative reproductive organs holding the Present compound, the Compound of the present invention, or the Composition A can be obtained, for example, by applying the formulations comprising Present compound, the Present Compound, the Compound of the present invention, or the Composition A by the above-described application method to seeds or vegetative reproductive organs.

The amount of the Present compound, the Compound of the present invention, or the Composition A to be applied varies depending on the climate condition, the dosage form, the application period, the application method, the application site, diseases to be controlled, crops to be protected, and the like, and is usually within the range of 1 g to 500 g per 1000 m² of area for cultivating plants in case of foliage treatment or soil treatment. In case of seed treatment, the amount of the Present compound, the Compound of the present invention, or the Composition A is usually within the range of 0.001 g to 100 g per 1 Kg of seed. When the Present compound, the Compound of the present invention, or the Composition A is formulated into an emulsifiable concentrate, a wettable powder, a flowable, or the like, such formulation is usually applied after diluting it with water in such a way that a concentration of the active ingredient is within the range of 0.01 ppm to 10000 ppm, and then sparging it. In the case of being formulated into a dust formulation, a granule, or the like, such formulation is usually used as itself without diluting it.

The Present compound, the Compound of the present invention, or the Composition A may be used as an agent for controlling plant diseases in croplands such as fields, paddy fields, grasses, and orchards. Examples of the plants include the followings.
corn (dent corn, flint corn, flour corn, popcorn, waxy corn, sweet corn, and field corn), rice (long grain rice, short grain rice, medium grain rice, japonica rice, tropical japonica rice, indica rice, javanica rice, paddy rice, upland rice, floating rice, direct-seeded rice, transplanted rice, and glutinous rice), wheat (bread wheat (hard wheat, soft wheat, medium wheat, red wheat, and white wheat), durum wheat, spelt wheat, and club wheat, winter wheat and spring wheat of them), barley (two-rowed barley (= barley for brewery), six-rowed barley, hull-less barley, and pearl barley, winter barley and spring barley of them), rye (winter rye and spring rye), triticale (winter triticale and spring triticale), oat (winter oat and spring oat), sorghum, cotton (upland cotton and Pima cotton), soybean (ripe seed harvest soybean, green soybeans, and early harvest soybeans, indeterminate type, determinate type, and semi-determinate type of them), peanut, buckwheat, beet (beets for sugar production, beets for feed, beets for root vegetable, beets for leaf vegetable, and beets for fuel), rapeseed (winter rapeseed and spring rapeseed), canola (winter canola and spring canola), sunflower (sunflowers for oil extraction, edible sunflowers, and sunflowers for ornamental purpose), sugar cane, tobacco, tea, mulberry, solanaceous vegetables (for example, eggplant, tomato, pimento, pepper, and potato), cucurbitaceous vegetables (for example, cucumber, pumpkin, zucchini, water melon, and melon), cruciferous vegetables (for example, Japanese radish, white turnip, horseradish, kohlrabi, Chinese cabbage, cabbage, leaf mustard, broccoli, and cauliflower), asteraceous vegetables (for example, burdock, crown daisy, artichoke, and lettuce), liliaceous vegetables (for example, welsh onion, onion, garlic, and asparagus), ammiaceous vegetables (for example, carrot, parsley, celery, and parsnip), chenopodiaceous vegetables (for example, spinach and Swiss chard), lamiaceous vegetables (for example, perilla, mint, and basil), strawberry, sweet potato, glutinous yam, eddoe, pomaceous fruits (for example, apple, pear, Japanese pear, Chinese white pear, Chinese quince, and quince), stone fleshy fruits (for example, peach, plum, nectarine, Japanese apricot (Prunus mume), cherry fruit, apricot, and prune), citrus fruits (for example, Citrus unshiu, orange, lemon, lime, and grapefruit), nuts (for example, chestnuts, walnuts, hazelnuts, almond, pistachio, cashew nuts, and macadamia nuts), berry fruits (for example, blueberry, cranberry, blackberry, and raspberry), grapes, Japanese persimmon, fig, olive, Japanese plum, banana, coffee, date palm, coconuts, ornamental plants, forest plants, turfs, grasses, and the others.

The above plants are not specifically limited as long as they are generally cultivated cultivars. The above plants also include plants which may be produced by natural breeding, plants which may be generated by mutation, F1 hybrid plants, and genetically modified crops. Examples of the genetically modified crops include plants which have resistance to HPPD (4-hydroxyphenylpyruvate dioxygenase enzyme) inhibitors such as isoxaflutole, ALS (acetolactate synthase) inhibitors such as imazethapyr and thifensulfuron-methyl, EPSP (5-enolpyruvylshikimate-3-phosphate synthase) inhibitors, glutamine synthetase inhibitors, PPO (protoporphyrinogen oxidase) inhibitors, or herbicide such as bromoxynil and dicamba; plants which can synthesize a selective toxin known in Bacillus spp. such as Bacillus thuringiensis or the like; and plants which can synthesize a gene fragment or the like which is partially identical to an endogenous gene derived from a harmful insect, and induce a gene silencing (RNAi; RNA interference) in the target harmful insect to achieve a specific insecticidal activity.

### EXAMPLES

Hereinafter, the present invention is illustrated more in detail by Preparation Examples, Reference Preparation Examples, Formulation Examples, and Test Examples, but the present invention is not limited to these Examples only.

In the present description, Me represents a methyl group, Et represents an ethyl group, Pr represents a propyl group, i-Pr represents an isopropyl group, cPr represents a cyclopropyl group, Bu represents a butyl group, i-Bu represents an isobutyl group, s-Bu represents a sec-butyl group, Ph represents a phenyl group, and Bn represents benzyl group. When the Ph group has substituent(s), the substituent(s) is/are indicated before the symbols of these groups with the substitution position(s). For example, 4-OMe-Ph group represents a 4-methoxyphenyl group, and 2,6-Cl₂-Ph group represents a 2,6-dichlorophenyl group.

When a physical property of a compound is measured by liquid chromatography / mass spectrometry (hereinafter referred to as "LC/MS"), the measured molecular ion value [M+H]⁺, [M+H+MeCN]⁺ or [M-H]⁻, and retention time (hereinafter referred to as "RT") are described. The conditions of liquid chromatography (hereinafter referred to as "LC") and mass spectrometry (hereinafter referred to as "MS") are as follows.

### [LC Condition]

Column: L-cpyrazoleumn2 ODS, inner diameter: 4.6 mm, length: 30 mm, particle size: 3 µm (manufactured by Chemicals Evaluation and Research Institute, Japan)
UV measurement wavelength: 254 nm
Mobile phase: Solution A: 0.1 % aqueous formic acid solution, Solution B: 0.1 % formic acid in acetonitrile
Flow rate: 2.0 mL/min
Pump: LC-20AD (manufactured by Shimadzu Corporation) two machines (high pressure gradient)
Gradient conditions: sending a solution with the concentration gradient described in Table LC1.

**[Table LC1]**

| Time (min) | Solution A (%) | Solution B (%) |
|---|---|---|
| 0.01 | 90 | 10 |
| 2.00 | 0 | 100 |
| 4.00 | 0 | 100 |
| 4.01 | 90 | 10 |

### [MS condition]

Detector: LCMS-2020 (manufactured by Shimadzu Corporation)
Ionization method: DUIS method (ESI, APCI)

First, Preparation Examples of the Present compounds (including the Compounds of the present invention) are shown below.

### Preparation Example 1

Under nitrogen atmosphere, to a mixture of 4-bromo-3,5-difluoro-biphenyl (2 g), and THF (18 mL), isopropylmagnesium chloride lithium chloride complex (THF solution, 1.3 M) (6.3 mL) was added dropwise at 0°C, and the mixture was stirred at room temperature for 2 hours. Six(6) mL as a part of the resulting mixture was added to a solution of 1,3-oxazole-2-carboaldehyde (0.22 g) in THF (5 mL), and the mixture was stirred at room temperature for 2.5 hours. To the resulting mixture, an aqueous solution of ammonium chloride was added, and the mixture was extracted with ethyl acetate. The resulting organic layer was washed with saturated brine and concentrated under reduced pressure. The resulting residue was subjected to silica gel column chromatography (hexane : ethyl acetate = 4:1 to 1:19) to obtain the Present compound 1 represented by the following formula (0.41 g). 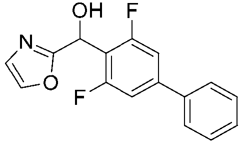 Present compound 1: ¹H-NMR (CDCl₃) δ: 7.67 (1H, s), 7.55-7.52 (2H, m), 7.49-7.39 (3H, m), 7.20-7.13 (3H, m), 6.26 (1H, d), 3.75-3.63 (1H, m).

### Preparation Example 1-1

The compounds prepared according to the Preparation Example 1 and physical properties thereof are shown below.

A compound represented by formula (IA-1): 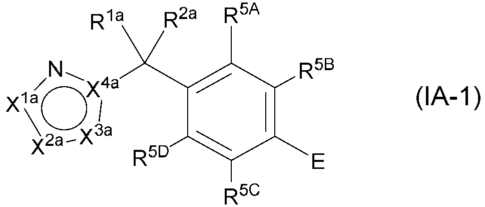 (hereinafter, referred to as "Compound (IA-1)") wherein the combination of R^{1a}, R^{2a}, X^{1a}, X^{2a}, X^{3a}, X^{4a}, R^{5A}, R^{5B}, R^{5C}, R^{5D}, and E represents any one combination indicated in Table A-1.

**[Table A-1]**

| Present compound | R^{1a} | R^{2a} | X^{1a} | X^{2a} | X^{3a} | X^{4a} | R^{5A} | R^{5B} | R^{5C} | R^{5D} | E |
|---|---|---|---|---|---|---|---|---|---|---|---|
| 2 | H | OH | CH | S | CH | C | F | H | H | F | Ph |
| 3 | H | OH | CH | CH | S | C | F | H | H | F | Ph |
| 51 | H | OH | NMe | CH | CH | C | F | H | H | F | 2-E-Ph |

| | | | | | | | | | | | |
|---|---|---|---|---|---|---|---|---|---|---|---|
| Present compound 2: ¹H-NMR (CDCl₃) δ: 8.79-8.76 (1H, m), 7.54-7.49 (2H, m), 7.46-7.36 (3H, m), 7.34 (1H, s), 7.15-7.11 (2H, m), 6.38 (1H, d), 3.84-3.54 (1H, m). Present compound 3: ¹H-NMR (CDCl₃) δ: 7.74-7.73 (1H, m), 7.56-7.50 (2H, m), 7.48-7.33 (4H, m), 7.19-7.10 (2H, m), 6.43 (1H, d), 4.03-3.90 (1H, m). Present compound 51: ¹H-NMR (CDCl₃) δ: 7.43-7.33 (2H, m), 7.31 (1H, d), 7.25-7.09 (4H, m), 6.27 (1H, d), 6.20 (1H, d), 3.89 (3H, s), 3.20 (1H, d). | | | | | | | | | | | |

### Preparation Example 2

To a mixture of the Present compound 1 (0.30 g) and THF (5 mL), phosphorus tribromide (0.30 mL) was added at room temperature, and the mixture was stirred at reflux for 3 hours. To the resulting mixture, 1 M aqueous solution of sodium hydroxide was added, and the mixture was extracted with chloroform. The resulting organic layer was washed with saturated brine and concentrated under reduced pressure. The resulting residue was subjected to a silica gel column chromatography (hexane : ethyl acetate = 1 : 1) to obtain the Present compound 4 represented by the following formula (0.2 g). 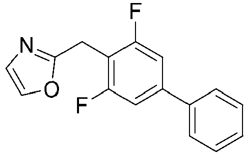 Present compound 4: ¹H-NMR (CDCl₃) δ: 7.59 (1H, d), 7.56-7.52 (2H, m), 7.48-7.36 (3H, m), 7.21-7.13 (2H, m), 7.05 (1H, d), 4.22 (2H, s).

### Preparation Example 2-1

The compounds prepared according to the Preparation Example 2 and physical properties thereof are shown below.

A compound represented by formula (IA-1) wherein the combination of R^{1a}, R^{2a}, X^{1a}, X^{2a}, X^{3a}, X^{4a}, R^{5A}, R^{5B}, R^{5C}, R^{5D}, and E represents any one combination indicated in Table A-2.

**[Table A-2]**

| Present compound | R^{1a} | R^{2a} | X^{1a} | X^{2a} | X^{3a} | X^{4a} | R^{5A} | R^{5B} | R^{5C} | R^{5D} | E |
|---|---|---|---|---|---|---|---|---|---|---|---|
| 6 | H | H | CH | CH | S | C | F | H | H | F | Ph |

| | | | | | | | | | | | |
|---|---|---|---|---|---|---|---|---|---|---|---|
| Present compound 6: LC/MS: 288 [M+H]⁺, RT = 2.083 min. | | | | | | | | | | | |

### Preparation Example 3

To a mixture of the Present compound 1 (0.067 g) and chloroform (5 ml), Dess-Martin periodinane (0.15 g) was added at room temperature, and the mixture was stirred at room temperature for 4 hours. To the resulting mixture, saturated aqueous sodium thiosulfate solution (5 mL) and saturated aqueous sodium hydrogen carbonate solution (5 mL) were added successively, and the mixture was stirred for 1 hour, and extracted with chloroform. The resulting organic layer was washed with saturated brine and concentrated under reduced pressure. The resulting residue was subjected to a silica gel column chromatography (hexane : ethyl acetate = 1 : 1) to obtain the Present compound 7 represented by the following formula (0.041 g). 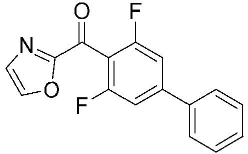 Present compound 7: ¹H-NMR (CDCl₃) δ: 7.94 (1H, s), 7.61-7.56 (2H, m), 7.52-7.42 (4H, m), 7.28-7.22 (2H, m).

### Preparation Example 4

Under nitrogen atmosphere, a mixture of the Intermediate compound 1 (0.43 g), 2,4-difluorophenyl boronic acid (0.27 g), {1,1'-bis(diphenylphosphino)ferrocene}dichloropalladium (II) (0.05 g), tripotassium phosphate (0.60 g), DME (5 mL) and water (1 mL) was stirred at reflux for 5 hours. To the resulting mixture, water was added, and the mixture was extracted with ethyl acetate. The resulting organic layer was concentrated under reduced pressure, and the resulting residue was subjected to a silica gel column chromatography (hexane : ethyl acetate = 1 : 1) to obtain the Present compound 8 represented by the following formula (0.41 g). 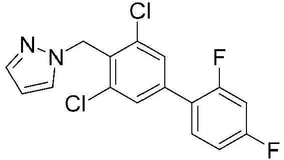 Present compound 8: ¹H-NMR (CDCl₃) δ: 7.56 (1H, d), 7.53 (2H, s), 7.47 (1H, d), 7.42-7.34 (1H, m), 7.00-6.91 (2H, m), 6.26 (1H, t), 5.66 (2H, s).

### Preparation Example 4-1

The compounds prepared according to the Preparation Example 4 and physical properties thereof are shown below.

A compound represented by formula (IA-1) wherein the combination of R^{1a}, R^{2a}, X^{1a}, X^{2a}, X^{3a}, X^{4a}, R^{5A}, R^{5B}, R^{5C}, R^{5D}, and E represents any one combination indicated in Table A-3.

**[Table A-3]**

| Present compound | R^{1a} | R^{2a} | X^{1a} | X^{2a} | X^{3a} | X^{4a} | R^{5A} | R^{5B} | R^{5C} | R^{5D} | E |
|---|---|---|---|---|---|---|---|---|---|---|---|
| 9 | H | H | CH | CH | CH | N | F | H | H | F | 4-Pr-Ph |
| 10 | H | H | CH | CH | CH | N | F | H | H | F | Ph |
| 11 | H | H | CH | CH | CH | N | F | H | H | H | Ph |
| 12 | H | H | CH | CH | CH | N | F | H | H | H | 3, 5-F₂-Ph |
| 13 | H | H | CH | CH | CH | N | Me | H | H | H | Ph |
| 14 | H | H | CH | CH | CH | N | OMe | H | H | H | Ph |
| 15 | H | H | CH | CH | CH | N | Cl | H | H | Cl | Ph |
| 16 | H | H | CH | CH | CH | N | pyrazol-1-yl | H | H | F | 2, 4-F₂-Ph |
| 17 | H | H | CH | CH | CH | N | Cl | H | H | Cl | 4-Me-Ph |
| 37 | H | H | CH | CH | CH | N | CN | H | H | H | Ph |
| 38 | H | H | CH | CH | CH | N | CN | H | H | H | 2-F-Ph |
| 41 | H | H | CH | CH | N | N | F | H | H | F | 2, 3-F₂-Ph |
| 42 | H | H | O | CH | N | C | F | H | H | H | 2,3-F₂-Ph |
| 43 | H | H | O | CMe | N | C | F | H | H | H | Ph |
| 44 | H | H | CH | CH | N | N | F | H | H | F | 2,4-F₂-Ph |
| 45 | H | H | O | CMe | N | C | F | H | H | F | Ph |
| 46 | H | H | CH | CH | N | N | F | H | H | H | Ph |
| 47 | H | H | CH | CH | N | N | F | H | H | H | 2-F-Ph |
| 48 | H | H | CH | CH | N | N | F | H | H | H | 2,4-F₂-Ph |
| 50 | H | H | CH | S | N | C | F | H | H | H | 2-F-Ph |
| 52 | H | H | S | CH | CH | C | F | H | H | F | 2-F-Ph |

| | | | | | | | | | | | |
|---|---|---|---|---|---|---|---|---|---|---|---|
| Present compound 9 : ¹H-NMR (CDCl₃) δ: 7.52 (2H, dd), 7.47-7.42 (2H, m), 7.29-7.24 (2H, m), 7.19-7.11 (2H, m), 6.25 (1H, t), 5.42 (2H, s), 2.63 (2H, t), 1.72-1.62 (2H, m), 0.96 (3H, t). Present compound 10: ¹H-NMR (CDCl₃) δ: 7.55-7.50 (4H, m), 7.48-7.37 (3H, m), 7.20-7.13 (2H, m), 6.26 (1H, t), 5.43 (2H, s). Present compound 11: ¹H-NMR (CDCl₃) δ: 7.58-7.48 (4H, m), 7.46-7.41 (2H, m), 7.40-7.28 (3H, m), 7.21 (1H, t), 6.30 (1H, t), 5.41 (2H, s). Present compound 12: ¹H-NMR (CDCl₃) δ: 7.57 (1H, t), 7.50 (1H, d), 7.30-7.19 (3H, m), 7.09-7.02 (2H, m), 6.85-6.78 (1H, m), 6.31 (1H, t), 5.42 (2H, s). Present compound 13: ¹H-NMR (CDCl₃) δ: 7.61-7.54 (3H, m), 7.47-7.39 (4H, m), 7.38-7.31 (2H, m), 7.10 (1H, d), 6.28 (1H, t), 5.37 (2H, s), 2.34 (3H, s). Present compound 14: ¹H-NMR (CDCl₃) δ: 7.59-7.54 (3H, m), 7.49-7.40 (3H, m), 7.39-7.33 (1H, m), 7.15-7.07 (3H, m), 6.27 (1H, t), 5.38 (2H, s), 3.92 (3H, s). Present compound 15: ¹H-NMR (CDCl₃) δ: 7.59 (2H, s), 7.56-7.52 (3H, m), 7.49-7.37 (4H, m), 6.25 (1H, t), 5.66 (2H, s). Present compound 16: ¹H-NMR (CDCl₃) δ: 7.85 (1H, d), 7.80 (1H, d), 7.47 (1H, d), 7.45-7.35 (3H, m), 7.20 (1H, d), 7.00-6.90 (2H, m), 6.47 (1H, t), 6.16 (1H, t), 5.47 (2H, d). Present compound 17: ¹H-NMR (CDCl₃) δ: 7.56 (3H, d), 7.44-7.39 (3H, m), 7.27 (2H, d), 6.25-6.24 (1H, m), 5.65 (2H, s), 2.41 (3H, s). Present compound 37: ¹H-NMR (CDCl₃) δ: 7.88 (1H, d), 7.76 (1H, dd), 7.60 (2H, dd), 7.54-7.52 (2H, m), 7.49-7.45 (2H, m), 7.43-7.39 (1H, m), 7.31 (1H, d), 6.34 (1H, t), 5.57 (2H, s). Present compound 38: ¹H-NMR (CDCl₃) δ: 7.87 (1H, br s), 7.73 (1H, dt), 7.61 (2H, t), 7.40-7.39 (1H, m), 7.37-7.36 (1H, m), 7.29 (1H, d), 7.24-7.22 (1H, m), 7.19-7.16 (1H, m), 6.35 (1H, t), 5.58 (2H, s). Present compound 41: ¹H-NMR (CDCl₃) δ: 7.64 (2H, s), 7.24-7.15 (5H, m), 5.75 (2H, s). Present compound 42: ¹H-NMR (CDCl₃) δ: 8.68 (1H, s), 7.39 (1H, t), 7.33-7.29 (2H, m), 7.20-7.13 (3H, m), 4.25 (2H, s). Present compound 43: ¹H-NMR (CDCl₃) δ: 7.57-7.54 (2H, m), 7.46-7.42 (2H, m), 7.38-7.29 (4H, m), 4.13 (2H, s), 2.57 (3H, s). Present compound 44: ¹H-NMR (CDCl₃) δ: 7.63 (2H, s), 7.40-7.37 (1H, m), 7.14-7.11 (2H, m), 7.00-6.92 (2H, m), 5.74 (2H, s). Present compound 45: ¹H-NMR (CDCl₃) δ: 7.55-7.52 (2H, m), 7.47-7.36 (3H, m), 7.18-7.13 (2H, m), 4.14 (2H, s), 2.56 (3H, s). Present compound 46: ¹H-NMR (CDCl₃) δ: 7.66 (2H, s), 7.56-7.52 (2H, m), 7.47-7.41 (2H, m), 7.39-7.30 (3H, m), 7.28-7.23 (1H, m), 5.72 (2H, s). Present compound 47: ¹H-NMR (CDCl₃) δ: 7.67 (2H, s), 7.43-7.38 (1H, m), 7.36-7.29 (3H, m), 7.26-7.13 (3H, m), 5.73 (2H, s). Present compound 48: ¹H-NMR (CDCl₃) δ: 7.66 (2H, s), 7.40-7.33 (1H, m), 7.30-7.22 (3H, m), 6.99-6.88 (2H, m), 5.73 (2H, s). Present compound 50: ¹H-NMR (CDCl₃) δ: 9.81 (1H, s), 7.42 (1H, td), 7.38 (1H, t), 7.3,5-7.30 (3H, m), 7.21 (1H, td), 7.17-7.13 (1H, m), 4.49 (2H, s). Present compound 52: ¹H-NMR (CDCl₃) δ: 8.59 (1H, d), 7.44-7.32 (2H, m), 7.25-7.21 (1H, m), 7.21-7.11 (4H, m), 4.29 (2H, s). | | | | | | | | | | | |

### Preparation Example 5

Under nitrogen atmosphere, to a mixture of (3,5-difluoro-[1,1'-biphenyl]-4-yl)methanol (0.20 g), 1H-tetrazole (0.14 g), and toluene (6 mL), cyanomethylene tributylphosphorane (0.54 mL) was added at room temperature, and the mixture was stirred at 100°C for 7 hours. To the resulting mixture, water was added, and the mixture was extracted with ethyl aetate, and the resulting organic layer was concentrated under reduced pressure. The resulting residue was subjected to a silica gel column chromatography to obtain the Present compound 18 represented by the following formula (0.05 g) and the Present compound 19 represented by the following formula (0.06 g). 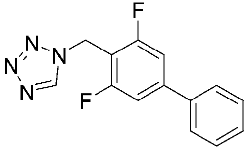 Present compound 18: ¹H-NMR (CDCl₃) δ: 8.52 (1H, s), 7.56-7.54 (2H, m), 7.49-7.42 (3H, m), 7.24-7.19 (2H, m), 5.93 (2H, s). 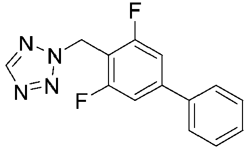 Present compound 19: ¹H-NMR (CDCl₃) δ: 8.68 (1H, s), 7.55-7.41 (5H, m), 7.24-7.21 (2H, m), 5.72 (2H, s).

### Preparation Example 5-1

The compounds prepared according to the Preparation Example 5 and physical properties thereof are shown below.

A compound represented by formula (IA-1) wherein the combination of R^{1a}, R^{2a}, X^{1a}, X^{2a}, X^{3a}, X^{4a}, R^{5A}, R^{5B}, R^{5C}, R^{5D}, and E represents any one combination indicated in Table A-4.

**[Table A-4]**

| Present compound | R^{1a} | R^{2a} | X^{1a} | X^{2a} | X^{3a} | X^{4a} | R^{5A} | R^{5B} | R^{5C} | R^{5D} | E |
|---|---|---|---|---|---|---|---|---|---|---|---|
| 20 | H | H | CH | N | CH | N | F | H | H | F | Ph |
| 21 | H | H | CH | CH | N | N | F | H | H | F | Ph |
| 22 | H | H | N | CH | CH | N | F | H | H | F | Ph |
| 23 | H | H | CMe | CH | CH | N | F | H | H | F | Ph |
| 24 | H | H | CH | CH | CMe | N | F | H | H | F | Ph |
| 25 | H | H | CH | CF | CH | N | F | H | H | F | Ph |
| 26 | H | H | CH | CH | N | N | F | H | H | F | 2-F-Ph |
| 27 | H | H | CMe | CH | N | N | F | H | H | F | Ph |
| 28 | H | H | N | CH | CH | N | Cl | H | H | Cl | Ph |
| 29 | H | H | CH | CH | N | N | Cl | H | H | Cl | Ph |

| | | | | | | | | | | | |
|---|---|---|---|---|---|---|---|---|---|---|---|
| Present compound 20: ¹H-NMR (CDCl₃) δ: 8.20 (1H, s), 7.95 (1H, s), 7.55-7.52 (2H, m), 7.49-7.40 (3H, m), 7.22-7.17 (2H, m), 5.47 (2H, s). Present compound 21: ¹H-NMR (CDCl₃) δ: 7.63 (2H, s), 7.55-7.52 (2H, m), 7.48-7.41 (3H, m), 7.22-7.17 (2H, m), 5.73 (2H, s). Present compound 22: ¹H-NMR (CDCl₃) δ: 7.71 (1H, d), 7.65 (1H, s), 7.55-7.52 (2H, m), 7.49-7.40 (3H, m), 7.22-7.17 (2H, m), 5.69 (2H, s). Present compounds 23 + 24 (which is a mixture of the Present compound 23 and the Present compound 24; and in the mixture the Present compound 23 : the Present compound 24 = 3 : 7 (molar ratio): ¹H-NMR (CDCl₃) δ: 7.54-7.52 (2H, m), 7.47-7.37 (4H, m), 7.19-7.13 (2H, m), 6.02 (0.7H, d), 6.00 (0.3H, d), 5.34 (1.4H, s), 5.30 (0.6H, s), 2.41 (0.9H, s), 2.27 (2.1H, s). Present compound 25: ¹H-NMR (CDCl₃) δ: 7.55-7.52 (2H, m), 7.48-7.38 (4H, m), 7.35 (1H, d), 7.20-7.15 (2H, m), 5.32 (2H, s). Present compound 26: ¹H-NMR (CDCl₃) δ: 7.64 (2H, s), 7.43-7.35 (2H, m), 7.25-7.15 (4H, m), 5.74 (2H, s). Present compound 27: ¹H-NMR (CDCl₃) δ: 7.55-7.52 (2H, m), 7.48-7.38 (3H, m), 7.37 (1H, s), 7.20-7.14 (2H, m), 5.64 (2H, s), 2.30 (3H, s). Present compound 28: ¹H-NMR (CDCl₃) δ: 7.70 (1H, d), 7.62 (2H, s), 7.59 (1H, d), 7.56-7.53 (2H, m), 7.50-7.41 (3H, m), 5.92 (2H, s). Present compound 29: ¹H-NMR (CDCl₃) δ: 7.64 (2H, s), 7.61 (2H, s), 7.56-7.52 (2H, m), 7.49-7.39 (3H, m), 5.95 (2H, s). | | | | | | | | | | | |

### Preparation Example 6

Under nitrogen atmosphere, to a mixture of the Intermediate compound 18 (0.30 g), trimethyl orthoformate (0.28 mL), and DMF (2 mL), trifluoroacetic acid (4 µL) was added at room temperature, and the mixture was stirred at 100°C for 7 hours. To the resulting mixture, water was added, and the mixture was extracted with a mixed solvent of hexane / ethyl acetate. The resulting organic layer was dried over magnesium sulfate and concentrated under reduced pressure. The resulting residue was subjected to a silica gel column chromatography to obtain the Present compound 35 represented by the following formula (0.31 g). 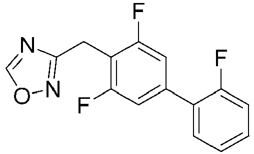 Present compound 35: ¹H-NMR (CDCl₃) δ: 8.67 (1H, s), 7.44-7.34 (2H, m), 7.25-7.14 (4H, m), 4.26 (2H, s).

### Preparation Example 7

Under nitrogen atmosphere, a mixture of the Intermediate compound 22 (0.17 g), 4-methylpyrazole (0.06 g), potassium carbonate (0.17 g), and DMF (3 mL) was stirred at room temperature for 2 hours and stirred at 60°C for 4 hours. To the resulting mixture, water was added, and the mixture was extracted with MTBE. The resulting organic layer was washed with saturated brine, and dried over magnesium sulfate, and concentrated under reduced pressure. The resulting residue was subjected to a silica gel column chromatography to obtain the Present compound 36 represented by the following formula (0.13 g). 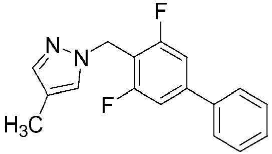 Present compound 36: ¹H-NMR (CDCl₃) δ: 7.54-7.50 (2H, m), 7.48-7.37 (3H, m), 7.32 (1H, s), 7.27 (1H, s), 7.18-7.13 (2H, m), 5.34 (2H, s), 2.05 (3H, s).

### Preparation Example 7-1

The compounds prepared according to the Preparation Example 7 and physical properties thereof are shown below.

A compound represented by formula (IA-1) wherein the combination of R^{1a}, R^{2a}, X^{1a}, X^{2a}, X^{3a}, X^{4a}, R^{5A}, R^{5B}, R^{5C}, R^{5D}, and E represents any one combination indicated in Table A-5.

**[Table A-5]**

| Present compound | R^{1a} | R^{2a} | X^{1a} | X^{2a} | X^{3a} | X^{4a} | R^{5A} | R^{5B} | R^{5C} | R^{5D} | E |
|---|---|---|---|---|---|---|---|---|---|---|---|
| 39 | H | H | CBr | CBr | N | N | F | H | H | F | Ph |
| 53 | H | H | CH | COMe | CH | N | F | H | H | F | 2-F-Ph |
| 56 | H | H | CMe | O | C=O | N | F | H | H | F | 2-F-Ph |

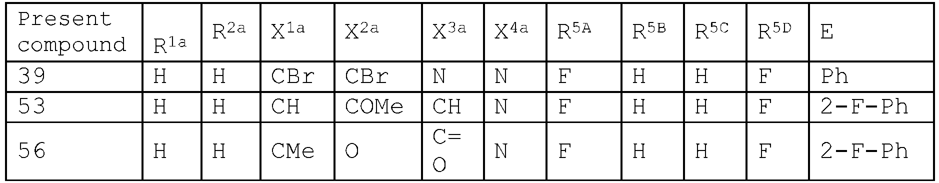

Present compound 39: ¹H-NMR (CDCl₃) δ: 7.56-7.53 (2H, m), 7.49-7.40 (3H, m), 7.22-7.16 (2H, m), 5.65 (2H, s). Present compound 53: LC/MS: 319 [M+H]⁺, RT = 1.995 min. Present compound 56: ¹H-NMR (CDCl₃) δ: 7.44-7.35 (2H, m), 7.26-7.13 (4H, m), 4.98 (2H, s), 2.23 (3H, s).

### Preparation Example 8

Under nitrogen atmosphere, a mixture of the Present compound 21 (0.20 g), propanethiol (14 mL), cesium carbonate (0.36 g), and DMF (5 mL) was stirred at 65°C for 4 hours. To the resulting mixture, water was added, and the mixture was extracted with ethyl acetate. The resulting organic layer was washed with saturated brine and concentrated under reduced pressure. The resulting residue was subjected to a silica gel column chromatography to obtain the Present compound 40 represented by the following formula (0.22 g). 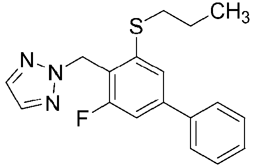 Present compound 40: ¹H-NMR (CDCl₃) δ: 7.62 (2H, s), 7.56-7.53 (2H, m), 7.48-7.44 (2H, m), 7.42-7.38 (2H, m), 7.21-7.18 (1H, m), 5.88 (2H, d), 2.92 (2H, t), 1.72-1.62 (2H, m), 1.00 (3H, t).

### Preparation Example 9

Under nitrogen atmosphere, to a mixture of the Present compound 40 (0.17 g), and chloroform (5 mL), meta-chloroperbenzoic acid (purity 75 %, 25 % water content) (0.22 g) was added at 0°C, and the mixture was stirred at room temperature for 2 hours. To the resulting mixture, an aqueous saturated sodium thiosulfate solution (5 ml) and an aqueous saturated sodium hydrogen carbonate (5 ml) were added successively, and the mixture was stirred for 1 hour, and the mixture was extracted with chloroform. The resulting organic layer was washed with saturated brine, and dried over sodium sulfate, and concentrated under reduced pressure. The resulting residue was subjected to a silica gel column chromatography to obtain the Present compound 34 represented by the following formula (0.15 g). 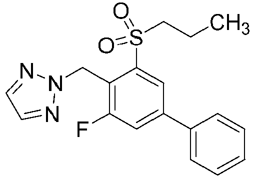 Present compound 34: ¹H-NMR (CDCl₃) δ: 8.17 (1H, t), 7.65-7.60 (5H, m), 7.52-7.43 (3H, m), 6.26 (2H, d), 3.24-3.20 (2H, m), 1.75-1.66 (2H, m), 0.94 (3H, t).

### Preparation Example 10

Under nitrogen atmosphere, a mixture of the Intermediate compound 23 (0.20 g), 2-chloroethyl isothiocyanate (0.10 mL), 4-dimethylaminopyridine (0.20 g), and toluene (8 mL) was stirred at 115°C for 8 hours. To the resulting mixture, water was added, and the mixture was extracted with ethyl acetate. The resulting organic layer was washed with saturated brine and concentrated under reduced pressure. The resulting residue was subjected to a silica gel column chromatography to obtain the Present compound 49 represented by the following formula (0.06 g). 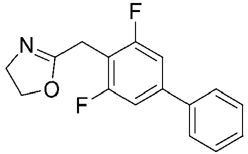 Present compound 49: ¹H-NMR (CDCl₃) δ: 7.56-7.51 (2H, m), 7.47-7.35 (3H, m), 7.17-7.10 (2H, m), 4.30 (2H, t), 3.85 (2H, t), 3.70 (2H, s).

### Preparation Example 11

Under nitrogen atmosphere, a mixture of the Intermediate compound 10 (0.21 g), pyridine (0.12 mL), trifluoroacetic anhydride (0.21 mL), and DMF (6 mL) was stirred at 60°C for 3 hours. To the resulting mixture, water was added, and the mixture was extracted with ethyl acetate. The resulting organic layer was washed with saturated brine and concentrated under reduced pressure. The resulting residue was subjected to a silica gel column chromatography to obtain the Present compound 54 represented by the following formula (0.22 g). 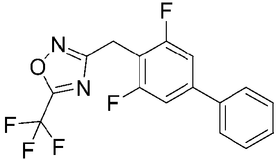 Present compound 54: ¹H-NMR (CDCl₃) δ: 7.56-7.54 (2H, m), 7.48-7.45 (2H, m), 7.43-7.39 (1H, m), 7.20-7.17 (2H, m), 4.28 (2H, s).

### Preparation Example 12

Under nitrogen atmosphere, a mixture of the Intermediate compound 29 (3.0 g), molecular sieves (4Å) (3.0 g), and toluene (30 mL) was stirred at 120°C for 15 hours. The resulting mixture was filtered, and the filtrates were concentrated under reduced pressure. The resulting residue was subjected to a silica gel column chromatography to obtain the Present compound 55 represented by the following formula (2.1 g). 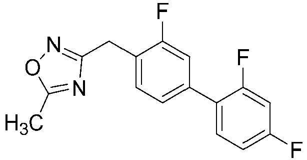 Present compound 55: ¹H-NMR (CDCl₃) δ: 7.42-7.33 (2H, m), 7.28-7.22 (2H, m), 6.99-6.88 (2H, m), 4.13 (2H, s), 2.57 (3H, s).

### Reference Preparation Example 1

Under nitrogen atmosphere, (4-bromo-2,6-dichlorophenyl)methanol (2.0 g), pyrazole (0.8 g), and toluene (20 mL), cyanomethylene tributylphosphorane (3.1 mL) was added at room temperature, and the mixture was stirred at 100°C for 7 hours. To the resulting mixture, water was added, and the mixture was extracted with ethyl acetate. The resulting organic layer was washed with saturated brine and concentrated under reduced pressure. The resulting residue was subjected to a silica gel column chromatography to obtain the Intermediate compound 1 represented by the following formula (2.2 g). 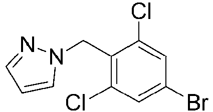 Intermediate compound 1: ¹H-NMR (CDCl₃) δ: 7.55 (2H, s), 7.53 (1H, d), 7.41 (1H, d), 6.24 (1H, t), 5.57 (2H, s).

### Reference Preparation Example 1-1

The compounds prepared according to the Reference Preparation Example 1 and physical properties thereof are shown below.

A compound represented by formula (IA-1) wherein the combination of R^{1a}, R^{2a}, X^{1a}, X^{2a}, X^{3a}, X^{4a}, R^{5A}, R^{5B}, R^{5C}, R^{5D}, and E represents any one combination indicated in Table B-2.

**[Table B-2]**

| Intermediate compound | R^{1a} | R^{2a} | X^{1a} | X^{2a} | X^{3a} | X^{4a} | R^{5A} | R^{5B} | R^{5C} | R^{5D} | E |
|---|---|---|---|---|---|---|---|---|---|---|---|
| 19 | H | H | CH | CH | N | N | F | H | H | F | Br |
| 20 | H | H | N | CH | CH | N | F | H | H | F | Br |

| | | | | | | | | | | | |
|---|---|---|---|---|---|---|---|---|---|---|---|
| Intermediate compound 19: ¹H-NMR (CDCl₃) δ: 7.60 (2H, s), 7.18-7.13 (2H, m), 5.64 (2H, s). Intermediate compound 20: ¹H-NMR (CDCl₃) δ: 7.70 (1H, d), 7.60 (1H, s), 7.20-7.16 (2H, m), 5.60 (2H, s). | | | | | | | | | | | |

### Reference Preparation Example 2

Under nitrogen atmosphere, a mixture of 4-bromo-1-(bromomethyl)-2-methyl benzene (0.44 g), pyrazole (0.17 g), potassium carbonate (0.46 g), and DMF (5 mL) was stirred at 65°C for 4 hours. To the resulting mixture, water was added, and the mixture was extracted with ethyl acetate. The resulting organic layer was washed with saturated brine and concentrated under reduced pressure. The resulting residue was subjected to a silica gel column chromatography to obtain the Intermediate compound 2 represented by the following formula (2.2 g). 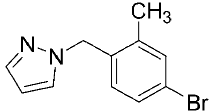 Intermediate compound 2: ¹H-NMR (CDCl₃) δ: 7.55 (1H, d), 7.37-7.27 (3H, m), 6.85 (1H, d), 6.27 (1H, t), 5.27 (2H, s), 2.25 (3H, s).

### Reference Preparation Example 2-1

The compounds prepared according to the Reference Preparation Example 2 and physical properties thereof are shown below.

A compound represented by formula (IA-1) wherein the combination of R^{1a}, R^{2a}, X^{1a}, X^{2a}, X^{3a}, X^{4a}, R^{5A}, R^{5B}, R^{5C}, R^{5D}, and E represents any one combination indicated in Table B-1.

**[Table B-1]**

| Intermediate compound | R^{1a} | R^{2a} | X^{1a} | X^{2a} | X^{3a} | X^{4a} | R^{5A} | R^{5B} | R^{5C} | R^{5D} | E |
|---|---|---|---|---|---|---|---|---|---|---|---|
| 3 | H | H | CH | CH | CH | N | F | H | H | F | Br |
| 4 | H | H | CH | CH | CH | N | OMe | H | H | H | Br |
| 5 | H | H | CH | CH | CH | N | F | H | H | H | Br |
| 6 | H | H | CH | CH | CH | N | pyrazol-1-yl | H | H | F | Br |
| 7 | H | H | CH | CH | N | N | F | H | H | H | Br |
| 8 | H | H | N | CH | CH | N | F | H | H | H | Br |
| 21 | H | H | CH | CH | CH | N | CN | H | H | H | Br |

| | | | | | | | | | | | |
|---|---|---|---|---|---|---|---|---|---|---|---|
| Intermediate compound 3: ¹H-NMR (CDCl₃) δ: 7.51 (1H, d), 7.47 (1H, d), 7.16-7.10 (2H, m), 6.24 (1H, t), 5.34 (2H, s). Intermediate compound 4: ¹H-NMR (CDCl₃) δ: 7.53 (1H, dd), 7.41 (1H, dd), 7.06-7.01 (2H, m), 6.85 (1H, d), 6.25 (1H, t), 5.27 (2H, s), 3.85 (3H, s). Intermediate compound 5: ¹H-NMR (CDCl₃) δ: 7.54 (1H, d), 7.45 (1H, d), 7.29-7.23 (2H, m), 7.00 (1H, t), 6.29 (1H, t), 5.32 (2H, s). Intermediate compound 6: ¹H-NMR (CDCl₃) δ: 7.84 (1H, dd), 7.79 (1H, d), 7.45-7.34 (3H, m), 7.16 (1H, d), 6.46 (1H, dd), 6.14 (1H, t), 5.41 (2H, d). Intermediate compound 7: ¹H-NMR (CDCl₃) δ: 7.64 (2H, s), 7.31-7.25 (2H, m), 7.06 (1H, t,), 5.63 (2H, s). Intermediate compound 8: ¹H-NMR (CDCl₃) δ: 7.73 (1H, s), 7.58 (1H, s), 7.33-7.29 (2H, m), 7.14 (1H, t), 5.58 (2H, s). Intermediate compound 21: ¹H-NMR (CDCl₃) δ: 7.80 (1H, d), 7.67 (1H, dd), 7.57 (2H, dd), 7.08 (1H, d), 6.33 (1H, t), 5.48 (2H, s). | | | | | | | | | | | |

### Reference Preparation Example 3

Under nitrogen atmosphere, a mixture of 2-(2',3-difluoro-[1,1'-biphenyl]-4-yl)acetonitrile (10.0 g), a 50% aqueous hydroxylamine solution (1.3 mL), and ethanol (14 mL) was stirred at 85°C for 2 and a half hours. To the resulting mixture, water was added, and the solids precipitated out were collected by filtration. The obtained solids were washed with sufficient amounts of water and dried under reduced pressure to obtain the Intermediate compound 9 represented by the following formula (2.91 g). 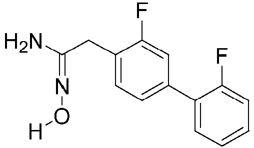 Intermediate compound 9:¹H-NMR (CDCl₃) δ: 7.44-7.36 (2H, m), 7.36-7.28 (3H, m), 7.23-7.13 (3H, m), 4.63 (2H, br s), 3.54 (2H, s).

### Reference Preparation Example 3-1

The compounds prepared according to the Reference Preparation Example 3 and physical properties thereof are shown below.

A compound represented by formula (IA-2) 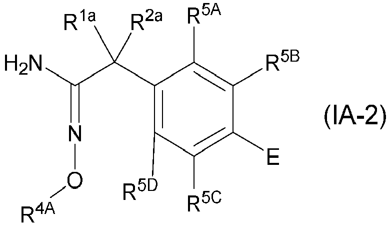 (hereinafter, referred to as "Compound (IA-2)")
wherein the combination of R^{1a}, R^{2a}, X^{1a}, X^{2a}, X^{3a}, X^{4a}, R^{5A}, R^{5B}, R^{5C}, R^{5D}, and E represents any one combination indicated in Table B-3.

**[Table B-3]**

| Intermediate compound | R^{1a} | R^{2a} | R^{4A} | R^{5A} | R^{5B} | R^{5C} | R^{5D} | E |
|---|---|---|---|---|---|---|---|---|
| 10 | H | H | H | F | H | H | F | Ph |
| 11 | H | H | H | F | H | H | F | Br |
| 12 | H | H | H | F | H | H | H | Ph |
| 13 | H | H | H | F | H | H | H | Br |
| 18 | H | H | H | F | H | H | F | 2-F-Ph |

| | | | | | | | | |
|---|---|---|---|---|---|---|---|---|
| Intermediate compound 10: ¹H-NMR (CDCl₃) δ: 7.54-7.51 (2H, m), 7.47-7.42 (2H, m), 7.41-7.36 (1H, m), 7.17-7.12 (2H, m), 6.95 (1H, br s), 4.65 (2H, br s), 3.57 (2H, s). Intermediate compound 11: ¹H-NMR (CDCl₃) δ: 7.14-7.09 (2H, m), 6.78 (1H, s), 4.59 (2H, s), 3.48 (2H, s). Intermediate compound 12: ¹H-NMR (DMSO-D₆) δ: 9.04 (1H, s), 7.72-7.66 (2H, m), 7.50-7.35 (6H, m), 5.50 (2H, s), 3.38 (2H, s). Intermediate compound 13: ¹H-NMR (CDCl₃) δ: 7.28-7.17 (3H, m), 6.02 (1H, br s), 4.54 (2H, br s), 3.44 (2H, s). Intermediate compound 18: ¹H-NMR (DMSO-D₆) δ: 9.07 (1H, s), 7.61-7.57 (1H, m), 7.50-7.44 (1H, m), 7.37-7.30 (2H, m), 7.28-7.23 (2H, m), 5.53 (2H, s), 3.44 (2H, s). | | | | | | | | |

### Reference Preparation Example 4

Under nitrogen atmosphere, a mixture of the Intermediate compound 13 (2.00 g), trimethyl orthoformate (2.2 mL), and DMF (10 mL) was stirred at 100°C for 3 hours. To the resulting mixture was added water, and the mixture was extracted with ethyl acetate, and the resulting organic layer was concentrated under reduced pressure. The resulting residue was subjected to a silica gel column chromatography to obtain the Intermediate compound 14 represented by the following formula (1.9 g). 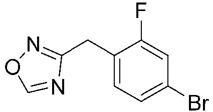 Intermediate compound 14: ¹H-NMR (CDCl₃) δ: 8.66 (1H, s), 7.30-7.26 (2H, m), 7.18 (1H, t), 4.14 (2H, s).

### Reference Preparation Example 4-1

The compounds prepared according to the Reference Preparation Example 4 and physical properties thereof are shown below.

A compound represented by formula (IA-1) wherein the combination of R^{1a}, R^{2a}, X^{1a}, X^{2a}, X^{3a}, X^{4a}, R^{5A}, R^{5B}, R^{5C} and E represents any one combination indicated in Table B-4.

**[Table B-4]**

| Intermediate compound | R^{1a} | R^{2a} | X^{1a} | X^{2a} | X^{3a} | X^{4a} | R^{5A} | R^{5B} | R^{5C} | R^{5D} | E |
|---|---|---|---|---|---|---|---|---|---|---|---|
| 15 | H | H | O | CH | N | C | F | H | H | F | Br |

### Reference Preparation Example 5

Under nitrogen atmosphere, a mixture of the Intermediate compound 13 (2.00 g), acetic anhydride (1.5 mL), pyridine (1.3 mL), and DMF (10 mL) was stirred at 100°C for 4 hours. To the resulting mixture, water was added, and the mixture was extracted with ethyl acetate, and the resulting organic layer was concentrated under reduced pressure. The resulting residue was subjected to a silica gel column chromatography to obtain the Intermediate compound 16 represented by the following formula (0.95 g). 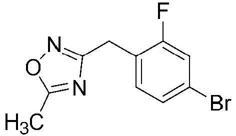 Intermediate compound 16:¹H-NMR (CDCl₃) δ: 7.28-7.25 (2H, m), 7.17 (1H, t), 4.04 (2H, s), 2.56 (3H, s).

### Reference Preparation Example 5-1

The compounds prepared according to the Reference Preparation Example 5 and physical properties thereof are shown below.

A compound represented by formula (IA-1) wherein the combination of R^{1a}, R^{2a}, X^{1a}, X^{2a}, X^{3a}, X^{4a}, R^{5A}, R^{5B}, R^{5C}, R^{5D}, and E represents any one combination indicated in Table B-5.

**[Table B-5]**

| Intermediate compound | R^{1a} | R^{2a} | X^{1a} | X^{2a} | X^{3a} | X^{4a} | R^{5A} | R^{5B} | R^{5C} | R^{5D} | E |
|---|---|---|---|---|---|---|---|---|---|---|---|
| 17 | H | H | O | CM e | N | C | F | H | H | F | Br |

Intermediate compound 17: ¹H-NMR (CDCl₃) δ: 7.15-7.10 (2H, m), 4.06 (2H, s), 2.55 (3H, s).

### Reference Preparation Example 6

Under nitrogen atmosphere, to a mixture of (3,5-difluoro-[1,1'-biphenyl]-4-yl)methanol (4.00 g), and chloroform (45 mL), phosphorus tribromide (9.83 g) was added dropwise at 0°C, and the mixture was stirred at room temperature for 2 hours. To the resulting mixture, an aqueous saturated sodium bicarbonate solution was added, and the mixture was extracted with chloroform, and the resulting organic layer was concentrated under reduced pressure. The resulting organic layer was subjected to silica gel column chromatography to obtain the Intermediate compound 22 represented by the following formula (3.42 g). 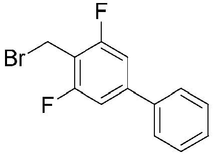 Intermediate compound 22:¹H-NMR (CDCl₃) δ: 7.55-7.52 (2H, m), 7.48-7.39 (3H, m), 7.17-7.11 (2H, m), 4.57 (2H, s).

### Reference Preparation Example 7

Under nitrogen atmosphere, a mixture of 4-bromo-2,6-difluorophenyl acetic acid (1.50 g), phenyl boronic acid (0.84 g), {1,1'-bis(diphenylphosphino)ferrocene}dichloropalladium (II) (0.22 g), tripotassium phosphate (2.54 g), DME (20 mL) and water (2.0 mL) was stirred at reflux for 2 hours. To the resulting mixture, 1M aqueous hydrochloride solution was added, and the mixture was extracted with MTBE, and the resulting organic layer was washed with saturated brine and dried over anhydrous sodium sulfate. The resulting organic layer was concentrated under reduced pressure to obtain the Intermediate compound 23 (1.86 g) represented by the following formula. 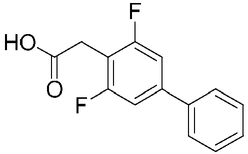 Intermediate compound 23:¹H-NMR (CDCl₃) δ: 7.56-7.51 (2H, m), 7.48-7.36 (3H, m), 7.18-7.11 (2H, m), 3.80 (2H, s).

### Reference Preparation Example 8-1

Under nitrogen atmosphere, to a mixture of 4-bromo-2-fluorophenyl acetamide (4.1 g), and toluene (100 mL), chlorocarbonylsulfenyl cloride (1.7 mL) was added at room temperature, and the mixture was stirred at 80°C for 6 hours. To the resulting mixture, water was added, and the mixture was extracted with ethyl acetate, and the resulting organic layer was washed with saturated brine and concentrated under reduced pressure. The resulting residue was subjected to a silica gel column chromatography to obtain the Intermediate compound 24 represented by the following formula (3.9 g). 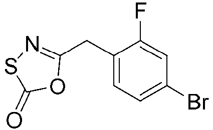 Intermediate compound 24: ¹H-NMR (CDCl₃) δ: 7.34-7.29 (2H, m), 7.19 (1H, t), 3.95 (2H, s).

### Reference Preparation Example 8-2

Under nitrogen atmosphere, a mixture of the Intermediate compound 24 (3.0 g), p-toluenesulfonyl cyanide (2.3 g), and 1,2-dichlorobenzene (25 mL) was stirred at 165°C for 6 hours. To the resulting mixture, water was added, and the mixture was extracted with ethyl acetate, and washed with saturated brine, and the resulting organic layer was concentrated under reduced pressure. The resulting residue was subjected to a silica gel column chromatography to obtain the Intermediate compound 25 represented by the following formula (3.5 g). 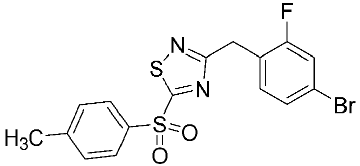 Intermediate compound 25: ¹H-NMR (CDCl₃) δ: 7.99 (2H, d), 7.41 (2H, d), 7.26-7.22 (2H, m), 7.12 (1H, t), 4.30 (2H, s), 2.48 (3H, s).

### Reference Preparation Example 8-3

Under nitrogen atmosphere, to a mixture of the Intermediate compound 25 (1.5 g), ethanol (10 mL) and chloroform (10 mL), sodium borohydride (0.27 g) was added at 0°C, and the mixture was stirred at room temperature for 1.5 hours. To the resulting mixture, an aqueous saturated ammonium chloride solution was added, and the mixture was extracted with chloroform, and the resulting organic layer was washed with saturated brine and concentrated under reduced pressure. The resulting residue was subjected to a silica gel column chromatography to obtain the Intermediate compound 26 represented by the following formula (0.78 g). 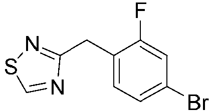 Intermediate compound 26: ¹H-NMR (CDCl₃) δ: 9.79 (1H, s), 7.30-7.25 (2H, m), 7.20 (1H, t), 4.40 (2H, s).

### Reference Preparation Example 8-4

Under nitrogen atmosphere, to a mixture of the Intermediate compound 25 (0.20 g), and THF (2 mL), methylmagnesium bromide (THF solution, about 1.0 M) (0.56 mL) was added at 0°C, and the mixture was stirred at room temperature for 1 hour. To the resulting mixture, water was added, the mixture was extracted with ethyl acetate, and the resulting organic layer was washed with saturated brine and concentrated under reduced pressure. The resulting residue was subjected to a silica gel column chromatography to obtain the Intermediate compound 30 represented by the following formula (0.13 g). 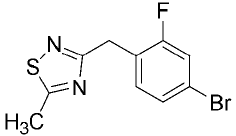 Intermediate compound 30: ¹H-NMR (CDCl₃) δ: 7.28-7.23 (2H, m), 7.17 (1H, t), 4.27 (2H, s), 2.78 (3H, s).

### Reference Preparation Example 9

Under nitrogen atmosphere, to a mixture of zinc powder (0.23 g), 1,2-dibromoethane (0.015 mL), and THF (3 mL), at room temperature, trimethylsilyl chloride (0.022 mL) was added at room temperature, and the mixture was stirred at 70°C for 15 minutes. To the resulting mixture, 4-bromo-2,6-difluorobenzyl bromide (0.50 g) was added at room temperature, and 3-bromo-isothiazole (0.30 g), and {1,1'-bis(diphenylphosphino)ferrocene}dichloropalladium (II) (0.04 g) were added successively, and the mixture was stirred at 70°C for 3 hours. To the resulting mixture, an aqueous saturated ammonium chloride solution was added, and the mixture was extracted with ethyl acetate, and the resulting organic layer was washed with saturated brine and concentrated under reduced pressure. The resulting residue was subjected to a silica gel column chromatography to obtain the Intermediate compound 27 (0.13 g) represented by the following formula. 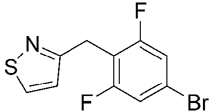 intermediate compound 27: ¹H-NMR (CDCl₃) δ: 8.58 (1H, d), 7.13-7.09 (2H, m), 7.07 (1H, d), 4.20 (2H, s).

### Reference Preparation Example 10

Under nitrogen atmosphere, to a mixture of the Intermediate compound 12 (5.0 g), triethylamine (6.0 mL), and acetonitrile (30 mL), acetic anhydride (1.5 mL) was added dropwise at 0°C, and the mixture was stirred at room temperature for 1 hour. To the resulting mixture, water was added, and the solids precipitated out were collected by filtration, and washed with hexane. A mixture of the obtained solids and toluene was heated to 110°C, and the mixture was stirred for 30 minutes. The resulting solution was left to stand to cool to room temperature, and the solids precipitated out were collected by filtration to obtain the Intermediate compound 28 represented by the following formula (2.1 g). 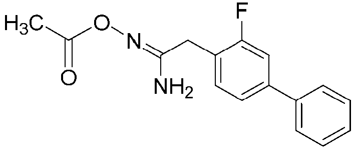 Intermediate compound 28: ¹H-NMR (DMSO-D₆) δ: 7.73-7.67 (2H, m), 7.54-7.44 (5H, m), 7.42-7.36 (1H, m), 6.52 (2H, s), 3.46 (2H, s), 2.05 (3H, s).

### Reference Preparation Example 10-1

The compounds prepared according to the Reference Preparation Example 10 and physical properties thereof are shown below.

A compound represented by formula (IA-2) wherein the combination of R¹³, R^{2a}, R^{4A}, R^{5A}, R^{5B}, R^{5C}, R^{5D}, and E represents any one combination indicated in Table B-6.

**[Table B-6]**

| Intermediate compound | R^{1a} | R^{2a} | R^{4A} | R^{5A} | R^{5B} | R^{5C} | R^{5D} | E |
|---|---|---|---|---|---|---|---|---|
| 29 | H | H | C(O)CH₃ | F | H | H | H | 2,4-F₂-Ph |

| | | | | | | | | |
|---|---|---|---|---|---|---|---|---|
| Intermediate compound 29: LC/MS: 323 [M+H]⁺, RT = 1.682 min. | | | | | | | | |

The compounds prepared according to the Reference Preparation Example 10 and physical properties thereof are shown below.

Examples of the above-mentioned Production Methods, and the Present compounds prepared according to the Preparation Examples (including the compounds of the Present invention) are shown below.

A compound represented by formula (A-1): 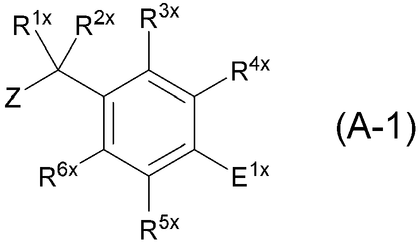 (hereinafter, referred to as "Compound (A-1)") wherein Z represents a 1H-pyrazol-1-yl group, and the combination of R^{1X}, R^{2X}, R^{3X}, R^{4X}, R^{5X}, R^{6X}, and E^{1X} represents any combinations indicated in the Combination A (hereinafter, referred to as "Compound group SX1").

The combination A consists of the substituent numbers A1 to A2112. The substituent numbers A1 to A2112 represents any combinations of R^{1X}, R^{2X}, R^{3X}, R^{4X}, R^{5X}, R^{6X}, and E^{1X} in the Compound (A-1) and is hereinafter expressed as [Substituent number: R^{1X}, R^{2X}, R^{3X}, R^{4X}, R^{5X}, R^{6X}, and E^{1X}].

For example, the substituent number A3 represents the combination wherein each of R^{1X}, R^{2X}, R^{4X}, R^{5X}, and R^{6X} represents a hydrogen atom, R^{3X} represents a methyl group, and E^{1X} represents a phenyl group.
Combination A: [A1;H,H,F,H,H,H,Ph], [A2;H,H,Cl,H,H,H,Ph], [A3;H,H,Me,H,H,H,Ph], [A4;H,H,CN,H,H,H,Ph], [A5;H,H,OH,H,H,H,Ph], [A6;H,H,cPr,H,H,H,Ph], [A7;H,H,F,F,H,H,Ph], [A8;H,H,F,H,F,H,Ph], [A9;H,H,F,H,H,F,Ph], [A10;H,H,Cl,H,H,Cl,Ph], [A11;H,H,Me,H,H,Me,Ph], [A12;H,H,F,F,F,F,Ph], [A13;F,H,F,H,H,H,Ph], [A14;F,H,Cl,H,H,H,Ph], [A15;F,H,Me,H,H,H,Ph], [A16;F,H,CN,H,H,H,Ph], [A17;F,H,OH,H,H,H,Ph], [A18;F,H,cPr,H,H,H,Ph], [A19;F,H,F,F,H,H,Ph], [A20;F,H,F,H, F,H, Ph], [A21;F,H,F,H,H,F,Ph], [A22;F,H,Cl,H,H,Cl,Ph], [A23;F,H,Me,H,H,Me,Ph], [A24;F,H,F,F,F,F, Ph], [A25;Me,H,F,H,H,H,Ph], [A26;Me,H,Cl,H,H,H, Ph], [A27;Me,H,Me,H,H,H,Ph], [A28;Me,H,CN,H,H,H, Ph], [A29;Me,H,OH,H,H,H,Ph], [A30;Me,H,cPr,H,H,H,Ph], [A31;Me,H,F,F,H,H,Ph], [A32;Me,H,F,H,F,H, Ph], [A33;Me,H,F,H,H,F,Ph], [A34;Me,H,Cl,H,H,Cl,Ph], [A35;Me,H,Me,H,H,Me,Ph], [A36;Me,H,F,F,F,F, Ph], [A37;OMe,H,F,H,H,H,Ph], [A38;OMe,H,Cl,H,H,H,Ph], [A39;OMe,H,Me,H,H,H,Ph], [A40;OMe,H,CN,H,H,H,Ph], [A41;OMe,H,OH,H,H,H,Ph], [A42;OMe,H,cPr,H,H,H,Ph], [A43;OMe,H,F,F,H,H,Ph], [A44;OMe,H,F,H,F,H,Ph], [A45;OMe,H,F,H,H,F,Ph], [A46;OMe,H,Cl,H,H,Cl,Ph], [A47;OMe,H,Me,H,H,Me,Ph], [A48;OMe,H,F,F,F,F, Ph], [A49;OH,H,F,H,H,H,Ph], [A50;OH,H,Cl,H,H,H,Ph], [A51;OH,H,Me,H,H,H,Ph], [A52;OH,H,CN,H,H,H,Ph], [A53;OH,H,OH,H,H,H,Ph], [A54;OH,H,cPr,H,H,H,Ph], [A55;OH,H,F,F,H,H,Ph], [A56;OH,H,F,H,F,H,Ph], [A57;OH,H,F,H,H,F,Ph], [A58;OH,H,Cl,H,H,Cl,Ph], [A59;OH,H,Me,H,H,Me,Ph], [A60;OH,H,F,F,F,F, Ph], [A61;Me,Me,F,H,H,H,Ph], [A62;Me,Me,Cl,H,H,H, Ph], [A63;Me,Me,Me,H,H,H,Ph], [A64;Me,Me,CN,H,H,H,Ph], [A65;Me,Me,OH,H,H,H,Ph], [A66;Me,Me,cPr,H,H,H,Ph], [A67;Me,Me,F,F,H,H,Ph], [A68;Me,Me,F,H,F,H,Ph], [A69;Me,Me,F,H,H,F,Ph], [A70;Me,Me,Cl,H,H,Cl,Ph], [A71;Me,Me,Me,H,H,Me,Ph], [A72;Me,Me,F,F,F,F,Ph], [A73;F,F,F,H,H,H,Ph], [A74;F,F,Cl,H,H,H,Ph], [A75;F,F,Me,H,H,H,Ph], [A76;F,F,CN,H,H,H,Ph], [A77;F,F,OH,H,H,H,Ph], [A78;F,F,cPr,H,H,H,Ph], [A79;F,F,F,F,H,H,Ph], [A80;F,F,F,H,F,H,Ph], [A81;F,F,F,H,H,F,Ph], [A82;F,F,Cl,H,H,Cl,Ph], [A83;F,F,Me,H,H,Me,Ph], [A84;F,F,F,F,F,F,Ph], [A85;OH,Me,F,H,H,H,Ph], [A86;OH,Me,Cl,H,H,H,Ph], [A87;OH,Me,Me,H,H,H,Ph], [A88;OH,Me,CN,H,H,H,Ph], [A89;OH,Me,OH,H,H,H,Ph], [A90;OH,Me,cPr,H,H,H,Ph], [A91;OH,Me,F,F,H,H,Ph], [A92;OH,Me,F,H,F,H,Ph], [A93;OH,Me,F,H,H,F,Ph], [A94;OH,Me,Cl,H,H,Cl,Ph], [A95;OH,Me,Me,H,H,Me,Ph], [A96;OH,Me,F,F,F,F,Ph], [A97;H,H,F,H,H,H,2-F-Ph], [A98;H,H,Cl,H,H,H,2-F-Ph], [A99;H,H,Me,H,H,H,2-F-Ph], [A100;H,H,CN,H,H,H,2-F-Ph], [A101;H,H,OH,H,H,H,2-F-Ph], [A102;H,H,cPr,H,H,H,2-F-Ph], [A103;H,H,F,F,H,H,2-F-Ph], [A104;H,H,F,H,F,H,2-F-Ph], [A105;H,H,F,H,H,F,2-F-Ph], [A106;H,H,Cl,H,H,Cl,2-F-Ph], [A107;H,H,Me,H,H,Me,2-F-Ph], [A108;H,H,F,F,F,F,2-F-Ph], [A109;F,H,F,H,H,H,2-F-Ph], [A110;F,H,Cl,H,H,H,2-F-Ph], [A111;F,H,Me,H,H,H,2-F-Ph], [A112;F,H,CN,H,H,H,2-F-Ph], [A113;F,H,OH,H,H,H,2-F-Ph], [A114;F,H,cPr,H,H,H,2-F-Ph], [A115;F,H,F,F,H4,H,2-F-Ph], [A116;F,H,F,H,F,H,2-F-Ph], [A117;F,H,F,H4,H,F,2-F-Ph], [A118;F,H,Cl,H,H,Cl,2-F-Ph], [A119;F,H,Me,H,H,Me,2-F-Ph], [A120;F,H,F,F,F,F,2-F-Ph], [A121;Me,H,F,H,H,H,2-F-Ph], [A122;Me,H,Cl,H,H,H,2-F-Ph], [A123;Me,H,Me,H,H,H,2-F-Ph], [A124;Me,H,CN,H,H,H,2-F-Ph], [A125;Me,H,OH,H,H,H,2-F-Ph], [A126;Me,H,cPr,H,H,H,2-F-Ph], [A127;Me,H,F,F,H,H,2-F-Ph], [A128;Me,H,F,H,F,H,2-F-Ph], [A129;Me,H,F,H,H,F,2-F-Ph], [A130;Me,H,Cl,H,H,Cl,2-F-Ph], [A131;Me,H,Me,H,H,Me,2-F-Ph], [A132;Me,H,F,F,F,F,2-F-Ph], [A133;OMe,H,F,H,H,H,2-F-Ph], [A134;OMe,H,Cl,H,H,H,2-F-Ph], [A135;OMe,H,Me,H,H,H,2-F-Ph], [A136;OMe,H,CN,H,H,H,2-F-Ph], [A137;OMe,H,OH,H,H,H,2-F-Ph], [A138;OMe,H,cPr,H,H,H,2-F-Ph], [A139;OMe,H,F,F,H,H,2-F-Ph], [A140;OMe,H,F,H,F,H,2-F-Ph], [A141;OMe,H,F,H,H,F,2-F-Ph], [A142;OMe,H,Cl,H,H,Cl,2-F-Ph], [A143;OMe,H,Me,H,H,Me,2-F-Ph], [A144;OMe,H,F,F,F,F,2-F-Ph], [A145;OH,H,F,H,H,H,2-F-Ph], [A146;OH,H,Cl,H,H,H,2-F-Ph], [A147;OH,H,Me,H,H,H,2-F-Ph], [A148;OH,H,CN,H,H,H,2-F-Ph], [A149;OH,H,OH,H,H,H,2-F-Ph], [A150;OH,H,cPr,H,H,H,2-F-Ph], [A151;OH,H,F,F,H,H,2-F-Ph], [A152;OH,H,F,H,F,H,2-F-Ph], [A153;OH,H,F,H,H,F,2-F-Ph], [A154;OH,H,Cl,H,H,Cl,2-F-Ph], [A155;OH,H,Me,H,H,Me,2-F-Ph], [A156;OH,H,F,F,F,F,2-F-Ph], [A157;Me,Me,F,H,H,H,2-F-Ph], [A158;Me,Me,Cl,H,H,H,2-F-Ph], [A159;Me,Me,Me,H,H,H,2-F-Ph], [A160;Me,Me,CN,H,H,H,2-F-Ph], [A161;Me,Me,OH,H,H,H,2-F-Ph], [A162;Me,Me,cPr,H,H,H,2-F-Ph], [A163;Me,Me,F,F,H,H,2-F-Ph], [A164;Me,Me,F,H,F,H,2-F-Ph], [A165;Me,Me,F,H,H,F,2-F-Ph], [A166;Me,Me,Cl,H,H,Cl,2-F-Ph], [A167;Me,Me,Me,H,H,Me,2-F-Ph], [A168;Me,Me,F,F,F,F,2-F-Ph], [A169;F,F,F,H,H,H,2-F-Ph], [A170;F,F,Cl,H,H,H,2-F-Ph], [A171;F,F,Me,H,H,H,2-F-Ph], [A172;F,F,CN,H,H,H,2-F-Ph], [A173;F,F,OH,H,H,H,2-F-Ph], [A174;F,F,cPr,H,H,H,2-F-Ph], [A175;F,F,F,F,H,H,2-F-Ph], [A176;F,F,F,H,F,H,2-F-Ph], [A177;F,F,F,H,H,F,2-F-Ph], [A178;F,F,Cl,H,H,Cl,2-F-Ph], [A179;F,F,Me,H,H,Me,2-F-Ph], [A180;F,F,F,F,F,F,2-F-Ph], [A181;OH,Me,F,H,H,H,2-F-Ph], [A182;OH,Me,Cl,H,H,H,2-F-Ph], [A183;OH,Me,Me,H,H,H,2-F-Ph], [A184;OH,Me,CN,H,H,H,2-F-Ph], [A185;OH,Me,OH,H,H,H,2-F-Ph], [A186;OH,Me,cPr,H,H,H,2-F-Ph], [A187;OH,Me,F,F,H,H,2-F-Ph], [A188;OH,Me,F,H,F,H,2-F-Ph], [A189;OH,Me,F,H,H,F,2-F-Ph], [A190;OH,Me,Cl,H,H, Cl,2-F-Ph], [A191;OH,Me,Me,H,H,Me,2-F-Ph], [A192;OH,Me,F,F,F,F,2-F-Ph], [A193;H,H,F,H,H,H,3-F-Ph], [A194;H,H,Cl,H,H,H,3-F-Ph], [A195;H,H,Me,H,H,H,3-F-Ph], [A196;H,H,CN,H,H,H,3-F-Ph], [A197;H,H,OH,H,H,H,3-F-Ph], [A198;H,H,cPr,H,H,H,3-F-Ph], [A199;H,H,F,F,H,H,3-F-Ph], [A200;H,H,F,H,F,H,3-F-Ph], [A201;H,H,F,H,H,F,3-F-Ph], [A202;H,H,Cl,H,H,Cl,3-F-Ph], [A203;H,H,Me,H,H,Me,3-F-Ph], [A204;H,H,F,F,F,F,3-F-Ph], [A205;F,H,F,H,H,H,3-F-Ph], [A206;F,H,Cl,H,H,H,3-F-Ph], [A207;F,H,Me,H,H,H,3-F-Ph], [A208;F,H,CN,H,H,H,3-F-Ph], [A209;F,H,OH,H,H,H,3-F-Ph], [A210;F,H,cPr,H,H,H,3-F-Ph], [A211;F,H,F,F,H,H,3-F-Ph], [A212;F,H,F,H,F,H,3-F-Ph], [A213;F,H,F,H,H,F,3-F-Ph], [A214;F,H,Cl,H,H,Cl,3-F-Ph], [A215;F,H,Me,H,H,Me,3-F-Ph], [A216;F,H,F,F,F,F,3-F-Ph], [A217;Me,H,F,H,H,H,3-F-Ph], [A218;Me,H,Cl,H,H,H,3-F-Ph], [A219;Me,H,Me,H,H,H,3-F-Ph], [A220;Me,H,CN,H,H,H,3-F-Ph], [A221;Me,H,OH,H,H,H,3-F-Ph], [A222;Me,H,cPr,H,H,H,3-F-Ph], [A223;Me,H,F,F,H,H,3-F-Ph], [A224;Me,H,F,H,F,H,3-F-Ph], [A225;Me,H,F,H,H,F,3-F-Ph], [A226;Me,H,Cl,H,H,Cl,3-F-Ph], [A227;Me,H,Me,H,H,Me,3-F-Ph], [A228;Me,H,F,F,F,F,3-F-Ph], [A229;OMe,H,F,H,H,H,3-F-Ph], [A230;OMe,H,Cl,H,H,H,3-F-Ph], [A231;OMe,H,Me,H,H,H,3-F-Ph], [A232;OMe,H,CN,H,H,H,3-F-Ph], [A233;OMe,H,OH,H,H,H,3-F-Ph], [A234;OMe,H,cPr,H,H,H,3-F-Ph], [A235;OMe,H,F,F,H,H,3-F-Ph], [A236;OMe,H,F,H,F,H,3-F-Ph], [A237;OMe,H,F,H,H,F,3-F-Ph], [A238;OMe,H,Cl,H,H,Cl,3-F-Ph], [A239;OMe,H,Me,H,H,Me,3-F-Ph], [A240;OMe,H,F,F,F,F,3-F-Ph], [A241;OH,H,F,H,H,H,3-F-Ph], [A242;OH,H,Cl,H,H,H,3-F-Ph], [A243;OH,H,Me,H,H,H,3-F-Ph], [A244;OH,H,CN,H,H,H,3-F-Ph], [A245;OH,H,OH,H,H,H,3-F-Ph], [A246;OH,H,cPr,H,H,H,3-F-Ph], [A247;OH,H,F,F,H,H,3-F-Ph], [A248;OH,H,F,H,F,H,3-F-Ph], [A249;OH,H,F,H,H,F,3-F-Ph], [A250;OH,H,Cl,H,H,Cl,3-F-Ph], [A251;OH,H,Me,H,H,Me,3-F-Ph], [A252;OH,H,F,F,F,F,3-F-Ph], [A253;Me,Me,F,H,H,H,3-F-Ph], [A254;Me,Me,Cl,H,H,H,3-F-Ph], [A255;Me,Me,Me,H,H,H,3-F-Ph], [A256;Me,Me,CN,H,H,H,3-F-Ph], [A257;Me,Me,OH,H,H,H,3-F-Ph], [A258;Me,Me,cPr,H,H,H,3-F-Ph], [A259;Me,Me,F,F,H,H,3-F-Ph], [A260;Me,Me,F,H,F,H,3-F-Ph], [A261;Me,Me,F,H,H,F,3-F-Ph], [A262;Me,Me,Cl,H,H,Cl,3-F-Ph], [A263;Me,Me,Me,H,H,Me,3-F-Ph], [A264;Me,Me,F,F,F,F,3-F-Ph], [A265;F,F,F,H,H,H,3-F-Ph], [A266;F,F,Cl,H,H,H,3-F-Ph], [A267;F,F,Me,H,H,H,3-F-Ph], [A268;F,F,CN,H,H,H,3-F-Ph], [A269;F,F,OH,H,H,H,3-F-Ph] , [A270;F,F,cPr,H,H,H,3-F-Ph], [A271;F,F,F,F,H,H,3-F-Ph], [A272;F,F,F,H,F,H,3-F-Ph], [A273;F,F,F,H,H,F,3-F-Ph], [A274;F,F,Cl,H,H,Cl,3-F-Ph], [A275;F,F,Me,H,H,Me,3-F-Ph], [A276;F,F,F,F,F,F,3-F-Ph], [A277;OH,Me,F,H,H,H,3-F-Ph], [A278;OH,Me,Cl,H,H,H,3-F-Ph], [A279;OH,Me,Me,H,H,H,3-F-Ph], [A280;OH,Me,CN,H,H,H,3-F-Ph], [A281;OH,Me,OH,H,H,H,3-F-Ph], [A282;OH,Me,cPr,H,H,H,3-F-Ph], [A283;OH,Me,F,F,H,H,3-F-Ph], [A284;OH,Me,F,H,F,H,3-F-Ph], [A285;OH,Me,F,H,H,F,3-F-Ph], [A286;OH,Me,Cl,H,H,Cl,3-F-Ph], [A287;OH,Me,Me,H,H,Me,3-F-Ph], [A288;OH,Me,F,F,F,F,3-F-Ph], [A289;H,H,F,H,H,H,4-F-Ph], [A290;H,H,Cl,H,H,H,4-F-Ph], [A291;H,H,Me,H,H,H,4-F-Ph], [A292;H,H,CN,H,H,H,4-F-Ph], [A293;H,H,OH,H,H,H,4-F-Ph], [A294;H,H,cPr,H,H,H,4-F-Ph], [A295;H,H,F,F,H,H, 4-F-Ph], [A296;H,H,F,H,F,H,4-F-Ph], [A297;H,H,F,H,H,F,4-F-Ph], [A298;H,H,Cl,H,H,Cl,4-F-Ph], [A299;H,H,Me,H,H,Me,4-F-Ph], [A300;H,H,F,F,F,F,4-F-Ph], [A301;F,H,F,H,H,H,4-F-Ph], [A302;F,H,Cl,H,H,H,4-F-Ph], [A303;F,H,Me,H,H,H,4-F-Ph], [A304;F,H,CN,H,H,H,4-F-Ph], [A305;F,H,OH,H,H,H,4-F-Ph], [A306;F,H,cPr,H,H,H,4-F-Ph], [A307;F,H,F,F,H,H,4-F-Ph], [A308;F,H,F,H,F,H,4-F-Ph], [A309;F,H,F,H,H,F,4-F-Ph], [A310;F,H,Cl,H,H,Cl,4-F-Ph], [A311;F,H,Me,H,H,Me,4-F-Ph], [A312;F,H,F,F,F,F,4-F-Ph], [A313;Me,H,F,H,H,H,4-F-Ph], [A314;Me,H,Cl,H,H,H,4-F-Ph], [A315;Me,H,Me,H,H,H,4-F-Ph], [A316;Me,H,CN,H,H,H,4-F-Ph], [A317;Me,H,OH,H,H,H,4-F-Ph], [A318;Me,H,cPr,H,H,H,4-F-Ph], [A319;Me,H,F,F,H,H,4-F-Ph], [A320;Me,H,F,H,F,H,4-F-Ph], [A321;Me,H,F,H,H,F,4-F-Ph], [A322;Me,H,Cl,H,H,Cl,4-F-Ph], [A323;Me,H,Me,H,H,Me,4-F-Ph], [A324;Me,H,F,F,F,F,4-F-Ph], [A325;OMe,H,F,H,H,H,4-F-Ph], [A326;OMe,H,Cl,H,H,H,4-F-Ph], [A327;OMe,H,Me,H,H,H,4-F-Ph], [A328;OMe,H,CN,H,H,H,4-F-Ph], [A329;OMe,H,OH,H,H,H,4-F-Ph], [A330;OMe,H,cPr,H,H,H,4-F-Ph], [A331;OMe,H,F,F,H,H,4-F-Ph], [A332;OMe,H,F,H,F,H,4-F-Ph], [A333;OMe,H,F,H,H,F,4-F-Ph], [A334;OMe,H,Cl,H,H,Cl,4-F-Ph], [A335;OMe,H,Me,H,H,Me,4-F-Ph], [A336;OMe,H,F,F,F,F,4-F-Ph], [A337;OH,H,F,H,H,H,4-F-Ph], [A338;OH,H,Cl,H,H,H,4-F-Ph], [A339;OH,H,Me,H,H,H,4-F-Ph], [A340;OH,H,CN,H,H,H,4-F-Ph], [A341;OH,H,OH,H,H,H,4-F-Ph], [A342;OH,H,cPr,H,H,H,4-F-Ph], [A343;OH,H,F,F,H,H,4-F-Ph], [A344;OH,H,F,H,F,H,4-F-Ph], [A345;OH,H,F,H,H,F,4-F-Ph], [A346;OH,H,Cl,H,H,Cl,4-F-Ph], [A347;OH,H,Me,H,H,Me,4-F-Ph], [A348;OH,H,F,F,F,F,4-F-Ph], [A349;Me,Me,F,H,H,H,4-F-Ph], [A350;Me,Me,Cl,H,H,H,4-F-Ph], [A351;Me,Me,Me,H,H,H,4-F-Ph], [A352;Me,Me,CN,H,H,H,4-F-Ph], [A353;Me,Me,OH,H,H,H,4-F-Ph], [A354;Me,Me,cPr,H,H,H,4-F-Ph], [A355;Me,Me,F,F,H,H,4-F-Ph], [A356;Me,Me,F,H,F,H,4-F-Ph], [A357;Me,Me,F,H,H,F,4-F-Ph], [A358;Me,Me,Cl,H,H,Cl,4-F-Ph], [A359;Me,Me,Me,H,H,Me,4-F-Ph], [A360;Me,Me,F,F,F,F,4-F-Ph], [A361;F,F,F,H,H,H,4-F-Ph], [A362;F,F,Cl,H,H,H,4-F-Ph], [A363;F,F,Me,H,H,H,4-F-Ph], [A364;F,F,CN,H,H,H,4-F-Ph], [A365;F,F,OH,H,H,H,4-F-Ph], [A366;F,F,cPr,H,H,H,4-F-Ph], [A367;F,F,F,F,H,H,4-F-Ph], [A368;F,F,F,H,F,H,4-F-Ph], [A369;F,F,F,H,H,F,4-F-Ph], [A370;F,F,Cl,H,H,Cl,4-F-Ph], [A371;F,F,Me,H,H,Me,4-F-Ph], [A372;F,F,F,F,F,F,4-F-Ph], [A373;OH,Me,F,H,H,H,4-F-Ph], [A374;OH,Me,Cl,H,H,H,4-F-Ph], [A375;OH,Me,Me,H,H,H,4-F-Ph], [A376;OH,Me,CN,H,H,H,4-F-Ph], [A377;OH,Me,OH,H,H,H,4-F-Ph], [A378;OH,Me,cPr,H,H,H,4-F-Ph], [A379;OH,Me,F,F,H,H,4-F-Ph], [A380;OH,Me,F,H,F,H,4-F-Ph], [A381;OH,Me,F,H,H,F,4-F-Ph], [A382;OH,Me,Cl,H,H,Cl,4-F-Ph], [A383;OH,Me,Me,H,H,Me,4-F-Ph], [A384;OH,Me,F,F,F,F,4-F-Ph], [A385;H,H,F,H,H,H,2-Cl-Ph], [A386;H,H,Cl,H,H,H,2-Cl-Ph], [A387;H,H,Me,H,H,H,2-Cl-Ph], [A388;H,H,CN,H,H,H,2-Cl-Ph], [A389;H,H,OH,H,H,H,2-Cl-Ph], [A390;H,H,cPr,H,H,H,2-Cl-Ph], [A391;H,H,F,F,H,H,2-Cl-Ph], [A392;H,H,F,H,F,H,2-Cl-Ph], [A393;H,H,F,H,H,F,2-Cl-Ph], [A394;H,H,Cl,H,H,Cl,2-Cl-Ph], [A395;H,H,Me,H,H,Me,2-Cl-Ph], [A396;H,H,F,F,F,F,2-Cl-Ph], [A397;F,H,F,H,H,H,2-Cl-Ph], [A398;F,H,Cl,H,H,H,2-Cl-Ph], [A399;F,H,Me,H,H,H,2-Cl-Ph], [A400;F,H,CN,H,H,H,2-Cl-Ph], [A401;F,H,OH,H,H,H,2-Cl-Ph], [A402;F,H,cPr,H,H,H,2-Cl-Ph], [A403;F,H,F,F,H,H,2-Cl-Ph], [A404;F,H,F,H,F,H,2-Cl-Ph], [A405;F,H,F,H,H,F,2-Cl-Ph], [A406;F,H,Cl,H,H,Cl,2-Cl-Ph], [A407;F,H,Me,H,H,Me,2-Cl-Ph], [A408;F,H,F,F,F,F,2-Cl-Ph], [A409;Me,H,F,H,H,H,2-Cl-Ph], [A410;Me,H,Cl,H,H,H,2-Cl-Ph], [A411;Me,H,Me,H,H,H,2-Cl-Ph], [A412;Me,H,CN,H,H,H,2-Cl-Ph], [A413;Me,H,OH,H,H,H,2-Cl-Ph], [A414;Me,H,cPr,H,H,H,2-Cl-Ph], [A415;Me,H,F,F,H,H,2-Cl-Ph], [A416;Me,H,F,H,F,H,2-Cl-Ph], [A417;Me,H,F,H,H,F,2-Cl-Ph], [A418;Me,H,Cl,H,H,Cl,2-Cl-Ph], [A419;Me,H,Me,H,H,Me,2-Cl-Ph], [A420;Me,H,F,F,F,F,2-Cl-Ph], [A421;OMe,H,F,H,H,H,2-Cl-Ph], [A422;OMe,H,Cl,H,H,H,2-Cl-Ph], [A423;OMe,H,Me,H,H,H,2-Cl-Ph], [A424;OMe,H,CN,H,H,H,2-Cl-Ph], [A425;OMe,H,OH,H,H,H,2-Cl-Ph], [A426;OMe,H,cPr,H,H,H,2-Cl-Ph], [A427;OMe,H,F,F,H,H,2-Cl-Ph], [A428;OMe,H,F,H,F,H,2-Cl-Ph], [A429;OMe,H,F,H,H,F,2-Cl-Ph], [A430;OMe,H,Cl,H,H,Cl,2-Cl-Ph], [A431;OMe,H,Me,H,H,Me,2-Cl-Ph], [A432;OMe,H,F,F,F,F,2-Cl-Ph], [A433;OH,H,F,H,H,H,2-Cl-Ph], [A434;OH,H,Cl,H,H,H,2-Cl-Ph], [A435;OH,H,Me,H,H,H,2-Cl-Ph], [A436;OH,H,CN,H,H,H,2-Cl-Ph], [A437;OH,H,OH,H,H,H,2-Cl-Ph], [A438;OH,H,cPr,H,H,H,2-Cl-Ph], [A439;OH,H,F,F,H,H,2-Cl-Ph], [A440;OH,H,F,H,F,H,2-Cl-Ph], [A441;OH,H,F,H,H,F,2-Cl-Ph], [A442;OH,H,Cl,H,H,Cl,2-Cl-Ph], [A443;OH,H,Me,H,H,Me,2-Cl-Ph], [A444;OH,H,F,F,F,F,2-Cl-Ph], [A445;Me,Me,F,H,H,H,2-Cl-Ph], [A446;Me,Me,Cl,H,H,H,2-Cl-Ph], [A447;Me,Me,Me,H,H,H,2-Cl-Ph], [A448;Me,Me,CN,H,H,H,2-Cl-Ph], [A449;Me,Me,OH,H,H,H,2-Cl-Ph], [A450;Me,Me,cPr,H,H,H,2-Cl-Ph], [A451;Me,Me,F,F,H,H,2-Cl-Ph], [A452;Me,Me,F,H,F,H,2-Cl-Ph], [A453;Me,Me,F,H,H,F,2-Cl-Ph], [A454;Me,Me,Cl,H,H,Cl,2-Cl-Ph], [A455;Me,Me,Me,H,H,Me,2-Cl-Ph], [A456;Me,Me,F,F,F,F,2-Cl-Ph], [A457;F,F,F,H,H,H,2-Cl-Ph], [A458;F,F,Cl,H,H,H,2-Cl-Ph], [A459;F,F,Me,H,H,H,2-Cl-Ph], [A460;F,F,CN,H,H,H,2-Cl-Ph], [A461;F,F,OH,H,H,H,2-Cl-Ph], [A462;F,F,cPr,H,H,H,2-Cl-Ph], [A463;F,F,F,F,H,H,2-Cl-Ph], [A464;F,F,F,H,F,H,2-Cl-Ph], [A465;F,F,F,H,H,F,2-Cl-Ph], [A466;F,F,Cl,H,H,Cl,2-Cl-Ph], [A467;F,F,Me,H,H,Me,2-Cl-Ph], [A468;F,F,F,F,F,F,2-Cl-Ph], [A469;OH,Me,F,H,H,H,2-Cl-Ph], [A470;OH,Me,Cl,H,H,H,2-Cl-Ph], [A471;OH,Me,Me,H,H,H,2-Cl-Ph], [A472;OH,Me,CN,H,H,H,2-Cl-Ph], [A473;OH,Me,OH,H,H,H,2-Cl-Ph], [A474;OH,Me,cPr,H,H,H,2-Cl-Ph], [A475;OH,Me,F,F,H,H,2-Cl-Ph], [A476;OH,Me,F,H,F,H,2-Cl-Ph], [A477;OH,Me,F,H,H,F,2-Cl-Ph], [A478;OH,Me,Cl,H,H,Cl,2-Cl-Ph], [A479;OH,Me,Me,H,H,Me,2-Cl-Ph], [A480;OH,Me,F,F,F,F,2-Cl-Ph], [A481;H,H,F,H,H,H,3-Cl-Ph], [A482;H,H,Cl,H,H,H,3-Cl-Ph], [A483;H,H,Me,H,H,H,3-Cl-Ph], [A484;H,H,CN,H,H,H,3-Cl-Ph], [A485;H,H,OH,H,H,H,3-Cl-Ph], [A486;H,H,cPr,H,H,H,3-Cl-Ph], [A487;H,H,F,F,H,H,3-Cl-Ph], [A488;H,H,F,H,F,H,3-Cl-Ph], [A489;H,H,F,H,H,F,3-Cl-Ph], [A490;H,H,Cl,H,H,Cl,3-Cl-Ph], [A491;H,H,Me,H,H,Me,3-Cl-Ph], [A492;H,H,F,F,F,F,3-Cl-Ph], [A493;F,H,F,H,H,H,3-Cl-Ph], [A494;F,H,Cl,H,H,H,3-Cl-Ph], [A495;F,H,Me,H,H,H,3-Cl-Ph], [A496;F,H,CN,H,H,H,3-Cl-Ph], [A497;F,H,OH,H,H,H,3-Cl-Ph], [A498;F,H,cPr,H,H,H,3-Cl-Ph], [A499;F,H,F,F,H,H,3-Cl-Ph], [A500;F,H,F,H,F,H,3-Cl-Ph], [A501;F,H,F,H,H,F,3-Cl-Ph], [A502;F,H,Cl,H,H,Cl,3-Cl-Ph], [A503;F,H,Me,H,H,Me,3-Cl-Ph], [A504;F,H,F,F,F,F,3-Cl-Ph], [A505;Me,H,F,H,H,H,3-Cl-Ph], [A506;Me,H,Cl,H,H,H,3-Cl-Ph], [A507;Me,H,Me,H,H,H,3-Cl-Ph], [A508;Me,H,CN,H,H,H,3-Cl-Ph], [A509;Me,H,OH,H,H,H,3-Cl-Ph], [A510;Me,H,cPr,H,H,H,3-Cl-Ph], [A511;Me,H,F,F,H,H,3-Cl-Ph], [A512;Me,H,F,H,F,H,3-Cl-Ph], [A513;Me,H,F,H,H,F,3-Cl-Ph], [A514;Me,H,Cl,H,H,Cl,3-Cl-Ph], [A515;Me,H,Me,H,H,Me,3-Cl-Ph], [A516;Me,H,F,F,F,F,3-Cl-Ph], [A517;OMe,H,F,H,H,H,3-Cl-Ph], [A518;OMe,H,Cl,H,H,H,3-Cl-Ph], [A519;OMe,H,Me,H,H,H,3-Cl-Ph], [A520;OMe,H,CN,H,H,H,3-Cl-Ph], [A521;OMe,H,OH,H,H,H,3-Cl-Ph], [A522;OMe,H,cPr,H,H,H,3-Cl-Ph], [A523;OMe,H,F,F,H,H,3-Cl-Ph], [A524;OMe,H,F,H,F,H,3-Cl-Ph], [A525;OMe,H,F,H,H,F,3-Cl-Ph], [A526;OMe,H,Cl,H,H,Cl,3-Cl-Ph], [A527;OMe,H,Me,H,H,Me,3-Cl-Ph], [A528;OMe,H,F,F,F,F,3-Cl-Ph], [A529;OH,H,F,H,H,H,3-Cl-Ph], [A530;OH,H,Cl,H,H,H,3-Cl-Ph], [A531;OH,H,Me,H,H,H,3-Cl-Ph], [A532;OH,H,CN,H,H,H,3-Cl-Ph], [A533;OH,H,OH,H,H,H,3-Cl-Ph], [A534;OH,H,cPr,H,H,H,3-Cl-Ph], [A535;OH,H,F,F,H,H,3-Cl-Ph], [A536;OH,H,F,H,F,H,3-Cl-Ph], [A537;OH,H,F,H,H,F,3-Cl-Ph], [A538;OH,H,Cl,H,H,Cl,3-Cl-Ph], [A539;OH,H,Me,H,H,Me,3-Cl-Ph], [A540;OH,H,F,F,F,F,3-Cl-Ph], [A541;Me,Me,F,H,H,H,3-Cl-Ph], [A542;Me,Me,Cl,H,H,H,3-Cl-Ph], [A543;Me,Me,Me,H,H,H,3-Cl-Ph], [A544;Me,Me,CN,H,H,H,3-Cl-Ph], [A545;Me,Me,OH,H,H,H,3-Cl-Ph], [A546;Me,Me,cPr,H,H,H,3-Cl-Ph], [A547;Me,Me,F,F,H,H,3-Cl-Ph], [A548;Me,Me,F,H,F,H,3-Cl-Ph], [A549;Me,Me,F,H,H,F,3-Cl-Ph], [A550;Me,Me,Cl,H,H,Cl,3-Cl-Ph], [A551;Me,Me,Me,H,H,Me,3-Cl-Ph], [A552;Me,Me,F,F,F,F,3-Cl-Ph], [A553;F,F,F,H,H,H,3-Cl-Ph], [A554;F,F,Cl,H,H,H,3-Cl-Ph], [A555;F,F,Me,H,H,H,3-Cl-Ph], [A556;F,F,CN,H,H,H,3-Cl-Ph], [A557;F,F,OH,H,H,H,3-Cl-Ph], [A558;F,F,cPr,H,H,H,3-Cl-Ph], [A559;F,F,F,F,H,H,3-Cl-Ph], [A560;F,F,F,H,F,H,3-Cl-Ph], [A561;F,F,F,H,H,F,3-Cl-Ph], [A562;F,F,Cl,H,H,Cl,3-Cl-Ph], [A563;F,F,Me,H,H,Me,3-Cl-Ph], [A564;F,F,F,F,F,F,3-Cl-Ph], [A565;OH,Me,F,H,H,H,3-Cl-Ph], [A566;OH,Me,Cl,H,H,H,3-Cl-Ph], [A567;OH,Me,Me,H,H,H,3-Cl-Ph], [A568;OH,Me,CN,H,H,H,3-Cl-Ph], [A569;OH,Me,OH,H,H,H,3-Cl-Ph], [A570;OH,Me,cPr,H,H,H,3-Cl-Ph], [A571;OH,Me,F,F,H,H,3-Cl-Ph], [A572;OH,Me,F,H,F,H,3-Cl-Ph], [A573;OH,Me,F,H,H,F,3-Cl-Ph], [A574;OH,Me,Cl,H,H,Cl,3-Cl-Ph], [A575;OH,Me,Me,H,H,Me,3-Cl-Ph], [A576;OH,Me,F,F,F,F,3-Cl-Ph], [A577;H,H,F,H,H,H,4-Cl-Ph], [A578;H,H,Cl,H,H,H,4-Cl-Ph], [A579;H,H,Me,H,H,H,4-Cl-Ph], [A580;H,H,CN,H,H,H,4-Cl-Ph], [A581;H,H,OH,H,H,H,4-Cl-Ph], [A582;H,H,cPr,H,H,H,4-Cl-Ph], [A583;H,H,F,F,H,H,4-Cl-Ph], [A584;H,H,F,H,F,H,4-Cl-Ph], [A585;H,H,F,H,H,F,4-Cl-Ph], [A586;H,H,Cl,H,H,Cl,4-Cl-Ph], [A587;H,H,Me,H,H,Me,4-Cl-Ph], [A588;H,H,F,F,F,F,4-Cl-Ph], [A589;F,H,F,H,H,H,4-Cl-Ph], [A590;F,H,Cl,H,H,H,4-Cl-Ph], [A591;F,H,Me,H,H,H,4-Cl-Ph], [A592;F,H,CN,H,H,H,4-Cl-Ph], [A593;F,H,OH,H,H,H,4-Cl-Ph], [A594;F,H,cPr,H,H,H,4-Cl-Ph], [A595;F,H,F,F,H,H,4-Cl-Ph], [A596;F,H,F,H,F,H,4-Cl-Ph], [A597;F,H,F,H,H,F,4-Cl-Ph], [A598;F,H,Cl,H,H,Cl,4-Cl-Ph], [A599;F,H,Me,H,H,Me,4-Cl-Ph], [A600;F,H,F,F,F,F,4-Cl-Ph], [A601;Me,H,F,H,H,H,4-Cl-Ph], [A602;Me,H,Cl,H,H,H,4-Cl-Ph], [A603;Me,H,Me,H,H,H,4-Cl-Ph], [A604;Me,H,CN,H,H,H,4-Cl-Ph], [A605;Me,H,OH,H,H,H,4-Cl-Ph], [A606;Me,H,cPr,H,H,H,4-Cl-Ph], [A607;Me,H,F,F,H,H,4-Cl-Ph], [A608;Me,H,F,H,F,H,4-Cl-Ph], [A609;Me,H,F,H,H,F,4-Cl-Ph], [A610;Me,H,Cl,H,H,Cl,4-Cl-Ph], [A611;Me,H,Me,H,H,Me,4-Cl-Ph], [A612;Me,H,F,F,F,F,4-Cl-Ph], [A613;OMe,H,F,H,H,H,4-Cl-Ph], [A614;OMe,H,Cl,H,H,H,4-Cl-Ph], [A615;OMe,H,Me,H,H,H,4-Cl-Ph], [A616;OMe,H,CN,H,H,H,4-Cl-Ph], [A617;OMe,H,OH,H,H,H,4-Cl-Ph], [A618;OMe,H,cPr,H,H,H,4-Cl-Ph], [A619;OMe,H,F,F,H,H,4-Cl-Ph], [A620;OMe,H,F,H,F,H,4-Cl-Ph], [A621;OMe,H,F,H,H,F,4-Cl-Ph], [A622;OMe,H,Cl,H,H,Cl,4-Cl-Ph], [A623;OMe,H,Me,H,H,Me,4-Cl-Ph], [A624;OMe,H,F,F,F,F,4-Cl-Ph], [A625;OH,H,F,H,H,H,4-Cl-Ph], [A626;OH,H,Cl,H,H,H,4-Cl-Ph], [A627;OH,H,Me,H,H,H,4-Cl-Ph], [A628;OH,H,CN,H,H,H,4-Cl-Ph], [A629;OH,H,OH,H,H,H,4-Cl-Ph], [A630;OH,H,cPr,H,H,H,4-Cl-Ph], [A631;OH,H,F,F,H,H,4-Cl-Ph], [A632;OH,H,F,H,F,H,4-Cl-Ph], [A633;OH,H,F,H,H,F,4-Cl-Ph], [A634;OH,H,Cl,H,H,Cl,4-Cl-Ph], [A635;OH,H,Me,H,H,Me,4-Cl-Ph], [A636;OH,H,F,F,F,F,4-Cl-Ph], [A637;Me,Me,F,H,H,H,4-Cl-Ph], [A638;Me,Me,Cl,H,H,H,4-Cl-Ph], [A639;Me,Me,Me,H,H,H,4-Cl-Ph], [A640;Me,Me,CN,H,H,H,4-Cl-Ph], [A641;Me,Me,OH,H,H,H,4-Cl-Ph], [A642;Me,Me,cPr,H,H,H,4-Cl-Ph], [A643;Me,Me,F,F,H,H,4-Cl-Ph], [A644;Me,Me,F,H,F,H,4-Cl-Ph], [A645;Me,Me,F,H,H,F,4-Cl-Ph], [A646;Me,Me,Cl,H,H,Cl,4-Cl-Ph], [A647;Me,Me,Me,H,H,Me,4-Cl-Ph], [A648;Me,Me,F,F,F,F,4-Cl-Ph], [A649;F,F,F,H,H,H,4-Cl-Ph], [A650;F,F,Cl,H,H,H,4-Cl-Ph], [A651;F,F,Me,H,H,H,4-Cl-Ph], [A652;F,F,CN,H,H,H,4-Cl-Ph], [A653;F,F,OH,H,H,H,4-Cl-Ph], [A654;F,F,cPr,H,H,H,4-Cl-Ph], [A655;F,F,F,F,H,H,4-Cl-Ph], [A656;F,F,F,H,F,H,4-Cl-Ph], [A657;F,F,F,H,H,F,4-Cl-Ph], [A658;F,F,Cl,H,H,Cl,4-Cl-Ph], [A659;F,F,Me,H,H,Me,4-Cl-Ph], [A660;F,F,F,F,F,F,4-Cl-Ph], [A661;OH,Me,F,H,H,H,4-Cl-Ph], [A662;OH,Me,Cl,H,H,H,4-Cl-Ph], [A663;OH,Me,Me,H,H,H,4-Cl-Ph], [A664;OH,Me,CN,H,H,H,4-Cl-Ph], [A665;OH,Me,OH,H,H,H,4-Cl-Ph], [A666;OH,Me,cPr,H,H,H,4-Cl-Ph], [A667;OH,Me,F,F,H,H,4-Cl-Ph], [A668;OH,Me,F,H,F,H,4-Cl-Ph], [A669;OH,Me,F,H,H,F,4-Cl-Ph], [A670;OH,Me,Cl,H,H,Cl,4-Cl-Ph], [A671;OH,Me,Me,H,H,Me,4-Cl-Ph], [A672;OH,Me,F,F,F,F,4-Cl-Ph], [A673;H,H,F,H,H,H,2-Me-Ph], [A674;H,H,C1,H,H,H,2-Me-Ph], [A675;H,H,Me,H,H,H,2-Me-Ph], [A676;H,H,CN,H,H,H,2-Me-Ph], [A677;H,H,OH,H,H,H,2-Me-Ph], [A678;H,H,cPr,H,H,H,2-Me-Ph], [A679;H,H,F,F,H,H,2-Me-Ph], [A680;H,H,F,H,F,H,2-Me-Ph], [A681;H,H,F,H,H,F,2-Me-Ph], [A682;H,H,Cl,H,H,Cl,2-Me-Ph], [A683;H,H,Me,H,H,Me,2-Me-Ph], [A684;H,H,F,F,F,F,2-Me-Ph], [A685;F,H,F,H,H,H,2-Me-Ph], [A686;F,H,Cl,H,H,H,2-Me-Ph], [A687;F,H,Me,H,H,H,2-Me-Ph], [A688;F,H,CN,H,H,H,2-Me-Ph], [A689;F,H,OH,H,H,H,2-Me-Ph], [A690;F,H,cPr,H,H,H,2-Me-Ph], [A691;F,H,F,F,H,H,2-Me-Ph], [A692;F,H,F,H,F,H,2-Me-Ph], [A693;F,H,F,H,H,F,2-Me-Ph], [A694;F,H,Cl,H,H,Cl,2-Me-Ph], [A695;F,H,Me,H,H,Me,2-Me-Ph], [A696;F,H,F,F,F,F,2-Me-Ph], [A697;Me,H,F,H,H,H,2-Me-Ph], [A698;Me,H,Cl,H,H,H,2-Me-Ph], [A699;Me,H,Me,H,H,H,2-Me-Ph], [A700;Me,H,CN,H,H,H,2-Me-Ph], [A701;Me,H,OH,H,H,H,2-Me-Ph], [A702;Me,H,cPr,H,H,H,2-Me-Ph], [A703;Me,H,F,F,H,H,2-Me-Ph], [A704;Me,H,F,H,F,H,2-Me-Ph], [A705;Me,H,F,H,H,F,2-Me-Ph], [A706;Me,H,Cl,H,H,Cl,2-Me-Ph], [A707;Me,H,Me,H,H,Me,2-Me-Ph], [A708;Me,H,F,F,F,F,2-Me-Ph], [A709;OMe,H,F,H,H,H,2-Me-Ph], [A710;OMe,H,Cl,H,H,H,2-Me-Ph], [A711;OMe,H,Me,H,H,H,2-Me-Ph], [A712;OMe,H,CN,H,H,H,2-Me-Ph], [A713;OMe,H,OH,H,H,H,2-Me-Ph], [A714;OMe,H,cPr,H,H,H,2-Me-Ph], [A715;OMe,H,F,F,H,H,2-Me-Ph], [A716;OMe,H,F,H,F,H,2-Me-Ph], [A717;OMe,H,F,H,H,F,2-Me-Ph], [A718;OMe,H,Cl,H,H,C1,2-Me-Ph], [A719;OMe,H,Me,H,H,Me,2-Me-Ph], [A720;OMe,H,F,F,F,F,2-Me-Ph], [A721;OH,H,F,H,H,H,2-Me-Ph], [A722;OH,H,Cl,H,H,H,2-Me-Ph], [A723;OH,H,Me,H,H,H,2-Me-Ph], [A724;OH,H,CN,H,H,H,2-Me-Ph], [A725;OH,H,OH,H,H,H,2-Me-Ph], [A726;OH,H,cPr,H,H,H,2-Me-Ph], [A727;OH,H,F,F,H,H,2-Me-Ph], [A728;OH,H,F,H,F,H,2-Me-Ph], [A729;OH,H,F,H,H,F,2-Me-Ph], [A730;OH,H,Cl,H,H,Cl,2-Me-Ph], [A731;OH,H,Me,H,H,Me,2-Me-Ph], [A732;OH,H,F,F,F,F,2-Me-Ph], [A733;Me,Me,F,H,H,H,2-Me-Ph], [A734;Me,Me,Cl,H,H,H,2-Me-Ph], [A735;Me,Me,Me,H,H,H,2-Me-Ph], [A736;Me,Me,CN,H,H,H,2-Me-Ph], [A737;Me,Me,OH,H,H,H,2-Me-Ph], [A738;Me,Me,cPr,H,H,H,2-Me-Ph], [A739;Me,Me,F,F,H,H,2-Me-Ph], [A740;Me,Me,F,H,F,H,2-Me-Ph], [A741;Me,Me,F,H,H,F,2-Me-Ph], [A742;Me,Me,Cl,H,H,Cl,2-Me-Ph], [A743;Me,Me,Me,H,H,Me,2-Me-Ph], [A744;Me,Me,F,F,F,F,2-Me-Ph], [A745;F,F,F,H,H,H,2-Me-Ph], [A746;F,F,Cl,H,H,H,2-Me-Ph], [A747;F,F,Me,H,H,H,2-Me-Ph], [A748;F,F,CN,H,H,H,2-Me-Ph], [A749;F,F,OH,H,H,H,2-Me-Ph], [A750;F,F,cPr,H,H,H,2-Me-Ph], [A751;F,F,F,F,H,H,2-Me-Ph], [A752;F,F,F,H,F,H,2-Me-Ph], [A753;F,F,F,H,H,F,2-Me-Ph], [A754;F,F,Cl,H,H,Cl,2-Me-Ph], [A755;F,F,Me,H,H,Me,2-Me-Ph], [A756;F,F,F,F,F,F,2-Me-Ph], [A757;OH,Me,F,H,H,H,2-Me-Ph], [A758;OH,Me,Cl,H,H,H,2-Me-Ph], [A759;OH,Me,Me,H,H,H,2-Me-Ph], [A760;OH,Me,CN,H,H,H,2-Me-Ph], [A761;OH,Me,OH,H,H,H,2-Me-Ph], [A762;OH,Me,cPr,H,H,H,2-Me-Ph], [A763;OH,Me,F,F,H,H,2-Me-Ph], [A764;OH,Me,F,H,F,H,2-Me-Ph], [A765;OH,Me,F,H,H,F,2-Me-Ph], [A766;OH,Me,Cl,H,H,Cl,2-Me-Ph], [A767;OH,Me,Me,H,H,Me,2-Me-Ph], [A768;OH,Me,F,F,F,F,2-Me-Ph], [A769;H,H,F,H,H,H,3-Me-Ph], [A770;H,H,Cl,H,H,H,3-Me-Ph], [A771;H,H,Me,H,H,H,3-Me-Ph], [A772;H,H,CN,H,H,H,3-Me-Ph], [A773;H,H,OH,H,H,H,3-Me-Ph], [A774;H,H,cPr,H,H,H,3-Me-Ph], [A775;H,H,F,F,H,H,3-Me-Ph], [A776;H,H,F,H,F,H,3-Me-Ph], [A777;H,H,F,H,H,F,3-Me-Ph], [A778;H,H,Cl,H,H,Cl,3-Me-Ph], [A779;H,H,Me,H,H,Me,3-Me-Ph], [A780;H,H,F,F,F,F,3-Me-Ph], [A781;F,H,F,H,H,H,3-Me-Ph], [A782;F,H,Cl,H,H,H,3-Me-Ph], [A783;F,H,Me,H,H,H,3-Me-Ph], [A784;F,H,CN,H,H,H,3-Me-Ph], [A785;F,H,OH,H,H,H,3-Me-Ph], [A786;F,H,cPr,H,H,H,3-Me-Ph], [A787;F,H,F,F,H,H,3-Me-Ph], [A788;F,H,F,H,F,H,3-Me-Ph], [A789;F,H,F,H,H,F,3-Me-Ph], [A790;F,H,Cl,H,H,Cl,3-Me-Ph], [A791;F,H,Me,H,H,Me,3-Me-Ph], [A792;F,H,F,F,F,F,3-Me-Ph], [A793;Me,H,F,H,H,H,3-Me-Ph], [A794;Me,H,Cl,H,H,H,3-Me-Ph], [A795;Me,H,Me,H,H,H,3-Me-Ph], [A796;Me,H,CN,H,H,H,3-Me-Ph], [A797;Me,H,OH,H,H,H,3-Me-Ph], [A798;Me,H,cPr,H,H,H,3-Me-Ph], [A799;Me,H,F,F,H,H,3-Me-Ph], [A800;Me,H,F,H,F,H,3-Me-Ph], [A801;Me,H,F,H,H,F,3-Me-Ph], [A802;Me,H,Cl,H,H,Cl,3-Me-Ph], [A803;Me,H,Me,H,H,Me,3-Me-Ph], [A804;Me,H,F,F,F,F,3-Me-Ph], [A805;OMe,H,F,H,H,H,3-Me-Ph], [A806;OMe,H,Cl,H,H,H,3-Me-Ph], [A807;OMe,H,Me,H,H,H,3-Me-Ph], [A808;OMe,H,CN,H,H,H,3-Me-Ph], [A809;OMe,H,OH,H,H,H,3-Me-Ph], [A810;OMe,H,cPr,H,H,H,3-Me-Ph], [A811;OMe,H,F,F,H,H,3-Me-Ph], [A812;OMe,H,F,H,F,H,3-Me-Ph], [A813;OMe,H,F,H,H,F,3-Me-Ph], [A814;OMe,H,Cl,H,H,Cl,3-Me-Ph], [A815;OMe,H,Me,H,H,Me,3-Me-Ph], [A816;OMe,H,F,F,F,F,3-Me-Ph], [A817;OH,H,F,H,H,H,3-Me-Ph], [A818;OH,H,Cl,H,H,H,3-Me-Ph], [A819;OH,H,Me,H,H,H,3-Me-Ph], [A820;OH,H,CN,H,H,H,3-Me-Ph], [A821;OH,H,OH,H,H,H,3-Me-Ph], [A822;OH,H,cPr,H,H,H,3-Me-Ph], [A823;OH,H,F,F,H,H,3-Me-Ph], [A824;OH,H,F,H,F,H,3-Me-Ph], [A825;OH,H,F,H,H,F,3-Me-Ph], [A826;OH,H,Cl,H,H,Cl,3-Me-Ph], [A827;OH,H,Me,H,H,Me,3-Me-Ph], [A828;OH,H,F,F,F,F,3-Me-Ph], [A829;Me,Me,F,H,H,H,3-Me-Ph], [A830;Me,Me,Cl,H,H,H,3-Me-Ph], [A831;Me,Me,Me,H,H,H,3-Me-Ph], [A832;Me,Me,CN,H,H,H,3-Me-Ph], [A833;Me,Me,OH,H,H,H,3-Me-Ph], [A834;Me,Me,cPr,H,H,H,3-Me-Ph], [A835;Me,Me,F,F,H,H,3-Me-Ph], [A836;Me,Me,F,H,F,H,3-Me-Ph], [A837;Me,Me,F,H,H,F,3-Me-Ph], [A838;Me,Me,Cl,H,H,Cl,3-Me-Ph], [A839;Me,Me,Me,H,H,Me,3-Me-Ph], [A840;Me,Me,F,F,F,F,3-Me-Ph], [A841;F,F,F,H,H,H,3-Me-Ph], [A842;F,F,Cl,H,H,H,3-Me-Ph], [A843;F,F,Me,H,H,H,3-Me-Ph], [A844;F,F,CN,H,H,H,3-Me-Ph], [A845;F,F,OH,H,H,H,3-Me-Ph], [A846;F,F,cPr,H,H,H,3-Me-Ph], [A847;F,F,F,F,H,H,3-Me-Ph], [A848;F,F,F,H,F,H,3-Me-Ph], [A849;F,F,F,H,H,F,3-Me-Ph], [A850;F,F,Cl,H,H,Cl,3-Me-Ph], [A851;F,F,Me,H,H,Me,3-Me-Ph], [A852;F,F,F,F,F,F,3-Me-Ph], [A853;OH,Me,F,H,H,H,3-Me-Ph], [A854;OH,Me,Cl,H,H,H,3-Me-Ph], [A855;OH,Me,Me,H,H,H,3-Me-Ph], [A856;OH,Me,CN,H,H,H,3-Me-Ph], [A857;OH,Me,OH,H,H,H,3-Me-Ph], [A858;OH,Me,cPr,H,H,H,3-Me-Ph], [A859;OH,Me,F,F,H,H,3-Me-Ph], [A860;OH,Me,F,H,F,H,3-Me-Ph], [A861;OH,Me,F,H,H,F,3-Me-Ph], [A862;OH,Me,Cl,H,H,Cl,3-Me-Ph], [A863;OH,Me,Me,H,H,Me,3-Me-Ph], [A864;OH,Me,F,F,F,F,3-Me-Ph], [A865;H,H,F,H,H,H,4-Me-Ph], [A866;H,H,C1,H,H,H,4-Me-Ph], [A867;H,H,Me,H,H,H,4-Me-Ph], [A868;H,H,CN,H,H,H,4-Me-Ph], [A869;H,H,OH,H,H,H,4-Me-Ph], [A870;H,H,cPr,H,H,H,4-Me-Ph], [A871;H,H,F,F,H,H,4-Me-Ph], [A872;H,H,F,H,F,H,4-Me-Ph], [A873;H,H,F,H,H,F,4-Me-Ph], [A874;H,H,Cl,H,H,Cl,4-Me-Ph], [A875;H,H,Me,H,H,Me,4-Me-Ph], [A876;H,H,F,F,F,F,4-Me-Ph], [A877;F,H,F,H,H,H,4-Me-Ph], [A878;F,H,Cl,H,H,H,4-Me-Ph], [A879;F,H,Me,H,H,H,4-Me-Ph], [A880;F,H,CN,H,H,H,4-Me-Ph], [A881;F,H,OH,H,H,H,4-Me-Ph], [A882;F,H,cPr,H,H,H,4-Me-Ph], [A883;F,H,F,F,H,H,4-Me-Ph], [A884;F,H,F,H,F,H,4-Me-Ph], [A885;F,H,F,H,H,F,4-Me-Ph], [A886;F,H,Cl,H,H,Cl,4-Me-Ph], [A887;F,H,Me,H,H,Me,4-Me-Ph], [A888;F,H,F,F,F,F,4-Me-Ph], [A889;Me,H,F,H,H,H,4-Me-Ph], [A890;Me,H,Cl,H,H,H,4-Me-Ph], [A891;Me,H,Me,H,H,H,4-Me-Ph], [A892;Me,H,CN,H,H,H,4-Me-Ph], [A893;Me,H,OH,H,H,H,4-Me-Ph], [A894;Me,H,cPr,H,H,H,4-Me-Ph], [A895;Me,H,F,F,H,H,4-Me-Ph], [A896;Me,H,F,H,F,H,4-Me-Ph], [A897;Me,H,F,H,H,F,4-Me-Ph], [A898;Me,H,Cl,H,H,Cl,4-Me-Ph], [A899;Me,H,Me,H,H,Me,4-Me-Ph], [A900;Me,H,F,F,F,F,4-Me-Ph], [A901;OMe,H,F,H,H,H,4-Me-Ph], [A902;OMe,H,Cl,H,H,H,4-Me-Ph], [A903;OMe,H,Me,H,H,H,4-Me-Ph], [A904;OMe,H,CN,H,H,H,4-Me-Ph], [A905;OMe,H,OH,H,H,H,4-Me-Ph], [A906;OMe,H,cPr,H,H,H,4-Me-Ph], [A907;OMe,H,F,F,H,H,4-Me-Ph], [A908;OMe,H,F,H,F,H,4-Me-Ph], [A909;OMe,H,F,H,H,F,4-Me-Ph], [A910;OMe,H,Cl,H,H,Cl,4-Me-Ph], [A911;OMe,H,Me,H,H,Me,4-Me-Ph], [A912;OMe,H,F,F,F,F,4-Me-Ph], [A913;OH,H,F,H,H,H,4-Me-Ph], [A914;OH,H,Cl,H,H,H,4-Me-Ph], [A915;OH,H,Me,H,H,H,4-Me-Ph], [A916;OH,H,CN,H,H,H,4-Me-Ph], [A917;OH,H,OH,H,H,H,4-Me-Ph], [A918;OH,H,cPr,H,H,H,4-Me-Ph], [A919;OH,H,F,F,H,H,4-Me-Ph], [A920;OH,H,F,H,F,H,4-Me-Ph], [A921;OH,H,F,H,H,F,4-Me-Ph], [A922;OH,H,Cl,H,H,Cl,4-Me-Ph], [A923;OH,H,Me,H,H,Me,4-Me-Ph], [A924;OH,H,F,F,F,F,4-Me-Ph], [A925;Me,Me,F,H,H,H,4-Me-Ph], [A926;Me,Me,Cl,H,H,H,4-Me-Ph], [A927;Me,Me,Me,H,H,H,4-Me-Ph], [A928;Me,Me,CN,H,H,H,4-Me-Ph], [A929;Me,Me,OH,H,H,H,4-Me-Ph], [A930;Me,Me,cPr,H,H,H,4-Me-Ph], [A931;Me,Me,F,F,H,H,4-Me-Ph], [A932;Me,Me,F,H,F,H,4-Me-Ph], [A933;Me,Me,F,H,H,F,4-Me-Ph], [A934;Me,Me,Cl,H,H,Cl,4-Me-Ph], [A935;Me,Me,Me,H,H,Me,4-Me-Ph], [A936;Me,Me,F,F,F,F,4-Me-Ph], [A937;F,F,F,H,H,H,4-Me-Ph], [A938;F,F,C1,H,H,H,4-Me-Ph], [A939;F,F,Me,H,H,H,4-Me-Ph], [A940;F,F,CN,H,H,H,4-Me-Ph], [A941;F,F,OH,H,H,H,4-Me-Ph], [A942;F,F,cPr,H,H,H,4-Me-Ph], [A943;F,F,F,F,H,H,4-Me-Ph], [A944;F,F,F,H,F,H,4-Me-Ph], [A945;F,F,F,H,H,F,4-Me-Ph], [A946;F,F,Cl,H,H,Cl,4-Me-Ph], [A947;F,F,Me,H,H,Me,4-Me-Ph], [A948;F,F,F,F,F,F,4-Me-Ph], [A949;OH,Me,F,H,H,H,4-Me-Ph], [A950;OH,Me,Cl,H,H,H,4-Me-Ph], [A951;OH,Me,Me,H,H,H,4-Me-Ph], [A952;OH,Me,CN,H,H,H,4-Me-Ph], [A953;OH,Me,OH,H,H,H,4-Me-Ph], [A954;OH,Me,cPr,H,H,H,4-Me-Ph], [A955;OH,Me,F,F,H,H,4-Me-Ph], [A956;OH,Me,F,H,F,H,4-Me-Ph], [A957;OH,Me,F,H,H,F,4-Me-Ph], [A958;OH,Me,Cl,H,H,Cl,4-Me-Ph], [A959;OH,Me,Me,H,H,Me,4-Me-Ph], [A960;OH,Me,F,F,F,F,4-Me-Ph], [A961;H,H,F,H,H,H,2-OMe-Ph], [A962;H,H,Cl,H,H,H,2-OMe-Ph], [A963;H,H,Me,H,H,H,2-OMe-Ph], [A964;H,H,CN,H,H,H,2-OMe-Ph], [A965;H,H,OH,H,H,H,2-OMe-Ph], [A966;H,H,cPr,H,H,H,2-OMe-Ph], [A967;H,H,F,F,H,H,2-OMe-Ph], [A968;H,H,F,H,F,H,2-OMe-Ph], [A969;H,H,F,H,H,F,2-OMe-Ph], [A970;H,H,Cl,H,H,Cl,2-OMe-Ph], [A971;H,H,Me,H,H,Me,2-OMe-Ph], [A972;H,H,F,F,F,F,2-OMe-Ph], [A973;F,H,F,H,H,H,2-OMe-Ph], [A974;F,H,Cl,H,H,H,2-OMe-Ph], [A975;F,H,Me,H,H,H,2-OMe-Ph], [A976;F,H,CN,H,H,H,2-OMe-Ph], [A977;F,H,OH,H,H,H,2-OMe-Ph], [A978;F,H,cPr,H,H,H,2-OMe-Ph], [A979;F,H,F,F,H,H,2-OMe-Ph], [A980;F,H,F,H,F,H,2-OMe-Ph], [A981;F,H,F,H,H,F,2-OMe-Ph], [A982;F,H,Cl,H,H,Cl,2-OMe-Ph], [A983;F,H,Me,H,H,Me,2-OMe-Ph], [A984;F,H,F,F,F,F,2-OMe-Ph], [A985;Me,H,F,H,H,H,2-OMe-Ph], [A986;Me,H,Cl,H,H,H,2-OMe-Ph], [A987;Me,H,Me,H,H,H,2-OMe-Ph], [A988;Me,H,CN,H,H,H,2-OMe-Ph], [A989;Me,H,OH,H,H,H,2-OMe-Ph], [A990;Me,H,cPr,H,H,H,2-OMe-Ph], [A991;Me,H,F,F,H,H,2-OMe-Ph], [A992;Me,H,F,H,F,H,2-OMe-Ph], [A993;Me,H,F,H,H,F,2-OMe-Ph], [A994;Me,H,Cl,H,H,Cl,2-OMe-Ph], [A995;Me,H,Me,H,H,Me,2-OMe-Ph], [A996;Me,H,F,F,F,F,2-OMe-Ph], [A997;OMe,H,F,H,H,H,2-OMe-Ph], [A998;OMe,H,Cl,H,H,H,2-OMe-Ph], [A999;OMe,H,Me,H,H,H,2-OMe-Ph], [A1000;OMe,H,CN,H,H,H,2-OMe-Ph], [A1001;OMe,H,OH,H,H,H,2-OMe-Ph], [A1002;OMe,H,cPr,H,H,H,2-OMe-Ph], [A1003;OMe,H,F,F,H,H,2-OMe-Ph], [A1004;OMe,H,F,H,F,H,2-OMe-Ph], [A1005;OMe,H,F,H,H,F,2-OMe-Ph], [A1006;OMe,H,Cl,H,H,Cl,2-OMe-Ph], [A1007;OMe,H,Me,H,H,Me,2-OMe-Ph], [A1008;OMe,H,F,F,F,F,2-OMe-Ph], [A1009;OH,H,F,H,H,H,2-OMe-Ph], [A1010;OH,H,Cl,H,H,H,2-OMe-Ph], [A1011;OH,H,Me,H,H,H,2-OMe-Ph], [A1012;OH,H,CN,H,H,H,2-OMe-Ph], [A1013;OH,H,OH,H,H,H,2-OMe-Ph], [A1014;OH,H,cPr,H,H,H,2-OMe-Ph], [A1015;OH,H,F,F,H,H,2-OMe-Ph], [A1016;OH,H,F,H,F,H,2-OMe-Ph], [A1017;OH,H,F,H,H,F,2-OMe-Ph], [A1018;0H,H,Cl,H,H,Cl,2-OMe-Ph], [A1019;OH,H,Me,H,H,Me,2-OMe-Ph], [A1020;OH,H,F,F,F,F,2-OMe-Ph], [A1021;Me,Me,F,H,H,H,2-OMe-Ph], [A1022;Me,Me,Cl,H,H,H,2-OMe-Ph], [A1023;Me,Me,Me,H,H,H,2-OMe-Ph], [A1024;Me,Me,CN,H,H,H,2-OMe-Ph], [A1025;Me,Me,OH,H,H,H,2-OMe-Ph], [A1026;Me,Me,cPr,H,H,H,2-OMe-Ph], [A1027;Me,Me,F,F,H,H,2-OMe-Ph], [A1028;Me,Me,F,H,F,H,2-OMe-Ph], [A1029;Me,Me,F,H,H,F,2-OMe-Ph], [A1030;Me,Me,Cl,H,H,Cl,2-OMe-Ph], [A1031;Me,Me,Me,H,H,Me,2-OMe-Ph], [A1032;Me,Me,F,F,F,F,2-OMe-Ph], [A1033;F,F,F,H,H,H,2-OMe-Ph], [A1034;F,F,Cl,H,H,H,2-OMe-Ph], [A1035;F,F,Me,H,H,H,2-OMe-Ph], [A1036;F,F,CN,H,H,H,2-OMe-Ph], [A1037;F,F,OH,H,H,H,2-OMe-Ph], [A1038;F,F,cPr,H,H,H,2-OMe-Ph], [A1039;F,F,F,F,H,H,2-OMe-Ph], [A1040;F,F,F,H,F,H,2-OMe-Ph], [A1041;F,F,F,H,H,F,2-OMe-Ph], [A1042;F,F,Cl,H,H,Cl,2-OMe-Ph], [A1043;F,F,Me,H,H,Me,2-OMe-Ph], [A1044;F,F,F,F,F,F,2-OMe-Ph], [A1045;OH,Me,F,H,H,H,2-OMe-Ph], [A1046;OH,Me,Cl,H,H,H,2-OMe-Ph], [A1047;OH,Me,Me,H,H,H,2-OMe-Ph], [A1048;OH,Me,CN,H,H,H,2-OMe-Ph], [A1049;OH,Me,OH,H,H,H,2-OMe-Ph], [A1050;OH,Me,cPr,H,H,H,2-OMe-Ph], [A1051;OH,Me,F,F,H,H,2-OMe-Ph], [A1052;OH,Me,F,H,F,H,2-OMe-Ph], [A1053;OH,Me,F,H,H,F,2-OMe-Ph], [A1054;OH,Me,Cl,H,H,C1,2-OMe-Ph], [A1055;OH,Me,Me,H,H,Me,2-OMe-Ph], [A1056;OH,Me,F,F,F,F,2-OMe-Ph], [A1057;H,H,F,H,H,H,3-OMe-Ph], [A1058;H,H,Cl,H,H,H,3-OMe-Ph], [A1059;H,H,Me,H,H,H,3-OMe-Ph], [A1060;H,H,CN,H,H,H,3-OMe-Ph], [A1061;H,H,OH,H,H,H,3-OMe-Ph], [A1062;H,H,cPr,H,H,H,3-OMe-Ph], [A1063;H,H,F,F,H,H,3-OMe-Ph], [A1064;H,H,F,H,F,H,3-OMe-Ph], [A1065;H,H,F,H,H,F,3-OMe-Ph], [A1066;H,H,Cl,H,H,Cl,3-OMe-Ph], [A1067;H,H,Me,H,H,Me,3-OMe-Ph], [A1068;H,H,F,F,F,F,3-OMe-Ph], [A1069;F,H,F,H,H,H,3-OMe-Ph], [A1070;F,H,Cl,H,H,H,3-OMe-Ph], [A1071;F,H,Me,H,H,H,3-OMe-Ph], [A1072;F,H,CN,H,H,H,3-OMe-Ph], [A1073;F,H,OH,H,H,H,3-OMe-Ph], [A1074;F,H,cPr,H,H,H,3-OMe-Ph], [A1075;F,H,F,F,H,H,3-OMe-Ph], [A1076;F,H,F,H,F,H,3-OMe-Ph], [A1077;F,H,F,H,H,F,3-OMe-Ph], [A1078;F,H,Cl,H,H,Cl,3-OMe-Ph], [A1079;F,H,Me,H,H,Me,3-OMe-Ph], [A1080;F,H,F,F,F,F,3-OMe-Ph], [A1081;Me,H,F,H,H,H,3-OMe-Ph], [A1082;Me,H,Cl,H,H,H,3-OMe-Ph], [A1083;Me,H,Me,H,H,H,3-OMe-Ph], [A1084;Me,H,CN,H,H,H,3-OMe-Ph], [A1085;Me,H,OH,H,H,H,3-OMe-Ph], [A1086;Me,H,cPr,H,H,H,3-OMe-Ph], [A1087;Me,H,F,F,H,H,3-OMe-Ph], [A1088;Me,H,F,H,F,H,3-OMe-Ph], [A1089;Me,H,F,H,H,F,3-OMe-Ph], [A1090;Me,H,Cl,H,H,Cl,3-OMe-Ph], [A1091;Me,H,Me,H,H,Me,3-OMe-Ph], [A1092;Me,H,F,F,F,F,3-OMe-Ph], [A1093;OMe,H,F,H,H,H,3-OMe-Ph], [A1094;OMe,H,Cl,H,H,H,3-OMe-Ph], [A1095;OMe,H,Me,H,H,H,3-OMe-Ph], [A1096;OMe,H,CN,H,H,H,3-OMe-Ph], [A1097;OMe,H,OH,H,H,H,3-OMe-Ph], [A1098;OMe,H,cPr,H,H,H,3-OMe-Ph], [A1099;OMe,H,F,F,H,H,3-OMe-Ph], [A1100;OMe,H,F,H,F,H,3-OMe-Ph], [A1101;OMe,H,F,H,H,F,3-OMe-Ph], [A1102;OMe,H,Cl,H,H,C1,3-OMe-Ph], [A1103;OMe,H,Me,H,H,Me,3-OMe-Ph], [A1104;OMe,H,F,F,F,F,3-OMe-Ph], [A1105;OH,H,F,H,H,H,3-OMe-Ph], [A1106;OH,H,Cl,H,H,H,3-OMe-Ph], [A1107;OH,H,Me,H,H,H,3-OMe-Ph], [A1108;OH,H,CN,H,H,H,3-OMe-Ph], [A1109;OH,H,OH,H,H,H,3-OMe-Ph], [A1110;OH,H,cPr,H,H,H,3-OMe-Ph], [A1111;OH,H,F,F,H,H,3-OMe-Ph], [A1112;OH,H,F,H,F,H,3-OMe-Ph], [A1113;OH,H,F,H,H,F,3-OMe-Ph], [A1114;OH,H,Cl,H,H,C1,3-OMe-Ph], [A1115;OH,H,Me,H,H,Me,3-OMe-Ph], [A1116;OH,H,F,F,F,F,3-OMe-Ph], [A1117;Me,Me,F,H,H,H,3-OMe-Ph], [A1118;Me,Me,Cl,H,H,H,3-OMe-Ph], [A1119;Me,Me,Me,H,H,H,3-OMe-Ph], [A1120;Me,Me,CN,H,H,H,3-OMe-Ph], [A1121;Me,Me,OH,H,H,H,3-OMe-Ph], [A1122;Me,Me,cPr,H,H,H,3-OMe-Ph], [A1123;Me,Me,F,F,H,H,3-OMe-Ph], [A1124;Me,Me,F,H,F,H,3-OMe-Ph], [A1125;Me,Me,F,H,H,F,3-OMe-Ph], [A1126;Me,Me,Cl,H,H,Cl,3-OMe-Ph], [A1127;Me,Me,Me,H,H,Me,3-OMe-Ph], [A1128;Me,Me,F,F,F,F,3-OMe-Ph], [A1129;F,F,F,H,H,H,3-OMe-Ph], [A1130;F,F,Cl,H,H,H,3-OMe-Ph], [A1131;F,F,Me,H,H,H,3-OMe-Ph], [A1132;F,F,CN,H,H,H,3-OMe-Ph], [A1133;F,F,OH,H,H,H,3-OMe-Ph], [A1134;F,F,cPr,H,H,H,3-OMe-Ph], [A1135;F,F,F,F,H,H,3-OMe-Ph], [A1136;F,F,F,H,F,H,3-OMe-Ph], [A1137;F,F,F,H,H,F,3-OMe-Ph], [A1138;F,F,Cl,H,H,Cl,3-OMe-Ph], [A1139;F,F,Me,H,H,Me,3-OMe-Ph], [A1140;F,F,F,F,F,F,3-OMe-Ph], [A1141;OH,Me,F,H,H,H,3-OMe-Ph], [A1142;OH,Me,Cl,H,H,H,3-OMe-Ph], [A1143;OH,Me,Me,H,H,H,3-OMe-Ph], [A1144;OH,Me,CN,H,H,H,3-OMe-Ph], [A1145;OH,Me,OH,H,H,H,3-OMe-Ph], [A1146;OH,Me,cPr,H,H,H,3-OMe-Ph], [A1147;OH,Me,F,F,H,H,3-OMe-Ph], [A1148;OH,Me,F,H,F,H,3-OMe-Ph], [A1149;OH,Me,F,H,H,F,3-OMe-Ph], [A1150;OH,Me,Cl,H,H,Cl,3-OMe-Ph], [A1151;OH,Me,Me,H,H,Me,3-OMe-Ph], [A1152;OH,Me,F,F,F,F,3-OMe-Ph], [A1153;H,H,F,H,H,H,4-OMe-Ph], [A1154;H,H,Cl,H,H,H, 4-OMe-Ph], [A1155;H,H,Me,H,H,H,4-OMe-Ph], [A156;H,H,CN,H,H,H,4-OMe-Ph], [A1157;H,H,OH,H,H,H,4-OMe-Ph], [A1158;H,H,cPr,H,H,H,4-OMe-Ph], [A1159;H,H,F,F,H,H,4-OMe-Ph], [A1160;H,H,F,H,F,H,4-OMe-Ph], [A1161;H,H,F,H,H,F,4-OMe-Ph], [A1162;H,H,Cl,H,H,Cl,4-OMe-Ph], [A1163;H,H,Me,H,H,Me,4-OMe-Ph], [A1164;H,H,F,F,F,F,4-OMe-Ph], [A1165;F,H,F,H,H,H,4-OMe-Ph], [A1166;F,H,Cl,H,H,H, 4-OMe-Ph], [A1167;F,H,Me,H,H,H,4-OMe-Ph], [A1168;F,H,CN,H,H,H,4-OMe-Ph], [A1169;F,H,OH,H,H,H,4-OMe-Ph], [A1170;F,H,cPr,H,H,H,4-OMe-Ph], [A1171;F,H,F,F,H,H,4-OMe-Ph], [A1172;F,H,F,H,F,H,4-OMe-Ph], [A1173;F,H,F,H,H,F,4-OMe-Ph], [A1174;F,H,Cl,H,H,Cl,4-OMe-Ph], [A1175;F,H,Me,H,H,Me,4-OMe-Ph], [A1176;F,H,F,F,F,F,4-OMe-Ph], [A1177;Me,H,F,H,H,H,4-OMe-Ph], [A1178;Me,H,Cl,H,H,H,4-OMe-Ph], [A1179;Me,H,Me,H,H,H,4-OMe-Ph], [A1180;Me,H,CN,H,H,H,4-OMe-Ph], [A1181;Me,H,OH,H,H,H,4-OMe-Ph], [A1182;Me,H,cPr,H,H,H,4-OMe-Ph], [A1183;Me,H,F,F,H,H,4-OMe-Ph], [A1184;Me,H,F,H,F,H,4-OMe-Ph], [A1185;Me,H,F,H,H,F,4-OMe-Ph], [A1186;Me,H,Cl,H,H,Cl,4-OMe-Ph], [A1187;Me,H,Me,H,H,Me,4-OMe-Ph], [A1188;Me,H,F,F,F,F,4-OMe-Ph], [A1189;OMe,H,F,H,H,H,4-OMe-Ph], [A1190;OMe,H,Cl,H,H,H,4-OMe-Ph], [A1191;OMe,H,Me,H,H,H,4-OMe-Ph], [A1192;OMe,H,CN,H,H,H,4-OMe-Ph], [A1193;OMe,H,OH,H,H,H,4-OMe-Ph], [A1194;OMe,H,cPr,H,H,H,4-OMe-Ph], [A1195;OMe,H,F,F,H,H,4-OMe-Ph], [A1196;OMe,H,F,H,F,H,4-OMe-Ph], [A1197;OMe,H,F,H,H,F,4-OMe-Ph], [A1198;OMe,H,Cl,H,H,C1,4-OMe-Ph], [A1199;OMe,H,Me,H,H,Me,4-OMe-Ph], [A1200;OMe,H,F,F,F,F,4-OMe-Ph], [A1201;OH,H,F,H,H,H,4-OMe-Ph], [A1202;OH,H,Cl,H,H,H,4-OMe-Ph], [A1203;OH,H,Me,H,H,H,4-OMe-Ph], [A1204;OH,H,CN,H,H,H,4-OMe-Ph], [A1205;OH,H,OH,H,H,H,4-OMe-Ph], [A1206;OH,H,cPr,H,H,H,4-OMe-Ph], [A1207;OH,H,F,F,H,H,4-OMe-Ph], [A1208;OH,H,F,H,F,H,4-OMe-Ph], [A1209;OH,H,F,H,H,F,4-OMe-Ph], [A1210;OH,H,Cl,H,H,Cl,4-OMe-Ph], [A1211;OH,H,Me,H,H,Me,4-OMe-Ph], [A1212;OH,H,F,F,F,F,4-OMe-Ph], [A1213;Me,Me,F,H,H,H,4-OMe-Ph], [A1214;Me,Me,Cl,H,H,H,4-OMe-Ph], [A1215;Me,Me,Me,H,H,H,4-OMe-Ph], [A1216;Me,Me,CN,H,H,H,4-OMe-Ph], [A1217;Me,Me,OH,H,H,H,4-OMe-Ph], [A1218;Me,Me,cPr,H,H,H,4-OMe-Ph], [A1219;Me,Me,F,F,H,H,4-OMe-Ph], [A1220;Me,Me,F,H,F,H,4-OMe-Ph], [A1221;Me,Me,F,H,H,F,4-OMe-Ph], [A1222;Me,Me,Cl,H,H,Cl,4-OMe-Ph], [A1223;Me,Me,Me,H,H,Me,4-OMe-Ph], [A1224;Me,Me,F,F,F,F,4-OMe-Ph], [A1225;F,F,F,H,H,H,4-OMe-Ph], [A1226;F,F,Cl,H,H,H,4-OMe-Ph], [A1227;F,F,Me,H,H,H,4-OMe-Ph], [A1228;F,F,CN,H,H,H,4-OMe-Ph], [A1229;F,F,OH,H,H,H,4-OMe-Ph], [A1230;F,F,cPr,H,H,H,4-OMe-Ph], [A1231;F,F,F,F,H,H,4-OMe-Ph], [A1232;F,F,F,H,F,H,4-OMe-Ph], [A1233;F,F,F,H,H,F,4-OMe-Ph], [A1234;F,F,Cl,H,H,Cl,4-OMe-Ph], [A1235;F,F,Me,H,H,Me,4-OMe-Ph], [A1236;F,F,F,F,F,F,4-OMe-Ph], [A1237;OH,Me,F,H,H,H,4-OMe-Ph], [A1238;OH,Me,Cl,H,H,H,4-OMe-Ph], [A1239;OH,Me,Me,H,H,H,4-OMe-Ph], [A1240;OH,Me,CN,H,H,H,4-OMe-Ph], [A1241;OH,Me,OH,H,H,H,4-OMe-Ph], [A1242;OH,Me,cPr,H,H,H,4-OMe-Ph], [A1243;OH,Me,F,F,H,H,4-OMe-Ph], [A1244;OH,Me,F,H,F,H,4-OMe-Ph], [A1245;OH,Me,F,H,H,F,4-OMe-Ph], [A1246;OH,Me,Cl,H,H,Cl,4-OMe-Ph], [A1247;OH,Me,Me,H,H,Me,4-OMe-Ph], [A1248;OH,Me,F,F,F,F,4-OMe-Ph], [A1249;H,H,F,H,H,H,2-cPr-Ph], [A1250;H,H,Cl,H,H,H,2-cPr-Ph], [A1251;H,H,Me,H,H,H,2-cPr-Ph], [A1252;H,H,CN,H,H,H,2-cPr-Ph], [A1253;H,H,OH,H,H,H,2-cPr-Ph], [A1254;H,H,cPr,H,H,H,2-cPr-Ph], [A1255;H,H,F,F,H,H,2-cPr-Ph], [A1256;H,H,F,H,F,H,2-cPr-Ph], [A1257;H,H,F,H,H,F,2-cPr-Ph], [A1258;H,H,Cl,H,H,Cl,2-cPr-Ph], [A1259;H,H,Me,H,H,Me,2-cPr-Ph], [A1260;H,H,F,F,F,F,2-cPr-Ph], [A1261;F,H,F,H,H,H,2-cPr-Ph], [A1262;F,H,Cl,H,H,H,2-cPr-Ph], [A1263;F,H,Me,H,H,H,2-cPr-Ph], [A1264;F,H,CN,H,H,H,2-cPr-Ph], [A1265;F,H,OH,H,H,H,2-cPr-Ph], [A1266;F,H,cPr,H,H,H,2-cPr-Ph], [A1267;F,H,F,F,H,H,2-cPr-Ph], [A1268;F,H,F,H,F,H,2-cPr-Ph], [A1269;F,H,F,H,H,F,2-cPr-Ph], [A1270;F,H,Cl,H,H,Cl,2-cPr-Ph], [A1271;F,H,Me,H,H,Me,2-cPr-Ph], [A1272;F,H,F,F,F,F,2-cPr-Ph], [A1273;Me,H,F,H,H,H,2-cPr-Ph], [A1274;Me,H,Cl,H,H,H,2-cPr-Ph], [A1275;Me,H,Me,H,H,H,2-cPr-Ph], [A1276;Me,H,CN,H,H,H,2-cPr-Ph], [A1277;Me,H,OH,H,H,H,2-cPr-Ph], [A1278;Me,H,cPr,H,H,H,2-cPr-Ph], [A1279;Me,H,F,F,H,H,2-cPr-Ph], [A1280;Me,H,F,H,F,H,2-cPr-Ph], [A1281;Me,H,F,H,H,F,2-cPr-Ph], [A1282;Me,H,Cl,H,H,Cl,2-cPr-Ph], [A1283;Me,H,Me,H,H,Me,2-cPr-Ph], [A1284;Me,H,F,F,F,F,2-cPr-Ph], [A1285;OMe,H,F,H,H,H,2-cPr-Ph], [A1286;OMe,H,Cl,H,H,H,2-cPr-Ph], [A1287;OMe,H,Me,H,H,H,2-cPr-Ph], [A1288;OMe,H,CN,H,H,H,2-cPr-Ph], [A1289;OMe,H,OH,H,H,H,2-cPr-Ph], [A1290;OMe,H,cPr,H,H,H,2-cPr-Ph], [A1291;OMe,H,F,F,H,H,2-cPr-Ph], [A1292;OMe,H,F,H,F,H,2-cPr-Ph], [A1293;OMe,H,F,H,H,F,2-cPr-Ph], [A1294;OMe,H,Cl,H,H,C1,2-cPr-Ph], [A1295;OMe,H,Me,H,H,Me,2-cPr-Ph], [A1296;OMe,H,F,F,F,F,2-cPr-Ph], [A1297;OH,H,F,H,H,H,2-cPr-Ph], [A1298;OH,H,Cl,H,H,H,2-cPr-Ph], [A1299;OH,H,Me,H,H,H,2-cPr-Ph], [A1300;OH,H,CN,H,H,H,2-cPr-Ph], [A1301;OH,H,OH,H,H,H,2-cPr-Ph], [A1302;OH,H,cPr,H,H,H,2-cPr-Ph], [A1303;OH,H,F,F,H,H,2-cPr-Ph], [A1304;OH,H,F,H,F,H,2-cPr-Ph], [A1305;OH,H,F,H,H,F,2-cPr-Ph], [A1306;OH,H,Cl,H,H,Cl,2-cPr-Ph], [A1307;OH,H,Me,H,H,Me,2-cPr-Ph], [A1308;OH,H,F,F,F,F,2-cPr-Ph], [A1309;Me,Me,F,H,H,H,2-cPr-Ph], [A1310;Me,Me,Cl,H,H,H,2-cPr-Ph], [A1311;Me,Me,Me,H,H,H,2-cPr-Ph], [A1312;Me,Me,CN,H,H,H,2-cPr-Ph], [A1313;Me,Me,OH,H,H,H,2-cPr-Ph], [A1314;Me,Me,cPr,H,H,H,2-cPr-Ph], [A1315;Me,Me,F,F,H,H,2-cPr-Ph], [A1316;Me,Me,F,H,F,H,2-cPr-Ph], [A1317;Me,Me,F,H,H,F,2-cPr-Ph], [A1318;Me,Me,Cl,H,H,Cl,2-cPr-Ph], [A1319;Me,Me,Me,H,H,Me,2-cPr-Ph], [A1320;Me,Me,F,F,F,F,2-cPr-Ph], [A1321;F,F,F,H,H,H,2-cPr-Ph], [A1322;F,F,Cl,H,H,H,2-cPr-Ph], [A1323;F,F,Me,H,H,H,2-cPr-Ph], [A1324;F,F,CN,H,H,H,2-cPr-Ph], [A1325;F,F,OH,H,H,H,2-cPr-Ph], [A1326;F,F,cPr,H,H,H,2-cPr-Ph], [A1327;F,F,F,F,H,H,2-cPr-Ph], [A1328;F,F,F,H,F,H,2-cPr-Ph], [A1329;F,F,F,H,H,F,2-cPr-Ph], [A1330;F,F,Cl,H,H,Cl,2-cPr-Ph], [A1331;F,F,Me,H,H,Me,2-cPr-Ph], [A1332;F,F,F,F,F,F,2-cPr-Ph], [A1333;OH,Me,F,H,H,H,2-cPr-Ph], [A1334;OH,Me,Cl,H,H,H,2-cPr-Ph], [A1335;OH,Me,Me,H,H,H,2-cPr-Ph], [A1336;OH,Me,CN,H,H,H,2-cPr-Ph], [A1337;OH,Me,OH,H,H,H,2-cPr-Ph], [A1338;OH,Me,cPr,H,H,H,2-cPr-Ph], [A1339;OH,Me,F,F,H,H,2-cPr-Ph], [A1340;OH,Me,F,H,F,H,2-cPr-Ph], [A1341;OH,Me,F,H,H,F,2-cPr-Ph], [A1342;OH,Me,Cl,H,H,Cl,2-cPr-Ph], [A1343;OH,Me,Me,H,H,Me,2-cPr-Ph], [A1344;OH,Me,F,F,F,F,2-cPr-Ph], [A1345;H,H,F,H,H,H,3-cPr-Ph], [A1346;H,H,Cl,H,H,H,3-cPr-Ph], [A1347;H,H,Me,H,H,H,3-cPr-Ph], [A1348;H,H,CN,H,H,H,3-cPr-Ph], [A1349;H,H,OH,H,H,H,3-cPr-Ph], [A1350;H,H,cPr,H,H,H,3-cPr-Ph], [A1351;H,H,F,F,H,H,3-cPr-Ph], [A1352;H,H,F,H,F,H,3-cPr-Ph], [A1353;H,H,F,H,H,F,3-cPr-Ph], [A1354;H,H,Cl,H,H,Cl,3-cPr-Ph], [A1355;H,H,Me,H,H,Me,3-cPr-Ph], [A1356;H,H,F,F,F,F,3-cPr-Ph], [A1357;F,H,F,H,H,H,3-cPr-Ph], [A1358;F,H,Cl,H,H,H,3-cPr-Ph], [A1359;F,H,Me,H,H,H,3-cPr-Ph], [A1360;F,H,CN,H,H,H,3-cPr-Ph], [A1361;F,H,OH,H,H,H,3-cPr-Ph], [A1362;F,H,cPr,H,H,H,3-cPr-Ph], [A1363;F,H,F,F,H,H,3-cPr-Ph], [A1364;F,H,F,H,F,H,3-cPr-Ph], [A1365;F,H,F,H,H,F,3-cPr-Ph], [A1366;F,H,Cl,H,H,Cl,3-cPr-Ph], [A1367;F,H,Me,H,H,Me,3-cPr-Ph], [A1368;F,H,F,F,F,F,3-cPr-Ph], [A1369;Me,H,F,H,H,H,3-cPr-Ph], [A1370;Me,H,Cl,H,H,H,3-cPr-Ph], [A1371;Me,H,Me,H,H,H,3-cPr-Ph], [A1372;Me,H,CN,H,H,H,3-cPr-Ph], [A1373;Me,H,OH,H,H,H,3-cPr-Ph], [A1374;Me,H,cPr,H,H,H,3-cPr-Ph], [A1375;Me,H,F,F,H,H,3-cPr-Ph], [A1376;Me,H,F,H,F,H,3-cPr-Ph], [A1377;Me,H,F,H,H,F,3-cPr-Ph], [A1378;Me,H,Cl,H,H,C1,3-cPr-Ph], [A1379;Me,H,Me,H,H,Me,3-cPr-Ph], [A1380;Me,H,F,F,F,F,3-cPr-Ph], [A1381;OMe,H,F,H,H,H,3-cPr-Ph], [A1382;OMe,H,Cl,H,H,H,3-cPr-Ph], [A1383;OMe,H,Me,H,H,H,3-cPr-Ph], [A1384;OMe,H,CN,H,H,H,3-cPr-Ph], [A1385;OMe,H,OH,H,H,H,3-cPr-Ph], [A1386;OMe,H,cPr,H,H,H,3-cPr-Ph], [A1387;OMe,H,F,F,H,H,3-cPr-Ph], [A1388;OMe,H,F,H,F,H,3-cPr-Ph], [A1389;OMe,H,F,H,H,F,3-cPr-Ph], [A1390;OMe,H,Cl,H,H,C1,3-cPr-Ph], [A1391;OMe,H,Me,H,H,Me,3-cPr-Ph], [A1392;OMe,H,F,F,F,F,3-cPr-Ph], [A1393;OH,H,F,H,H,H,3-cPr-Ph], [A1394;OH,H,Cl,H,H,H,3-cPr-Ph], [A1395;OH,H,Me,H,H,H,3-cPr-Ph], [A1396;OH,H,CN,H,H,H,3-cPr-Ph], [A1397;OH,H,OH,H,H,H,3-cPr-Ph], [A1398;OH,H,cPr,H,H,H,3-cPr-Ph], [A1399;OH,H,F,F,H,H,3-cPr-Ph], [A1400;OH,H,F,H,F,H,3-cPr-Ph], [A1401;OH,H,F,H,H,F,3-cPr-Ph], [A1402;OH,H,Cl,H,H,Cl,3-cPr-Ph], [A1403;OH,H,Me,H,H,Me,3-cPr-Ph], [A1404;OH,H,F,F,F,F,3-cPr-Ph], [A1405;Me,Me,F,H,H,H,3-cPr-Ph], [A1406;Me,Me,Cl,H,H,H,3-cPr-Ph], [A1407;Me,Me,Me,H,H,H,3-cPr-Ph], [A1408;Me,Me,CN,H,H,H,3-cPr-Ph], [A1409;Me,Me,OH,H,H,H,3-cPr-Ph], [A1410;Me,Me,cPr,H,H,H,3-cPr-Ph], [A1411;Me,Me,F,F,H,H,3-cPr-Ph], [A1412;Me,Me,F,H,F,H,3-cPr-Ph], [A1413;Me,Me,F,H,H,F,3-cPr-Ph], [A1414;Me,Me,Cl,H,H,Cl,3-cPr-Ph], [A1415;Me,Me,Me,H,H,Me,3-cPr-Ph], [A1416;Me,Me,F,F,F,F,3-cPr-Ph], [A1417;F,F,F,H,H,H,3-cPr-Ph], [A1418;F,F,Cl,H,H,H,3-cPr-Ph], [A1419;F,F,Me,H,H,H,3-cPr-Ph], [A1420;F,F,CN,H,H,H,3-cPr-Ph], [A1421;F,F,OH,H,H,H,3-cPr-Ph], [A1422;F,F,cPr,H,H,H,3-cPr-Ph], [A1423;F,F,F,F,H,H,3-cPr-Ph], [A1424;F,F,F,H,F,H,3-cPr-Ph], [A1425;F,F,F,H,H,F,3-cPr-Ph], [A1426;F,F,Cl,H,H,Cl,3-cPr-Ph], [A1427;F,F,Me,H,H,Me,3-cPr-Ph], [A1428;F,F,F,F,F,F,3-cPr-Ph], [A1429;OH,Me,F,H,H,H,3-cPr-Ph], [A1430;OH,Me,Cl,H,H,H,3-cPr-Ph], [A1431;OH,Me,Me,H,H,H,3-cPr-Ph], [A1432;OH,Me,CN,H,H,H,3-cPr-Ph], [A1433;OH,Me,OH,H,H,H,3-cPr-Ph], [A1434;OH,Me,cPr,H,H,H,3-cPr-Ph], [A1435;OH,Me,F,F,H,H,3-cPr-Ph], [A1436;OH,Me,F,H,F,H,3-cPr-Ph], [A1437;OH,Me,F,H,H,F,3-cPr-Ph], [A1438;OH,Me,Cl,H,H,Cl,3-cPr-Ph], [A1439;OH,Me,Me,H,H,Me,3-cPr-Ph], [A1440;OH,Me,F,F,F,F,3-cPr-Ph], [A1441;H,H,F,H,H,H,4-cPr-Ph], [A1442;H,H,Cl,H,H,H,4-cPr-Ph], [A1443;H,H,Me,H,H,H,4-cPr-Ph], [A1444;H,H,CN,H,H,H,4-cPr-Ph], [A1445;H,H,OH,H,H,H,4-cPr-Ph], [A1446;H,H,cPr,H,H,H,4-cPr-Ph], [A1447;H,H,F,F,H,H,4-cPr-Ph], [A1448;H,H,F,H,F,H,4-cPr-Ph], [A1449;H,H,F,H,H,F,4-cPr-Ph], [A1450;H,H,Cl,H,H,C1,4-cPr-Ph], [A1451;H,H,Me,H,H,Me,4-cPr-Ph], [A1452;H,H,F,F,F,F,4-cPr-Ph], [A1453;F,H,F,H,H,H,4-cPr-Ph], [A1454;F,H,Cl,H,H,H,4-cPr-Ph], [A1455;F,H,Me,H,H,H,4-cPr-Ph], [A1456;F,H,CN,H,H,H,4-cPr-Ph], [A1457;F,H,OH,H,H,H,4-cPr-Ph], [A1458;F,H,cPr,H,H,H,4-cPr-Ph], [A1459;F,H,F,F,H,H,4-cPr-Ph], [A1460;F,H,F,H,F,H,4-cPr-Ph], [A1461;F,H,F,H,H,F,4-cPr-Ph], [A1462;F,H,Cl,H,H,Cl,4-cPr-Ph], [A1463;F,H,Me,H,H,Me,4-cPr-Ph], [A1464;F,H,F,F,F,F,4-cPr-Ph], [A1465;Me,H,F,H,H,H,4-cPr-Ph], [A1466;Me,H,Cl,H,H,H,4-cPr-Ph], [A1467;Me,H,Me,H,H,H,4-cPr-Ph], [A1468;Me,H,CN,H,H,H,4-cPr-Ph], [A1469;Me,H,OH,H,H,H,4-cPr-Ph], [A1470;Me,H,cPr,H,H,H,4-cPr-Ph], [A1471;Me,H,F,F,H,H,4-cPr-Ph], [A1472;Me,H,F,H,F,H,4-cPr-Ph], [A1473;Me,H,F,H,H,F,4-cPr-Ph], [A1474;Me,H,Cl,H,H,C1,4-cPr-Ph], [A1475;Me,H,Me,H,H,Me,4-cPr-Ph], [A1476;Me,H,F,F,F,F,4-cPr-Ph], [A1477;OMe,H,F,H,H,H,4-cPr-Ph], [A1478;OMe,H,Cl,H,H,H,4-cPr-Ph], [A1479;OMe,H,Me,H,H,H,4-cPr-Ph], [A1480;OMe,H,CN,H,H,H,4-cPr-Ph], [A1481;OMe,H,OH,H,H,H,4-cPr-Ph], [A1482;OMe,H,cPr,H,H,H,4-cPr-Ph], [A1483;OMe,H,F,F,H,H,4-cPr-Ph], [A1484;OMe,H,F,H,F,H,4-cPr-Ph], [A1485;OMe,H,F,H,H,F,4-cPr-Ph], [A1486;OMe,H,Cl,H,H,Cl,4-cPr-Ph], [A1487;OMe,H,Me,H,H,Me,4-cPr-Ph], [A1488;OMe,H,F,F,F,F,4-cPr-Ph], [A1489;OH,H,F,H,H,H,4-cPr-Ph], [A1490;OH,H,Cl,H,H,H,4-cPr-Ph], [A1491;OH,H,Me,H,H,H,4-cPr-Ph], [A1492;OH,H,CN,H,H,H,4-cPr-Ph], [A1493;OH,H,OH,H,H,H,4-cPr-Ph], [A1494;OH,H,cPr,H,H,H,4-cPr-Ph], [A1495;OH,H,F,F,H,H,4-cPr-Ph], [A1496;OH,H,F,H,F,H,4-cPr-Ph], [A1497;OH,H,F,H,H,F,4-cPr-Ph], [A1498;OH,H,Cl,H,H,Cl,4-cPr-Ph], [A1499;OH,H,Me,H,H,Me,4-cPr-Ph], [A1500;OH,H,F,F,F,F,4-cPr-Ph], [A1501;Me,Me,F,H,H,H,4-cPr-Ph], [A1502;Me,Me,Cl,H,H,H,4-cPr-Ph], [A1503;Me,Me,Me,H,H,H,4-cPr-Ph], [A1504;Me,Me,CN,H,H,H,4-cPr-Ph], [A1505;Me,Me,OH,H,H,H,4-cPr-Ph], [A1506;Me,Me,cPr,H,H,H,4-cPr-Ph], [A1507;Me,Me,F,F,H,H,4-cPr-Ph], [A1508;Me,Me,F,H,F,H,4-cPr-Ph], [A1509;Me,Me,F,H,H,F,4-cPr-Ph], [A1510;Me,Me,Cl,H,H,C1,4-cPr-Ph], [A1511;Me,Me,Me,H,H,Me,4-cPr-Ph], [A1512; Me,Me,F,F,F,F,4-cPr-Ph], [A1513;F,F,F,H,H,H,4-cPr-Ph], [A1514;F,F,Cl,H,H,H,4-cPr-Ph], [A1515;F,F,Me,H,H,H,4-cPr-Ph], [A1516;F,F,CN,H,H,H,4-cPr-Ph], [A1517;F,F,OH,H,H,H,4-cPr-Ph], [A1518;F,F,cPr,H,H,H,4-cPr-Ph], [A1519;F,F,F,F,H,H,4-cPr-Ph], [A1520;F,F,F,H,F,H,4-cPr-Ph], [A1521;F,F,F,H,H,F,4-cPr-Ph], [A1522;F,F,Cl,H,H,Cl,4-cPr-Ph], [A1523;F,F,Me,H,H,Me,4-cPr-Ph], [A1524;F,F,F,F,F,F,4-cPr-Ph], [A1525;OH,Me,F,H,H,H,4-cPr-Ph], [A1526;OH,Me,Cl,H,H,H,4-cPr-Ph], [A1527;OH,Me,Me,H,H,H,4-cPr-Ph], [A1528;OH,Me,CN,H,H,H,4-cPr-Ph], [A1529;OH,Me,OH,H,H,H,4-cPr-Ph], [A1530;OH,Me,cPr,H,H,H,4-cPr-Ph], [A1531;OH,Me,F,F,H,H,4-cPr-Ph], [A1532;OH,Me,F,H,F,H,4-cPr-Ph], [A1533;OH,Me,F,H,H,F,4-cPr-Ph], [A1534;OH,Me,Cl,H,H,C1,4-cPr-Ph], [A1535;OH,Me,Me,H,H,Me,4-cPr-Ph], [A1536;OH,Me,F,F,F,F,4-cPr-Ph], [A1537;H,H,F,H,H,H, (2,3-F₂-Ph)], [A1538;H,H,Cl,H,H,H,(2,3-F₂-Ph)], [A1539;H,H,Me,H,H,H,(2,3-F₂-Ph)], [A1540;H,H,CN,H,H,H,(2,3-F₂-Ph)], [A1541;H,H,OH,H,H,H, (2,3-F₂-Ph)], [A1542;H,H,cPr,H,H,H,(2,3-F₂-Ph)], [A1543;H,H,F,F,H,H,(2,3-F₂-Ph)], [A1544;H,H,F,H,F,H,(2,3-F₂-Ph)], [A1545;H,H,F,H,H,F,(2,3-F₂-Ph)], [A1546;H,H,Cl,H,H,Cl,(2,3-F₂-Ph)], [A1547;H,H,Me,H,H,Me,(2,3-F₂-Ph)], [A1548;H,H,F,F,F,F,(2,3-F₂-Ph)], [A1549;F,H,F,H,H,H, (2,3-F₂-Ph)], [A1550;F,H,Cl,H,H,H,(2,3-F₂-Ph)], [A1551;F,H,Me,H,H,H,(2,3-F₂-Ph)], [A1552;F,H,CN,H,H,H,(2,3-F₂-Ph)], [A1553;F,H,OH,H,H,H,(2,3-F₂-Ph)], [A1554;F,H,cPr,H,H,H,(2,3-F₂-Ph)], [A1555;F,H,F,F,H,H, (2,3-F₂-Ph)], [A1556;F,H,F,H,F,H,(2,3-F₂-Ph)], [A1557;F,H,F,H,H,F,(2,3-F₂-Ph)], [A1558;F,H,Cl,H,H,Cl,(2,3-F₂-Ph)], [A1559;F,H,Me,H,H,Me,(2,3-F₂-Ph)], [A1560;F,H,F,F,F,F,(2,3-F₂-Ph)], [A1561;Me,H,F,H,H,H, (2,3-F₂-Ph)], [A1562;Me,H,Cl,H,H,H,(2,3-F₂-Ph)], [A1563;Me,H,Me,H,H,H,(2,3-F₂-Ph)], [A1564;Me,H,CN,H,H,H,(2,3-F₂-Ph)], [A1565;Me,H,OH,H,H,H,(2,3-F₂-Ph)], [A1566;Me,H,cPr,H,H,H,(2,3-F₂-Ph)], [A1567;Me,H,F,F,H,H,(2,3-F₂-Ph)], [A1568;Me,H,F,H,F,H,(2,3-F₂-Ph)], [A1569;Me,H,F,H,H,F,(2,3-F₂-Ph)], [A1570;Me,H,Cl,H,H,Cl,(2,3-F₂-Ph)], [A1571;Me,H,Me,H,H,Me,(2,3-F₂-Ph)], [A1572;Me,H,F,F,F,F,(2,3-F₂-Ph)], [A1573;OMe,H,F,H,H,H,(2,3-F₂-Ph)], [A1574;OMe,H,Cl,H,H,H,(2,3-F₂-Ph)], [A1575;OMe,H,Me,H,H,H,(2,3-F₂-Ph)], [A1576;OMe,H,CN,H,H,H,(2,3-F₂-Ph)], [A1577;OMe,H,OH,H,H,H,(2,3-F₂-Ph)], [A1578;OMe,H,cPr,H,H,H,(2,3-F₂-Ph)], [A1579;OMe,H,F,F,H,H,(2,3-F₂-Ph)], [A1580;OMe,H,F,H,F,H,(2,3-F₂-Ph)], [A1581;OMe,H,F,H,H,F,(2,3-F₂-Ph)], [A1582;OMe,H,Cl,H,H,Cl,(2,3-F₂-Ph)], [A1583;OMe,H,Me,H,H,Me,(2,3-F₂-Ph)], [A1584;OMe,H,F,F,F,F,(2,3-F₂-Ph)], [A1585;OH,H,F,H,H,H,(2,3-F₂-Ph)], [A1586;OH,H,Cl,H,H,H,(2,3-F₂-Ph)], [A1587;OH,H,Me,H,H,H,(2,3-F₂-Ph)], [A1588;OH,H,CN,H,H,H,(2,3-F₂-Ph)], [A1589;OH,H,OH,H,H,H,(2,3-F₂-Ph)], [A1590;OH,H,cPr,H,H,H,(2,3-F₂-Ph)], [A1591;OH,H,F,F,H,H,(2,3-F₂-Ph)], [A1592;OH,H,F,H,F,H,(2,3-F₂-Ph)], [A1593;OH,H,F,H,H,F,(2,3-F₂-Ph)], [A1594;OH,H,Cl,H,H,Cl,(2,3-F₂-Ph)], [A1595;OH,H,Me,H,H,Me,(2,3-F₂-Ph)], [A1596;OH,H,F,F,F,F,(2,3-F₂-Ph)], [A1597;Me,Me,F,H,H,H,(2,3-F₂-Ph)], [A1598;Me,Me,Cl,H,H,H,(2,3-F₂-Ph)], [A1599;Me,Me,Me,H,H,H,(2,3-F₂-Ph)], [A1600;Me,Me,CN,H,H,H,(2,3-F₂-Ph)], [A1601;Me,Me,OH,H,H,H,(2,3-F₂-Ph)], [A1602;Me,Me,cPr,H,H,H,(2,3-F₂-Ph)], [A1603;Me,Me,F,F,H,H,(2,3-F₂-Ph)], [A1604;Me,Me,F,H,F,H,(2,3-F₂-Ph)], [A1605;Me,Me,F,H,H,F,(2,3-F₂-Ph)], [A1606;Me,Me,Cl,H,H,Cl,(2,3-F₂-Ph)], [A1607;Me,Me,Me,H,H,Me,(2,3-F₂-Ph)], [A1608;Me,Me,F,F,F,F,(2,3-F₂-Ph)], [A1609;F,F,F,H,H,H, (2,3-F₂-Ph)], [A1610;F,F,Cl,H,H,H,(2,3-F₂-Ph)], [A1611;F,F,Me,H,H,H,(2,3-F₂-Ph)], [A1612;F,F,CN,H,H,H,(2,3-F₂-Ph)], [A1613;F,F,OH,H,H,H,(2,3-F₂-Ph)], [A1614;F,F,cPr,H,H,H, (2,3-F₂-Ph)], [A1615;F,F,F,F,H,H, (2,3-F₂-Ph)], [A1616;F,F,F,H,F,H,(2,3-F₂-Ph)], [A1617;F,F,F,H,H,F,(2,3-F₂-Ph)], [A1618;F,F,Cl,H,H,Cl,(2,3-F₂-Ph)], [A1619;F,F,Me,H,H,Me,(2,3-F₂-Ph)], [A1620;F,F,F,F,F,F,(2,3-F₂-Ph)], [A1621;OH,Me,F,H,H,H,(2,3-F₂-Ph)], [A1622;OH,Me,Cl,H,H,H,(2,3-F₂-Ph)], [A1623;OH,Me,Me,H,H,H, (2,3-F₂-Ph)], [A1624;OH,Me,CN,H,H,H, (2,3-F₂-Ph)], [A1625;OH,Me,OH,H,H,H,(2,3-F₂-Ph)], [A1626;OH,Me,cPr,H,H,H, (2,3-F₂-Ph)], [A1627;OH,Me,F,F,H,H,(2,3-F₂-Ph)], [A1628;OH,Me,F,H,F,H,(2,3-F₂-Ph)], [A1629;OH,Me,F,H,H,F,(2,3-F₂-Ph)], [A1630;OH,Me,Cl,H,H,Cl, (2,3-F₂-Ph)], [A1631;OH,Me,Me,H,H,Me, (2,3-F₂-Ph)], [A1632;OH,Me,F,F,F,F,(2,3-F₂-Ph)], [A1633;H,H,F,H,H,H,(2,4-F₂-Ph)], [A1634;H,H,Cl,H,H,H,(2,4-F₂-Ph)], [A1635;H,H,Me,H,H,H,(2,4-F₂-Ph)], [A1636;H,H,CN,H,H,H, (2,4-F₂-Ph)], [A1637;H,H,OH,H,H,H,(2,4-F₂-Ph)], [A1638;H,H,cPr,H,H,H,(2,4-F₂-Ph)], [A1639;H,H,F,F,H,H, (2,4-F₂-Ph)], [A1640;H,H,F,H,F,H,(2,4-F₂-Ph)], [A1641;H,H,F,H,H,F,(2,4-F₂-Ph)], [A1642;H,H,Cl,H,H,Cl,(2,4-F₂-Ph)], [A1643;H,H,Me,H,H,Me,(2,4-F₂-Ph)], [A1644;H,H,F,F,F,F,(2,4-F₂-Ph)], [A1645;F,H,F,H,H,H, (2,4-F₂ -Ph)], [A1646;F,H,Cl,H,H,H,(2,4-F₂-Ph)], [A1647;F,H,Me,H,H,H,(2,4-F₂-Ph)], [A1648;F,H,CN,H,H,H, (2,4-F₂-Ph)], [A1649;F,H,OH,H,H,H,(2,4-F₂-Ph)], [A1650;F,H,cPr,H,H,H,(2,4-F₂-Ph)], [A1651;F,H,F,F,H,H, (2,4-F₂-Ph)], [A1652;F,H,F,H,F,H,(2,4-F₂-Ph)], [A1653;F,H,F,H,H,F,(2,4-F₂-Ph)], [A1654;F,H,Cl,H,H,Cl,(2,4-F₂-Ph)], [A1655;F,H,Me,H,H,Me,(2,4-F₂-Ph)], [A1656;F,H,F,F,F,F,(2,4-F₂-Ph)], [A1657;Me,H,F,H,H,H, (2,4-F₂-Ph)], [A1658;Me,H,Cl,H,H,H,(2,4-F₂-Ph)], [A1659;Me,H,Me,H,H,H,(2,4-F₂-Ph)], [A1660;Me,H,CN,H,H,H,(2,4-F₂-Ph)], [A1661;Me,H,OH,H,H,H,(2,4-F₂-Ph)], [A1662;Me,H,cPr,H,H,H,(2,4-F₂-Ph)], [A1663;Me,H,F,F,H,H,(2,4-F₂-Ph)], [A1664;Me,H,F,H,F,H,(2,4-F₂-Ph)], [A1665;Me,H,F,H,H,F,(2,4-F₂-Ph)], [A1666;Me,H,Cl,H,H,Cl,(2,4-F₂-Ph)], [A1667;Me,H,Me,H,H,Me,(2,4-F₂-Ph)], [A1668;Me,H,F,F,F,F,(2,4-F₂-Ph)], [A1669;OMe,H,F,H,H,H,(2,4-F₂-Ph)], [A1670;OMe,H,Cl,H,H,H,(2,4-F₂-Ph)], [A1671;OMe,H,Me,H,H,H,(2,4-F₂-Ph)], [A1672;OMe,H,CN,H,H,H,(2,4-F₂-Ph)], [A1673;OMe,H,OH,H,H,H,(2,4-F₂-Ph)], [A1674;OMe,H,cPr,H,H,H,(2,4-F₂-Ph)], [A1675;OMe,H,F,F,H,H,(2,4-F₂-Ph)], [A1676;OMe,H,F,H,F,H,(2,4-F₂-Ph)], [A1677;OMe,H,F,H,H,F,(2,4-F₂-Ph)], [A1678;OMe,H,Cl,H,H,Cl,(2,4-F₂-Ph)], [A1679;OMe,H,Me,H,H,Me,(2,4-F₂-Ph)], [A1680;OMe,H,F,F,F,F,(2,4-F₂-Ph)], [A1681;OH,H,F,H,H,H,(2,4-F₂-Ph)], [A1682;OH,H,Cl,H,H,H,(2,4-F₂-Ph)], [A1683;OH,H,Me,H,H,H,(2,4-F₂-Ph)], [A1684;OH,H,CN,H,H,H,(2,4-F₂-Ph)], [A1685;OH,H,OH,H,H,H,(2,4-F₂-Ph)], [A1686;OH,H,cPr,H,H,H,(2,4-F₂-Ph)], [A1687;OH,H,F,F,H,H,(2,4-F₂-Ph)], [A1688;OH,H,F,H,F,H,(2,4-F₂-Ph)], [A1689;OH,H,F,H,H,F,(2,4-F₂-Ph)], [A1690;OH,H,Cl,H,H,Cl,(2,4-F₂-Ph)], [A1691;OH,H,Me,H,H,Me,(2,4-F₂-Ph)], [A1692;OH,H,F,F,F,F,(2,4-F₂-Ph)], [A1693;Me,Me,F,H,H,H,(2,4-F₂-Ph)], [A1694;Me,Me,Cl,H,H,H,(2,4-F₂-Ph)], [A1695;Me,Me,Me,H,H,H,(2,4-F₂-Ph)], [A1696;Me,Me,CN,H,H,H,(2,4-F₂-Ph)], [A1697;Me,Me,OH,H,H,H,(2,4-F₂-Ph)], [A1698;Me,Me,cPr,H,H,H,(2,4-F₂-Ph)], [A1699;Me,Me,F,F,H,H,(2,4-F₂-Ph)], [A1700;Me,Me,F,H,F,H,(2,4-F₂-Ph)], [A1701;Me,Me,F,H,H,F,(2,4-F₂-Ph)], [A1702;Me,Me,Cl,H,H,Cl,(2,4-F₂-Ph)], [A1703;Me,Me,Me,H,H,Me,(2,4-F₂-Ph)], [A1704;Me,Me,F,F,F,F,(2,4-F₂-Ph)], [A1705;F,F,F,H,H,H, (2,4-F₂-Ph)], [A1706;F,F,Cl,H,H,H,(2,4-F₂-Ph)], [A1707;F,F,Me,H,H,H,(2,4-F₂-Ph)], [A1708;F,F,CN,H,H,H,(2,4-F₂-Ph)], [A1709;F,F,OH,H,H,H,(2,4-F₂-Ph)], [A1710;F,F,cPr,H,H,H,(2,4-F₂-Ph)], [A1711;F,F,F,F,H,H,(2,4-F₂-Ph)], [A1712;F,F,F,H,F,H,(2,4-F₂-Ph)], , [A1713;F,F,F,H,H,F,(2,4-F₂-Ph)], [A1714;F,F,Cl,H,H,Cl,(2,4-F₂-Ph)], [A1715;F,F,Me,H,H,Me,(2,4-F₂-Ph)], [A1716;F,F,F,F,F,F,(2,4-F₂-Ph)], [A1717;OH,Me,F,H,H,H,(2,4-F₂-Ph)], [A1718;OH,Me,Cl,H,H,H,(2,4-F₂-Ph)], [A1719;OH,Me,Me,H,H,H,(2,4-F₂-Ph)], [A1720;OH,Me,CN,H,H,H,(2,4-F₂-Ph)], [A1721;OH,Me,OH,H,H,H,(2,4-F₂-Ph)], [A1722;OH,Me,cPr,H,H,H,(2,4-F₂-Ph)], [A1723;OH,Me,F,F,H,H,(2,4-F₂-Ph)], [A1724;OH,Me,F,H,F,H,(2,4-F₂-Ph)], [A1725;OH,Me,F,H,H,F,(2,4-F₂-Ph)], [A1726;OH,Me,Cl,H,H,Cl,(2,4-F₂-Ph)], [A1727;OH,Me,Me,H,H,Me,(2,4-F₂-Ph)], [A1728;OH,Me,F,F,F,F,(2,4-F₂-Ph)], [A1729;H,H,F,H,H,H, (2,5-F₂-Ph)], [A1730;H,H,Cl,H,H,H,(2,5-F₂-Ph)], [A1731;H,H,Me,H,H,H,(2,5-F₂-Ph)], [A1732;H,H,CN,H,H,H, (2,5-F₂-Ph)], [A1733;H,H,OH,H,H,H,(2,5-F₂-Ph)], [A1734;H,H,cPr,H,H,H,(2,5-F₂-Ph)], [A1735;H,H,F,F,H,H, (2,5-F₂-Ph)], [A1736;H,H,F,H,F,H,(2,5-F₂-Ph)], [A1737;H,H,F,H,H,F,(2,5-F₂-Ph)], [A1738;H,H,Cl,H,H,Cl,(2,5-F₂-Ph)], [A1739;H,H,Me,H,H,Me,(2,5-F₂-Ph)], [A1740;H,H,F,F,F,F,(2,5-F₂-Ph)], [A1741;F,H,F,H,H,H, (2,5-F₂-Ph)], [A1742;F,H,Cl,H,H,H,(2,5-F₂-Ph)], [A1743;F,H,Me,H,H,H,(2,5-F₂-Ph)], [A1744;F,H,CN,H,H,H,(2,5-F₂-Ph)], [A1745;F,H,OH,H,H,H,(2,5-F₂-Ph)], [A1746;F,H,cPr,H,H,H,(2,5-F₂-Ph)], [A1747;F,H,F,F,H,H,(2,5-F₂-Ph)], [A1748;F,H,F,H,F,H,(2,5-F₂-Ph)], [A1749;F,H,F,H,H,F,(2,5-F₂-Ph)], [A1750;F,H,Cl,H,H,Cl,(2,5-F₂-Ph)], [A1751;F,H,Me,H,H,Me,(2,5-F₂-Ph)], [A1752;F,H,F,F,F,F,(2,5-F₂-Ph)], [A1753;Me,H,F,H,H,H,(2,5-F₂-Ph)], [A1754;Me,H,Cl,H,H,H,(2,5-F₂-Ph)], [A1755;Me,H,Me,H,H,H,(2,5-F₂-Ph)], [A1756;Me,H,CN,H,H,H,(2,5-F₂-Ph)], [A1757;Me,H,OH,H,H,H,(2,5-F₂-Ph)], [A1758;Me,H,cPr,H,H,H,(2,5-F₂-Ph)], [A1759;Me,H,F,F,H,H,(2,5-F₂-Ph)], [A1760;Me,H,F,H,F,H,(2,5-F₂-Ph)], [A1761;Me,H,F,H,H,F, (2,5-F₂-Ph)], [A1762;Me,H,Cl,H,H,Cl,(2,5-F₂-Ph)], [A1763;Me,H,Me,H,H,Me,(2,5-F₂-Ph)], [A1764;Me,H,F,F,F,F,(2,5-F₂-Ph)], [A1765;OMe,H,F,H,H,H,(2,5-F₂-Ph)], [A1766;OMe,H,Cl,H,H,H,(2,5-F₂-Ph)], [A1767;OMe,H,Me,H,H,H,(2,5-F₂-Ph)], [A1768;OMe,H,CN,H,H,H,(2,5-F₂-Ph)], [A1769;OMe,H,OH,H,H,H,(2,5-F₂-Ph)], [A1770;OMe,H,cPr,H,H,H,(2,5-F₂-Ph)], [A1771;OMe,H,F,F,H,H,(2,5-F₂-Ph)], [A1772;OMe,H,F,H,F,H,(2,5-F₂-Ph)], [A1773;OMe,H,F,H,H,F,(2,5-F₂-Ph)], [A1774;OMe,H,Cl,H,H,Cl,(2,5-F₂-Ph)], [A1775;OMe,H,Me,H,H,Me,(2,5-F₂-Ph)], [A1776;OMe,H,F,F,F,F,(2,5-F₂-Ph)], [A1777;OH,H,F,H,H,H,(2,5-F₂-Ph)], [A1778;OH,H,Cl,H,H,H,(2,5-F₂-Ph)], [A1779;OH,H,Me,H,H,H,(2,5-F₂-Ph)], [A1780;OH,H,CN,H,H,H,(2,5-F₂-Ph)], [A1781;OH,H,OH,H,H,H,(2,5-F₂-Ph)], [A1782;OH,H,cPr,H,H,H,(2,5-F₂-Ph)], [A1783;OH,H,F,F,H,H,(2,5-F₂-Ph)], [A1784;OH,H,F,H,F,H,(2,5-F₂-Ph)], [A1785;OH,H,F,H,H,F,(2,5-F₂-Ph)], [A1786;OH,H,Cl,H,H,Cl,(2,5-F₂-Ph)], [A1787;OH,H,Me,H,H,Me,(2,5-F₂-Ph)], [A1788;OH,H,F,F,F,F,(2,5-F₂-Ph)], [A1789;Me,Me,F,H,H,H,(2,5-F₂-Ph)], [A1790;Me,Me,Cl,H,H,H,(2,5-F₂-Ph)], [A1791;Me,Me,Me,H,H,H,(2,5-F₂-Ph)], [A1792;Me,Me,CN,H,H,H,(2,5-F₂-Ph)], [A1793;Me,Me,OH,H,H,H,(2,5-F₂-Ph)], [A1794;Me,Me,cPr,H,H,H,(2,5-F₂-Ph)], [A1795;Me,Me,F,F,H,H,(2,5-F₂-Ph)], [A1796;Me,Me,F,H,F,H,(2,5-F₂-Ph)], [A1797;Me,Me,F,H,H,F,(2,5-F₂-Ph)], [A1798;Me,Me,Cl,H,H,Cl,(2,5-F₂-Ph)], [A1799;Me,Me,Me,H,H,Me,(2,5-F₂-Ph)], [A1800;Me,Me,F,F,F,F,(2,5-F₂-Ph)], [A1801;F,F,F,H,H,H,(2,5-F₂-Ph)], [A1802;F,F,Cl,H,H,H,(2,5-F₂-Ph)], [A1803;F,F,Me,H,H,H,(2,5-F₂-Ph)], [A1804;F,F,CN,H,H,H,(2,5-F₂-Ph)], [A1805;F,F,OH,H,H,H,(2,5-F₂-Ph)], [A1806;F,F,cPr,H,H,H,(2,5-F₂-Ph)], [A1807;F,F,F,F,H,H,(2,5-F₂-Ph)], [A1808;F,F,F,H,F,H,(2,5-F₂-Ph)], [A1809;F,F,F,H,H,F,(2,5-F₂-Ph)], [A1810;F,F,Cl,H,H,Cl,(2,5-F₂-Ph)], [A1811;F,F,Me,H,H,Me,(2,5-F₂-Ph)], [A1812;F,F,F,F,F,F, (2,5-F₂-Ph)], [A1813;OH,Me,F,H,H,H,(2,5-F₂-Ph)], [A1814;OH,Me,Cl,H,H,H,(2,5-F₂-Ph)], [A1815;OH,Me,Me,H,H,H,(2,5-F₂-Ph)], [A1816;OH,Me,CN,H,H,H,(2,5-F₂-Ph)], [A1817;OH,Me,OH,H,H,H,(2,5-F₂-Ph)], [A1818;OH,Me,cPr,H,H,H, (2,5-F₂-Ph)], [A1819;OH,Me,F,F,H,H,(2,5-F₂-Ph)], [A1820;OH,Me,F,H,F,H,(2,5-F₂-Ph)], [A1821;OH,Me,F,H,H,F,(2,5-F₂-Ph)], [A1822;OH,Me,Cl,H,H,Cl, (2,5-F₂-Ph)], [A1823;OH,Me,Me,H,H,Me,(2,5-F₂-Ph)], [A1824;OH,Me,F,F,F,F,(2,5-F₂-Ph)], [A1825;H,H,F,H,H,H, (2,6-F₂-Ph)], [A1826;H,H,Cl,H,H,H,(2,6-F₂-Ph)], [A1827;H,H,Me,H,H,H,(2,6-F₂-Ph)], [A1828;H,H,CN,H,H,H, (2,6-F₂-Ph)], [A1829;H,H,OH,H,H,H,(2,6-F₂-Ph)], [A1830;H,H,cPr,H,H,H,(2,6-F₂-Ph)], [A1831;H,H,F,F,H,H, (2,6-F₂-Ph)], [A1832;H,H,F,H,F,H,(2,6-F₂-Ph)], [A1833;H,H,F,H,H,F,(2,6-F₂-Ph)], [A1834;H,H,Cl,H,H,Cl,(2,6-F₂-Ph)], [A1835;H,H,Me,H,H,Me, (2,6-F₂-Ph)], [A1836;H,H,F,F,F,F,(2,6-F₂-Ph)], [A1837;F,H,F,H,H,H, (2,6-F₂-Ph)], [A1838;F,H,Cl,H,H,H,(2,6-F₂-Ph)], [A1839;F,H,Me,H,H,H,(2,6-F₂-Ph)], [A1840;F,H,CN,H,H,H,(2,6-F₂-Ph)], [A1841;F,H,OH,H,H,H,(2,6-F₂-Ph)], [A1842;F,H,cPr,H,H,H,(2,6-F₂-Ph)], [A1843;F,H,F,F,H,H,(2,6-F₂-Ph)], [A1844;F,H,F,H,F,H,(2,6-F₂-Ph)], [A1845;F,H,F,H,H,F,(2,6-F₂-Ph)], [A1846;F,H,Cl,H,H,Cl,(2,6-F₂-Ph)], [A1847;F,H,Me,H,H,Me, (2,6-F₂-Ph)], [A1848;F,H,F,F,F,F,(2,6-F₂-Ph)], [A1849;Me,H,F,H,H,H, (2,6-F₂-Ph)], [A1850;Me,H,Cl,H,H,H,(2,6-F₂-Ph)], [A1851;Me,H,Me,H,H,H, (2,6-F₂-Ph)], [A1852;Me,H,CN,H,H,H,(2,6-F₂-Ph)], [A1853;Me,H,OH,H,H,H,(2,6-F₂-Ph)], [A1854;Me,H,cPr,H,H,H,(2,6-F₂-Ph)], [A1855;Me,H,F,F,H,H,(2,6-F₂-Ph)], [A1856;Me,H,F,H,F,H, (2,6-F₂-Ph)], [A1857;Me,H,F,H,H,F,(2,6-F₂-Ph)], [A1858;Me,H,Cl,H,H,Cl, (2,6-F₂-Ph)], [A1859;Me,H,Me,H,H,Me, (2,6-F₂-Ph)], [A1860;Me,H,F,F,F,F,(2,6-F₂-Ph)], [A1861;OMe,H,F,H,H,H,(2,6-F₂-Ph)], [A1862;OMe,H,Cl,H,H,H,(2,6-F₂-Ph)], [A1863;OMe,H,Me,H,H,H,(2,6-F₂-Ph)], [A1864;OMe,H,CN,H,H,H,(2,6-F₂-Ph)], [A1865;OMe,H,OH,H,H,H,(2,6-F₂-Ph)], [A1866;OMe,H,cPr,H,H,H,(2,6-F₂-Ph)], [A1867;OMe,H,F,F,H,H,(2,6-F₂-Ph)], [A1868;OMe,H,F,H,F,H,(2,6-F₂-Ph)], [A1869;OMe,H,F,H,H,F,(2,6-F₂-Ph)], [A1870;OMe,H,Cl,H,H,Cl,(2,6-F₂-Ph)], [A1871;OMe,H,Me,H,H,Me,(2,6-F₂-Ph)], [A1872;OMe,H,F,F,F,F, (2,6-F₂-Ph)], [A1873;OH,H,F,H,H,H,(2,6-F₂-Ph)], [A1874;OH,H,Cl,H,H,H,(2,6-F₂-Ph)], [A1875;OH,H,Me,H,H,H,(2,6-F₂-Ph)], [A1876;OH,H,CN,H,H,H,(2,6-F₂-Ph)], [A1877;OH,H,OH,H,H,H,(2,6-F₂-Ph)], [A1878;OH,H,cPr,H,H,H,(2,6-F₂-Ph)], [A1879;OH,H,F,F,H,H,(2,6-F₂-Ph)], [A1880;OH,H,F,H,F,H,(2,6-F₂-Ph)], [A1881;OH,H,F,H,H,F,(2,6-F₂-Ph)], [A1882;OH,H,Cl,H,H,Cl,(2,6-F₂-Ph)], [A1883;OH,H,Me,H,H,Me,(2,6-F₂-Ph)], [A1884;OH,H,F,F,F,F,(2,6-F₂-Ph)], [A1885;Me,Me,F,H,H,H,(2,6-F₂-Ph)], [A1886;Me,Me,Cl,H,H,H,(2,6-F₂-Ph)], [A1887;Me,Me,Me,H,H,H,(2,6-F₂-Ph)], [A1888;Me,Me,CN,H,H,H,(2,6-F₂-Ph)], [A1889;Me,Me,OH,H,H,H,(2,6-F₂-Ph)], [A1890;Me,Me,cPr,H,H,H,(2,6-F₂-Ph)], [A1891;Me,Me,F,F,H,H,(2,6-F₂-Ph)], [A1892;Me,Me,F,H,F,H,(2,6-F₂-Ph)], [A1893;Me,Me,F,H,H,F,(2,6-F₂-Ph)], [A1894;Me,Me,Cl,H,H,Cl,(2,6-F₂-Ph)], [A1895;Me,Me,Me,H,H,Me,(2,6-F₂-Ph)], [A1896;Me,Me,F,F,F,F,(2,6-F₂-Ph)], [A1897;F,F,F,H,H,H, (2,6-F₂-Ph)], [A1898;F,F,Cl,H,H,H,(2,6-F₂-Ph)], [A1899;F,F,Me,H,H,H,(2,6-F₂-Ph)], [A1900;F,F,CN,H,H,H, (2,6-F₂-Ph)], [A1901;F,F,OH,H,H,H,(2,6-F₂-Ph)], [A1902;F,F,cPr,H,H,H,(2,6-F₂-Ph)], [A1903;F,F,F,F,H,H,(2,6-F₂-Ph)], [A1904;F,F,F,H,F,H,(2,6-F₂-Ph)], [A1905;F,F,F,H,H,F,(2,6-F₂-Ph)], [A1906;F,F,Cl,H,H,Cl,(2,6-F₂-Ph)], [A1907;F,F,Me,H,H,Me,(2,6-F₂-Ph)], [A1908;F,F,F,F,F,F,(2,6-F₂-Ph)], [A1909;OH,Me,F,H,H,H,(2,6-F₂-Ph)], [A1910;OH,Me,Cl,H,H,H,(2,6-F₂-Ph)], [A1911;OH,Me,Me,H,H,H,(2,6-F₂-Ph)], [A1912;OH,Me,CN,H,H,H,(2,6-F₂-Ph)], [A1913;OH,Me,OH,H,H,H,(2,6-F₂-Ph)], [A1914;OH,Me,cPr,H,H,H,(2,6-F₂-Ph)], [A1915;OH,Me,F,F,H,H,(2,6-F₂-Ph)], [A1916;OH,Me,F,H,F,H,(2,6-F₂-Ph)], [A1917;OH,Me,F,H,H,F,(2,6-F₂-Ph)], [A1918;OH,Me,Cl,H,H,Cl,(2,6-F₂-Ph)], [A1919;OH,Me,Me,H,H,Me,(2,6-F₂-Ph)], [A1920;OH,Me,F,F,F,F,(2,6-F₂-Ph)], [A1921;H,H,F,H,H,H, (3,4-F₂-Ph)], [A1922;H,H,Cl,H,H,H,(3,4-F₂-Ph)], [A1923;H,H,Me,H,H,H,(3,4-F₂-Ph)], [A1924;H,H,CN,H,H,H, (3,4-F₂-Ph)], [A1925;H,H,OH,H,H,H,(3,4-F₂-Ph)], [A1926;H,H,cPr,H,H,H,(3,4-F₂-Ph)], [A1927;H,H,F,F,H,H, (3,4-F₂-Ph)], [A1928;H,H,F,H,F,H,(3,4-F₂-Ph)], [A1929;H,H,F,H,H,F,(3,4-F₂-Ph)], [A1930;H,H,Cl,H,H,Cl,(3,4-F₂-Ph)], [A1931;H,H,Me,H,H,Me,(3,4-F₂-Ph)], [A1932;H,H,F,F,F,F,(3,4-F₂-Ph)], [A1933;F,H,F,H,H,H,(3,4-F₂-Ph)], [A1934;F,H,Cl,H,H,H,(3,4-F₂-Ph)], [A1935;F,H,Me,H,H,H,(3,4-F₂-Ph)], [A1936;F,H,CN,H,H,H, (3,4-F₂-Ph)], [A1937;F,H,OH,H,H,H,(3,4-F₂-Ph)], [A1938;F,H,cPr,H,H,H,(3,4-F₂-Ph)], [A1939;F,H,F,F,H,H, (3,4-F₂-Ph)], [A1940;F,H,F,H,F,H,(3,4-F₂-Ph)], [A1941;F,H,F,H,H,F,(3,4-F₂-Ph)], [A1942;F,H,Cl,H,H,Cl,(3,4-F₂-Ph)], [A1943;F,H,Me,H,H,Me,(3,4-F₂-Ph)], [A1944;F,H,F,F,F,F,(3,4-F₂-Ph)], [A1945;Me,H,F,H,H,H, (3,4-F₂-Ph)], [A1946;Me,H,Cl,H,H,H,(3,4-F₂-Ph)], [A1947;Me,H,Me,H,H,H, (3,4-F₂-Ph)], [A1948;Me,H,CN,H,H,H, (3,4-F₂-Ph)], [A1949;Me,H,OH,H,H,H, (3,4-F₂-Ph)], [A1950;Me,H,cPr,H,H,H, (3,4-F₂-Ph)], [A1951;Me,H,F,F,H,H,(3,4-F₂-Ph)], [A1952;Me,H,F,H,F,H, (3,4-F₂-Ph)], [A1953;Me,H,F,H,H,F,(3,4-F₂-Ph)], [A1954;Me,H,Cl,H,H,Cl, (3,4-F₂-Ph)], [A1955;Me,H,Me,H,H,Me, (3,4-F₂-Ph)], [A1956;Me,H,F,F,F,F,(3,4-F₂-Ph)], [A1957;OMe,H,F,H,H,H,(3,4-F₂-Ph)], [A1958;OMe,H,Cl,H,H,H,(3,4-F₂-Ph)], [A1959;OMe,H,Me,H,H,H,(3,4-F₂-Ph)], [A1960;OMe,H,CN,H,H,H,(3,4-F₂-Ph)], [A1961;OMe,H,OH,H,H,H,(3,4-F₂-Ph)], [A1962;OMe,H,cPr,H,H,H,(3,4-F₂-Ph)], [A1963;OMe,H,F,F,H,H,(3,4-F₂-Ph)], [A1964;OMe,H,F,H,F,H,(3,4-F₂-Ph)], [A1965;OMe,H,F,H,H,F,(3,4-F₂-Ph)], [A1966;OMe,H,Cl,H,H,Cl,(3,4-F₂-Ph)], [A1967;OMe,H,Me,H,H,Me,(3,4-F₂-Ph)], [A1968;OMe,H,F,F,F,F,(3,4-F₂-Ph)], [A1969;OH,H,F,H,H,H,(3,4-F₂-Ph)], [A1970;OH,H,Cl,H,H,H,(3,4-F₂-Ph)], [A1971;OH,H,Me,H,H,H,(3,4-F₂-Ph)], [A1972;OH,H,CN,H,H,H,(3,4-F₂-Ph)], [A1973;OH,H,OH,H,H,H,(3,4-F₂-Ph)], [A1974;OH,H,cPr,H,H,H,(3,4-F₂-Ph)], [A1975;OH,H,F,F,H,H,(3,4-F₂-Ph)], [A1976;OH,H,F,H,F,H, (3,4-F₂-Ph)], [A1977;OH,H,F,H,H,F,(3,4-F₂-Ph)], [A1978;OH,H,Cl,H,H,Cl,(3,4-F₂-Ph)], [A1979;OH,H,Me,H,H,Me,(3,4-F₂-Ph)], [A1980;OH,H,F,F,F,F,(3,4-F₂-Ph)], [A1981;Me,Me,F,H,H,H,(3,4-F₂-Ph)], [A1982;Me,Me,Cl,H,H,H,(3,4-F₂-Ph)], [A1983;Me,Me,Me,H,H,H,(3,4-F₂-Ph)], [A1984;Me,Me,CN,H,H,H,(3,4-F₂-Ph)], [A1985;Me,Me,OH,H,H,H,(3,4-F₂-Ph)], [A1986;Me,Me,cPr,H,H,H,(3,4-F₂-Ph)], [A1987;Me,Me,F,F,H,H,(3,4-F₂-Ph)], [A1988;Me,Me,F,H,F,H,(3,4-F₂-Ph)], [A1989;Me,Me,F,H,H,F,(3,4-F₂-Ph)], [A1990;Me,Me,Cl,H,H,Cl,(3,4-F₂-Ph)], [A1991;Me,Me,Me,H,H,Me,(3,4-F₂-Ph)], [A1992;Me,Me,F,F,F,F,(3,4-F₂-Ph)], [A1993;F,F,F,H,H,H, (3,4-F₂-Ph)], [A1994;F,F,Cl,H,H,H,(3,4-F₂-Ph)], [A1995;F,F,Me,H,H,H,(3,4-F₂-Ph)], [A1996;F,F,CN,H,H,H, (3,4-F₂-Ph)], [A1997;F,F,OH,H,H,H,(3,4-F₂-Ph)], [A1998;F,F,cPr,H,H,H,(3,4-F₂-Ph)], [A1999;F,F,F,F,H,H, (3,4-F₂-Ph)], [A2000;F,F,F,H,F,H,(3,4-F₂-Ph)], [A2001;F,F,F,H,H,F,(3,4-F₂-Ph)], [A2002;F,F,Cl,H,H,Cl,(3,4-F₂-Ph)], [A2003;F,F,Me,H,H,Me,(3,4-F₂-Ph)], [A2004;F,F,F,F,F,F,(3,4-F₂-Ph)], [A2005;OH,Me,F,H,H,H,(3,4-F₂-Ph)], [A2006;OH,Me,Cl,H,H,H,(3,4-F₂-Ph)], [A2007;OH,Me,Me,H,H,H,(3,4-F₂-Ph)], [A2008;OH,Me,CN,H,H,H,(3,4-F₂-Ph)], [A2009;OH,Me,OH,H,H,H,(3,4-F₂-Ph)], [A2010;OH,Me,cPr,H,H,H,(3,4-F₂-Ph)], [A2011;OH,Me,F,F,H,H,(3,4-F₂-Ph)], [A2012;OH,Me,F,H,F,H,(3,4-F₂-Ph)], [A2013;OH,Me,F,H,H,F,(3,4-F₂-Ph)], [A2014;OH,Me,Cl,H,H,Cl, (3,4-F₂-Ph)], [A2015;OH,Me,Me,H,H,Me,(3,4-F₂-Ph)], [A2016;OH,Me,F,F,F,F,(3,4-F₂-Ph)], [A2017;H,H,F,H,H,H, (3,5-F₂-Ph)], [A2018;H,H,Cl,H,H,H,(3,5-F₂-Ph)], [A2019;H,H,Me,H,H,H,(3,5-F₂-Ph)], [A2020;H,H,CN,H,H,H,(3,5-F₂-Ph)], [A2021;H,H,OH,H,H,H,(3,5-F₂-Ph)], [A2022;H,H,cPr,H,H,H,(3,5-F₂-Ph)], [A2023;H,H,F,F,H,H, (3,5-F₂-Ph)], [A2024;H,H,F,H,F,H,(3,5-F₂-Ph)], [A2025;H,H,F,H,H,F,(3,5-F₂-Ph)], [A2026;H,H,Cl,H,H,Cl,(3,5-F₂-Ph)], [A2027;H,H,Me,H,H,Me,(3,5-F₂-Ph)], [A2028;H,H,F,F,F,F,(3,5-F₂-Ph)], [A2029;F,H,F,H,H,H, (3,5-F₂-Ph)], [A2030;F,H,Cl,H,H,H,(3,5-F₂-Ph)], [A2031;F,H,Me,H,H,H,(3,5-F₂-Ph)], [A2032;F,H,CN,H,H,H, (3,5-F₂-Ph)], [A2033;F,H,OH,H,H,H,(3,5-F₂-Ph)], [A2034;F,H,cPr,H,H,H,(3,5-F₂-Ph)], [A2035;F,H,F,F,H,H, (3,5-F₂-Ph)], [A2036;F,H,F,H,F,H,(3,5-F₂-Ph)], [A2037;F,H,F,H,H,F,(3,5-F₂-Ph)], [A2038;F,H,Cl,H,H,Cl,(3,5-F₂-Ph)], [A2039;F,H,Me,H,H,Me,(3,5-F₂-Ph)], [A2040;F,H,F,F,F,F,(3,5-F₂-Ph)], [A2041;Me,H,F,H,H,H,(3,5-F₂-Ph)], [A2042;Me,H,Cl,H,H,H,(3,5-F₂-Ph)], [A2043;Me,H,Me,H,H,H,(3,5-F₂-Ph)], [A2044;Me,H,CN,H,H,H,(3,5-F₂-Ph)], [A2045;Me,H,OH,H,H,H, (3,5-F₂-Ph)], [A2046;Me,H,cPr,H,H,H,(3,5-F₂-Ph)], [A2047;Me,H,F,F,H,H,(3,5-F₂-Ph)], **[A2048;Me,H,F,H,F,H, (3,5-**F₂-Ph)], [A2049;Me,H,F,H,H,F,(3,5-F₂-Ph)], [A2050;Me,H,Cl,H,H,Cl,(3,5-F₂-Ph)], [A2051;Me,H,Me,H,H,Me,(3,5-F₂-Ph)], [A2052;Me,H,F,F,F,F,(3,5-F₂-Ph)], [A2053;OMe,H,F,H,H,H,(3,5-F₂-Ph)], [A2054;OMe,H,Cl,H,H,H,(3,5-F₂-Ph)], [A2055;OMe,H,Me,H,H,H,(3,5-F₂-Ph)], [A2056;OMe,H,CN,H,H,H,(3,5-F₂-Ph)], [A2057;OMe,H,OH,H,H,H,(3,5-F₂-Ph)], [A2058;OMe,H,cPr,H,H,H,(3,5-F₂-Ph)], [A2059;OMe,H,F,F,H,H,(3,5-F₂-Ph)], [A2060;OMe,H,F,H,F,H,(3,5-F₂-Ph)], [A2061;OMe,H,F,H,H,F,(3,5-F₂-Ph)], [A2062;OMe,H,Cl,H,H,Cl,(3,5-F₂-Ph)], [A2063;OMe,H,Me,H,H,Me,(3,5-F₂-Ph)], [A2064;OMe,H,F,F,F,F,(3,5-F₂-Ph)], [A2065;OH,H,F,H,H,H,(3,5-F₂-Ph)], [A2066;OH,H,C1,H,H,H,(3,5-F₂-Ph)], [A2067;OH,H,Me,H,H,H,(3,5-F₂-Ph)], [A2068;OH,H,CN,H,H,H,(3,5-F₂-Ph)], [A2069;OH,H,OH,H,H,H,(3,5-F₂-Ph)], [A2070;OH,H,cPr,H,H,H,(3,5-F₂-Ph)], [A2071;OH,H,F,F,H,H,(3,5-F₂-Ph)], [A2072;OH,H,F,H,F,H, (3,5-F₂-Ph)], [A2073;OH,H,F,H,H,F,(3,5-F₂-Ph)], [A2074;OH,H,Cl,H,H,Cl,(3,5-F₂-Ph)], [A2075;OH,H,Me,H,H,Me,(3,5-F₂-Ph)], [A2076;OH,H,F,F,F,F,(3,5-F₂-Ph)], [A2077;Me,Me,F,H,H,H,(3,5-F₂-Ph)], [A2078;Me,Me,Cl,H,H,H,(3,5-F₂-Ph)], [A2079;Me,Me,Me,H,H,H,(3,5-F₂-Ph)], [A2080;Me,Me,CN,H,H,H,(3,5-F₂-Ph)], [A2081;Me,Me,OH,H,H,H,(3,5-F₂-Ph)], [A2082;Me,Me,cPr,H,H,H,(3,5-F₂-Ph)], [A2083;Me,Me,F,F,H,H,(3,5-F₂-Ph)], [A2084;Me,Me,F,H,F,H,(3,5-F₂-Ph)], [A2085;Me,Me,F,H,H,F,(3,5-F₂-Ph)], [A2086;Me,Me,Cl,H,H,Cl,(3,5-F₂-Ph)], [A2087;Me,Me,Me,H,H,Me,(3,5-F₂-Ph)], [A2088;Me,Me,F,F,F,F,(3,5-F₂-Ph)], [A2089;F,F,F,H,H,H, (3,5-F₂-Ph)], [A2090;F,F,Cl,H,H,H,(3,5-F₂-Ph)], [A2091;F,F,Me,H,H,H,(3,5-F₂-Ph)], [A2092;F,F,CN,H,H,H, (3, 5-F₂-Ph)], [A2093;F,F,OH,H,H,H,(3,5-F₂-Ph)], [A2094;F,F,cPr,H,H,H,(3,5-F₂-Ph)], [A2095;F,F,F,F,H,H, (3,5-F₂-Ph)], [A2096;F,F,F,H,F,H,(3,5-F₂-Ph)], [A2097;F,F,F,H,H,F,(3,5-F₂-Ph)], [A2098;F,F,Cl,H,H,Cl,(3,5-F₂-Ph)], [A2099;F,F,Me,H,H,Me,(3,5-F₂-Ph)], [A2100;F,F,F,F,F,F,(3,5-F₂-Ph)], [A2101;OH,Me,F,H,H,H,(3,5-F₂-Ph)], [A2102;OH,Me,Cl,H,H,H,(3,5-F₂-Ph)], [A2103;OH,Me,Me,H,H,H,(3,5-F₂-Ph)], [A2104;OH,Me,CN,H,H,H,(3,5-F₂-Ph)], [A2105;OH,Me,OH,H,H,H,(3,5-F₂-Ph)], [A2106;OH,Me,cPr,H,H,H, (3,5-F₂-Ph)], [A2107;OH,Me,F,F,H,H,(3,5-F₂-Ph)], [A2108;OH,Me,F,H,F,H,(3,5-F₂-Ph)], [A2109;OH,Me,F,H,H,F,(3,5-F₂-Ph)], [A2110;OH,Me,Cl,H,H,Cl,(3,5-F₂-Ph)], [A2111;OH,Me,Me,H,H,Me,(3,5-F₂-Ph)], [A2112;OH,Me,F,F,F,F,(3,5-F₂-Ph)].

The Compound (A-1) wherein Z represents a 1-methyl-1H-pyrazole-3-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX2").

The Compound (A-1) wherein Z represents a 1-methyl-1H-pyrazole-2-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX3").

The Compound (A-1) wherein Z represents a 1-methyl-1H-pyrazole-4-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX4").

The Compound (A-1) wherein Z represents an oxazole-2-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX5").

The Compound (A-1) wherein Z represents a thiadiazole-2-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX6").

The Compound (A-1) wherein Z represents an oxazole-4-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX7").

The Compound (A-1) wherein Z represents a thiadiazole-4-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX8").

The Compound (A-1) wherein Z represents an isoxazole-3-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX9").

The Compound (A-1) wherein Z represents an isothiazole-3-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX10").

The Compound (A-1) wherein Z represents an 1,2,4-oxadiazole-5-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX11").

The Compound (A-1) wherein Z represents an 1,2,4-thidiazole-5-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX12").

The Compound (A-1) wherein Z represents an 1,2,4-oxadiazole-3-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX13").

The Compound (A-1) wherein Z represents an 1,2,4-thidiazole-3-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX14").

The Compound (A-1) wherein Z represents an 1,2,3-triazole-1-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX15").

The Compound (A-1) wherein Z represents an 1,2,3-triazole-2-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX16").

The Compound (A-1) wherein Z represents a tetrazole-1-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX17").

The Compound (A-1) wherein Z represents a tetrazole-2-yl group, and the combination of R^{1X}, R^{2X}, R^{3X}, R^{4X}, R^{5X}, R^{6X}, and E^{1X} represents any combination indicated in the Combination A (hereinafter referred to as "Compound group SX18").

A compound represented by formula (B-1): 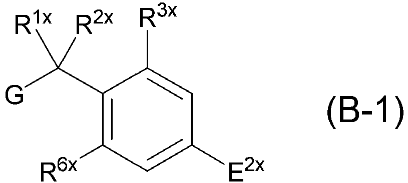 (hereinafter, referred to as "Compound (B-1)")
wherein R^{1X}, R^{2X}, and R^{3X} represent a hydrogen atom, R^{6X} represents a fluorine atom, and E^{2X} represents a phenyl group, and the combination of a group indicated by G, as well as a substituents R^{z1}, R^{z2}, and R^{z3} on the group indicated by G represents any combination indicated in the Combination B (hereinafter referred to as "Compound group SX19").

G1 to G24 represents a group represented by the following formula G1 to formula G24 respectively.

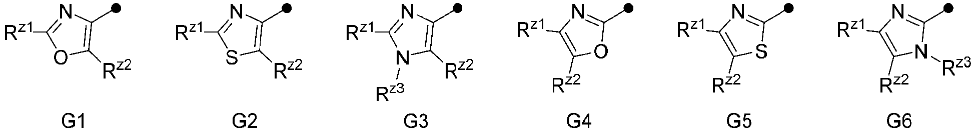

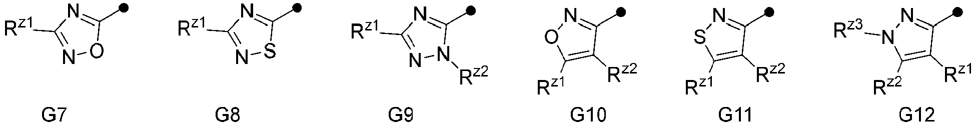

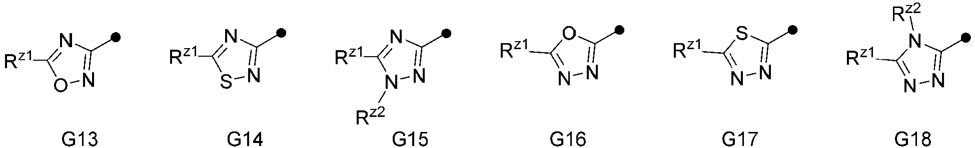

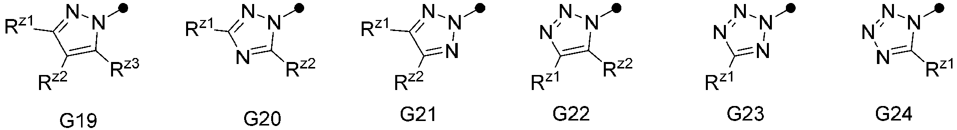

The combination B consists of the substituent numbers B1 to B374. The substituent numbers B1 to B374 represents any combinations of a group indicated by G, as well as a substituents R^{z1}, R^{z2}, and R^{z3} on the group indicated by G, and is hereinafter expressed as [Substituent number: G, R^{z1}, R^{z2}, R^{z3}].

For example, the substituent number B5 represents the combination wherein G represents a group represented by formula G1, R^{z1} represents an iodine atom, and R^{z2} represents a hydrogen atom.
Combination B: [B1; G1, H, H, -], [B2; G1, F, H, -], [B3; G1, Cl, H, -], [B4; G1, Br, H, -], [B5; G1, I, H, -], [B6; G1, Me, H, -], [B7; G1, OMe, H, -], [B8; G1, cPr, H, -], [B9; G1, CF₃, H, -], [B10; G1, CF₂H, H, -], [B11; G1, H, F, -], [B12; G1, H, Cl, -], [B13; G1, H, Br, -], [B14; G1, H, I, -], [B15; G1, H, Me, -], [B16; G1, H, OMe, -], [B17; G1, H, cPr, -], [B18; G1, H, CF₃, -], [B19; G1, H, CF₂H, -], [B20; G1, Me, Me, -], [B21; G1, Cl, Cl, -], [B22; G2, H, H, -], [B23; G2, F, H, -], [B24; G2, Cl, H, -], [B25; G2, Br, H, -], [B26; G2, I, H, -], [B27; G2, Me, H, -], [B28; G2, OMe, H, -], [B29; G2, cPr, H, -], [B30; G2, CF₃, H, -], [B31; G2, CF₂H, H, -], [B32; G2, H, F, -], [B33; G2, H, Cl, -], [B34; G2, H, Br, -], [B35; G2, H, I, -], [B36; G2, H, Me, -], [B37; G2, H, OMe, -], [B38; G2, H, cPr, -], [B39; G2, H, CF₃, -], [B40; G2, H, CF₂H, -], [B41; G2, Me, Me, -], [B42; G2, Cl, Cl, -], [B43; G3, H, H, Me], [B44; G3, F, H, Me], [B45; G3, Cl, H, Me], [B46; G3, Me, H, Me], [B47; G3, OMe, H, Me], [B48; G3, cPr, H, Me], [B49; G3, CF₃, H, Me], [B50; G3, CF₂H, H, Me], [B51; G3, H, F, Me], [B52; G3, H, Cl, Me], [B53; G3, H, Me, Me], [B54; G3, H, OMe, Me], [B55; G3, H, cPr, Me], [B56; G3, H, CF₃, Me], [B57; G3, H, CF₂H, Me], [B58; G3, Me, Me, Me], [B59; G3, Cl, Cl, Me], [B60; G3, H, H, H], [B61; G4, H, H, -], [B62; G4, F, H, -], [B63; G4, Cl, H, -], [B64; G4, Br, H, -], [B65; G4, I, H, -], [B66; G4, Me, H, -], [B67; G4, OMe, H, -], [B68; G4, cPr, H, -], [B69; G4, CF₃, H, -], [B70; G4, CF₂H, H, -], [B71; G4, H, F, -], [B72; G4, H, Cl, -], [B73; G4, H, Br, -], [B74; G4, H, I, -], [B75; G4, H, Me, -], [B76; G4, H, OMe, -], [B77; G4, H, cPr, -], [B78; G4, H, CF₃, -], [B79; G4, H, CF₂H, -], [B80; G4, Me, Me, -], [B81; G4, Cl, Cl, -], [B82; G5, H, H, -], [B83; G5, F, H, -], [B84; G5, Cl, H, -], [B85; G5, Br, H, -], [B86; G5, I, H, -], [B87; G5, Me, H, -], [B88; G5, OMe, H, -], [B89; G5, cPr, H, -], [B90; G5, CF₃, H, -], [B91; G5, CF₂H, H, -], [B92; G5, H, F, -], [B93; G5, H, Cl, -], [B94; G5, H, Br, -], [B95; G5, H, I, -], [B96; G5, H, Me, -], [B97; G5, H, OMe, -], [B98; G5, H, cPr, -], [B99; G5, H, CF₃, -], [B100; G5, H, CF₂H, -], [B101; G5, Me, Me, -], [B102; G5, Cl, Cl, -], [B103; G6, H, H, Me], [B104; G6, F, H, Me], [B105; G6, Cl, H, Me], [B106; G6, Me, H, Me], [B107; G6, OMe, H, Me], [B108; G6, cPr, H, Me], [B109; G6, CF₃, H, Me], [B110; G6, CF₂H, H, Me], [B111; G6, H, F, Me], [B112; G6, H, Cl, Me], [B113; G6, H, Me, Me], [B114; G6, H, OMe, Me], [B115; G6, H, cPr, Me], [B116; G6, H, CF₃, Me], [B117; G6, H, CF₂H, Me], [B118; G6, H, H, H], [B119; G7, H, -, -], [B120; G7, F, -, -], [B121; G7, Cl, -, -], [B122; G7, Br, -, -], [B123; G7, I, -, -], [B124; G7, Me, -, -], [B125; G7, OMe, -, -], [B126; G7, cPr, -, -], [B127; G7, CF₃, -, -], [B128; G7, CF₂H, -, -], [B129; G8, H, -, -], [B130; G8, F, -, -], [B131; G8, Cl, -, -], [B132; G8, Br, -, -], [B133; G8, I, -, -], [B134; G8, Me, -, -], [B135; G8, OMe, -, -], [B136; G8, cPr, -, -], [B137; G8, CF₃₋, -, -], [B138; G8, CF₂H, -, -], [B139; G9, H, Me, -], [B140; G9, F, Me, -], [B141; G9, Cl, Me, -], [B142; G9, Br, Me, -], [B143; G9, I, Me, -], [B144; G9, Me, Me, -], [B145; G9, OMe, Me, -], [B146; G9, cPr, Me, -], [B147; G9, CF₃, Me, -], [B148; G9, CF₂H, Me, -], [B149; G9, H, H, -], [B150; G10, H, H, -], [B151; G10, F, H, -], [B152; G10, Cl, H, -], [B153; G10, Br, H, -], [B154; G10, I, H, -], [B155; G10, Me, H, -], [B156; G10, OMe, H, -], [B157; G10, cPr, H, -], [B158; G10, CF₃, H, -], [B159; G10, CF₂H, H, -], [B160; G10, H, F, -], [B161; G10, H, Cl, -], [B162; G10, H, Br, -], [B163; G10, H, I, -], [B164; G10, H, Me, -], [B165; G10, H, OMe, -], [B166; G10, H, cPr, -], [B167; G10, H, CF₃, -], [B168; G10, H, CF₂H, -], [B169; G10, Me, Me, -], [B170; G10, Cl, Cl, -], [B171; G11, H, H, -], [B172; G11, F, H, -], [B173; G11, Cl, H, -], [B174; G11, Br, H, -], [B175; G11, I, H, -], [B176; G11, Me, H, -], [B177; G11, OMe, H, -], [B178; G11, cPr, H, -], [B179; G11, CF₃, H, -], [B180; G11, CF₂H, H, -], [B181; G11, H, F, -], [B182; G11, H, Cl, -], [B183; G11, H, Br, -], [B184; G11, H, I, -], [B185; G11, H, Me, -], [B186; G11, H, OMe, -], [B187; G11, H, cPr, -], [B188; G11, H, CF₃, -], [B189; G11, H, CF₂H, -], [B190; G11, Me, Me, -], [B191; G11, Cl, Cl, -], [B192; G12, H, H, Me], [B193; G12, F, H, Me], [B194; G12, Cl, H, Me], [B195; G12, Br, H, Me], [B196; G12, I, H, Me], [B197; G12, Me, H, Me], [B198; G12, OMe, H, Me], [B199; G12, cPr, H, Me], [B200; G12, CF₃, H, Me], [B201; G12, CF₂H, H, Me], [B202; G12, H, F, Me], [B203; G12, H, Cl, Me], [B204; G12, H, Br, Me], [B205; G12, H, I, Me], [B206; G12, H, Me, Me], [B207; G12, H, OMe, Me], [B208; G12, H, cPr, Me], [B209; G12, H, CF₃, Me], [B210; G12, H, CF₂H, Me], [B211; G12, Me, Me, Me], [B212; G12, Cl, Cl, Me], [B213; G12, H, H, Et], [B214; G12, H, H, Pr], [B215; G12, H, H, iPr], [B216; G12, H, H, cPr], [B217; G13, H, -, -], [B218; G13, F, -, -], [B219; G13, Cl, -, -], [B220; G13, Br, -, -], [B221; G13, I, -, -], [B222; G13, Me, -, -], [B223; G13, OMe, -, -], [B224; G13, cPr, -, -], [B225; G13, CF₃, -, -], [B226; G13, CF₂H, -, -], [B227; G14, H, -, -], [B228; G14, F, -, -], [B229; G14, Cl, -, -], [B230; G14, Br, -, -], [B231; G14, I, -, -], [B232; G14, Me, -, -], [B233; G14, OMe, -, -], [B234; G14, cPr, -, -], [B235; G14, CF₃, -, -], [B236; G14, CF₂H, -, -], [B237; G15, H, Me, -], [B238; G15, F, Me, -], [B239; G15, Cl, Me, -], [B240; G15, Br, Me, -], [B241; G15, I, Me, -], [B242; G15, Me, Me, -], [B243; G15, OMe, Me, -], [B244; G15, cPr, Me, -], [B245; G15, CF₃, Me, -], [B246; G15, CF₂H, Me, -], [B247; G15, H, H, -], [B248; G16, H, -, -], [B249; G16, F, -, -], [B250; G16, Cl, -, -], [B251; G16, Br, -, -], [B252; G16, I, -, -], [B253; G16, Me, -, -], [B254; G16, OMe, -, -], [B255; G16, cPr, -, -], [B256; G16, CF₃₋, -, -], [B257; G16, CF₂H, -, -], [B258; G17, H, -, -], [B259; G17, F, -, -], [B260; G17, Cl, -, -], [B261; G17, Br, -, -], [B262; G17, I, -, -], [B263; G17, Me, -, -], [B264; G17, OMe, -, -], [B265; G17, cPr, -, -], [B266; G17, CF₃, -, -], [B267; G17, CF₂H, -, -], [B268; G18, H, Me, -], [B269; G18, F, Me, -], [B270; G18, Cl, Me, -], [B271; G18, Br, Me, -], [B272; G18, I, Me, -], [B273; G18, Me, Me, -], [B274; G18, OMe, Me, -], [B275; G18, cPr, Me, -], [B276; G18, CF₃, Me, -], [B277; G18, CF₂H, Me, -], [B278; G18, H, H, -], [B279; G19, H, H, H], [B280; G19, F, H, H], [B281; G19, Cl, H, H], [B282; G19, Br, H, H], [B283; G19, I, H, H], [B284; G19, Me, H, H], [B285; G19, OMe, H, H], [B286; G19, cPr, H, H], [B287; G19, CF₃, H, H], [B288; G19, CF₂H, H, H], [B289; G19, H, F, H], [B290; G19, H, Cl, H], [B291; G19, H, Br, H], [B292; G19, H, I, H], [B293; G19, H, Me, H], [B294; G19, H, OMe, H], [B295; G19, H, cPr, H], [B296; G19, H, CF₃, H], [B297; G19, H, CF₂H, H], [B298; G19, Me, Me, H], [B299; G19, Cl, Cl, H], [B300; G19, H, H, F], [B301; G19, H, H, Cl], [B302; G19, H, H, Br], [B303; G19, H, H, I], [B304; G19, H, H, Me], [B305; G19, H, H, OMe], [B306; G19, H, H, cPr], [B307; G19, H, H, CF₃], [B308; G19, H, H, CF₂H], [B309; G19, H, Me, Me], [B310; G19, H, Cl, Cl], [B311; G19, Me, H, Me], [B312; G19, CF₂H, H, CF₂H], [B313; G19, Me, H, CF₃], [B314; G20, H, H, -], [B315; G20, F, H, -], [B316; G20, Cl, H, -], [B317; G20, Me, H, -], [B318; G20, OMe, H, -], [B319; G20, cPr, H, -], [B320; G20, CF₃, H, -], [B321; G20, CF₂H, H, -], [B322; G20, H, F, -], [B323; G20, H, Cl, -], [B324; G20, H, Me, -], [B325; G20, H, OMe, -], [B326; G20, H, cPr, -], [B327; G20, H, CF₃, -], [B328; G20, H, CF₂H, -], [B329; G20, Me, Me, -], [B330; G21, H, H, -], [B331; G21, F, H, -], [B332; G21, Cl, H, -], [B333; G21, Me, H, -], [B334; G21, OMe, H, -], [B335; G21, cPr, H, -], [B336; G21, CF₃, H, -], [B337; G21, CF₂H, H, -], [B338; G21, Me, Me, -], [B339; G22, H, H, -], [B340; G22, F, H, -], [B341; G22, Cl, H, -], [B342; G22, Me, H, -], [B343; G22, OMe, H, -], [B344; G22, cPr, H, -], [B345; G22, CF₃, H, -], [B346; G22, CF₂H, H, -], [B347; G22, H, F, -], [B348; G22, H, Cl, -], [B349; G22, H, Me, -], [B350; G22, H, OMe, -], [B351; G22, H, cPr, -], [B352; G22, H, CF₃, -], [B353; G22, H, CF₂H, -], [B354; G22, Me, Me, -], [B355; G23, H, -, -], [B356; G23, F, -, -], [B357; G23, Cl, -, -], [B358; G23, Br, -, -], [B359; G23, I, -, -], [B360; G23, Me, -, -], [B361; G23, OMe, -, -], [B362; G23, cPr, -, -], [B363; G23, CF₃₋, -], [B364; G23, CF₂H, -, -], [B365; G24, H, -, -], [B366; G24, F, -, -], [B367; G24, Cl, -, -], [B368; G24, Br, -, -], [B369; G24, I, -, -], [B370; G24, Me, -, -], [B371; G24, OMe, -, -], [B372; G24, cPr, -, -], [B373; G24, CF₃ , -, -], [B374; G24, CF₂H, -, -].

The Compound (B-1) wherein R^{1X} and R^{2X} represent a hydrogen atom, R^{3X} and R^{6X} represent a fluorine atom, E^{2X} represents a phenyl group, and the combination of a group indicated by G, as well as a substituents R^{z1}, R^{z2}, and R^{z3} on the group indicated by G represents any combination indicated in the Combination B (hereinafter referred to as "Compound group SX20").

The Compound (B-1) wherein R^{1X} and R^{2X} represent a hydrogen atom, R^{3X} and R^{6X} represent a chlorine atom, E^{2X} represents a phenyl group, and the combination of a group indicated by G, as well as a substituents R^{z1}, R^{z2}, and R^{z3} on the group indicated by G represents any combination indicated in the Combination B (hereinafter referred to as "Compound group SX21").

The Compound (B-1) wherein R^{1X} and R^{2X} represent a hydrogen atom, R^{3X} and R^{6X} represent a methyl group, E^{2X} represents a phenyl group, and the combination of a group indicated by G, as well as a substituents R^{z1}, R^{z2}, and R^{z3} on the group indicated by G represents any combination indicated in the Combination B (hereinafter referred to as "Compound group SX22").

The Compound (B-1) wherein R^{1X} represents a hydroxy group, R^{2X} and R^{3X} represent a hydrogen atom, R^{6X} represents a fluorine atom, E^{2X} represents a phenyl group, and the combination of a group indicated by G, as well as a substituents R^{z1}, R^{z2}, and R^{z3} on the group indicated by G represents any combination indicated in the Combination B (hereinafter referred to as "Compound group SX23").

The Compound (B-1) wherein R^{1X} represents a hydroxy group, R^{2X} represents a hydrogen atom, R^{3X} and R^{6X} represent a fluorine atom, E^{2X} represents a phenyl group, and the combination of a group indicated by G, as well as a substituents R^{z1}, R^{z2}, and R^{z3} on the group indicated by G represents any combination indicated in the Combination B (hereinafter referred to as "Compound group SX24").

The Compound (B-1) wherein R^{1X} represents a hydroxy group, R^{2X} represents a hydrogen atom, R^{3X} and R^{6X} represent a chlorine atom, E^{2X} represents a phenyl group, and the combination of a group indicated by G, as well as a substituents R^{z1}, R^{z2}, and R^{z3} on the group indicated by G represents any combination indicated in the Combination B (hereinafter referred to as "Compound group SX25").

The Compound (B-1) wherein R^{1X} represents a hydroxy group, R^{2X} represents a hydrogen atom, R^{3X} and R^{6X} represent a methyl group, E^{2X} represents a phenyl group, and the combination of a group indicated by G, as well as a substituents R^{z1}, R^{z2}, and R^{z3} on the group indicated by G represents any combination indicated in the Combination B (hereinafter referred to as "Compound group SX26").

The Compound (B-1) wherein R^{1X}, R^{2X}, and R^{3X} represent a hydrogen atom, R^{6X} represents a fluorine atom, E^{2X} represents a 2-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX27").

The Compound (B-1) wherein R^{1X} and R^{2X} represent a hydrogen atom, R^{3X} and R^{6X} represent a fluorine atom, E^{2X} represents a 2-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX28").

The Compound (B-1) wherein R^{1X} and R^{2X} represent a hydrogen atom, R^{3X} and R^{6X} represent a chlorine atom, E^{2X} represents a 2-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX29").

The Compound (B-1) wherein R^{1X} and R^{2X} represent a hydrogen atom, R^{3X} and R^{6X} represent a methyl group, E^{2X} represents a 2-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX30").

The Compound (B-1) wherein R^{1X} represents a hydroxy group, R^{2X} and R^{3X} represent a hydrogen atom, R^{6X} represents a fluorine atom, E^{2X} represents a 2-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX31").

The Compound (B-1) wherein R^{1X} represents a hydroxy group, R^{2X} represents a hydrogen atom, R^{3X} and R^{6X} represent a fluorine atom, E^{2X} represents a 2-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX32").

The Compound (B-1) wherein R^{1X} represents a hydroxy group, R^{2X} represents a hydrogen atom, R^{3X} and R^{6X} represent a chlorine atom, E^{2X} represents a 2-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX33").

The Compound (B-1) wherein R^{1X} represents a hydroxy group, R^{2X} represents a hydrogen atom, R^{3X} and R^{6X} represent a methyl group, E^{2X} represents a 2-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX34").

The Compound (B-1) wherein R^{1X}, R^{2X} , and R^{3X} represent a hydrogen atom, R^{6X} represents a fluorine atom, E^{2X} represents a 3-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX35").

The Compound (B-1) wherein R^{1X} and R^{2X} represent a hydrogen atom, R^{3X} and R^{6X} represent a fluorine atom, E^{2X} represents a 3-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX36").

The Compound (B-1) wherein R^{1X} and R^{2X} represent a hydrogen atom, R^{3X} and R^{6X} represent a chlorine atom, E^{2X} represents a 3-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX37").

The Compound (B-1) wherein R^{1X} and R^{2X} represent a hydrogen atom, R^{3X} and R^{6X} represent a methyl group, E^{2X} represents a 3-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX38").

The Compound (B-1) wherein R^{1X} represents a hydroxy group, R^{2X} and R^{3X} represent a hydrogen atom, R^{6X} represents a fluorine atom, E^{2X} represents a 3-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX39").

The Compound (B-1) wherein R^{1X} represents a hydroxy group, R^{2X} represents a hydrogen atom, R^{3X} and R^{6X} represents a fluorine atom, E^{2X} represents a 3-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX40").

The Compound (B-1) wherein R^{1X} represents a hydroxy group, R^{2X} represents a hydrogen atom, R^{3X} and R^{6X} represents a chlorine atom, E^{2X} represents a 3-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX41").

The Compound (B-1) wherein R^{1X} represents a hydroxy group, R^{2X} represents a hydrogen atom, R^{3X} and R^{6X} represents a methyl group, E^{2X} represents a 3-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX42").

The Compound (B-1) wherein R^{1X}, R^{2X}, and R^{3X} represent a hydrogen atom, R⁶⁷ represents a fluorine atom, E^{2X} represents a 4-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX43").

The Compound (B-1) wherein R^{1X} and R^{2X} represent a hydrogen atom, R^{3X} and R^{6X} represent a fluorine atom, E^{2X} represents a 4-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX44").

The Compound (B-1) wherein R^{1X} and R^{2X} represent a hydrogen atom, R^{3X} and R^{6X} represent a chlorine atom, E^{2X} represents a 4-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX45").

The Compound (B-1) wherein R^{1X} and R^{2X} represent a hydrogen atom, R^{3X} and R^{6X} represent a methyl group, E^{2X} represents a 4-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX46").

The Compound (B-1) wherein R^{1X} represents a hydroxy group, R^{2X} and R^{3X} represent a hydrogen atom, R^{6X} represents a fluorine atom, E^{2X} represents a 4-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX47").

The Compound (B-1) wherein R^{1X} represents a hydroxy group, R^{2X} represents a hydrogen atom, R^{3X} and R^{6X} represent a fluorine atom, E^{2X} represents a 4-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX48").

The Compound (B-1) wherein R^{1X} represents a hydroxy group, R^{2X} represents a hydrogen atom, R^{3X} and R^{6X} represent a chlorine atom, E^{2X} represents a 4-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX49").

The Compound (B-1) wherein R^{1X} represents a hydroxy group, R^{2X} represents a hydrogen atom, R^{3X} and R^{6X} represent a methyl group, E^{2X} represents a 4-fluorophenyl group, and the combination of a group indicated by G, as well as a substituents R^{z1}, R^{z2}, and R^{z3} on the group indicated by G represents any combination indicated in the Combination B (hereinafter referred to as "Compound group SX50").

The Compound (B-1) wherein R^{1X}, R^{2X}, and R^{3X} represent a hydrogen atom, R^{6X} represents a fluorine atom, E^{2X} represents a 2-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX51").

The Compound (B-1) wherein R^{1X} and R^{2X} represent a hydrogen atom, R^{3X} and R^{6X} represent a fluorine atom, E^{2X} represents a 2-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX52").

The Compound (B-1) wherein R^{1X} and R^{2X} represent a hydrogen atom, R^{3X} and R^{6X} represent a chlorine atom, E^{2X} represents a 2-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX53").

The Compound (B-1) wherein R^{1X} and R^{2X} represent a hydrogen atom, R^{3X} and R^{6X} represent a methyl group, E^{2X} represents a 2-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX54").

The Compound (B-1) wherein R^{1X} represents a hydroxy group, R^{2X} and R^{3X} represent a hydrogen atom, R^{6X} represents a fluorine atom, E^{2X} represents a 2-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX55").

The Compound (B-1) wherein R^{1X} represents a hydroxy group, R^{2X} represents a hydrogen atom, R^{3X} and R^{6X} represent a fluorine atom, E^{2X} represents a 2-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX56").

The Compound (B-1) wherein R^{1X} represents a hydroxy group, R^{2X} represents a hydrogen atom, R^{3X} and R^{6X} represent a chlorine atom, E^{2X} represents a 2-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX57").

The Compound (B-1) wherein R^{1X} represents a hydroxy group, R^{2X} represents a hydrogen atom, R^{3X} and R^{6X} represent a methyl group, E^{2X} represents a 2-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX58").

The Compound (B-1) wherein R^{1X}, R^{2X}, and R^{3X} represent a hydrogen atom, R^{6X} represents a fluorine atom, E^{2X} represents a 3-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX59").

The Compound (B-1) wherein R^{1X} and R^{2X} represent a hydrogen atom, R^{3X} and R^{6X} represent a fluorine atom, E^{2X} represents a 3-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX60").

The Compound (B-1) wherein R^{1X} and R^{2X} represent a hydrogen atom, R^{3X} and R^{6X} represent a chlorine atom, E^{2X} represents a 3-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX61").

The Compound (B-1) wherein R^{1X} and R^{2X} represent a hydrogen atom, R^{3X} and R^{6X} represent a methyl group, E^{2X} represents a 3-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX62").

The Compound (B-1) wherein R^{1X} represents a hydroxy group, R^{2X} and R^{3X} represent a hydrogen atom, R^{6X} represents a fluorine atom, E^{2X} represents a 3-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX63").

The Compound (B-1) wherein R^{1X} represents a hydroxy group, R^{2X} represents a hydrogen atom, R^{3X} and R^{6X} represent a fluorine atom, E^{2X} represents a 3-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX64").

The Compound (B-1) wherein R^{1X} represents a hydroxy group, R^{2X} represents a hydrogen atom, R^{3X} and R^{6X} represent a chlorine atom, E^{2X} represents a 3-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX65").

The Compound (B-1) wherein R^{1X} represents a hydroxy group, R^{2X} represents a hydrogen atom, R^{3X} and R^{6X} represent a methyl group, E^{2X} represents a 3-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX66").

The Compound (B-1) wherein R^{1X}, R^{2X}, and R^{3X} represent a hydrogen atom, R^{6X} represents a fluorine atom, E^{2X} represents a 4-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX67").

The Compound (B-1) wherein R^{1X} and R^{2X} represent a hydrogen atom, R^{3X} and R^{6X} represent a fluorine atom, E^{2X} represents a 4-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX68").

The Compound (B-1) wherein R^{1X} and R^{2X} represent a hydrogen atom, R^{3X} and R^{6X} represent a chlorine atom, E^{2X} represents a 4-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX69").

The Compound (B-1) wherein R^{1X} and R^{2X} represent a hydrogen atom, R^{3X} and R^{6X} represent a methyl group, E^{2X} represents a 4-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX70").

The Compound (B-1) wherein R^{1X} represents a hydroxy group, R^{2X} and R^{3X} represent a hydrogen atom, R^{6X} represents a fluorine atom, E^{2X} represents a 4-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX71").

The Compound (B-1) wherein R^{1X} represents a hydroxy group, R^{2X} represents a hydrogen atom, R^{3X} and R^{6X} represent a fluorine atom, E^{2X} represents a 4-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX72").

The Compound (B-1) wherein R^{1X} represents a hydroxy group, R^{2X} represents a hydrogen atom, R^{3X} and R^{6X} represent a chlorine atom, E^{2X} represents a 4-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX73").

The Compound (B-1) wherein R^{1X} represents a hydroxy group, R^{2X} represents a hydrogen atom, R^{3X} and R^{6X} represent a methyl group, E^{2X} represents a 4-chlorophenyl group, and the combination of a group indicated by G, as well as a substituents R^{z1}, R^{z2}, and R^{z3} on the group indicated by G represents any combination indicated in the Combination B (hereinafter referred to as "Compound group SX74").

The Compound (B-1) wherein R^{1X}, R^{2X}, and R^{3X} represent a hydrogen atom, R^{6X} represents a fluorine atom, E^{2X} represents a 2-methylphenyl group, and the combination of a group indicated by G, as well as a substituents R^{z1}, R^{z2}, and R^{z3} on the group indicated by G represents any combination indicated in the Combination B (hereinafter referred to as "Compound group SX75").

The Compound (B-1) wherein R^{1X} and R^{2X} represent a hydrogen atom, R^{3X} and R^{6X} represent a fluorine atom, E^{2X} represents a 2-methylphenyl group, and the combination of a group indicated by G, as well as a substituents R^{z1}, R^{z2}, and R^{z3} on the group indicated by G represents any combination indicated in the Combination B (hereinafter referred to as "Compound group SX76").

The Compound (B-1) wherein R^{1X} and R^{2X} represent a hydrogen atom, R^{3X} and R^{6X} represent a chlorine atom, E^{2X} represents a 2-methylphenyl group, and the combination of a group indicated by G, as well as a substituents R^{z1}, R^{z2}, and R^{z3} on the group indicated by G represents any combination indicated in the Combination B (hereinafter referred to as "Compound group SX77").

The Compound (B-1) wherein R^{1X} and R^{2X} represent a hydrogen atom, R^{3X} and R^{6X} represent a methyl group, E^{2X} represents a 2-methylphenyl group, and the combination of a group indicated by G, as well as a substituents R^{z1}, R^{z2}, and R^{z3} on the group indicated by G represents any combination indicated in the Combination B (hereinafter referred to as "Compound group SX78").

The Compound (B-1) wherein R^{1X} represents a hydroxy group, R^{2X} and R^{3X} represent a hydrogen atom, R^{6X} represents a fluorine atom, E^{2X} represents a 2-methylphenyl group, and the combination of a group indicated by G, as well as a substituents R^{z1}, R^{z2}, and R^{z3} on the group indicated by G represents any combination indicated in the Combination B (hereinafter referred to as "Compound group SX79").

The Compound (B-1) wherein R^{1X} represents a hydroxy group, R^{2X} represents a hydrogen atom, R^{3X} and R^{6X} represent a fluorine atom, E^{2X} represents a 2-methylphenyl group, and the combination of a group indicated by G, as well as a substituents R^{z1}, R^{z2}, and R^{z3} on the group indicated by G represents any combination indicated in the Combination B (hereinafter referred to as "Compound group SX80").

The Compound (B-1) wherein R^{1X} represents a hydroxy group, R^{2X} represents a hydrogen atom, R^{3X} and R^{6X} represent a chlorine atom, E^{2X} represents a 2-methylphenyl group, and the combination of a group indicated by G, as well as a substituents R^{z1}, R^{z2}, and R^{z3} on the group indicated by G represents any combination indicated in the Combination B (hereinafter referred to as "Compound group SX81").

The Compound (B-1) wherein R^{1X} represents a hydroxy group, R^{2X} represents a hydrogen atom, R^{3X} and R^{6X} represent a methyl group, E^{2X} represents a 2-methylphenyl group, and the combination of a group indicated by G, as well as a substituents R^{z1}, R^{z2}, and R^{z3} on the group indicated by G represents any combination indicated in the Combination B (hereinafter referred to as "Compound group SX82").

The Compound (B-1) wherein R^{1X}, R^{2X}, and R^{3X} represent a hydrogen atom, R^{6X} represents a fluorine atom, E^{2X} represents a 3-methylphenyl group, and the combination of a group indicated by G, as well as a substituents R^{z1}, R^{z2}, and R^{z3} on the group indicated by G represents any combination indicated in the Combination B (hereinafter referred to as "Compound group SX83").

The Compound (B-1) wherein R^{1X} and R^{2X} represent a hydrogen atom, R^{3X} and R^{6X} represent a fluorine atom, E^{2X} represents a 3-methylphenyl group, and the combination of a group indicated by G, as well as a substituents R^{z1}, R^{z2}, and R^{z3} on the group indicated by G represents any combination indicated in the Combination B (hereinafter referred to as "Compound group SX84").

The Compound (B-1) wherein R^{1X} and R^{2X} represent a hydrogen atom, R^{3X} and R^{6X} represent a chlorine atom, E^{2X} represents a 3-methylphenyl group, and the combination of a group indicated by G, as well as a substituents R^{z1}, R^{z2}, and R^{z3} on the group indicated by G represents any combination indicated in the Combination B (hereinafter referred to as "Compound group SX85").

The Compound (B-1) wherein R^{1X} and R^{2X} represent a hydrogen atom, R^{3X} and R^{6X} represent a methyl group, E^{2X} represents a 3-methylphenyl group, and the combination of a group indicated by G, as well as a substituents R^{z1}, R^{z2}, and R^{z3} on the group indicated by G represents any combination indicated in the Combination B (hereinafter referred to as "Compound group SX86").

The Compound (B-1) wherein R^{1X} represents a hydroxy group, R^{2X} and R^{3X} represent a hydrogen atom, R^{6X} represents a fluorine atom, E^{2X} represents a 3-methylphenyl group, and the combination of a group indicated by G, as well as a substituents R^{z1}, R^{z2}, and R^{z3} on the group indicated by G represents any combination indicated in the Combination B (hereinafter referred to as "Compound group SX87").

The Compound (B-1) wherein R^{1X} represents a hydroxy group, R^{2X} represents a hydrogen atom, R^{3X} and R^{6X} represents a fluorine atom, E^{2X} represents a 3-methylphenyl group, and the combination of a group indicated by G, as well as a substituents R^{z1}, R^{z2}, and R^{z3} on the group indicated by G represents any combination indicated in the Combination B (hereinafter referred to as "Compound group SX88").

The Compound (B-1) wherein R^{1X} represents a hydroxy group, R^{2X} represents a hydrogen atom, R^{3X} and R^{6X} represents a chlorine atom, E^{2X} represents a 3-methylphenyl group, and the combination of a group indicated by G, as well as a substituents R^{z1}, R^{z2}, and R^{z3} on the group indicated by G represents any combination indicated in the Combination B (hereinafter referred to as "Compound group SX89").

The Compound (B-1) wherein R^{1X} represents a hydroxy group, R^{2X} represents a hydrogen atom, R^{3X} and R^{6X} represents a methyl group, E^{2X} represents a 3-methylphenyl group, and the combination of a group indicated by G, as well as a substituents R^{z1}, R^{z2}, and R^{z3} on the group indicated by G represents any combination indicated in the Combination B (hereinafter referred to as "Compound group SX90").

The Compound (B-1) wherein R^{1X}, R^{2X}, and R^{3X} represent a hydrogen atom, R^{6X} represents a fluorine atom, E^{2X} represents a 4-methylphenyl group, and the combination of a group indicated by G, as well as a substituents R^{z1}, R^{z2}, and R^{z3} on the group indicated by G represents any combination indicated in the Combination B (hereinafter referred to as "Compound group SX91").

The Compound (B-1) wherein R^{1X} and R^{2X} represent a hydrogen atom, R^{3X} and R^{6X} represents a fluorine atom, E^{2X} represents a 4-methylphenyl group, and the combination of a group indicated by G, as well as a substituents R^{z1}, R^{z2}, and R^{z3} on the group indicated by G represents any combination indicated in the Combination B (hereinafter referred to as "Compound group SX92").

The Compound (B-1) wherein R^{1X} and R^{2X} represent a hydrogen atom, R^{3X} and R^{6X} represents a chlorine atom, E^{2X} represents a 4-methylphenyl group, and the combination of a group indicated by G, as well as a substituents R^{z1}, R^{z2}, and R^{z3} on the group indicated by G represents any combination indicated in the Combination B (hereinafter referred to as "Compound group SX93").

The Compound (B-1) wherein R^{1X} and R^{2X} represent a hydrogen atom, R^{3X} and R^{6X} represents a methyl group, E^{2X} represents a 4-methylphenyl group, and the combination of a group indicated by G, as well as a substituents R^{z1}, R^{z2}, and R^{z3} on the group indicated by G represents any combination indicated in the Combination B (hereinafter referred to as "Compound group SX94").

The Compound (B-1) wherein R^{1X} represents a hydroxy group, R^{2X} and R^{3X} represent a hydrogen atom, R^{6X} represents a fluorine atom, E^{2X} represents a 4-methylphenyl group, and the combination of a group indicated by G, as well as a substituents R^{z1}, R^{z2}, and R^{z3} on the group indicated by G represents any combination indicated in the Combination B (hereinafter referred to as "Compound group SX95").

The Compound (B-1) wherein R^{1X} represents a hydroxy group, R^{2X} represents a hydrogen atom, R^{3X} and R^{6X} represent a fluorine atom, E^{2X} represents a 4-methylphenyl group, and the combination of a group indicated by G, as well as a substituents R^{z1}, R^{z2}, and R^{z3} on the group indicated by G represents any combination indicated in the Combination B (hereinafter referred to as "Compound group SX96").

The Compound (B-1) wherein R^{1X} represents a hydroxy group, R^{2X} represents a hydrogen atom, R^{3X} and R^{6X} represent a chlorine atom, E^{2X} represents a 4-methylphenyl group, and the combination of a group indicated by G, as well as a substituents R^{z1}, R^{z2}, and R^{z3} on the group indicated by G represents any combination indicated in the Combination B (hereinafter referred to as "Compound group SX97").

The Compound (B-1) wherein R^{1X} represents a hydroxy group, R^{2X} represents a hydrogen atom, R^{3X} and R^{6X} represent a methyl group, E^{2X} represents a 4-methylphenyl group, and the combination of a group indicated by G, as well as a substituents R^{z1}, R^{z2}, and R^{z3} on the group indicated by G represents any combination indicated in the Combination B (hereinafter referred to as "Compound group SX98").

Next, Formulation Examples are shown below. The "part(s)" represents "part(s) by weight". Also, the expression of "Present compound S" represents the compounds described in the Compound groups SX1 to SX98.

### Formulation Example 1

Thirty-five (35) parts of a mixture of ammonium polyoxyethylene alkyl ether sulfate and wet silica (weight ratio: 1:1), 10 parts of any one of the present compound S, and 55 parts of water are mixed, and the mixture is then finely-ground by a wet grinding method to obtain a formulation.

### Formulation Example 2

Fifty (50) parts of any one of the present compound S, 3 parts of calcium lignin sulfonate, 2 parts of sodium lauryl sulfate, and 45 parts of silica are ground and mixed to obtain a formulation.

### Formulation Example 3

Five (5) parts of any one of the present compound S, 9 parts of polyoxyethylene styryl phenyl ether, 5 parts of polyoxyethylene decyl ether (Number of ethylene oxide additions: 5), 6 parts of calcium dodecylbenzene sulfonate and 75 parts of xylene are mixed to obtain a formulation.

### Formulation Example 4

Two (2) parts of any one of the present compound S, 1 part of silica, 2 parts of calcium lignin sulfonate, 30 parts of bentonite and 65 parts of kaolin clay are mixed-grinding, and thereto is added an appropriate amount of water, and the mixture is kneaded and is then granulated with a granulator and dried to obtain a formulation.

### Formulation Example 5

Ten (10) parts of any one of the present compound S is mixed with a mixture of 18 parts of benzyl alcohol and 9 parts of DMSO, and thereto are added 6.3 parts of GERONOL (registered trademark) TE250, 2.7 parts of Ethylan (registered trademark) NS-500LQ, and 54 parts of Solvent naphtha, and the mixture is mixed to obtain a formulation.

Next, Test Examples are shown below.

The term of "non-treated" in Test Example 15 represents a test wherein a test was carried out under the same conditions as those described in the Test Example except for dispensing DMSO instead of a diluted solution with DMSO comprising the Present compound. Also, the term of "non-treated leaf disk" in Test Example 7 represents a leaf disk wherein a test was carried out under the same conditions as those described in the Test Example except for dispensing DMSO instead of a diluted solution with DMSO comprising the Present compound. Also, the term of "non-treated" in Test Example 11 means that a diluted solution with water of a formulation comprising the Present compound was not sprayed. The descriptions of "Present compounds 23 + 24" in the Test Examples means a mixture of the Present compound 23 and the Present compound 24; and in the mixture, the molar ratio of the Present compound 23 : the Present compound 24 = 3 : 7.

### Test Example 1 Control test against wheat leaf blotch fungus (Septoria tritici)

Each Present compound is diluted with DMSO to obtain a diluted solution containing 150 ppm of the compound, 1 µL of the diluted solution is dispensed into a titer plate (96 well), and thereto is then dispensed 150 µL of a YBG medium to which spores of Septoria tritici are inoculated in advance. This plate is cultured at 18°C for 3 days, thereby allowing Septoria tritici to undergo proliferation, and the absorbance at 550 nm of each well of the titer plate is then measured to determine a degree of growth of Septoria tritici. As a result, each Present compound shows a control effect against the plant disease.

### Test Example 2 Control test against corn smut fungus (Ustilago maydis)

Each Present compound is diluted with DMSO to obtain a diluted solution containing 150 ppm of the compound, 1 µL of the diluted solution is dispensed into a titer plate (96 well), and thereto is then dispensed 150 µL of a potato dextrose broth medium (PDB medium) to which spores of Ustilago maydis are inoculated in advance. This plate is cultured at 18°C for 4 days, thereby allowing Ustilago maydis to undergo proliferation, and the absorbance at 550 nm of each well of the titer plate is then measured to determine a degree of growth of Ustilago maydis. As a result, each Present compound shows a control effect against the plant disease.

### Test Example 3 Control test against barley scald fungus (Rhynchosporium secalis)

Each Present compound is diluted with DMSO to obtain a diluted solution containing 150 ppm of the compound, 1 µL of the diluted solution is dispensed into a titer plate (96 well), and thereto is then dispensed 150 µL of a potato dextrose broth medium (PDB medium) to which spores of Rhynchosporium secalis are inoculated in advance. This plate is cultured at 18°C for 7 days, thereby allowing Rhynchosporium secalis to undergo proliferation, and the absorbance at 550 nm of each well of the titer plate is then measured to determine a degree of growth of Rhynchosporium secalis. As a result, each Present compound shows a control effect against the plant disease.

### Test Example 4 Control test against cucumber Botrytis rot fungus (Botrytis cinerea)

Each Present compound is diluted with DMSO to obtain a diluted solution containing 150 ppm of the compound, 1 µL of the diluted solution is dispensed into a titer plate (96 well), and thereto is then dispensed 150 µL of a complete medium to which spores of Botrytis cinerea are inoculated in advance. This plate is cultured at 18°C for 4 days, thereby allowing Botrytis cinerea to undergo proliferation, and the absorbance at 550 nm of each well of the titer plate is then measured to determine a degree of growth of Botrytis cinerea. As a result, each Present compound shows a control effect against the plant disease.

### Test Example 5 Control test against peach scab fungus (Cladosporium carpophilum)

Each Present compound is diluted with DMSO to obtain a diluted solution containing 150 ppm of the compound, 1 µL of the diluted solution is dispensed into a titer plate (96 well), and thereto is then dispensed 150 µL of a potato dextrose broth medium (PDB medium) to which spores of Cladosporium carpophilum are inoculated in advance. This plate is cultured at 18°C for 5 days, thereby allowing Cladosporium carpophilum to undergo proliferation, and the absorbance at 550 nm of each well of the titer plate is then measured to determine a degree of growth of Cladosporium carpophilum. As a result, each Present compound shows a control effect against the plant disease.

### Test Example 6 Control test against rice brown spot fungus (Cochliobolus miyabeanus)

Each Present compound is diluted with DMSO to obtain a diluted solution containing 150 ppm of the compound, 1 µL of the diluted solution is dispensed into a titer plate (96 well), and thereto is then dispensed 150 µL of a YB liquid medium to which spores of Cochliobolus miyabeanus are inoculated in advance. This plate is cultured at 23°C for 3 days, thereby allowing Cochliobolus miyabeanus to undergo proliferation, and the absorbance at 550 nm of each well of the titer plate is then measured to determine a degree of growth of Cochliobolus miyabeanus. As a result, each Present compound shows a control effect against the plant disease.

### Test Example 7 Control test against soybean rust (Phakopsora pachyrhizi)

A true leaf of a soybean (cultivar: Kurosengoku) was cut into a diameter of 1 cm to prepare each leaf disk. To each well of a 24 well microplate was dispensed 1 mL of an agar medium (agar concentration: 1.2%), and then one of said leaf disk was placed on the agar medium in each well. To a mixture of Sorpol (registered trademark) 1200KX (0.5 µL), DMSO (4.5 µL), and xylene (5 µL) was added a DMSO solution (20 µL) comprising a test compound (10000 ppm), and the resulting mixture was mixed. The resulting mixture was diluted with ion exchange water to prepare a mixture comprising a prescribed concentration of the test compound. The resulting mixture was sprayed into each leaf disk at a ratio of 10 µL per leaf disk. After 1 day, an aqueous suspension of spores of soybean rust fungus (Phakopsora pachyrhizi) having an amino acid substitution of F129L in a mitochondrial cytochrome b protein (1.0 × 10⁵ / mL) was inoculated by spraying on each leaf disk. After the inoculation, the microplate was placed into an artificial climate chamber (Lighting: 6 hours, Lights-out: 18 hours, Temperature: 23°C, Humidity: 60%). After 1 day, the leaf disks were air-dried until water droplets on the surfaces of the leaf disks disappeared, and the microplate was placed into the artificial climate chamber again for 12 days. Then, lesion area of soybean rust was investigated. As a result, when the prescribed concentration was 200 ppm, each lesion area of the leaf disk treated with any one of the Present compound 1, 2, 3, 4, 7, 8, 9, 10, 11, 12, 13, 15, 16, 17, 18, 19, 20, 21, 22, 23 + 24 (mixture of the present compound 23 : the present compound 24 = 3 : 7), 25, 26, 27, 28, 29, 35, 36, 37, 38, 39, 41, 42, 43, 44, 45, 46, 47, 48, or 53 as the test compound was 30% or less relative to the lesion area of the non-treated leaf disk.

### Test Example 8 Control test against barley net blotch (Pyrenophora teres)

A plastic pot is filled with soil, barley (cultivar: Nishinohoshi) is seeded thereto, and grown in a greenhouse for 7 days. The Present compound formulated according to the method described in the Formulation Example 1 is mixed with water in such a way that the concentration thereof is 200 ppm, and the resulting mixture is sprayed to foliage of the above barley so as to adequately adhere onto the surfaces of leaves of the above barley. After spraying the mixture, the barley is air-dried. After 1 day, an aqueous suspension of spores of Pyrenophora teres is inoculated by spraying to the barley. After the inoculation, the barley is placed in a greenhouse at 23°C in daytime and at 20°C in nighttime under a humid condition for 3 days, then cultured in a greenhouse for 7 days, and then a lesion area is investigated. As a result, each Present compound shows a control effect against the plant disease.

### Test Example 9 Control test against wheat leaf rust (Puccinia recondita)

A plastic pot was filled with soil, wheat (cultivar: Shirogane) is seeded thereto, and grown in a greenhouse for 9 days. The Present compound formulated according to the method described in the Formulation Example 1 is mixed with water in such a way that the concentration thereof is 200 ppm, and the resulting mixture is sprayed to foliage of the above wheat so as to adequately adhere onto the surfaces of leaves of the above wheat. After spraying the mixture, the wheat is air-dried, cultured at 20°C under lighting for 5 to 7 days, and then spores of Puccinia recondita are inoculated by dusting thereto. After the inoculation, the wheat is placed at 23°C under a dark humid condition for 1 day, then cultured at 20°C under lighting for 8 days, and a lesion area is investigated. As a result, each Present compound shows a control effect against the plant disease.

### Test Example 10 Control test against wheat leaf blotch (Septoria tritici)

A plastic pot is filled with soil, wheat (cultivar: Apogee) is seeded thereto, and cultured in a greenhouse for 10 days. The Present compound formulated according to the method described in the Formulation Example 1 is mixed with water in such a way that the concentration thereof is 200 ppm, and the resulting mixture is sprayed to foliage of the above wheat so as to adequately adhere onto the surfaces of leaves of the above wheat. After spraying the mixture, the wheat is air-dried. After 4 days, an aqueous suspension comprising spores of Septoria tritici is inoculated by spraying to the wheat. After the inoculation, the wheat is placed at 18°C under a humid condition for 3 days, then cultured under lighting for 14 to 18 days, and then a lesion area is investigated. As a result, each Present compound shows a control effect against the plant disease.

### Test Example 11 Control test against soybean rust (Phakopsora pachyrhizi)

A plastic pot was filled with soil, soybeans (cultivar: Kurosengoku) were seeded thereto, and cultured in a greenhouse for 10 to 14 days. The Present compound 1, 2, 3, 4, 7, 8, 9, 10, 13, 14, 15, 16, 17, 18, 19, 20, 21, 22, 23 + 24 (mixture of the present compound 23 : the present compound 24 = 3 : 7), or 25 formulated according to the method described in the Formulation Example 1 was mixed with water in such a way that the concentration thereof was 200 ppm, and the resulting mixture was sprayed to foliage of the above soybeans so as to adequately adhere onto the surfaces of leaves of the above soybeans. After spraying the mixture, the soybeans were air-dried. After 2 to 5 days, an aqueous suspension of spores of Phakopsora pachyrhizi was inoculated by spraying to the soybeans. After the inoculation, the soybeans were placed in a greenhouse at 23°C in daytime and at 20°C in nighttime under a humid condition for 1 to 2 day(s), then cultured in a greenhouse for 12 days, and then a lesion area was investigated. As a result, the lesion area in the soybeans treated with each of the above Present compound was 30% or less relative to the lesion area in the non-treated soybeans.

### Test Example 12 Control test against soybean rust (Phakopsora pachyrhizi)

A plastic pot is filled with soil, soybeans (cultivar: Kurosengoku) are seeded thereto, and cultured in a greenhouse for 10 days. An aqueous suspension comprising spores of Phakopsora pachyrhizi is inoculated by spraying to the soybeans. After the inoculation, the soybeans are placed in a greenhouse at 23°C in daytime and at 20°C in nighttime under a humid condition for 1 day, and then cultured in a greenhouse for 2 days. Then, the Present compound formulated according to the method described in the Formulation Example 1 is mixed with water in such a way that the concentration thereof is 200 ppm, and the resulting mixture is sprayed to foliage of the above soybeans so as to adequately adhere onto the surfaces of leaves of the above soybeans. After spraying the mixture, the soybeans are air-dried, then cultured in a greenhouse for 8 days, and then a lesion area is investigated. As a result, each Present compound shows a control effect against the plant disease.

### Test Example 13 Control test against soybean Cercospora leaf spot (Cercospora sojina)

A plastic pot is filled with soil, soybeans (cultivar: Tachinagaha) are seeded thereto, and cultured in a greenhouse for 13 days. The Present compound formulated according to the method described in the Formulation Example 1 is mixed with water in such a way that the concentration thereof is 200 ppm, and the resulting mixture is sprayed to foliage of the above soybeans so as to adequately adhere onto the surfaces of leaves of the above soybeans. After spraying the mixture, the soybeans are air-dried. After 1 day, an aqueous suspension of spores of Cercospora sojina is inoculated by spraying to the soybeans. After the inoculation, the soybeans are placed in a greenhouse at 23°C in daytime and at 20°C in nighttime under a humid condition for 3 days, then cultured in a greenhouse for 16 days, and then a lesion area is investigated. As a result, each Present compound shows a control effect against the plant disease.

### Test Example 14 Control test against tomato early blight (Alternaria solani)

A plastic pot is filled with soil, tomatoes (cultivar: Patio) are seeded thereto, and cultured in a greenhouse for 20 days. The Present compound formulated according to the method described in the Formulation Example 1 is mixed with water in such a way that the concentration thereof is 200 ppm, and the resulting mixture is sprayed to foliage of the above tomatoes so as to adequately adhere onto the surfaces of leaves of the above tomatoes. After spraying the mixture, the tomatoes are air-dried. After 1 day, an aqueous suspension of spores of Alternaria solani is inoculated by spraying to the tomatoes. After the inoculation, the tomatoes are placed at 18°C under a humid condition for 6 days, and then a lesion area is investigated. As a result, each Present compound shows a control effect against the plant disease.

### Test Example 15 Control test against soybean rust fungus (Phakopsora pachyrhizi)

Each test compound was diluted with DMSO to obtain a diluted solution containing 150 ppm of the compound, 1 µL of the diluted solution was dispensed into a titer plate (96 well), and thereto was then dispensed 149 µL of an aqueous suspension of spores of Phakopsora pachyrhizi to which spores of Phakopsora pachyrhizi were suspended in advance (1.0 × 10⁴ / mL). This plate was cultured at 23°C for several hours, and then germinated spores of Phakopsora pachyrhizi were counted. As a result, when the treatment concentration was 1 ppm, the number of germinated spores in each well comprising any one of the Present compound 1, 2, 3, 4, 6, 7, 8, 9, 10, 11, 12, 13, 14, 15, 16, 17, 18, 19, 20, 21, 22, 23 + 24 (mixture of the present compound 23 : the present compound 24 = 3 : 7), 25, 26, 27, 28, 29, 34, 35, 36, 37, 38, 39, 41, 42, 43, 44, 45, 46, 47, 48, 49, 50, 51, 52, 53, 54, 55, or 56 as the test compound was 50% or less relative to the number of germinated spores in the non-treated well.

### Test Example 16 Control test against soybean rust fungus (Phakopsora pachyrhizi)

A test is carried out by the method according to the Test Example 15 and using each Present compound as the test compound with the treatment concentration of 10 ppm. As a result, each Present compound shows a control effect against the plant disease.

### INDUSTRIAL APPLICABILITY

The Present compounds (including the Compounds of the present invention) have control effects against plant diseases, and can be used in controlling plant diseases.

## Claims

1. A method for controlling a plant disease which comprises applying a compound represented by formula (I): 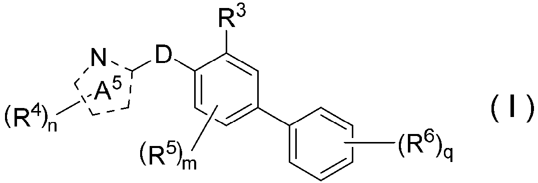 [wherein
the ring A⁵ represents a five(5) membered nonaromatic nitrogen-containing heterocycle or a five(5) membered aromatic nitrogen-containing heterocycle (with the proviso that in the ring A⁵, an atom binding to D represents a carbon atom or a nitrogen atom, and among the atoms which is adjacent to the atom binding to the D and constitutes the ring A⁵, at least one thereof represents a nitrogen atom),
D represents CR¹R², C=O, C=S, or C=N-OR⁷,
n is 0, 1, 2, or 3,
m is 0, 1, 2, or 3,
q is 0, 1, 2, 3, 4, or 5,
R¹ and R² are identical to or different from each other and each represents a C1-C6 chain hydrocarbon group optionally substituted with one or more substituent(s) selected from Group A, a C3-C8 alicyclic hydrocarbon group, a three(3)- eight(8) membered nonaromatic heterocyclic group {the C3-C8 alicyclic hydrocarbon group, and the three (3)-eight(8) membered nonaromatic heterocyclic group may be optionally substituted with one or more substituents selected from Group B}, a phenyl group, a five(5) membered aromatic heterocyclic group {the phenyl group and the five(5) membered aromatic heterocyclic group may be optionally substituted with one or more substituents selected from Group C}, OR⁸, SR⁹, S(O)R¹⁰, S(O)₂R¹¹, NR¹²R¹³, C(O)R¹⁴, C(R¹⁵)=NOR¹⁶, a cyano group, a nitro group, a halogen atom, or a hydrogen atom,
R¹ and R² may combine together with a carbon atom to which they are attached to form a C3-C8 alicyclic hydrocarbon, or a three(3)- eight(8) membered nonaromatic heterocycle {the C3-C8 alicyclic hydrocarbon, and the three(3)- eight(8) membered nonaromatic heterocycle may be optionally substituted with one or more substituents selected from Group B},
R³ and R⁵ are identical to or different from each other and each represents a C1-C6 chain hydrocarbon group optionally substituted with one or more substituent(s) selected from Group A, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B}, a five(5)- six(6) membered aromatic heterocyclic group optionally substituted with one or more substituents selected from Group C, OR¹⁷, SR¹⁸, S(O)R¹⁹, S(O)₂R²⁰, NR²¹R²², C(O)R²³, C(R²⁴)=NOR²⁵, a cyano group, a nitro group, or a halogen atom,
R⁴ represents a C1-C6 chain hydrocarbon group optionally substituted with one or more substituent(s) selected from Group A, a C3-C8 alicyclic hydrocarbon group, a three(3)-eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)-six(6) membered aromatic heterocyclic group {the phenyl group, and the five(5)-six(6) membered aromatic heterocyclic group may be optionally substituted with one or more substituents selected from Group C}, OR²⁶, NR²⁷R²⁸, C(O)R²⁹, C(R³⁰)=NOR³¹, a cyano group, a nitro group, or a halogen atom,
R⁶ represents a C1-C6 chain hydrocarbon group optionally substituted with one or more substituent(s) selected from Group D, a C3-C8 alicyclic hydrocarbon group, a three(3)-eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, OR³², SR³³, S(O)R³⁴, S(O)₂R³⁵, NR³⁶R³⁷, C(O)R³⁸, C(R³⁹)=NOR⁴⁰, a cyano group, a nitro group, or a halogen atom,
R⁸, R¹², R¹³, R¹⁷, R²¹, R²², R²⁶, R²⁷, R²⁸, R³², R³⁶, and R³⁷ are identical to or different from each other and each represents a C1-C6 chain hydrocarbon group optionally substituted with one or more substituent(s) selected from Group A, a C1-C6 alkylsulfonyl group, a (C1-C6 alkyl)carbonyl group, a (C1-C6 alkoxy)carbonyl group, a (C1-C6 alkylamino) carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, or a hydrogen atom,
R⁷, R⁹, R¹⁶, R¹⁸, R²¹, R³¹, R³³, and R⁴⁰ are identical to or different from each other and each represents a C1-C6 chain hydrocarbon group optionally substituted with one or more substituent(s) selected from Group A, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, or a hydrogen atom,
R¹⁰, R¹¹, R¹⁹, R²⁰, R³⁴, and R³⁵ are identical to or different from each other and each represents a C1-C6 chain hydrocarbon group optionally substituted with one or more substituent(s) selected from Group A, a C1-C6 alkylamino group, a C2-C8 dialkylamino group {the C1-C6 alkylamino group, and the C2-C8 dialkylamino group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, or a five(5)- six(6) membered aromatic heterocyclic group {the phenyl group, and the five(5)- six (6) membered aromatic heterocyclic group may be optionally substituted with one or more substituents selected from Group C},
R¹⁴, R²³, R²⁹, and R³⁸ are identical to or different from each other and each represents a C1-C6 chain hydrocarbon group, a C1-C6 alkoxy group, a C1-C6 alkylamino group, a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms}, or a hydroxy group,
R¹⁵, R²⁴, R³⁰, and R³⁹ are identical to or different from each other and each represents a C1-C6 chain hydrocarbon group optionally substituted with one or more substituent(s) selected from Group A, or a hydrogen atom,
when q is 2, 3, 4, or 5, the neighboring two R⁶ and R⁶ may combine together with carbon atoms to which they are attached to form a C5-C6 alicyclic hydrocarbon, a five(5)-six(6) membered nonaromatic heterocycle {the C5-C6 alicyclic hydrocarbon, and the five(5)- six(6) membered nonaromatic heterocycle may be optionally substituted with one or more substituents selected from Group B}, a benzene ring, or five(5)- six(6) membered aromatic heterocycle {the benzene ring, and the five(5)- six(6) membered aromatic heterocycle may be optionally substituted with one or more substituents selected from Group C}.
Group A: a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, a halogen atom, a cyano group, a nitro group, a hydroxy group, and a sulfanyl group.
Group B: a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group C: a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino) carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group optionally substituted with one or more substituents selected from Group B, an amino group, a nitro group, a cyano group, a hydroxy group, a sulfanyl group, and a halogen atom.
Group D: a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5) membered aromatic heterocyclic group {the phenyl group, and the five(5) membered aromatic heterocyclic group may be optionally substituted with one or more substituents selected from Group C}, a halogen atom, a cyano group, a nitro group, a hydroxy group, and a sulfanyl group.
(with the proviso that the following compounds are excluded:
3-((3,5-difluoro-[1,1'-biphenyl]-4-yl)methyl)-1,2,4-thiadiazole,
3-((3-fluoro-[1,1'-biphenyl]-4-yl)methyl)-1,2,4-thiadiazole,
3-((3,5-difluoro-[1,1'-biphenyl]-4-yl)difluoromethyl)-1,2,4-thiadiazole,
3-((3,5-difluoro-[1,1'-biphenyl]-4-yl)methyl)-1,3,4-thiadiazole-2(3H)-one,
2-((3,5-difluoro-[1,1'-biphenyl]-4-yl)methyl)-1,3,4-thiadiazole,
(3,5-difluoro-[1,1'-biphenyl]-4-yl)(1,2,4-thiadiazole-3-yl)methanone,
2-((3-fluoro-[1,1'-biphenyl]-4-yl)methyl)-1,3,4-oxadiazole,
(E)-(3,5-difluoro-[1,1'-biphenyl]-4-yl)(1,2,4-thiadiazole-3-yl)methanone O-methyl oxime,
(Z)-(3,5-difluoro-[1,1'-biphenyl]-4-yl)(1,2,4-thiadiazole-3-yl)methanone O-methyl oxime,
3-((3,5-difluoro-[1,1'-biphenyl]-4-yl)methyl)-5-ethyl-1,2,4-thiadiazole,
5-cyclopropyl-3-((3,5-difluoro-[1,1'-biphenyl]-4-yl)methyl)-1,2,4-thiadiazole,
3-((3,5-difluoro-[1,1'-biphenyl]-4-yl)methyl)-5-(trifluoromethyl)-1,2,4-thiadiazole,
3-((3,5-difluoro-[1,1'-biphenyl]-4-yl)methyl)-1,2,4-thiadiazole-5-carbonitrile,
3-((3,5-difluoro-[1,1'-biphenyl]-4-yl)methyl)isoxazole 5(4H)-one,
5-chloro-3-((3,5-difluoro-[1,1'-biphenyl]-4-yl)methyl)isoxazole,
3-((3,5-difluoro-[1,1'-biphenyl]-4-yl)methyl)-1,2,4-thiadiazole-5-ol,
3-((3,5-difluoro-[1,1'-biphenyl]-4-yl)methyl)-5-methoxy-1,2,4-thiadiazole,
(2-chlorothiazole-4-yl)(3-fluoro-[1,1'-biphenyl]-4-yl)methanol,
2-chloro-4-((3-fluoro-[1,1'-biphenyl]-4-yl)methyl)thiazole,
5-chloro-3-((3,5-difluoro-[1,1'-biphenyl]-4-yl)methyl)-1,2,4-thiadiazole,
5-bromo-3-((3,5-difluoro-[1,1'-biphenyl]-4-yl)methyl)- -1,2,4-thiadiazole, and
3-((3,5-difluoro-[1,1'-biphenyl]-4-yl)methyl)-1,2,4-thiadiazole-5-carboxamide)]
or an N-oxide thereof, or a salt thereof, to a plant or soil for cultivating a plant.

2. The method for controlling a plant disease according to claim 1 wherein the compound represented by formula (I) or an N-oxide or a salt thereof is a compound represented or an N-oxide or a salt thereof, in which the ring A⁵ represents a group represented by formula H1, formula H2, formula H3, or formula H4, 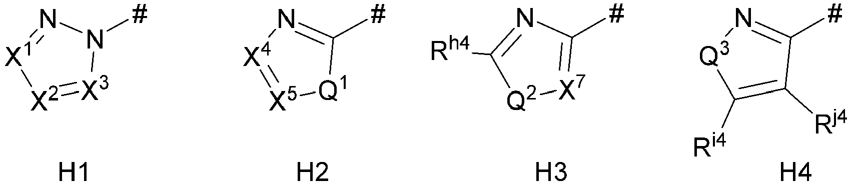 [wherein
# represents a binding position to D,
X¹ represents a nitrogen atom, or CR^{a4}, X² represents a nitrogen atom, or CR^{b4}, X³ represents a nitrogen atom, or CR^{c4} (with the proviso that among X¹, X², and X³, the number of the nitrogen atom is 0, or 1],
a combination of Q¹, X⁴, and X⁵ represents
a combination wherein X⁴ represents a nitrogen atom, Q¹ represents NR^{a1}, and X⁵ represents CR^{d4}, or
a combination wherein X⁴ represents CRe4, Q¹ represents an oxygen atom, a sulfur atom, or NR^{b1}, and X⁵ represents nitrogen atom, or CR^{f4};
Q² represents an oxygen atom, or NR^{c1}, X⁷ represents a nitrogen atom, or CR^{g4},
Q³ represents a sulfur atom, or NR^{d1},
R^{a4}, R^{b4}, R^{c4}, R^{d4}, R^{e4}, R^{f4}, R^{g4}, R^{h4}, Rⁱ⁴, and R^{j4} are identical to or different from each other and each represents a C1-C6 chain hydrocarbon group optionally substituted with one or more substituents selected from Group A, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, OR²⁶, NR²⁷R²⁸, C(O)R²⁹, C(R³⁰)=NOR³¹, a cyano group, a nitro group, a halogen atom, or a hydrogen atom,
R^{a1}, R^{b1}, R^{c1}, and R^{d1} are identical to or different from each other and each represents a C1-C6 chain hydrocarbon group optionally substituted with one or more substituents selected from Group A, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B}, a phenyl group, a five(5)- six(6) membered aromatic heterocyclic group {the phenyl group, and the five(5)- six(6) membered aromatic heterocyclic group may be optionally substituted with one or more substituents selected from Group C}, C(O)R²⁹, or a hydrogen].

3. The method for controlling a plant disease according to claim 2 wherein the compound represented by formula (I) or an N-oxide or a salt thereof is a compound represented by formula (I) in which the ring A⁵ represents the group represented by formula H1.

4. The method for controlling a plant disease according to claim 2 wherein the compound represented by formula (I) or an N-oxide or a salt thereof is a compound represented by formula (I) in which the ring A⁵ represents the group represented by formula H2.

5. The method for controlling a plant disease according to claim 2 wherein the compound represented by formula (I) or an N-oxide or a salt thereof is a compound represented by formula (I) in which the ring A⁵ represents the group represented by formula H3.

6. The method for controlling a plant disease according to claim 2 wherein the compound represented by formula (I) or an N-oxide or a salt thereof is a compound represented by formula (I) in which the ring A⁵ represents the group represented by formula H4.

7. A compound represented by formula (II) 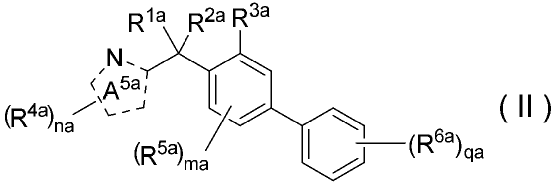 [wherein
the ring A^{5a} represents a five (5) membered aromatic nitrogen-containing heterocyclic group (with the proviso that in the ring A^{5a}, the binding atom to CR^{1a}R^{2a} represents a carbon atom or a nitrogen atom, and among the atoms which are adjacent to the atom binding to CR^{1a}R^{2a} and constitute the ring A^{5a}, at least one thereof represents a nitrogen atom),
na is 0, 1, 2, or 3,
ma is 0, 1, 2, or 3,
qa is 0, 1, 2, 3, 4, or 5,
R^{1a} and R^{2a} are identical to or different from each other and each represents a C1-C6 chain hydrocarbon group optionally substituted with one or more substituents selected from Group A^{a}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}}, a phenyl group optionally substituted with one or more substituents selected from Group C^{a}, OR^{8a}, SR^{9a}, S(O)R^{10a}, S(O)₂R^{11a}, NR^{12a}R^{13a}, C(O)R^{14a}, C(R^{15a})=NOR^{16a}, a cyano group, a nitro group, a halogen atom, or a hydrogen atom,
R^{1a} and R^{2a} may combine together with a carbon atom to which they are attached to form a C3-C8 alicyclic hydrocarbon, or a three(3)- eight(8) membered nonaromatic heterocycle {the C3-C8 alicyclic hydrocarbon, and the three(3)- eight(8) membered nonaromatic heterocycle may be optionally substituted with one or more substituents selected from Group Ba}
R^{3a} and R^{5a} are identical to or different from each other and each represents a C1-C6 chain hydrocarbon group optionally substituted with one or more substituents selected from Group A^{a}, a C3-C8 alicyclic hydrocarbon group, a three(3)-eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}}, a five(5)- six(6) membered aromatic heterocyclic group optionally substituted with one or more substituents selected from Group C^{a}, OR^{17a}, SR^{18a}, S(O)R^{19a}, S(O)₂R^{20a}, NR^{21a}R^{22a}, C(O)R^{23a}, C(R^{24a})=NOR^{25a}, a cyano group, a nitro group, or a halogen atom,
R^{4a} represents a C1-C6 chain hydrocarbon group optionally substituted with one or more substituents selected from Group D^{a}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}}, OR^{26a}, NR^{27a}R^{28a}, C(R^{29a})=NOR^{30a}, a cyano group, a nitro group, or a halogen atom,
R^{6a} represents a C1-C6 chain hydrocarbon group optionally substituted with one or more substituents selected from Group E^{a}, a C3-C8 alicyclic hydrocarbon group optionally substituted with one or more substituents selected from Group B^{a}, a phenyl group, a five(5)- six (6) membered aromatic heterocyclic group {the phenyl group, and the five(5)- six(6) membered aromatic heterocyclic group may be optionally substituted with one or more substituents selected from Group C^{a}}, OR^{31a}, SR^{32a}, S(O)R^{33a}, S(O)₂R^{34a}, NR^{35a}R^{36a}, C(O)R^{37a}, C(R^{38a})=NOR^{39a}, a cyano group, a nitro group, or a halogen atom,
R^{8a}, R^{12a}, R^{13a}, R^{17a}, R^{31a}, R^{21a}, R^{22a}, and R^{35a} are identical to or different from each other and each represents a C1-C6 chain hydrocarbon group optionally substituted with one or more substituents selected from Group A^{a}, a C1-C6 alkylsulfonyl group, a (C1-C6 alkyl)carbonyl group, a (C1-C6 alkoxy)carbonyl group, a (C1-C6 alkylamino)carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino)carbonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a phenyl group, a five(5)- six(6) membered aromatic heterocyclic group {the phenyl group, and the five(5)- six(6) membered aromatic heterocyclic group may be optionally substituted with one or more substituents selected from Group C^{a}}, or a hydrogen atom,
R^{36a} represents a C1-C6 chain hydrocarbon group optionally substituted with one or more substituents selected from Group A^{a}, a C1-C6 alkylsulfonyl group, a (C1-C6 alkyl)carbonyl group, a (C1-C6 alkoxy)carbonyl group, a (C1-C6 alkylamino)carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a phenyl group, or a five(5)- six(6) membered aromatic heterocyclic group{the phenyl group, and the five(5)- six(6) membered aromatic heterocyclic group may be optionally substituted with one or more substituents selected from Group C^{a}},
R^{7a}, R^{9a}, R^{10a}, R^{18a}, R^{19a}, R^{25a}, R^{32a}, R^{33a}, R^{34a}, and R^{39a} are identical to or different from each other and each represents a C1-C6 chain hydrocarbon group optionally substituted with one or more substituents selected from Group A^{a}, a C3-C8 alicyclic hydrocarbon group, a three(3)- eight (8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a phenyl group, or a five(5)- six(6) membered aromatic heterocyclic group{the phenyl group, and the five(5)- six(6) membered aromatic heterocyclic group may be optionally substituted with one or more substituents selected from Group C^{a}},
R^{11a} and R^{20a} are identical to or different from each other and each represents a C1-C6 chain hydrocarbon group optionally substituted with one or more substituents selected from Group A^{a}, a C1-C6 alkylamino group, a C2-C8 dialkylamino group {the C1-C6 alkylamino group, and the C2-C8 dialkylamino group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}}, a phenyl group, or a five(5)- six(6) membered aromatic heterocyclic group {the phenyl group, and the five(5)- six(6) membered aromatic heterocyclic group may be optionally substituted with one or more substituents selected from Group C^{a}},
R^{14a} and R^{23a} are identical to or different from each other and each represents a C1-C6 chain hydrocarbon group, a C1-C6 alkoxy group, a C1-C6 alkylamino group, a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms}, or a hydroxy group,
R^{15a}, R^{24a}, and R^{38a} are identical to or different from each other and each represents a C1-C6 chain hydrocarbon group optionally substituted with one or more substituents selected from Group A^{a}, or a hydrogen atom,
R^{26a}, R^{27a}, and R^{28a} are identical to or different from each other and each represents a C1-C6 chain hydrocarbon group optionally substituted with one or more substituents selected from Group D^{a}, a C1-C6 alkylsulfonyl group, a (C1-C6 alkyl)carbonyl group, a (C1-C6 alkoxy)carbonyl group, a (C1-C6 alkylamino)carbonyl group, a (C2-C8 dialkylamino)carbonyl group {the C1-C6 alkylsulfonyl group, the (C1-C6 alkyl)carbonyl group, the (C1-C6 alkoxy)carbonyl group, the (C1-C6 alkylamino)carbonyl group, and the (C2-C8 dialkylamino) carbonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, or a hydrogen atom,
R^{30a} represents a C1-C6 chain hydrocarbon group optionally substituted with one or more substituents selected from Group D^{a}, a C3-C8 alicyclic hydrocarbon group, a three(3)- eight(8) membered nonaromatic heterocyclic group {the C3-C8 alicyclic hydrocarbon group, and the three(3)-eight(8) membered nonaromatic heterocyclic group may be optionally substituted with one or more substituents selected from Group B^{a}},
R^{29a} represents a C1-C6 chain hydrocarbon group optionally substituted with one or more substituents selected from Group D^{a}, or a hydrogen atom,
R^{37a} represents a C1-C6 chain hydrocarbon group, a C1-C6 alkoxy group, a C1-C6 alkylamino group, or a C2-C8 dialkylamino group {the C1-C6 chain hydrocarbon group, the C1-C6 alkoxy group, the C1-C6 alkylamino group, and the C2-C8 dialkylamino group may be optionally substituted with one or more halogen atoms}.
Group A^{a}: a group consisting of a C1-C6 alkoxy group, a a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a phenyl group, a five(5)- six(6) membered aromatic heterocyclic group{the phenyl group, and the five(5)- six(6) membered aromatic heterocyclic group may be optionally substituted with one or more substituents selected from Group C^{a}}, a halogen atom, a cyano group, a nitro group, a hydroxy group, and a sulfanyl group.
Group B^{a}: a group consisting of an oxo group, a thioxo group, a C1-C6 chain hydrocarbon group, a C1-C6 alkoxy group, a C1-C6 alkylsulfanyl group, a C1-C6 alkylsulfinyl group, a C1-C6 alkylsulfonyl group {the C1-C6 chain hydrocarbon group, the C1-C6 alkoxy group, the C1-C6 alkylsulfanyl group, the C1-C6 alkylsulfinyl group, and the C1-C6 alkylsulfonyl group may be optionally substituted with one or more halogen atoms}, a hydroxy group, a sulfanyl group, a halogen atom, and a cyano group.
Group C^{a}: a group consisting of a C1-C6 chain hydrocarbon group, a C1-C6 alkoxy group, a C1-C6 alkylsulfanyl group, a C1-C6 alkylsulfinyl group, a C1-C6 alkylsulfonyl group, a C1-C6 alkylamino group, a C2-C8 dialkylamino group, a (C1-C6 alkyl)carbonyl group, a (C1-C6 alkoxy)carbonyl group, a (C1-C6 alkylamino)carbonyl group, a (C2-C8 dialkylamino)carbonyl group, a (C1-C6 alkyl)carbonylamino group, a (C1-C6 alkoxy)carbonylamino group {the C1-C6 chain hydrocarbon group, the C1-C6 alkoxy group, the C1-C6 alkylsulfanyl group, the C1-C6 alkylsulfinyl group, the C1-C6 alkylsulfonyl group, the C1-C6 alkylamino group, the C2-C8 dialkylamino group, the (C1-C6 alkyl)carbonyl group, the (C1-C6 alkoxy)carbonyl group, the (C1-C6 alkylamino)carbonyl group, the (C2-C8 dialkylamino)carbonyl group, the (C1-C6 alkyl)carbonylamino group, and the (C1-C6 alkoxy)carbonylamino group may be optionally substituted with one or more halogen atoms}, a C3-C8 alicyclic hydrocarbon group optionally substituted with one or more substituents selected from Group B^{a}, an amino group, a nitro group, a cyano group, a hydroxy group, a sulfanyl group, and a halogen atom.
Group D^{a}: a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a halogen atom, a cyano group, a nitro group, a hydroxy group, and a sulfanyl group.
Group E^{a}: a group consisting of a C1-C6 alkoxy group, a C1-C6 alkylsulfanyl group, a C1-C6 alkylsulfinyl group, a C1-C6 alkylsulfonyl group {the C1-C6 alkoxy group, the C1-C6 alkylsulfanyl group, the C1-C6 alkylsulfinyl group, and the C1-C6 alkylsulfonyl group may be optionally substituted with one or more halogen atoms}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, a phenyl group, five(5) membered aromatic heterocyclic group, a halogen atom, a cyano group, a nitro group, a hydroxy group, and a sulfanyl group], (with the proviso that the following compounds are excluded:
4-((3,4,5-tribromo-1H-pyrazole-1-yl)methyl)-[1,1'-biphenyl]-3-ol,
5-(1-(3-fluoro-[1,1'-biphenyl]-4-yl)ethyl)-3-methyl-1,2,4-oxadiazole,
3-(chloromethyl)-5-(1-(3-fluoro-[1,1'-biphenyl]-4-yl)ethyl)-1,2,4-oxadiazole,
3-((3,5-difluoro-[1,1'-biphenyl]-4-yl)methyl)-1,2,4-thiadiazole,
3-((3-fluoro-[1,1'-biphenyl]-4-yl)methyl)-1,2,4-thiadiazole,
3-((3,5-difluoro-[1,1'-biphenyl]-4-yl)difluoromethyl)-1,2,4-thiadiazole,
3-((3,5-difluoro-[1,1'-biphenyl]-4-yl)methyl)-1,3,4-thiadiazole-2(3H)-one,
2-((3,5-difluoro-[1,1'-biphenyl]-4-yl)methyl)-1,3,4-thiadiazole,
(3,5-difluoro-[1,1'-biphenyl]-4-yl) (1,2,4-thiadiazole-3-yl)methanone,
2-((3-fluoro-[1,1'-biphenyl]-4-yl)methyl)-1,3,4-oxadiazole,
(E)-(3,5-difluoro-[1,1'-biphenyl]-4-yl)(1,2,4-thiadiazole-3-yl)methanone O-methyl oxime,
(Z)-(3,5-difluoro-[1,1'-biphenyl]-4-yl)(1,2,4-thiadiazole-3-yl)methanone O-methyl oxime,
3-((3,5-difluoro-[1,1'-biphenyl]-4-yl)methyl)-5-ethyl-1,2,4-thiadiazole,
5-cyclopropyl-3-((3,5-difluoro-[1,1'-biphenyl]-4-yl)methyl)-1,2,4-thiadiazole,
3-((3,5-difluoro-[1,1'-biphenyl]-4-yl)methyl)-5-(trifluoromethyl)-1,2,4-thiadiazole,
3-((3,5-difluoro-[1,1'-biphenyl]-4-yl)methyl)-1,2,4-thiadiazole-5-carbonitrile,
3-((3,5-difluoro-[1,1'-biphenyl]-4-yl)methyl)isoxazole-5(4H)-one,
5-chloro-3-((3,5-difluoro-[1,1'-biphenyl]-4-yl)methyl)isoxazole,
3-((3,5-difluoro-[1,1'-biphenyl]-4-yl)methyl)-1,2,4-thiadiazole-5-ol,
3-((3,5-difluoro-[1,1'-biphenyl]-4-yl)methyl)-5-methoxy-1,2,4-thiadiazole,
(2-chlorothiazole-4-yl)(3-fluoro-[1,1'-biphenyl]-4-yl)methanol,
2-chloro-4-((3-fluoro-[1,1'-biphenyl]-4-yl)methyl)thiazole,
5-chloro-3-((3,5-difluoro-[1,1'-biphenyl]-4-yl)methyl)-1,2,4-thiadiazole,
5-bromo-3-((3,5-difluoro-[1,1'-biphenyl]-4-yl)methyl)-1,2,4-thiadiazole, and,
3-((3,5-difluoro-[1,1'-biphenyl]-4-yl)methyl)-1,2,4-thiadiazole-5-carboxamide),
or an N-oxide thereof, or a salt thereof.

8. The compound or an N-oxide thereof, or a salt thereof according to claim 7, wherein
the ring A^{5a} represents a group represented by formula H1a, formula H2a, formula H3a, or formula H4a, 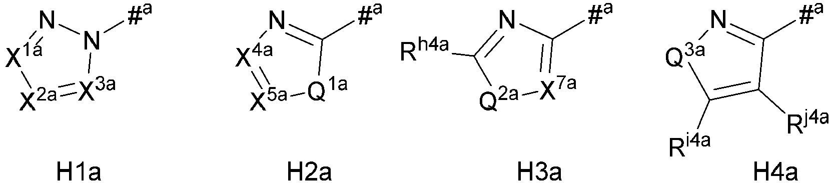 [wherein
#^{a} represents a binding position to CR^{1a}R^{2a},
X^{1a} represents a nitrogen atom, or CR^{a4a}, X^{2a} represents a nitrogen atom, or CR^{b4a}, X^{3a} represents a nitrogen atom, or CR^{c4a} (with the proviso that among X^{1a}, X^{2a}, and X^{3a}, the number of the nitrogen atom is 0, or 1],
a combination of Q^{1a}, X^{4a}, and X^{5a} represents
a combination wherein X^{4a} represents a nitrogen atom, Q¹ represents NR^{a1a}, and X^{5a} represents CR^{d4a}, or
a combination wherein X^{4a} represents CR^{e4a}, Q^{1a} represents an oxygen atom, a sulfur atom, or NR^{b1a}, and X^{5a} represents a nitrogen atom, or CR^{f4a};
Q^{2a} represents an oxygen atom, or NR^{c1a}, X^{7a} represents a nitrogen atom, or CR^{g4a},
Q^{3a} represents a sulfur atom, or NR^{d1a},
R^{a4a}, R^{b4a}, R^{c4a}, R^{d4a}, R^{e4a}, R^{f4a}, R^{g4a}, R^{h4a}, R^{i4a}, and R^{j4a} are identical to or different from each other and each represents a C1-C6 chain hydrocarbon group optionally substituted with one or more substituents selected from Group D^{a}, a C3-C8 alicyclic hydrocarbon group, a three(3)- eight(8) membered nonaromatic heterocyclic group {the C3-C8 alicyclic hydrocarbon group, and the three(3)- eight(8) membered nonaromatic heterocyclic group may be optionally substituted with one or more substituents selected from Group B^{a}}, OR^{26a}, NR^{27a}R^{28a}, C(R^{29a})=NOR^{30a}, a cyano group, a nitro group, a halogen atom, or a hydrogen atom,
R^{a1a}, R^{b1a}, R^{c1a}, and R^{d1a} are identical to or different from each other and each represents a C1-C6 chain hydrocarbon group optionally substituted with one or more substituents selected from Group D^{a}, a C3-C8 alicyclic hydrocarbon group, a three(3)- eight(8) membered nonaromatic heterocyclic group {the C3-C8 alicyclic hydrocarbon group, and the three (3)-eight(8) membered nonaromatic heterocyclic group may be optionally substituted with one or more substituents selected from Group B^{a}), a cyano group, or a hydrogen atom].

9. A composition comprising the compound or an N-oxide thereof, or a salt thereof according to claim 7 or 8, and an inert carrier.

10. A composition comprising one or more ingredient(s) selected from the group consisting of Group (a), Group (b), Group (c), and Group (d), and the compound or an N-oxide thereof, or a salt thereof according to claim 7 or 8:
Group (a): a group consisting of insecticidal active ingredients, miticidal active ingredients, and nematicidal active ingredients;
Group (b): fungicidal active ingredients;
Group (c): plant growth regulatory ingredients;
Group (d): repellent ingredients.

11. A method for controlling plant diseases which comprises applying an effective amount of the compound or an N-oxide thereof, or a salt thereof according to claim 7 or 8 or an effective amount of the composition according to claim 10, to a plant or soil for cultivating a plant.

12. Use of the compound or an N-oxide thereof, or a salt thereof according toclaim 7 or 8 or the composition according to claim 10 for controlling a plant disease.

13. A seed or a vegetative reproductive organ holding an effective amount of the compound or an N-oxide thereof, or a salt thereof according to claim 7 or 8 or an effective amount of the composition according to claim 10.

14. A compound represented by formula (III): 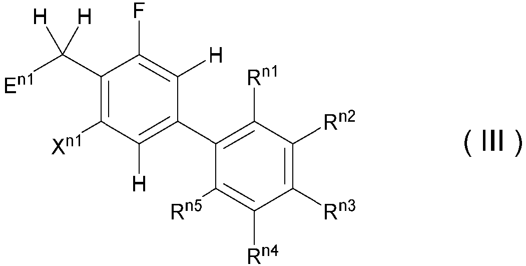 [wherein
Xⁿ¹ represents a halogen atom,
Rⁿ¹, Rⁿ², Rⁿ³, and Rⁿ⁵ are identical to or different from each other and each represents a hydrogen atom, a halogen atom, a methyl group, or a methoxy group,
a combination of Eⁿ¹ and Rⁿ⁴ represents
a combination wherein Eⁿ¹ represents a halogen atom, and Rⁿ⁴ represents a hydrogen atom, a methyl group, or a methoxy group; or
a combination wherein Eⁿ¹ represents a cyano group, C(O)OH, or C(NH₂)=NORⁿ⁶, and Rⁿ⁴ represents a hydrogen atom, a halogen atom, a methyl group, or a methoxy group,
Rⁿ⁶ represents a C1-C6 alkylcarbonyl group optionally substituted with one or more halogen atoms, or a hydrogen atom].

15. A compound represented by formula (IV): 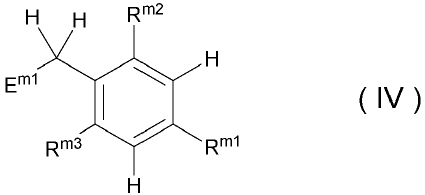 [wherein
R^{m1} represents a chlorine atom, a bromine atom, or an iodine atom,
R^{m2} represents a halogen atom,
R^{m3} represents a hydrogen atom, a halogen atom, a methyl group, or a methoxy group,
E^{m1} represents a 1,2,4-thiadiazole-3-yl group, a 1,2,4-oxadiazole-3-yl group {the 1,2,4-thiadiazole-3-yl group, and the 1,2,4-oxadiazole-3-yl group may be optionally substituted with one or more substituents selected from Group A^{m1}}, 1,2,3-triazole-2-yl group, or an isothiadiazole-3-yl group {the 1,2,3-triazole-2-yl group, and the isothiadiazole-3-yl group may be optionally substituted with one or more substituents selected from Group B^{m1}},
Group A^{m1}: a group consisting of a C1-C4 alkyl group, a C1-C4 alkoxy group, a cyclopropyl group, and a CF₃ group.
Group B^{m1}: a group consisting of a halogen atom, a C1-C4 alkyl group, a C1-C4aalkoxy group, a C1-C4 haloalkyl group, a C1-C4 haloalkoxy group, and a cyclopropyl group].
